# EUROPÄISCHE PATENTANMELDUNG

(11) **EP 2 186 805 A1**
(43) Veröffentlichungstag der Anmeldung: **19.05.2010**
(21) Anmeldenummer: 10151668.0
(22) Anmeldetag: 08.02.2007
(51) Int. Cl.: C07D 307/60, A01N 43/08

(54) **Cycloalkyl-phenylsubstituierte cyclische Ketoenole**

(62) Teilanmeldung aus: 07703352.0
(71) Anmelder: Bayer CropScience AG, 40789 Monheim (DE)
(72) Erfinder: Die Erfindernennung liegt noch nicht vor

(57) **Zusammenfassung**

Die Erfindung betrifft neue cycloalkyl-phenyl-substituierte cyclische Ketoenole der Formel (I) 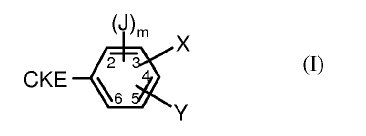 in welcher
J, X, Y, m und CKE die oben angegebene Bedeutung haben,

Verfahren und Zwischenprodukte zu ihrer Herstellung und ihre Verwendung als Schädlingsbekämpfungsmittel und/oder Herbizide.

Außerdem betrifft die Erfindung selektiv herbizide Mittel, die cycloalkyl-phenyl-substituierte cyclische Ketoenole einerseits und eine die Kulturpflanzenverträglichkeit verbesserende Verbindung andererseits enthalten.

Die Erfindung betrifft weiterhin die Steigerung der Wirkung von Pflanzenschutzmittel enthaltend Verbindungen der Formel (I) durch die Zugaben von Ammonium- oder Phosphoniumsalzen und gegebenenfalls Penetrationsförderer.

## Beschreibung

Die vorliegende Erfindung betrifft neue Cycloalkyl-phenylsubstituierte cyclische Ketoenole, mehrere Verfahren zu ihrer Herstellung und ihre Verwendung als Schädlingsbekämpfungsmittel und/oder Herbizide. Auch Gegenstand der Erfindung sind selektiv herbizide Mittel, die Cycloalkyl-phenylsubstituierte cyclische Ketoenole einerseits und eine die Kulturpflanzenverträglichkeit verbessernde Verbindung andererseits enthalten.

Die vorliegende Erfindung betrifft weiterhin die Steigerung der Wirkung von Pflanzenschutzmitteln enthaltend insbesondere cycloalkyl-phenylsubstituierte cyclische Ketoenole, durch die Zugabe von Ammonium- oder Phosphoniumsalzen und gegebenenfalls Penetrationsförderern, die entsprechenden Mittel, Verfahren zu ihrer Herstellung und ihre Anwendung im Pflanzenschutz als Insektizide und/oder Akarizide und/oder zur Verhinderung von unerwünschten Pflanzenwuchs.

Von 3-Acyl-pyrrolidin-2,4-dionen sind pharmazeutische Eigenschaften vorbeschrieben (S. Suzuki et al. Chem. Pharm. Bull. 15 1120 (1967)). Weiterhin wurden N-Phenylpyrrolidin-2,4-dione von R. Schmierer und H. Mildenberger (Liebigs Ann. Chem. 1985, 1095) synthetisiert. Eine biologische Wirksamkeit dieser Verbindungen wurde nicht beschrieben.

In EP-A-0 262 399 und GB-A-2 266 888 werden ähnlich strukturierte Verbindungen (3-Aryl-pyrrolidin-2,4-dione) offenbart, von denen jedoch keine herbizide, insektizide oder akarizide Wirkung bekannt geworden ist. Bekannt mit herbizider, insektizider oder akarizider Wirkung sind unsubstituierte, bicyclische 3-Aryl-pyrrolidin-2,4-dion-Derivate (EP-A-355 599, EP-A-415 211 und JP-A-12-053 670) sowie substituierte monocyclische 3-Aryl-pyrrolidin-2,4-dion-Derivate (EP-A-377 893 und EP-A-442 077).

Weiterhin bekannt sind polycyclische 3-Arylpyrrolidin-2,4-dion-Derivate (EP-A-442 073) sowie 1H-Arylpyrrolidin-dion-Derivate (EP-A-456 063, EP-A-521 334, EP-A-596 298, EP-A-613 884, EP-A-613 885, WO 95/01 997, WO 95/26 954, WO 95/20 572, EP-A-0 668 267, WO 96/25 395, WO 96/35 664, WO 97/01 535, WO 97/02 243, WO 97/36 868, WO 97/43275, WO 98/05638, WO 98/06721, WO 98/25928, WO 99/16748, WO 99/24437, WO 99/43649, WO 99/48869 und WO 99/55673, WO 01/17972, WO 01/23354, WO 01/74770, WO 03/013249, WO 04/007448, WO 04/024688, WO 04/065366, WO 04/080962, WO 04/111042, WO 05/044791, WO 05/044796, WO 05/048710, WO 05/049596, WO 05/066125, WO 05/092897, WO 06/000355, WO 06/029799, WO 06/056281, WO 06/056282, WO 06/089633, DE-A-05051325, DE-A-05059891).

Es ist bekannt, dass bestimmte substituierte Δ³-Dihydrofuran-2-on-Derivate herbizide Eigenschaften besitzen (vgl. DE-A-4 014 420). Die Synthese der als Ausgangsverbindungen verwendeten Tetronsäurederivate (wie z.B. 3-(2-Methyl-phenyl)-4-hydroxy-5-(4-fluorphenyl)-Δ³-dihydrofuranon-(2)) ist ebenfalls in DE-A-4 014 420 beschrieben. Ähnlich strukturierte Verbindungen ohne Angabe einer insektiziden und/oder akariziden Wirksamkeit sind aus der Publikation Campbell et al., J. Chem. Soc., Perkin Trans. 1, 1985, (8) 1567-76 bekannt. Weiterhin sind 3-Aryl-Δ³-dihydrofuranon-Derivate mit herbiziden, akariziden und insektiziden Eigenschaften aus EP-A-528 156, EP-A-0 647 637, WO 95/26 345, WO 96/20 196, WO 96/25 395, WO 96/35 664, WO 97/01 535, WO 97/02 243, WO 97/36 868, WO 98/05638, WO 98/25928, WO 99/16748, WO 99/43649, WO 99/48869, WO 99/55673, WO 01/17972, WO 01/23354 und WO 01/74770, WO 03/013 249, WO 04/024 688, WO 04/080 962, WO 04/111 042, WO 05/092897, WO 06/000355, WO06/029799, WO 06/089633, DE-A-05051325, DE-A-05059891 bekannt. Auch 3-Aryl-Δ³-dihydrothiophen-on-Derivate sind bekannt (WO 95/26 345, 96/25 395, WO 97/01 535, WO 97/02 243, WO 97/36 868, WO 98/05638, WO 98/25928, WO 99/16748, WO 99/43649, WO 99/48869, WO 99/55673, WO 01/ 17972, WO 01/23354, WO 01/74770, WO 03/013249, WO 04/080 962, WO 04/111 042, WO 05/092897, WO 06/029799).

Bestimmte, im Phenylring unsubstituierte Phenyl-pyron-Derivate sind bereits bekannt geworden (vgl. A.M. Chirazi, T. Kappe und E. Ziegler, Arch. Pharm. 309, 558 (1976) und K.-H. Boltze und K. Heidenbluth, Chem. Ber. 91, 2849), wobei für diese Verbindungen eine mögliche Verwendbarkeit als Schädlingsbekämpfungsmittel nicht angegeben wird. Im Phenylring substituierte Phenyl-pyron-Derivate mit herbiziden, akariziden und insektiziden Eigenschaften sind in EP-A-588 137, WO 96/25 395, WO 96/35 664, WO 97/01 535, WO 97/02 243, WO 97/16 436, WO 97/19 941, WO 97/36 868, WO 98/05638, WO 99/43649, WO 99/48869, WO 99/55673, WO 01/17972, WO 01/74770, WO 03/013249, WO 04/080 962, WO 04/111 042, WO 05/092897, WO 06/029799 beschrieben.

Bestimmte, im Phenylring unsubstituierte 5-Phenyl-1,3-thiazin-Derivate sind bereits bekannt geworden (vgl. E. Ziegler und E. Steiner, Monatsh. 95, 147 (1964), R. Ketcham, T. Kappe und E. Ziegler, J. Heterocycl. Chem. 10, 223 (1973)), wobei für diese Verbindungen eine mögliche Anwendung als Schädlingsbekämpfungsmittel nicht angegeben wird. Im Phenylring substituierte 5-Phenyl-1,3-thiazin-Derivate mit herbizider, akarizider und insektizider Wirkung sind in WO 94/14 785, WO 96/02 539, WO 96/35 664, WO 97/01 535, WO 97/02 243, WO 97/02 243, WO 97/36 868, WO 99/05638, WO 99/43649, WO 99/48869, WO 99/55673, WO 01/17972, WO 01/74770, WO 03/013249, WO 04/080 962, WO 04/111 042, WO 05/092897, WO 06/029799 beschrieben.

Es ist bekannt, dass bestimmte substituierte 2-Arylcyclopentandione herbizide, insektizide und akarizide Eigenschaften besitzen (vgl. z.B. US-4 283 348; 4 338 122; 4 436 666; 4 526 723; 4 551 547; 4 632 698; WO 96/01 798; WO 96/03 366, WO 97/14 667 sowie WO 98/39281, WO 99/43649, WO 99/48869, WO 99/55673, WO 01/17972, WO 01/74770, WO 03/013249, WO 04/080 962, WO 04/111 042, WO 05/092897, WO 06/029799). Außerdem sind ähnlich substituierte Verbindungen bekannt; 3-Hydroxy-5,5-dimethyl-2-phenylcyclopent-2-en-1-on aus der Publikation Micklefield et al., Tetrahedron, (1992), 7519-26 sowie der Naturstoff Involutin (-)-cis-5-(3,4-dihydroxyphenyl)-3,4-dihydroxy-2-(4-hydroxyphenyl)-cyclopent-2-en-one aus der Publikation Edwards et al., J. Chem. Soc. S, (1967), 405-9. Eine insektizide oder akarizide Wirkung wird nicht beschrieben. Außerdem ist 2-(2,4,6-Trimethylphenyl)-1,3-indandion aus der Publikation J. Economic Entomology, 66, (1973), 584 und der Offenlegungsschrift DE-A 2 361 084 bekannt, mit Angabe von herbiziden und akariziden Wirkungen.

Es ist bekannt, dass bestimmte substituierte 2-Arylcyclohexandione herbizide, insektizide und akarizide Eigenschaften besitzen (US-4 175 135, 4 209 432, 4 256 657, 4 256 658, 4 256 659, 4 257 858, 4 283 348, 4 303 669, 4 351 666, 4 409 153, 4 436 666, 4 526 723, 4 613 617, 4 659 372, DE-A 2 813 341, sowie Wheeler, T.N., J. Org. Chem. 44, 4906 (1979)), WO 99/43649, WO 99/48869, WO 99/55673, WO 01/17972, WO 01/74770, WO 03/013249, WO 04/080 962, WO 04/111 042, WO 05/092897, WO 06/029799).

Es ist bekannt, dass bestimmte substituierte 4-Aryl-pyrazolidin-3,5-dione akarizide, insektizide und herbizide Eigenschaften besitzen (vgl. z.B. WO 92/16 510, EP-A-508 126, WO 96/11 574, WO 96/21 652, WO 99/47525, WO 01/17 351, WO 01/17 352, WO 01/17 353, WO 01/17 972, WO 01/17 973, WO 03/028 466, WO 03/062 244, WO 04/080 962, WO 04/111 042, WO 05/005428, WO 05/016873, WO 05/092897, WO 06/029799).

Es ist bekannt, dass bestimmte Tetrahydropyridone herbizide Eigenschaften besitzen: JP-A-0 832 530. Außerdem sind spezielle 4-Hydroxytetrahydropyridone mit akariziden, insektiziden und herbiziden Eigenschaften bekannt: JP-A-11 152 273. Weiterhin wurden 4-Hydroxy-tetrahydropyridone als Schädlingsbekämpfungsmittel und Herbizide in WO 01/79204 bekannt.

Es ist weiterhin bekannt, dass bestimmte 5,6-Dihydropyron-Derivate als Proteaseinhibitoren antivirale Eigenschaften haben: WO 95/14012. Weiterhin ist das 4-Phenyl-6-(2-phenethyl)-5,6-dihydropyron aus der Synthese von Kawalakton-Derivaten bekannt: Kappe et al., Arch. Pharm. 309, 558-64 (1976). Außerdem sind 5,6-Dihydropyron-Derivate als Zwischenprodukte bekannt: White, J.D., Brenner, J.B., Deinsdale, M.J., J. Amer. Chem. Soc. 93, 281-2 (1971). 3-Phenyl-5,6-dihydropyron-Derivate mit Anwendungen als Pflanzenschutzmittel sind in WO 01/98288 beschrieben.

Die Wirksamkeit und Wirkungsbreite dieser Verbindungen ist jedoch insbesondere bei niedrigen Aufwandmengen und Konzentrationen nicht immer voll zufriedenstellend. Weiterhin ist die Pflanzenverträglichkeit dieser Verbindungen nicht immer ausreichend.

Es wurden nun neue Verbindungen der Formel (I) gefunden 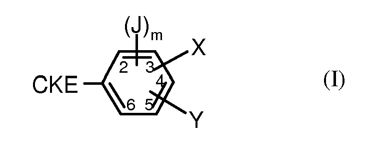 in welcher
- J: für gegebenenfalls substituiertes Cycloalkyl steht, welches gegebenenfalls durch Heteroatome unterbrochen sein kann,
- X: für Wasserstoff, Alkyl, Halogen, Halogenalkyl, Alkoxy oder Halogenalkoxy steht,
- Y: für Wasserstoff, Alkyl, Halogenalkyl, Halogen, Alkoxy oder Halogenalkoxy steht,
- m: für eine Zahl 1, 2 oder 3 steht,
mit der Maßgabe, dass mindestens einer der Reste J, X oder Y in der 2-Position des Phenylrestes steht und dabei ungleich Wasserstoff ist,
- CKE: für eine der Gruppen 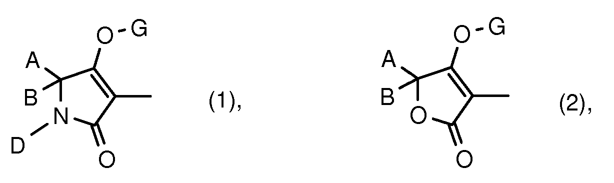 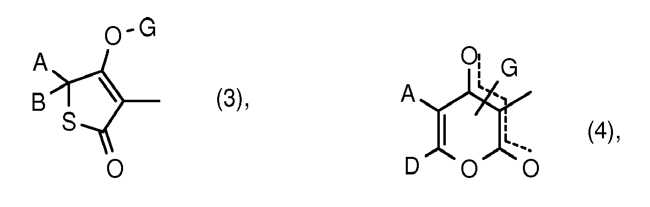 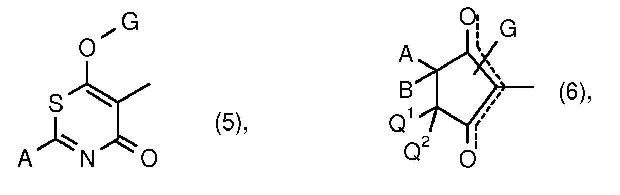 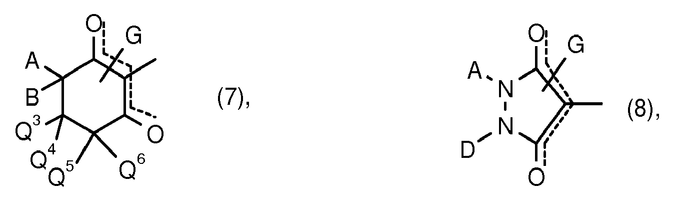 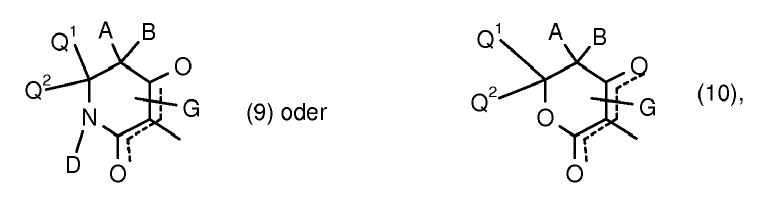
worin
- A: für Wasserstoff, jeweils gegebenenfalls durch Halogen substituiertes Alkyl, Alkenyl, Alkoxyalkyl, Alkylthioalkyl, gesättigtes oder ungesättigtes, gegebenenfalls substituiertes Cycloalkyl, in welchem gegebenenfalls mindestens ein Ringatom durch ein Heteroatom ersetzt ist, oder jeweils gegebenenfalls durch Halogen, Alkyl, Halogenalkyl, Alkoxy, Halogenalkoxy, Cyano oder Nitro substituiertes Aryl, Arylalkyl oder Hetaryl steht,
- B: für Wasserstoff, Alkyl oder Alkoxyalkyl steht, oder
- A und B: gemeinsam mit dem Kohlenstoffatom an das sie gebunden sind für einen gesättigten oder ungesättigten, gegebenenfalls mindestens ein Heteroatom enthaltenden unsubstituierten oder substituierten Cyclus stehen,
- D: für Wasserstoff oder einen gegebenenfalls substituierten Rest aus der Reihe Alkyl, Alkenyl, Alkinyl, Alkoxyalkyl, gesättigtes oder ungesättigtes Cycloalkyl, in welchem gegebenenfalls eines oder mehrere Ringglieder durch Heteroatome ersetzt sind, Arylalkyl, Aryl, Hetarylalkyl oder Hetaryl steht oder
- A und D: gemeinsam mit den Atomen an die sie gebunden sind für einen gesättigten oder ungesättigten und gegebenenfalls mindestens ein (im Falle CKE=8 ein weiteres) Heteroatom enthaltenden, im A,D-Teil unsubstituierten oder substituierten Cyclus stehen, bzw.
- A und Q¹: gemeinsam für gegebenenfalls durch eine Carbonylgruppe oder Heteroatome unterbrochenes gegebenenfalls durch Halogen, Hydroxy, jeweils gegebenenfalls substituiertes Alkyl, Alkoxy, Alkylthio, Cycloalkyl, Benzyloxy oder Aryl substituiertes Alkandiyl oder Alkendiyl stehen, in welchem gegebenenfalls zwei nicht direkt benachbarte Kohlenstoffatome einen weiteren gegebenenfalls substituierten Cyclus bilden, der gegebenenfalls durch Heteroatome unterbrochen sein kann oder
- D und Q¹: gemeinsam mit den Atomen, an die sie gebunden sind, für einen gesättigten oder ungesättigten und gegebenenfalls mindestens ein Heteroatom enthaltenden, im D, Q¹-Teil unsubstituierten oder substituierten Cyclus stehen,
- Q¹: für Wasserstoff, Alkyl, Alkoxyalkyl, gegebenenfalls substituiertes Cycloalkyl (worin gegebenenfalls eine Methylengruppe durch Sauerstoff oder Schwefel ersetzt ist) oder gegebenenfalls substituiertes Phenyl steht,
- Q² Q⁴ Q⁵ und Q⁶: unabhängig voneinander für Wasserstoff oder Alkyl stehen,
- Q³: für Wasserstoff, für gegebenenfalls substituiertes Alkyl, Alkoxyalkyl, Alkylthioalkyl, gegebenenfalls substituiertes Cycloalkyl (worin gegebenenfalls eine Methylengruppe durch Sauerstoff oder Schwefel ersetzt ist) oder gegebenenfalls substituiertes Phenyl steht, oder
- Q¹ und: Q² gemeinsam mit dem Kohlenstoffatom, an das sie gebunden sind, für einen gegebenenfalls ein Heteroatom enthaltenden unsubstituierten oder substituierten Cyclus stehen, oder
- Q³ und: Q⁴ gemeinsam mit dem Kohlenstoffatom, an das sie gebunden sind, für einen gesättigten oder ungesättigten, gegebenenfalls ein Heteroatom enthaltenden unsubstituierten oder substituierten Cyclus stehen,
- G: für Wasserstoff (a) oder für eine der Gruppen 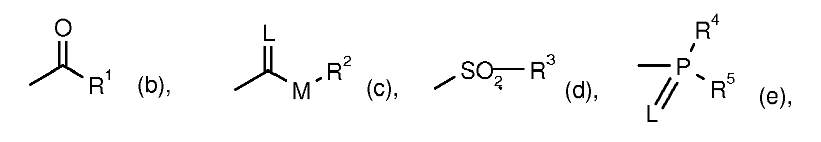 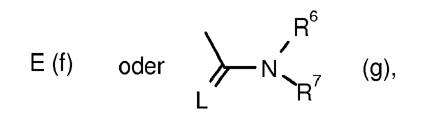
steht,
worin
- E: für ein Metallionäquivalent oder ein Ammoniumion steht,
- L: für Sauerstoff oder Schwefel steht,
- M: für Sauerstoff oder Schwefel steht,
- R¹: für jeweils gegebenenfalls durch Halogen substituiertes Alkyl, Alkenyl, Alkoxyalkyl, Alkylthioalkyl, Polyalkoxyalkyl oder gegebenenfalls durch Halogen, Alkyl oder Alkoxy substituiertes Cycloalkyl, das durch mindestens ein Heteroatom unterbrochen sein kann, jeweils gegebenenfalls substituiertes Phenyl, Phenylalkyl, Hetaryl, Phenoxyalkyl oder Hetaryloxyalkyl steht,
- R²: für jeweils gegebenenfalls durch Halogen substituiertes Alkyl, Alkenyl, Alkoxyalkyl, Polyalkoxyalkyl oder für jeweils gegebenenfalls substituiertes Cycloalkyl, Phenyl oder Benzyl steht,
- R³, R⁴ und R⁵: unabhängig voneinander für jeweils gegebenenfalls durch Halogen substituiertes Alkyl, Alkoxy, Alkylamino, Dialkylamino, Alkylthio, Alkenylthio, Cycloalkylthio oder für jeweils gegebenenfalls substituiertes Phenyl, Benzyl, Phenoxy oder Phenylthio stehen,
- R⁶ und R⁷: unabhängig voneinander für Wasserstoff, jeweils gegebenenfalls durch Halogen substituiertes Alkyl, Cycloalkyl, Alkenyl, Alkoxy, Alkoxyalkyl, für gegebenenfalls substituiertes Phenyl, für gegebenenfalls substituiertes Benzyl stehen, oder gemeinsam mit dem N-Atom, an das sie gebunden sind, für einen gegebenenfalls durch Sauerstoff oder Schwefel unterbrochenen Cyclus stehen.

Die Verbindungen der Formel (I) können, auch in Abhängigkeit von der Art der Substituenten, als geometrische und/oder optische Isomere oder Isomerengemische, in unterschiedlicher Zusammensetzung vorliegen, die gegebenenfalls in üblicher Art und Weise getrennt werden können. Sowohl die reinen Isomeren als auch die Isomerengemische, deren Herstellung und Verwendung sowie diese enthaltende Mittel sind Gegenstand der vorliegenden Erfindung. Im folgenden wird der Einfachheit halber jedoch stets von Verbindungen der Formel (I) gesprochen, obwohl sowohl die reinen Verbindungen als gegebenenfalls auch Gemische mit unterschiedlichen Anteilen an isomeren Verbindungen gemeint sind.

Unter Einbeziehung der Bedeutungen (1) bis (10) der Gruppe CKE ergeben sich folgende hauptsächliche Strukturen (I-1) bis (I-10): 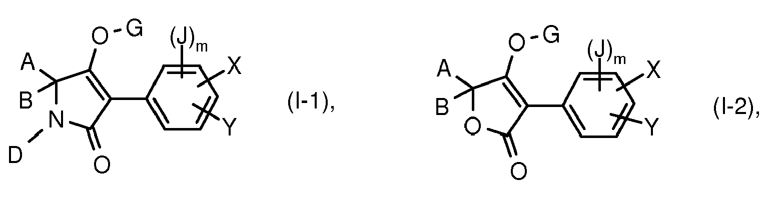 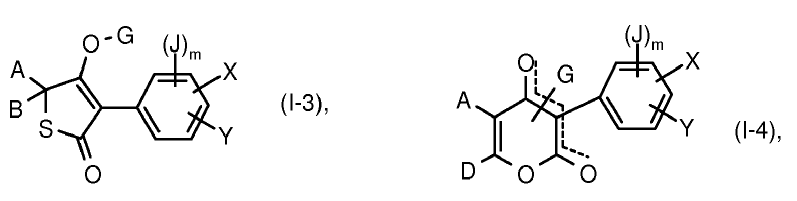 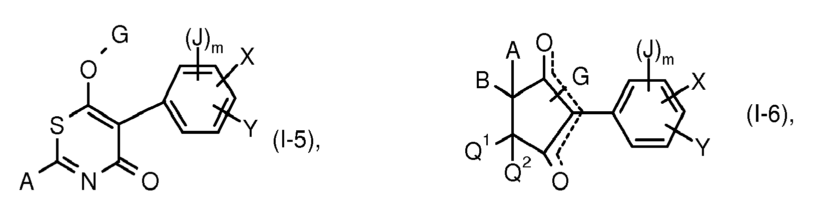 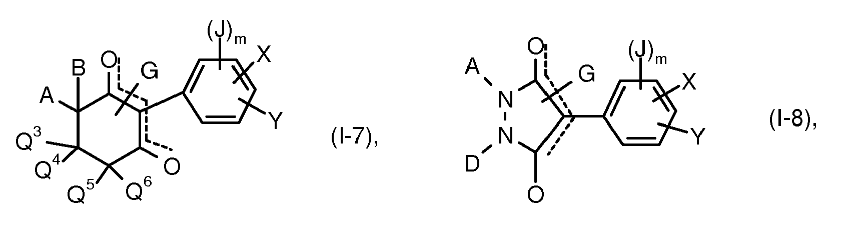 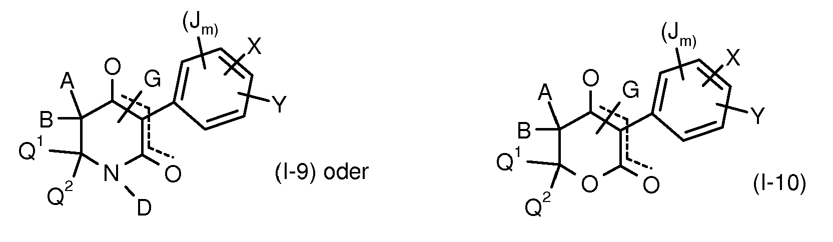 worin

A, B, D, G, J, m, Q¹, Q², Q³ Q⁴, Q⁵, Q⁶ X und Y die oben angegebene Bedeutung haben.

Unter Einbeziehung der verschiedenen Bedeutungen (a), (b), (c), (d), (e), (f) und (g) der Gruppe G ergeben sich folgende hauptsächliche Strukturen (I-1-a) bis (I-1-g), wenn CKE für die Gruppe (1) steht, 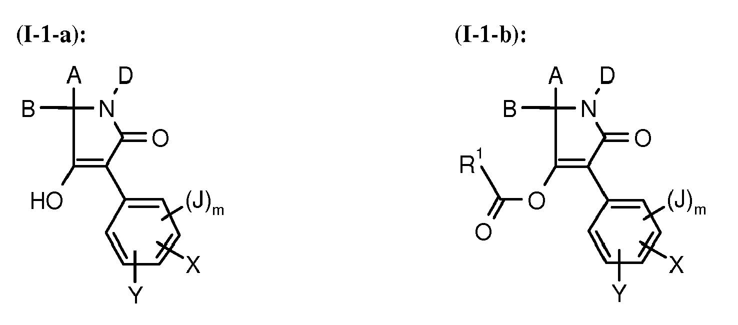 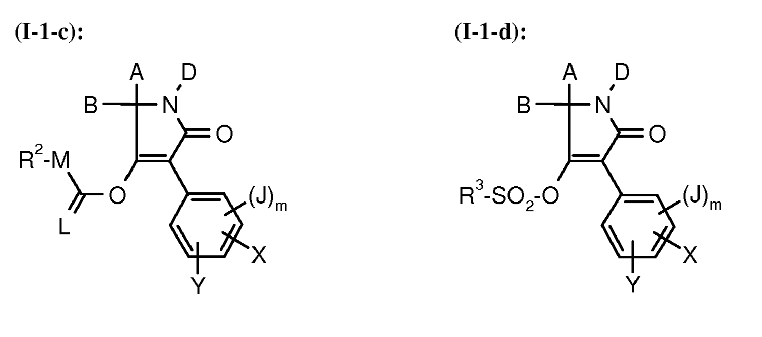 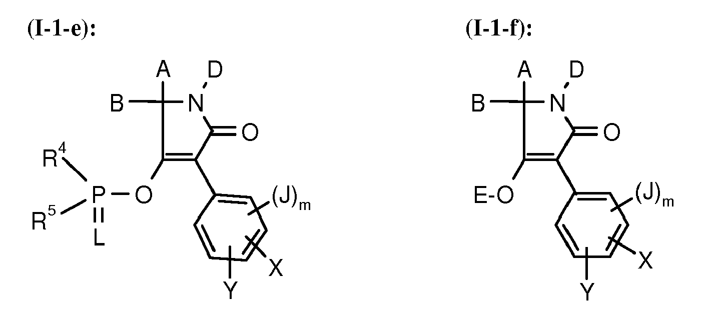 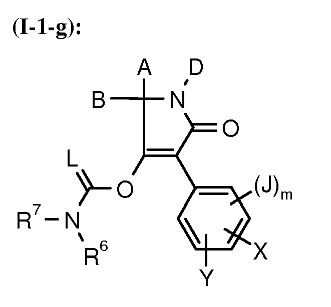 worin

A, B, D, E, J, L, m, M, X, Y, R¹, R², R³, R⁴, R⁵, R⁶ und R⁷ die oben angegebenen Bedeutungen besitzen.

Unter Einbeziehung der verschiedenen Bedeutungen (a), (b), (c), (d), (e), (f) und (g) der Gruppe G ergeben sich folgende hauptsächliche Strukturen (I-2-a) bis (I-2-g), wenn CKE für die Gruppe (2) steht, 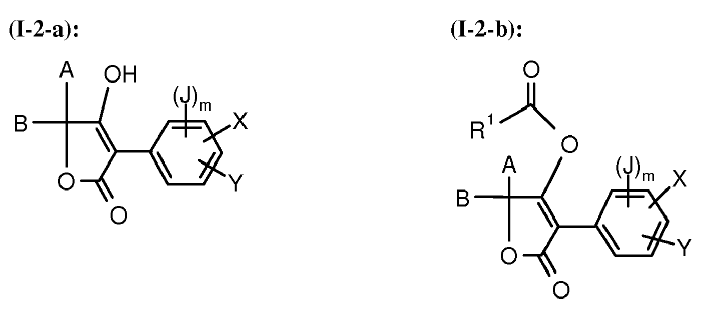 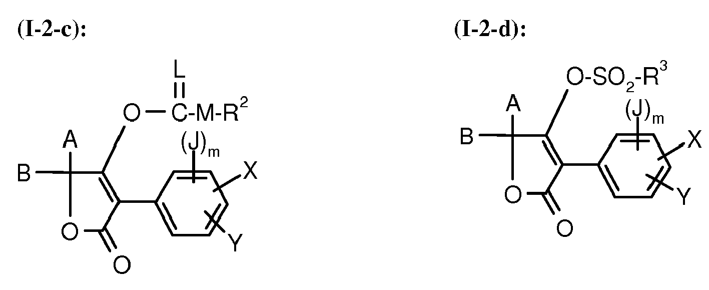 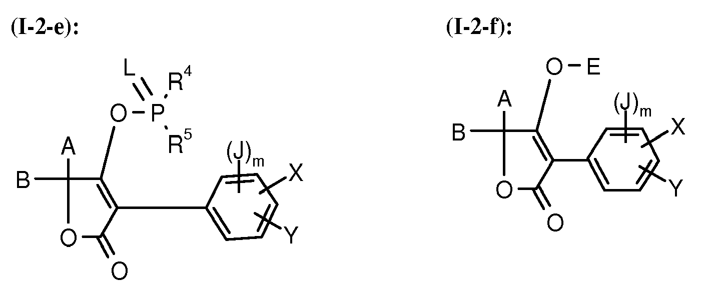 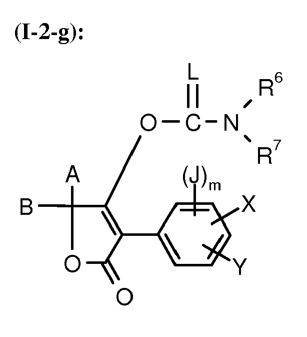 worin

A, B, E, J, L, m, M, X, Y, R¹, R², R³, R⁴, R⁵, R⁶ und R⁷ die oben angegebene Bedeutung haben.

Unter Einbeziehung der verschiedenen Bedeutungen (a), (b), (c), (d), (e), (f) und (g) der Gruppe G ergeben sich folgende hauptsächliche Strukturen (I-3-a) bis (I-3-g), wenn CKE für die Gruppe (3) steht, 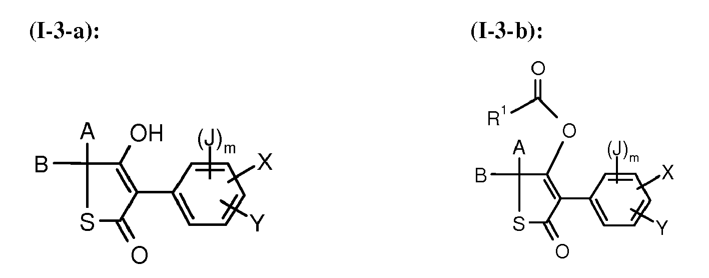 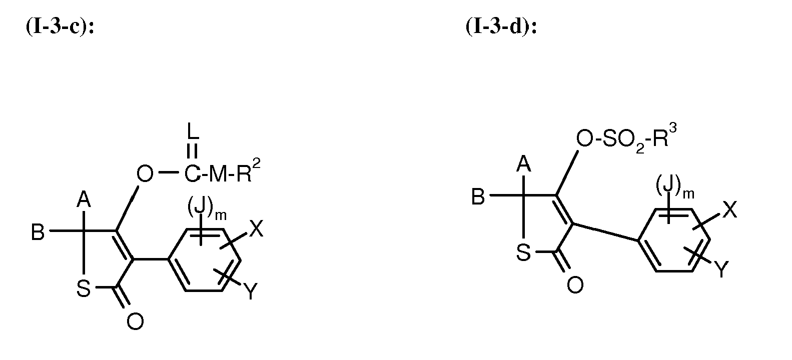 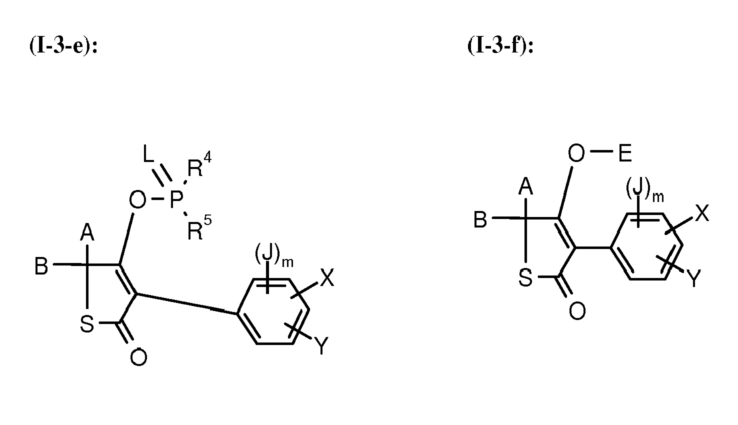 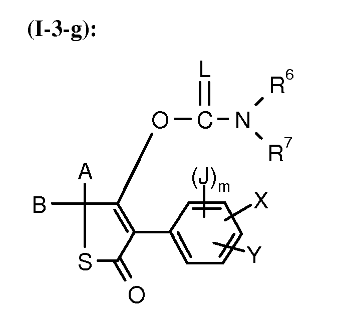 worin

A, B, E, J, L, m, M, X, Y, R¹, R², R³, R⁴, R⁵, R⁶ und R⁷ die oben angegebenen Bedeutung besitzen.

Die Verbindungen der Formel (I-4) können in Abhängigkeit von der Stellung des Substituenten G in den zwei isomeren Formen der Formeln (I-4-A) und (I-4-B) vorliegen, 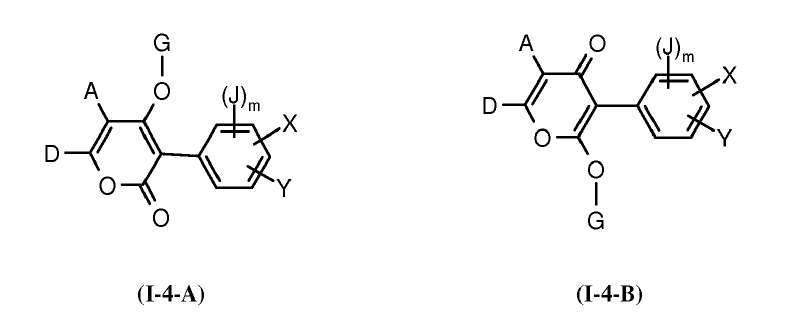 was durch die gestrichelte Linie in der Formel (I-4) zum Ausdruck gebracht werden soll.

Die Verbindungen der Formeln (I-4-A) und (I-4-B) können sowohl als Gemische als auch in Form ihrer reinen Isomeren vorliegen. Gemische der Verbindungen der Formeln (I-4-A) und (I-4-B) lassen sich gegebenenfalls in an sich bekannter Weise durch physikalische Methoden trennen, beispielsweise durch chromatographische Methoden.

Aus Gründen der besseren Übersichtlichkeit wird im folgenden jeweils nur eines der möglichen Isomeren aufgeführt. Das schließt nicht aus, dass die Verbindungen gegebenenfalls in Form der Isomerengemische oder in der jeweils anderen isomeren Form vorliegen können.

Unter Einbeziehung der verschiedenen Bedeutungen (a), (b), (c), (d), (e), (f) und (g) der Gruppe G ergeben sich folgende hauptsächliche Strukturen (I-4-a) bis (I-4-g), wenn CKE für die Gruppe (4) steht, 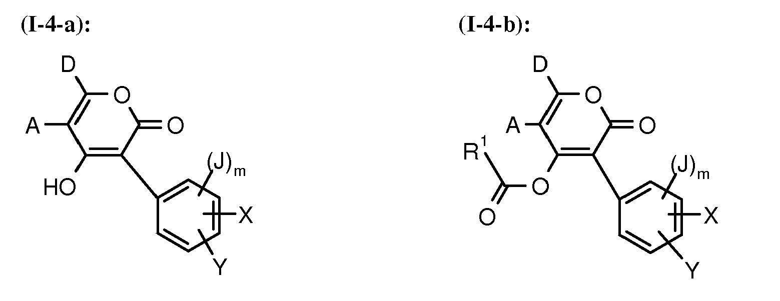 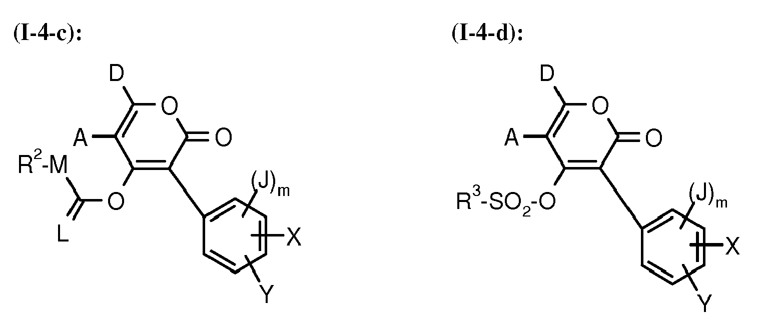 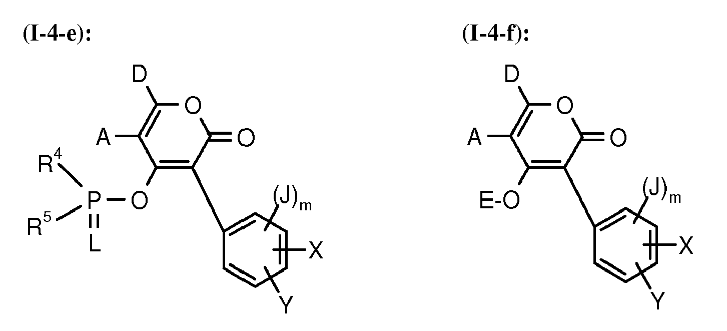 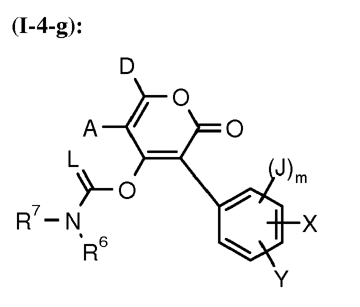 worin

A, D, E, J, L, m, M, X, Y, R¹, R², R³, R⁴, R⁵, R⁶ und R⁷ die oben angegebenen Bedeutungen besitzen.

Unter Einbeziehung der verschiedenen Bedeutungen (a), (b), (c), (d), (e), (f) und (g) der Gruppe G ergeben sich folgende hauptsächliche Strukturen (I-5-a) bis (I-5-g), wenn CKE für die Gruppe (5) steht, 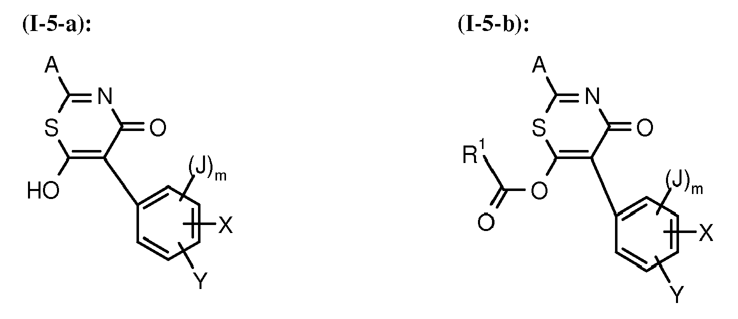 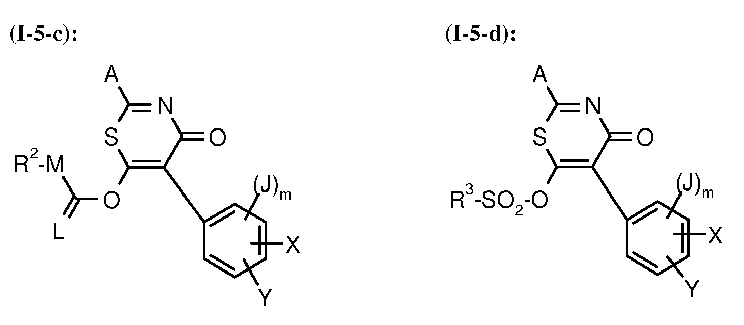 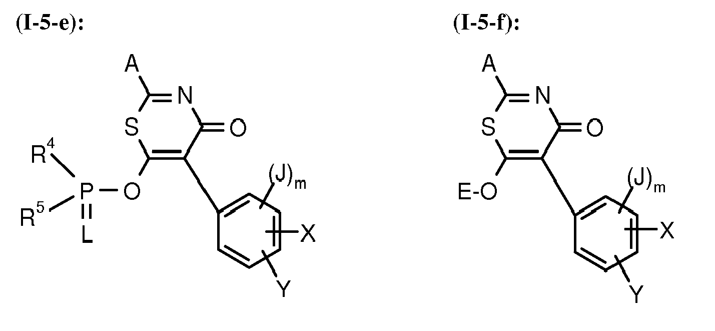 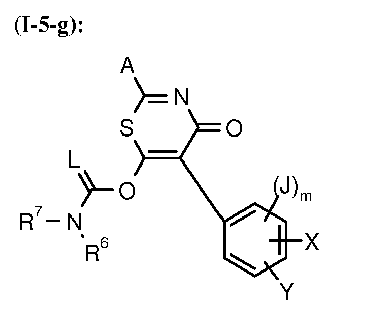 worin

A, E, J, L, m, M, X, Y, R¹, R², R³, R⁴, R⁵, R⁶ und R⁷ die oben angegebenen Bedeutungen besitzen.

Die Verbindungen der Formel (I-6) können in Abhängigkeit von der Stellung des Substituenten G in den zwei isomeren Formen der Formeln (I-6-A) und (I-6-B) vorliegen, 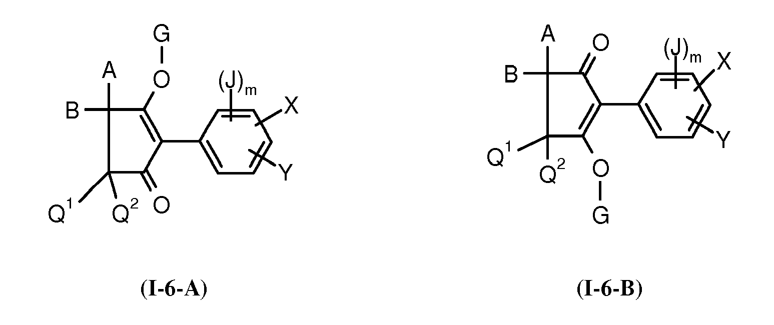 was durch die gestrichelte Linie in der Formel (I) zum Ausdruck gebracht werden soll.

Die Verbindungen der Formeln (I-6-A) und (I-6-B) können sowohl als Gemische als auch in Form ihrer reinen Isomeren vorliegen. Gemische der Verbindungen der Formeln (I-6-A) und (I-6-B) lassen sich gegebenenfalls durch physikalische Methoden trennen, beispielsweise durch chromatographische Methoden.

Aus Gründen der besseren Übersichtlichkeit wird im folgenden jeweils nur eines der möglichen Isomeren aufgeführt. Das schließt nicht aus, dass die Verbindungen gegebenenfalls in Form der Isomerengemische oder in der jeweils anderen isomeren Form vorliegen können.

Unter Einbeziehung der verschiedenen Bedeutungen (a), (b), (c), (d), (e), (f) und (g) der Gruppe G ergeben sich folgende hauptsächlichen Strukturen (I-6-a) bis (I-6-g): 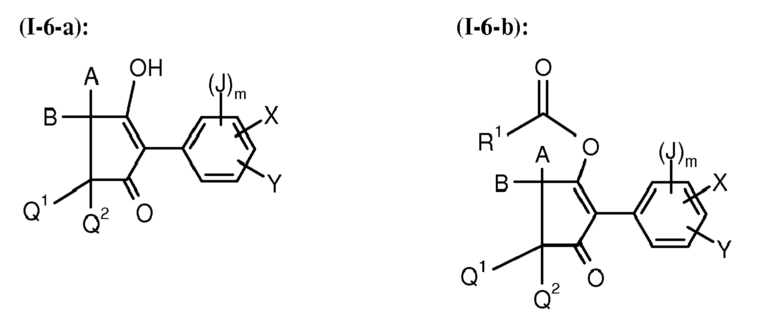 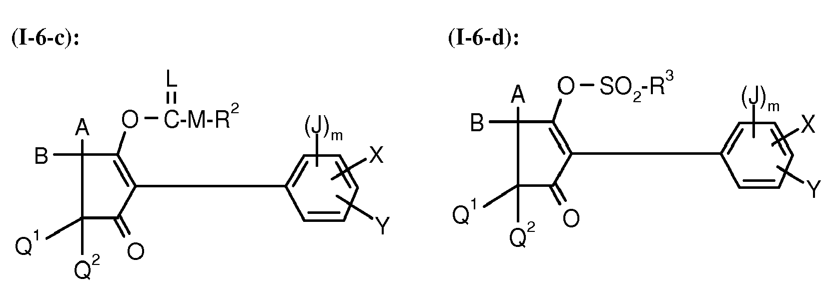 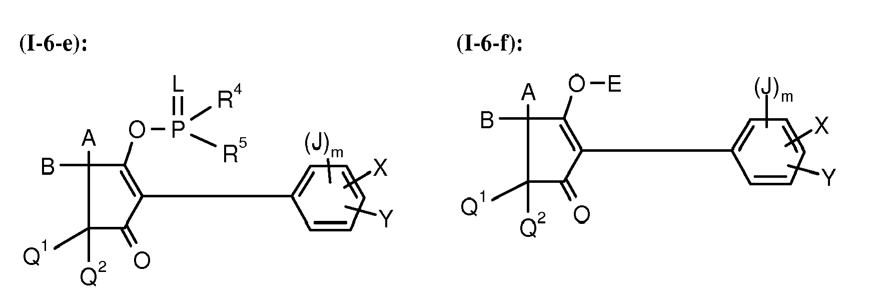 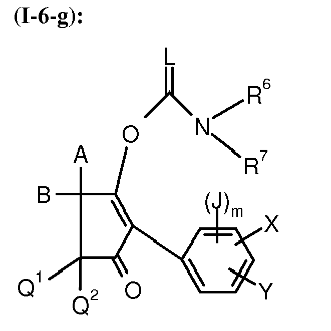 worin

A, B, J, Q¹, Q², E, L, m, M, X, Y, R¹, R², R³, R⁴, R⁵, R⁶ und R⁷ die oben angegebenen Bedeutungen besitzen.

Die Verbindungen der Formel (I-7) können in Abhängigkeit von der Stellung des Substituenten G in den zwei isomeren Formen der Formeln (I-7-A) bzw. (I-7-B) vorliegen, was durch die gestrichelte Linie in der Formel (I-7) zum Ausdruck gebracht werden soll:

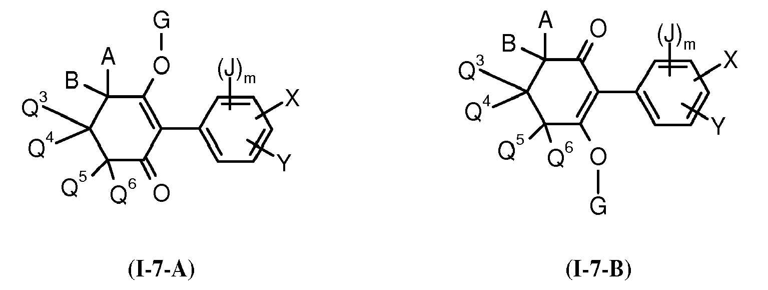

Die Verbindungen der Formeln (I-7-A) bzw. (I-7-B) können sowohl als Gemische als auch in Form ihrer reinen Isomeren vorliegen. Gemische der Verbindungen der Formeln (I-7-A) und (I-7-B) lassen sich gegebenenfalls durch physikalische Methoden trennen, beispielsweise durch chromatographische Methoden.

Aus Gründen der besseren Übersichtlichkeit wird im folgenden jeweils nur eines der möglichen Isomeren aufgeführt. Das schließt ein, dass die betreffende Verbindung gegebenenfalls als Isomerengemisch oder in der jeweils anderen isomeren Form vorliegen kann.

Unter Einbeziehung der verschiedenen Bedeutungen (a), (b), (c), (d), (e), (f) und (g) der Gruppe G ergeben sich folgende hauptsächlichen Strukturen (I-7-a) bis (I-7-g): 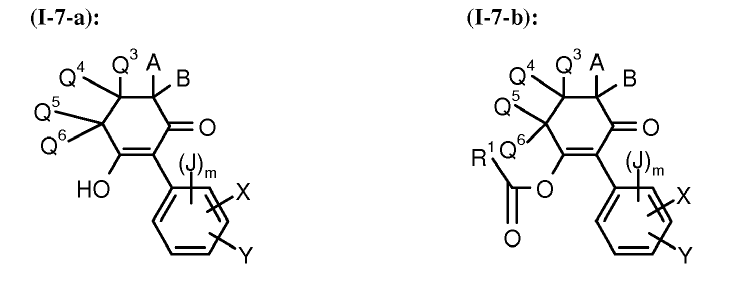 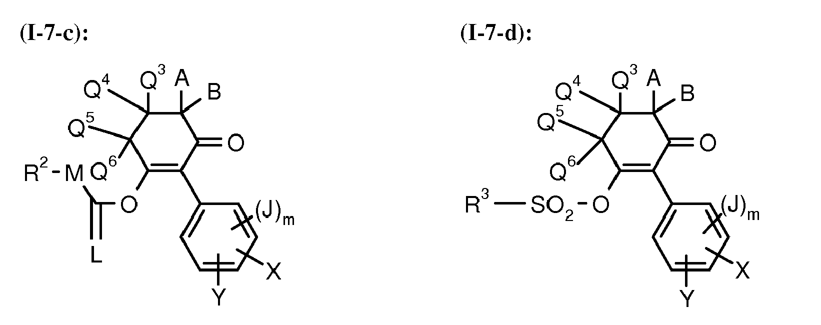 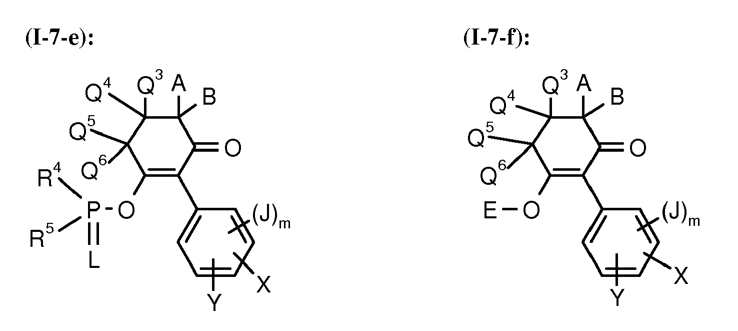 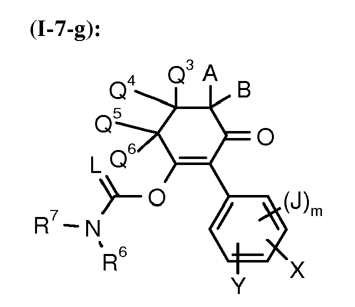 worin

A, B, J, E, L, m, M, Q³, Q⁴, Q⁵, Q⁶ X, Y, R¹, R² R³, R⁴, R⁵, R⁶ und R⁷ die oben angegebenen Bedeutungen besitzen.

Die Verbindungen der Formel (I-8) können in Abhängigkeit von der Stellung des Substituenten G in den zwei isomeren Formeln (I-8-A) und (I-8-B) vorliegen, 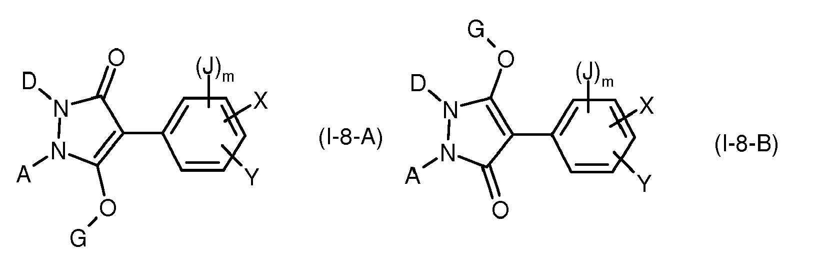 was durch die gestrichelte Linie in der Formel (I-8) zum Ausdruck gebracht werden soll.

Die Verbindungen der Formeln (I-8-A) und (I-8-B) können sowohl als Gemische als auch in Form ihrer reinen Isomeren vorliegen. Gemische der Verbindungen der Formel (I-8-A) und (I-8-B) lassen sich gegebenenfalls in an sich bekannter Weise durch physikalische Methoden trennen, beispielsweise durch chromatographische Methoden.

Aus Gründen der besseren Übersichtlichkeit wird im folgenden jeweils nur eines der möglichen Isomeren aufgeführt. Das schließt nicht aus, dass die Verbindungen gegebenenfalls in Form der Isomerengemische oder in der jeweils anderen isomeren Form vorliegen können.

Unter Einbeziehung der verschiedenen Bedeutungen (a), (b), (c), (d), (e), (f) und (g) der Gruppe G ergeben sich folgende hauptsächliche Strukturen (I-8-a) bis (I-8-g), wenn CKE für die Gruppe (8) steht, 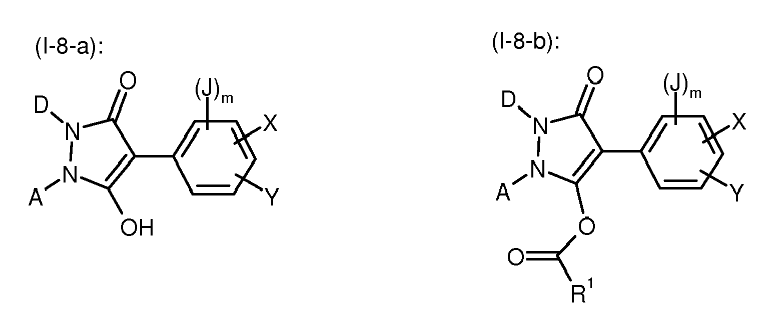 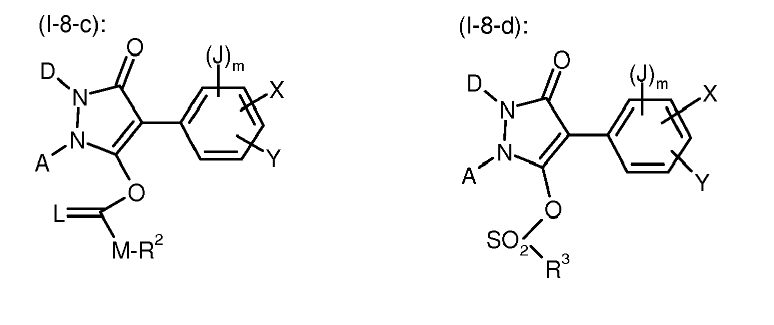 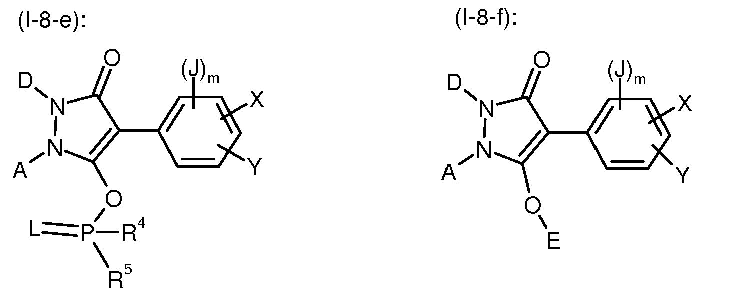 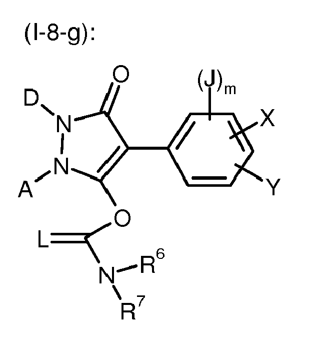 worin

A, D, E, J, L, M, m, X, Y, R¹, R², R³, R⁴, R⁵, R⁶ und R⁷ die oben angegebenen Bedeutungen besitzen.

Die Verbindungen der Formel (I-9) können in Abhängigkeit von der Stellung des Substituenten G in den zwei isomeren Formen der Formeln (I-9-A) und (I-9-B) vorliegen, 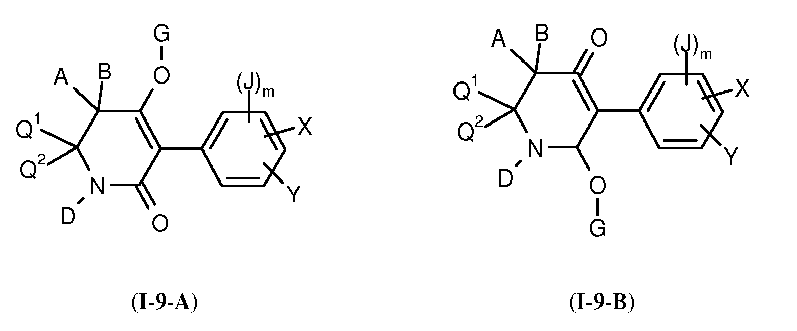 was durch die gestrichelte Linie in der Formel (I-9) zum Ausdruck gebracht werden soll.

Die Verbindungen der Formeln (I-9-A) und (I-9-B) können sowohl als Gemische als auch in Form ihrer reinen Isomeren vorliegen. Gemische der Verbindungen der Formeln (I-9-A) und (I-9-B) lassen sich gegebenenfalls in an sich bekannter Weise durch physikalische Methoden trennen, beispielsweise durch chromatographische Methoden.

Aus Gründen der besseren Übersichtlichkeit wird im folgenden jeweils nur eines der möglichen Isomeren aufgeführt. Das schließt nicht aus, dass die Verbindungen gegebenenfalls in Form der Isomerengemische oder in der jeweils anderen isomeren Form vorliegen können.

Unter Einbeziehung der verschiedenen Bedeutungen (a), (b), (c), (d), (e), (f) und (g) der Gruppe G ergeben sich folgende hauptsächliche Strukturen (I-9-a) bis (I-9-g) wenn CKE für die Gruppe (9) steht, 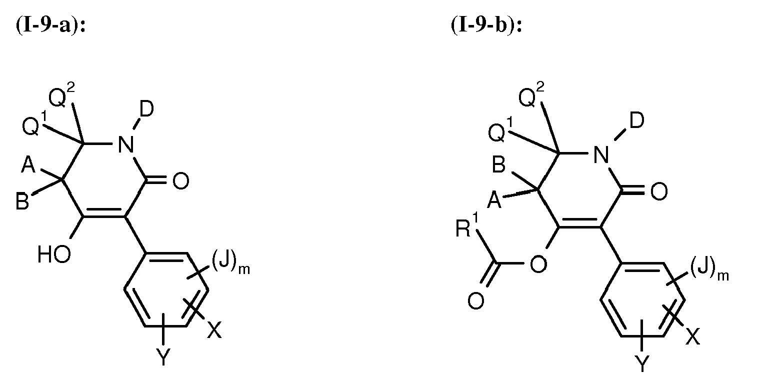 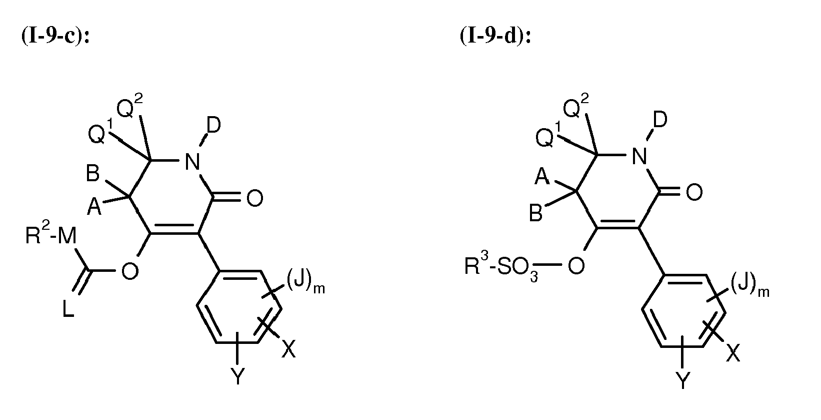 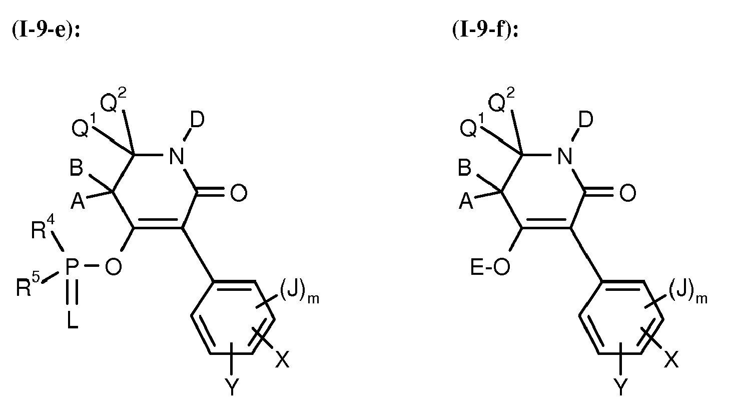 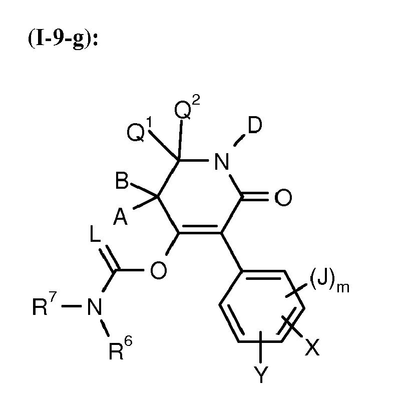 worin

A, B, D, E, J, L, m, M, Q¹, Q², X, Y, R¹, R², R³, R⁴, R⁵, R⁶ und R⁷ die oben angegebenen Bedeutungen besitzen.

Die Verbindungen der Formel (I-10) können in Abhängigkeit von der Stellung des Substituenten G in den zwei isomeren Formen der Formeln (I-10-A) und (I-10-B) vorliegen, 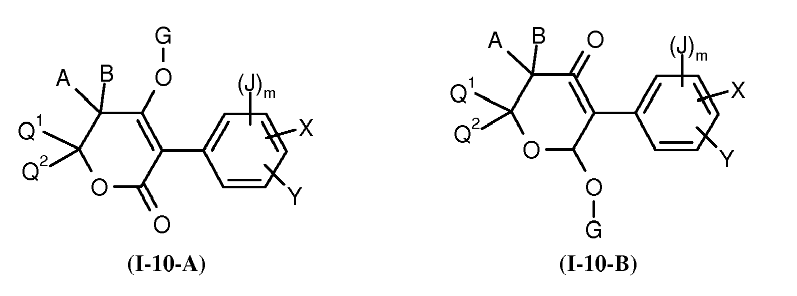 was durch die gestrichelte Linie in der Formel (I-10) zum Ausdruck gebracht werden soll.

Die Verbindungen der Formeln (I-10-A) und (I-10-B) können sowohl als Gemische als auch in Form ihrer reinen Isomeren vorliegen. Gemische der Verbindungen der Formeln (I-10-A) und (I-10-B) lassen sich gegebenenfalls in an sich bekannter Weise durch physikalische Methoden trennen, beispielsweise durch chromatographische Methoden.

Aus Gründen der besseren Übersichtlichkeit wird im folgenden jeweils nur eines der möglichen Isomeren aufgeführt. Das schließt nicht aus, dass die Verbindungen gegebenenfalls in Form der Isomerengemische oder in der jeweils anderen isomeren Form vorliegen können.

Unter Einbeziehung der verschiedenen Bedeutungen (a), (b), (c), (d), (e), (f) und (g) der Gruppe G ergeben sich folgende hauptsächliche Strukturen (I-10-a) bis (I-10-g) wenn CKE für die Gruppe (10) steht, 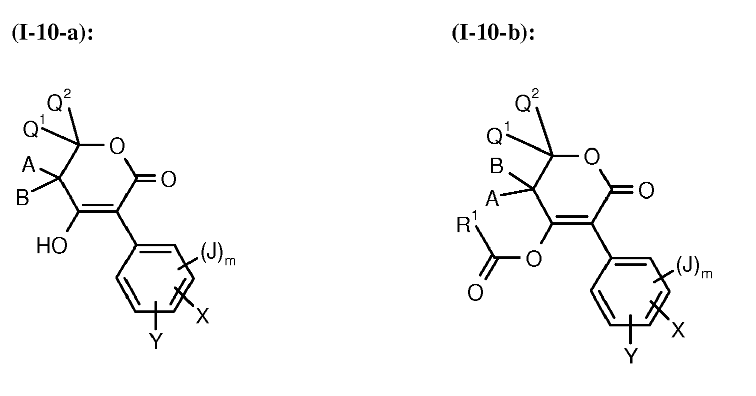 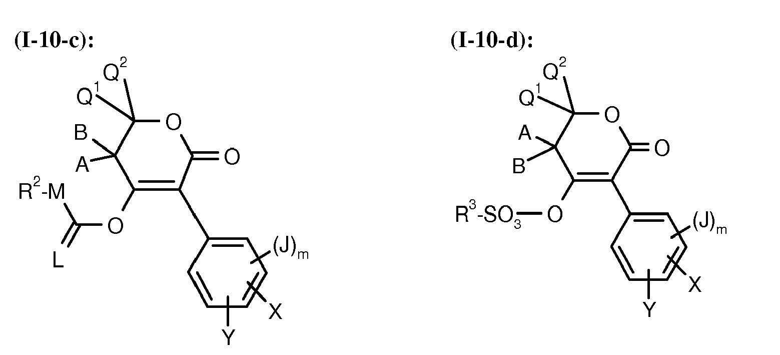 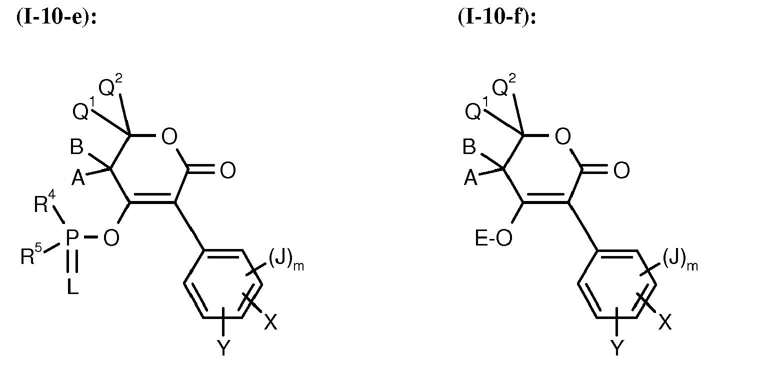 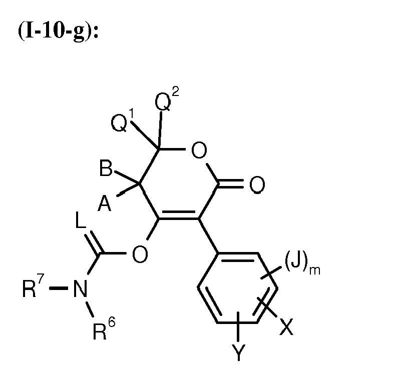 worin

A, B, E, L, m, M, Q¹, Q², X, Y, R¹, R², R³, R⁴, R⁵, R⁶ und R⁷ die oben angegebenen Bedeutungen besitzen.

Weiterhin wurde gefunden, dass man die neuen Verbindungen der Formel (I) nach einem der im folgenden beschriebenen Verfahren erhält:
(A) Man erhält substituierte 3-Phenylpyrrolidin-2,4-dione bzw. deren Enole der Formel (I-1-a) 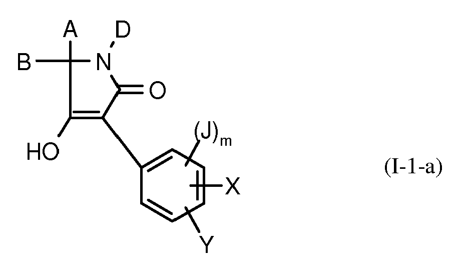 in welcher
   A, B, D, J, m, X und Y die oben angegebenen Bedeutungen haben,
   wenn man
   N-Acylaminosäureester der Formel (II) 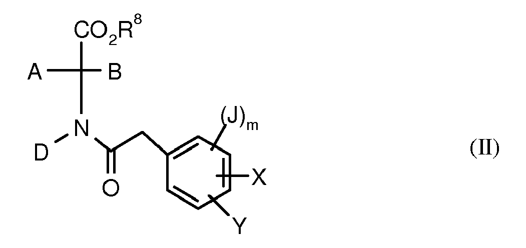 in welcher
   A, B, D, J, m, X und Y die oben angegebenen Bedeutungen haben,
   und
   R⁸ für Alkyl (bevorzugt C₁-C₆-Alkyl) steht,
   in Gegenwart eines Verdünnungsmittels und in Gegenwart einer Base intramolekular kondensiert.
(B) Außerdem wurde gefunden, dass man substituierte 3-Phenyl-4-hydroxy-Δ³-dihydrofuranon-Derivate der Formel (I-2-a) 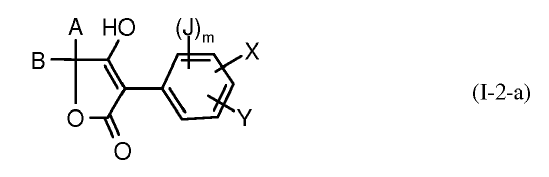 in welcher
   A, B, J, m, X und Y die oben angegebenen Bedeutungen haben,
   erhält, wenn man
   Carbonsäureester der Formel (III) 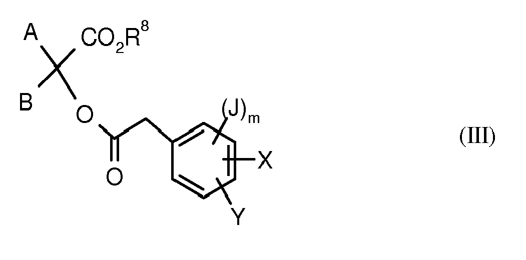 in welcher
   A, B, J, m, X , Y und R⁸ die oben angegebenen Bedeutungen haben,
   in Gegenwart eines Verdünnungsmittels und in Gegenwart einer Base intramolekular kondensiert.
(C) Weiterhin wurde gefunden, dass man substituierte 3-Phenyl-4-hydroxy-Δ³-dihydrothio-phenon-Derivate der Formel (I-3-a) 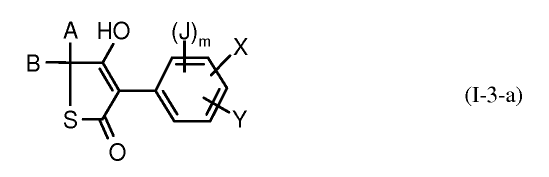 in welcher
   A, B, J, m, X und Y die oben angegebenen Bedeutungen haben,
   erhält, wenn man
   β-Ketocarbonsäureester der Formel (IV) 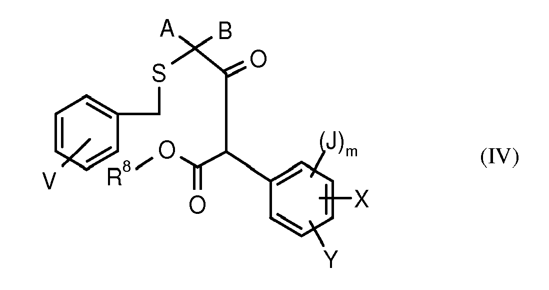 in welcher
   A, B, J, m, X, Y und R⁸ die oben angegebenen Bedeutungen haben und
   V für Wasserstoff, Halogen, Alkyl (bevorzugt C₁-C₆-Alkyl) oder Alkoxy (bevorzugt C₁-C₈-Alkoxy) steht,
   gegebenenfalls in Gegenwart eines Verdünnungsmittels und in Gegenwart einer Säure intramolekular cyclisiert.
(D) Weiterhin wurde gefunden, dass man die neuen substituierten 3-Phenylpyron-Derivate der Formel (I-4-a) 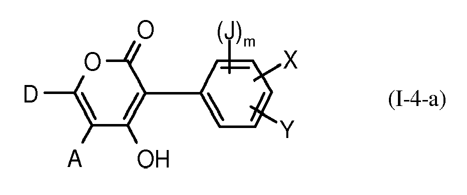 in welcher
   A, D, J, m, X und Y die oben angegebenen Bedeutungen haben,
   erhält, wenn man
   Carbonylverbindungen der Formel (V) 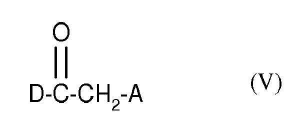 in welcher
   A und D die oben angegebenen Bedeutungen haben,
   oder deren Silylenolether der Formel (Va) 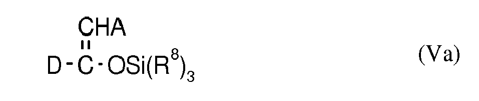 in welcher
   A, D und R⁸ die oben angegebene Bedeutung haben,
   mit Ketensäurehalogeniden der Formel (VI) 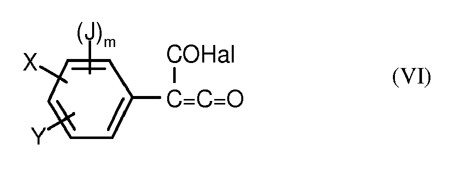 in welcher
   J, m, X und Y die oben angegebenen Bedeutungen haben und
   Hal für Halogen (vorzugsweise für Chlor oder Brom) steht,
   gegebenenfalls in Gegenwart eines Verdünnungsmittels und gegebenenfalls in Gegenwart eines Säureakzeptors umsetzt.
   Weiterhin wurde gefunden,
(E) dass man die neuen substituierten Phenyl-1,3-thiazin-Derivate der Formel (I-5-a) 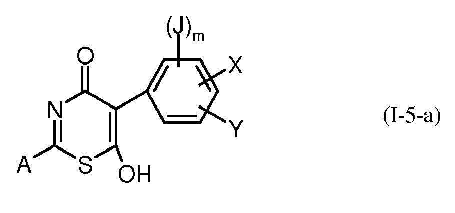 in welcher
   A, J, m, X und Y die oben angegebene Bedeutung haben,
   erhält, wenn man Thioamide der Formel (VII) 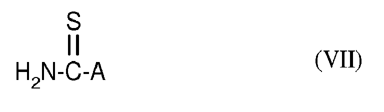 in welcher
   A die oben angegebene Bedeutung hat,
   mit Ketensäurehalogeniden der Formel (VI) 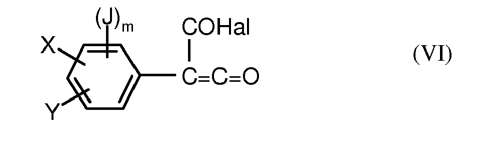 in welcher
   Hal, J, m, X und Y die oben angegebenen Bedeutungen haben,
   gegebenenfalls in Gegenwart eines Verdünnungsmittels und gegebenenfalls in Gegenwart eines Säureakzeptors umsetzt.
   Weiterhin wurde gefunden,
(F) dass man Verbindungen der Formel (I-6-a) 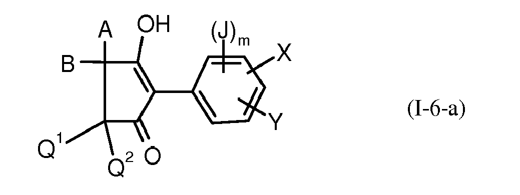 in welcher
   A, B, Q¹, Q², J, m, X und Y die oben angegebene Bedeutung haben,
   erhält, wenn man
   Ketocarbonsäureester der Formel (VIII) 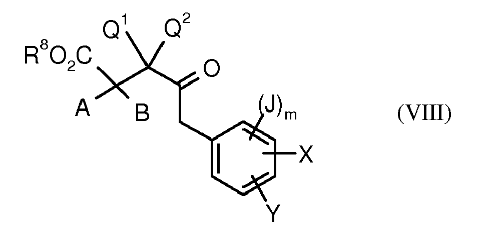 in welcher
   A, B, Q¹, Q², J, m, X und Y die oben angegebene Bedeutung haben, und
   R⁸ für Alkyl (insbesondere C₁-C₈-Alkyl) steht,
   gegebenenfalls in Gegenwart eines Verdünnungsmittels und in Gegenwart einer Base intramolekular cyclisiert.
   Außerdem wurde gefunden,
(G) dass man Verbindungen der Formel (I-7-a) 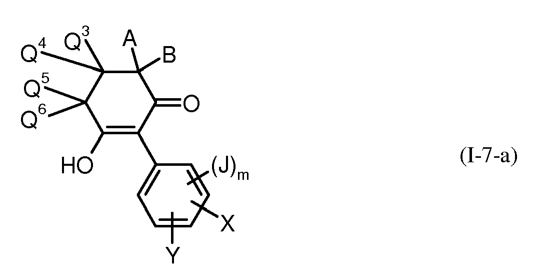 in welcher
   A, B, J, m, Q³, Q⁴, Q⁵, Q⁶, X und Y die oben angegebene Bedeutung haben,
   erhält, wenn man
   6-Aryl-5-keto-hexansäureester der Formel (IX) 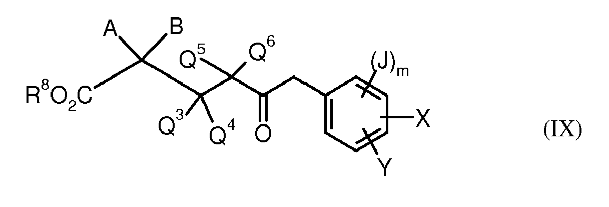 in welcher
   A, B, J, m, Q³, Q⁴, Q⁵, Q⁶, X und Y die oben angegebene Bedeutung haben
   und
   R⁸ für Alkyl (bevorzugt C₁-C₆-Alkyl) steht,
   in Gegenwart eines Verdünnungsmittels und in Gegenwart einer Base intramolekular kondensiert.
(H) Weiterhin wurde gefunden, dass man die Verbindungen der Formel (I-8-a) 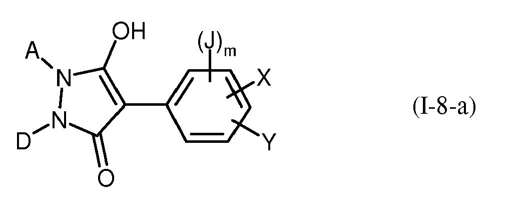 in welcher
   A, D, J, m, X und Y die oben angegebenen Bedeutungen haben,
   erhält, wenn man
   Verbindungen der Formel (X) 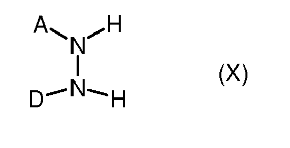 in welcher
   A und D die oben angegebene Bedeutung haben,
   α) mit Verbindungen der Formel (VI) 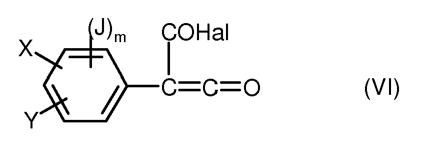 in welcher
   Hal, m, X, Y und J die oben angegebenen Bedeutungen haben,
   gegebenenfalls in Gegenwart eines Verdünnungsmittels und gegebenenfalls in Gegenwart eines Säureakzeptors umsetzt, oder
   β) mit Verbindungen der Formel (XI) 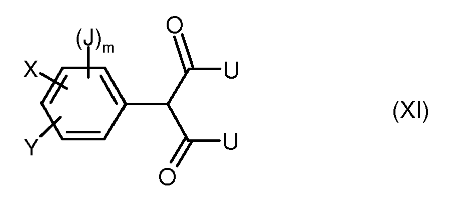 in welcher
   J, m, X und Y die oben angegebene Bedeutung haben,
   und U für NH₂ oder O-R⁸ steht, wobei R⁸ die oben genannte Bedeutung hat,
   gegebenenfalls in Gegenwart eines Verdünnungsmittels und gegebenenfalls in Gegenwart einer Base umsetzt, oder
   γ) mit Verbindungen der Formel (XII) 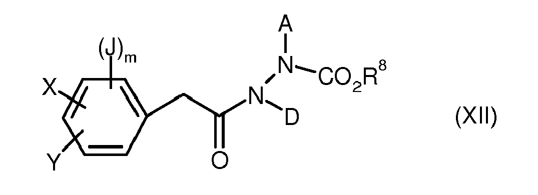 in welcher
   A, D, J, m, X, Y und R⁸ die oben angegebene Bedeutung haben,
   gegebenenfalls in Gegenwart eines Verdünnungsmittels und gegebenenfalls in Gegenwart einer Base umsetzt.
   Außerdem wurde gefunden, dass man die neuen Verbindungen der Formel (I) nach einem der im folgenden beschriebenen Verfahren erhält:
(I) Man erhält substituierte Tetrahydropyridin-2,4-dione bzw. deren Enole der Formel (I-9-a) 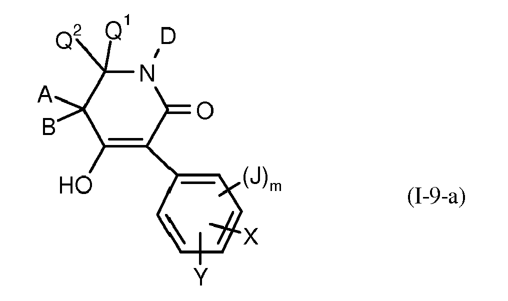 in welcher
   A, B, D, J, m, Q¹, Q², X und Y die oben angegebenen Bedeutungen haben,
   wenn man
   N-Acylaminosäureester der Formel (XIII) 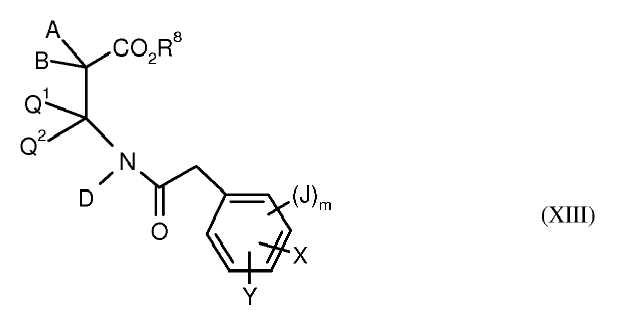 in welcher
   A, B, D, J, m, Q¹, Q², X und Y die oben angegebenen Bedeutungen haben,
   und
   R⁸ für Alkyl (bevorzugt C₁-C₆-Alkyl) steht,
   in Gegenwart eines Verdünnungsmittels und in Gegenwart einer Base intramolekular kondensiert.
   Weiterhin wurde gefunden,
(J) dass man substituierte 5,6-Dihydropyrone der Formel (I-10-a) erhält 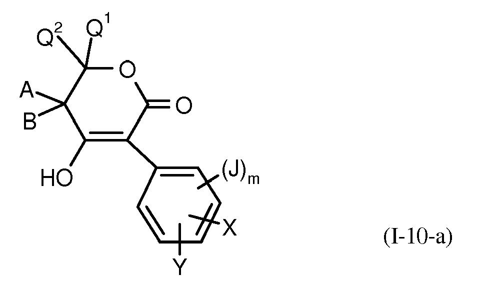 in welcher
   A, B, J, m, Q¹, Q², X und Y die oben angegebene Bedeutungen haben,
   wenn man
   O-Acylhydroxycarbonsäureester der Formel (XIV) 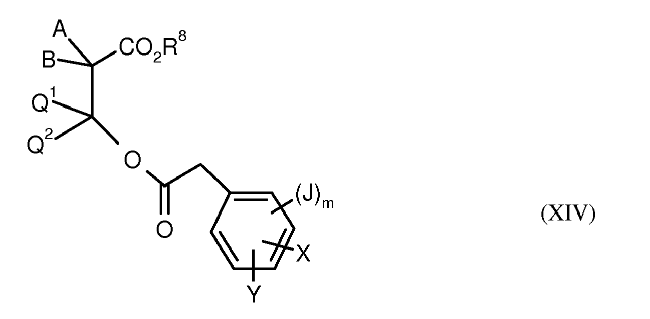 in welcher
   A, B, J, m, Q¹, Q², X und Y die oben angegebenen Bedeutungen haben,
   und
   R⁸ für Alkyl (bevorzugt C₁-C₆-Alkyl) steht,
   in Gegenwart eines Verdünnungsmittels und in Gegenwart einer Base intramolekular kondensiert.
   Außerdem wurde gefunden
(K) dass man die Verbindungen der oben gezeigten Formeln (I-1-b) bis (I-10-b), in welchen A, B, D, J, m, Q¹, Q², Q³, Q⁴, Q⁵, Q⁶ R¹, X und Y die oben angegebenen Bedeutungen haben, erhält, wenn man Verbindungen der oben gezeigten Formeln (I-1-a) bis (I-10-a), in welchen A, B, D, J, m, Q¹, Q², Q³, Q⁴, Q⁵, Q⁶ X und Y die oben angegebenen Bedeutungen haben, jeweils
   (α) mit Säurehalogeniden der Formel (XV) 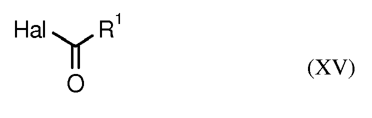 in welcher
   R¹ die oben angegebene Bedeutung hat und
   Hal für Halogen (insbesondere Chlor oder Brom) steht
   oder
   (β) mit Carbonsäureanhydriden der Formel (XVI)

   R¹-CO-O-CO-R¹ (XVI)

   in welcher
   R¹ die oben angegebene Bedeutung hat,
   gegebenenfalls in Gegenwart eines Verdünnungsmittels und gegebenenfalls in Gegenwart eines Säurebindemittels umsetzt;
(L) dass man die Verbindungen der oben gezeigten Formeln (I-1-c) bis (I-10-c), in welchen A, B, D, J, m, Q¹, Q², Q³, Q⁴, Q⁵, Q⁶ R² M X und Y die oben angegebenen Bedeutungen haben und L für Sauerstoff steht, erhält, wenn man Verbindungen der oben gezeigten Formeln (I-1-a) bis (I-10-a), in welchen A, B, D, J, m, Q¹, Q², Q³, Q⁴, Q⁵, Q⁶, X und Y die oben angegebenen Bedeutungen haben, jeweils
   mit Chlorameisensäureestern oder Chlorameisensäurethiolestern der Formel (XVII)

   R²-M-CO-Cl (XVII)

   in welcher
   R² und M die oben angegebenen Bedeutungen haben,
   gegebenenfalls in Gegenwart eines Verdünnungsmittels und gegebenenfalls in Gegenwart eines Säurebindemittels umsetzt;
(M) dass man Verbindungen der oben gezeigten Formeln (I-1-c) bis (I-10-c), in welchen A, B, D, J, m, Q¹, Q², Q³, Q⁴, Q⁵, Q⁶ R², M, X und Y die oben angegebenen Bedeutungen haben und L für Schwefel steht, erhält, wenn man Verbindungen der oben gezeigten Formeln (I-1-a) bis (I-10-a), in welchen A, B, D, J, m, Q¹, Q², Q³ Q⁴, Q⁵, Q⁶, X und Y die oben angegebenen Bedeutungen haben, jeweils
   mit Chlormonothioameisensäureestern oder Chlordithioameisensäureestern der Formel (XVIII) 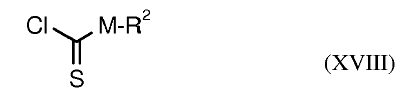 in welcher
   M und R² die oben angegebenen Bedeutungen haben,
   gegebenenfalls in Gegenwart eines Verdünnungsmittels und gegebenenfalls in Gegenwart eines Säurebindemittels umsetzt
   und
(N) dass man Verbindungen der oben gezeigten Formeln (I-1-d) bis (I-10-d), in welchen A, B, D, J, m, Q¹, Q², Q³, Q⁴, Q⁵, Q⁶, R³, X und Y die oben angegebenen Bedeutungen haben, erhält, wenn man Verbindungen der oben gezeigten Formeln (I-1-a) bis (I-10-a), in welchen A, B, D, J, m, Q¹, Q², Q³, Q⁴, Q⁵, Q⁶, X und Y die oben angegebenen Bedeutungen haben, jeweils
   mit Sulfonsäurechloriden der Formel (XIX)

   R³-SO₂-Cl (XIX)

   in welcher
   R³ die oben angegebene Bedeutung hat,
   gegebenenfalls in Gegenwart eines Verdünnungsmittels und gegebenenfalls in Gegenwart eines Säurebindemittels umsetzt,
(O) dass man Verbindungen der oben gezeigten Formeln (I-1-e) bis (I-10-e), in welchen A, B, D, J, m, L, Q¹, Q², Q³, Q⁴, Q⁵, Q⁶ R⁴, R⁵, X und Y die oben angegebenen Bedeutungen haben, erhält, wenn man Verbindungen der oben gezeigten Formeln (I-1-a) bis (I-10-a), in welchen A, B, D, J, m, Q¹, Q², Q³, Q⁴, Q⁵, Q⁶, X und Y die oben angegebenen Bedeutungen haben, jeweils
   mit Phosphorverbindungen der Formel (XX) 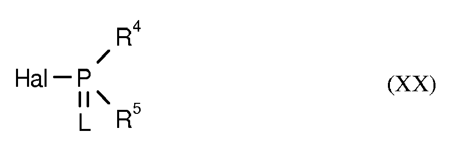 in welcher
   - L, R⁴ und R⁵: die oben angegebenen Bedeutungen haben und
   - Hal: für Halogen (insbesondere Chlor oder Brom) steht,
   gegebenenfalls in Gegenwart eines Verdünnungsmittels und gegebenenfalls in Gegenwart eines Säurebindemittels umsetzt,
(P) dass man Verbindungen der oben gezeigten Formeln (I-1-f) bis (I-10-f), in welchen A, B, D, E, J, m, Q¹, Q², Q³, Q⁴, Q⁵, Q⁶, X und Y die oben angegebenen Bedeutungen haben, erhält, wenn man Verbindungen der Formeln (I-1-a) bis (I-10-a), in welchen A, B, D, J, m, Q¹, Q², Q³, Q⁴, Q⁵, Q⁶, X und Y die oben angegebenen Bedeutungen haben, jeweils
   mit Metallverbindungen oder Aminen der Formeln (XXI) oder (XXII)

   Me(OR¹⁰)ₜ (XXI)

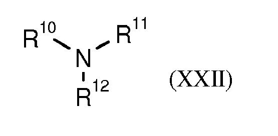

   in welchen
   - Me: für ein ein- oder zweiwertiges Metall (bevorzugt ein Alkali- oder Erdalkalimetall wie Lithium, Natrium, Kalium, Magnesium oder Calcium), oder für ein Ammoniumion

   - t: für die Zahl 1 oder 2 und
   - R¹⁰, R¹¹, R¹²: unabhängig voneinander für Wasserstoff oder Alkyl (bevorzugt C₁-C₈-Alkyl) stehen,
   gegebenenfalls in Gegenwart eines Verdünnungsmittels umsetzt,
(Q) dass man Verbindungen der oben gezeigten Formeln (I-1-g) bis (I-10-g), in welchen A, B, D, J, m, L, Q¹, Q², Q³, Q⁴, Q⁵, Q⁶ R⁶, R⁷, X und Y die oben angegebenen Bedeutungen haben, erhält, wenn man Verbindungen der oben gezeigten Formeln (I-1-a) bis (I-10-a), in welchen A, B, D, J, m, Q¹, Q², Q³, Q⁴, Q⁵, Q⁶, X und Y die oben angegebenen Bedeutungen haben, jeweils
   (α) mit Isocyanaten oder Isothiocyanaten der Formel (XXIII)

      R⁶-N=C=L (XXIII)

      in welcher
      - R⁶ und L: die oben angegebenen Bedeutungen haben,
      gegebenenfalls in Gegenwart eines Verdünnungsmittels und gegebenenfalls in Gegenwart eines Katalysators umsetzt oder
   (β) mit Carbamidsäurechloriden oder Thiocarbamidsäurechloriden der Formel (XXIV) 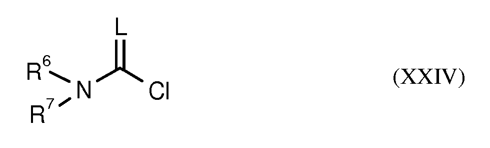 in welcher
      - L, R⁶ und R⁷: die oben angegebenen Bedeutungen haben,
      gegebenenfalls in Gegenwart eines Verdünnungsmittels und gegebenenfalls in Gegenwart eines Säurebindemittels, umsetzt,
   (R) dass man Verbindungen der oben gezeigten Formeln (I-1-a) bis (I-10-g), in welchen A, B, D, G, J, m, Q¹, Q², Q³, Q⁴, Q⁵, Q⁶, X und Y die oben angegebene Bedeutung haben, erhält, wenn man Verbindungen der Formeln (I-1') bis (I-10'-g), in welchen A, B, D, G, m, Q¹, Q², Q³, Q⁴, Q⁵, Q⁶, X und Y die oben genannte Bedeutung haben und J' bevorzugt für Brom oder Iod steht 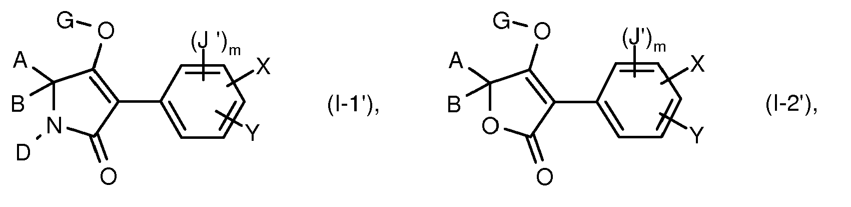 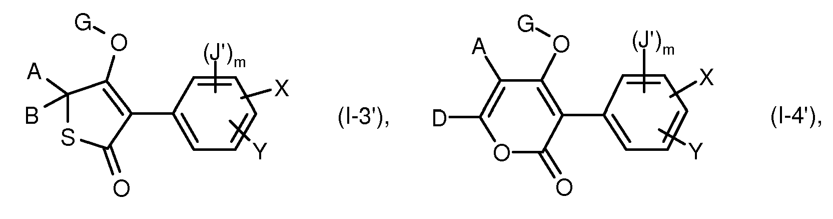 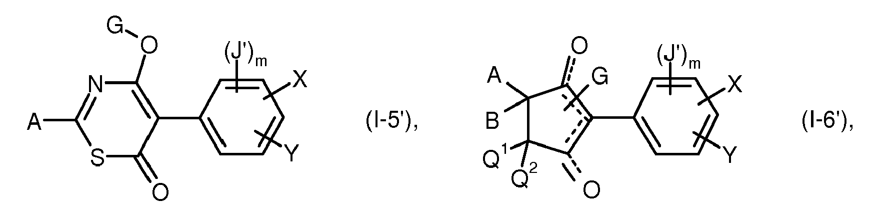 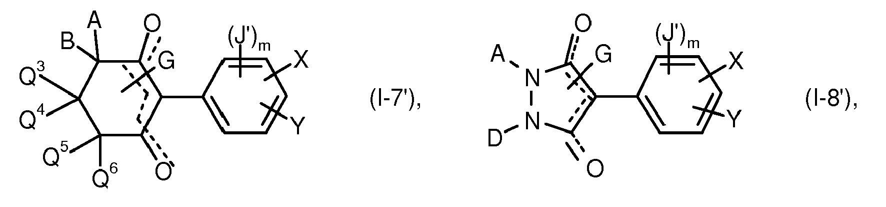 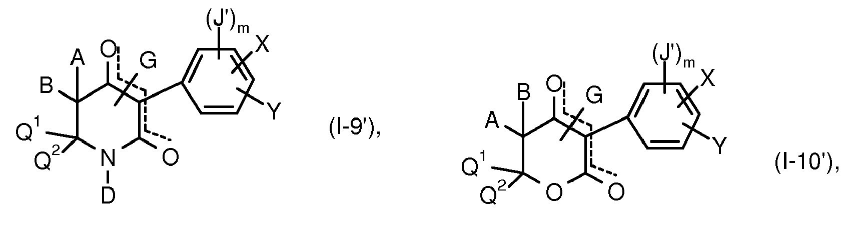 mit kupplungsfähigen Cycloalkylboronsäurederivaten, z.B. Cycloalkylboronsäuren der Formel (XXV) 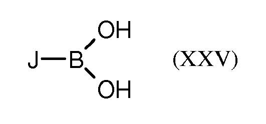
oder deren Ester in Gegenwart eines Lösungsmittels in Gegenwart eines Katalysators (z. B. Pd-Komplexe) und in Gegenwart einer Base (z.B. Natriumcarbonat, Kalium-dihydrogenphosphat) kuppelt.

Weiterhin wurde gefunden, dass die neuen Verbindungen der Formel (I) eine sehr gute Wirksamkeit als Schädlingsbekämpfungsmittel, vorzugsweise als Insektizide und/oder Akarizide und/oder Herbizide aufweisen.

Überraschenderweise wurde nun auch gefunden, dass bestimmte substituierte, cyclische Ketoenole bei gemeinsamer Anwendung mit den im weiteren beschriebenen, die Kulturpflanzen-Verträglichkeit verbessernden Verbindungen (Safenern/Antidots) ausgesprochen gut die Schädigung der Kulturpflanzen verhindern und besonders vorteilhaft als breit wirksame Kombinationspräparate zur selektiven Bekämpfung von unerwünschten Pflanzen in Nutzpflanzenkulturen, wie z.B. in Getreide aber auch Mais, Soja und Reis, verwendet werden können.

Gegenstand der Erfindung sind auch selektiv-herbizide Mittel enthaltend einen wirksamen Gehalt an einer Wirkstoffkombination umfassend als Komponenten
(a') mindestens ein substituiertes, cyclisches Ketoenol der Formel (I), in welcher CKE, J, m, X und Y die oben angegebene Bedeutung haben
   und
(b') zumindest eine die Kulturpflanzen-Verträglichkeit verbessernde Verbindung aus der folgenden Gruppe von Verbindungen:
   4-Dichloracetyl-1-oxa-4-aza-spiro[4.5]-decan (AD-67, MON-4660), 1-Dichloracetyl-hexahydro-3,3,8a-trimethylpyrrolo[1,2-a]-pyrimidin-6(2H)-on (Dicyclonon, BAS-145138), 4-Dichloracetyl-3,4-dihydro-3-methyl-2H-1,4-benzoxazin (Benoxacor), 5-Chlor-chinolin-8-oxy-essigsäure-(1-methyl-hexylester) (Cloquintocet-mexyl - vgl. auch verwandte Verbindungen in EP-A-86750, EP-A-94349, EP-A-191736, EP-A-492366), 3-(2-Chlor-benzyl)-1-(1-methyl-1-phenyl-ethyl)-harnstoff (Cumyluron), α-(Cyanomethoximino)-phenylacetonitril (Cyometrinil), 2,4-Dichlor-phenoxyessigsäure (2,4-D), 4-(2,4-Dichlor-phenoxy)-buttersäure (2,4-DB), 1-(1-Methyl-1-phenyl-ethyl)-3-(4-methyl-phenyl)-harnstoff (Daimuron, Dymron), 3,6-Dichlor-2-methoxy-benzoesäure (Dicamba), Piperidin-1-thiocarbonsäure-S-1-methyl-1-phenyl-ethylester (Dimepiperate), 2,2-Dichlor-N-(2-oxo-2-(2-propenylamino)-ethyl)-N-(2-propenyl)-acetamid (DKA-24), 2,2-Dichlor-N,N-di-2-propenyl-acetamid (Dichlormid), 4,6-Dichlor-2-phenyl-pyrimidin (Fenclorim), 1-(2,4-Dichlorphenyl)-5-trichlormethyl-1H-1,2,4-triazol-3-carbonsäure-ethylester (Fenchlorazole-ethyl - vgl. auch verwandte Verbindungen in EP-A-174562 und EP-A-346620), 2-Chlor-4-trifluormethyl-thiazol-5-carbonsäure-phenylmethylester (Flurazole), 4-Chlor-N-(1,3-dioxolan-2-yl-methoxy)-a-trifluor-acetophenonoxim (Fluxofenim), 3-Dichloracetyl-5-(2-furanyl)-2,2-dimethyl-oxazolidin (Furilazole, MON-13900), Ethyl-4,5-dihydro-5,5-diphenyl-3-isoxazolcarboxylat (Isoxadifen-ethyl - vgl. auch verwandte Verbindungen in WO-A-95/07897), 1-(Ethoxycarbonyl)-ethyl-3,6-dichlor-2-methoxybenzoat (Lactidichlor), (4-Chlor-o-tolyloxy)-essigsäure (MCPA), 2-(4-Chlor-o-tolyloxy)-propionsäure (Mecoprop), Diethyl-1-(2,4-dichlor-phenyl)-4,5-dihydro-5-methyl-1H-pyrazol-3,5-dicarboxylat (Mefenpyr-diethyl - vgl. auch verwandte Verbindungen in WO-A-91/07874) 2-Dichlormethyl-2-methyl-1,3-dioxolan (MG-191), 2-Propenyl-1-oxa-4-azaspiro[4.5]decane-4-carbodithioate (MG-838), 1,8-Naphthalsäureanhydrid, α-(1,3-Dioxolan-2-yl-methoximino)-phenylacetonitril (Oxabetrinil), 2,2-Dichlor-N-(1,3-dioxolan-2-yl-methyl)-N-(2-propenyl)-acetamid (PPG-1292), 3-Dichloracetyl-2,2-dimethyl-oxazolidin (R-28725), 3-Dichloracetyl-2,2,5-trimethyl-oxazolidin (R-29148), 4-(4-Chlor-o-tolyl)-buttersäure, 4-(4-Chlor-phenoxy)-buttersäure, Diphenylmethoxyessigsäure, Diphenylmethoxyessigsäure-methylester, Diphenylmethoxyessigsäure-ethylester, 1-(2-Chlor-phenyl)-5-phenyl-1H-pyrazol-3-carbonsäure-methylester, 1-(2,4-Dichlor-phenyl)-5-methyl-1H-pyrazol-3-carbonsäure-ethylester, 1-(2,4-Dichlor-phenyl)-5-isopropyl-1H-pyrazol-3-carbonsäure-ethylester, 1-(2,4-Dichlor-phenyl)-5-(l,l-dimethyl-ethyl)-1H-pyrazol-3-carbonsäure-ethylester, 1-(2,4-Dichlor-phenyl)-5-phenyl-1H-pyrazol-3-carbonsäure-ethylester (vgl. auch verwandte Verbindungen in EP-A-269806 und EP-A-333131), 5-(2,4-Dichlor-benzyl)-2-isoxazolin-3-carbonsäure-ethylester, 5-Phenyl-2-isoxazolin-3-carbonsäure-ethylester, 5-(4-Fluor-phenyl)-5-phenyl-2-isoxazolin-3-carbonsäure-ethylester (vgl. auch verwandte Verbindungen in WO-A-91/08202), 5-Chlor-chinolin-8-oxy-essigsäure-(1,3-dimethyl-but-1-yl)-ester, 5-Chlor-chinolin-8-oxy-essigsäure-4-allyloxy-butylester, 5-Chlor-chinolin-8-oxy-essigsäure-1-allyloxy-prop-2-yl-ester, 5-Chlor-chinoxalin-8-oxy-essigsäure-methylester, 5-Chlor-chinolin-8-oxy-essigsäure-ethylester, 5-Chlor-chinoxalin-8-oxy-essigsäure-allylester, 5-Chlor-chinolin-8-oxy-essigsäure-2-oxo-prop-1-yl-ester, 5-Chlor-chinolin-8-oxy-malonsäure-diethylester, 5-Chlor-chinoxalin-8-oxy-malonsäure-di-allylester, 5-Chlor-chinolin-8-oxy-malonsäure-diethylester (vgl. auch verwandte Verbindungen in EP-A-582198), 4-Carboxy-chroman-4-yl-essigsäure (AC-304415, vgl. EP-A-613618), 4-Chlor-phenoxy-essigsäure, 3,3'-Dimethyl-4-methoxy-benzophenon, 1-Brom-4-chlormethylsulfonyl-benzol, 1-[4-(N-2-Methoxybenzoylsulfamoyl)-phenyl]-3-methyl-harnstoff (alias N-(2-Methoxybenzoyl)-4-[(methylamino-carbonyl)-amino]-benzolsulfonamid), 1-[4-(N-2-Methoxybenzoyl-sulfamoyl)-phenyl]-3,3-dimethyl-harnstoff, 1-[4-(N-4,5-Dimethylbenzoylsulfamoyl)-phenyl]-3-methyl-harnstoff, 1-[4-(N-Naphthylsulfamoyl)-phenyl]-3,3-dimethyl-harnstoff, N-(2-Methoxy-5-methyl-benzoyl)-4-(cyclopropylaminocarbonyl)-benzolsulfonamid,
   und/oder eine der folgenden durch allgemeine Formeln definierten Verbindungen
   der allgemeinen Formel (IIa) 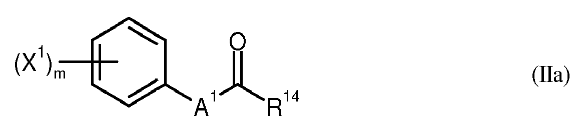 oder der allgemeinen Formel (IIb) 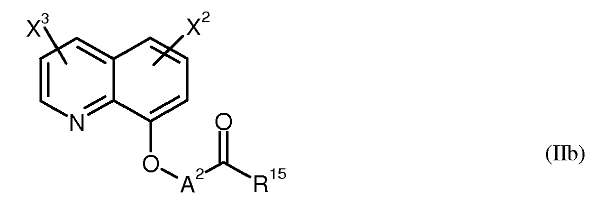 oder der Formel (IIc) 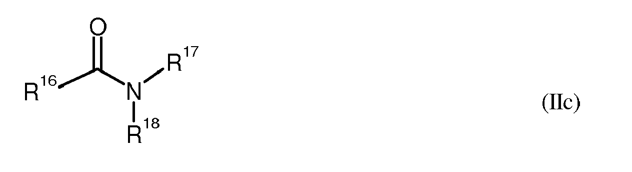
wobei
- m: für eine Zahl 0, 1, 2, 3, 4 oder 5 steht,
- A¹: für eine der nachstehend skizzierten divalenten heterocyclischen Gruppierungen steht, 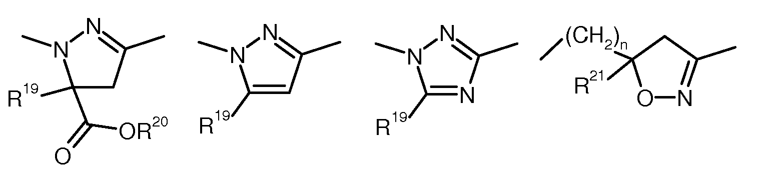
- n: für eine Zahl 0, 1, 2, 3, 4 oder 5 steht,
- A²: für gegebenenfalls durch C₁-C₄-Alkyl und/oder C₁-C₄-Alkoxy-carbonyl und/oder C₁-C₄-Alkenyloxy-carbonyl substituiertes Alkandiyl mit 1 oder 2 Kohlenstoffatomen steht,
- R¹⁴: für Hydroxy, Mercapto, Amino, C₁-C₆-Alkoxy, C₁-C₆-Alkylthio, C₁-C₆-Alkylamino oder Di- (C₁-C₄-alkyl)-amino steht,
- R¹⁵: für Hydroxy, Mercapto, Amino, C₁-C₇-Alkoxy, C₁-C₆-Alkenyloxy, C₁-C₆-Alkenyloxy-C₁-C₆-alkoxy, C₁-C₆-Alkylthio, C₁-C₆-Alkylamino oder Di-(C₁-C₄-alkyl)-amino steht,
- R¹⁶: für jeweils gegebenenfalls durch Fluor, Chlor und/oder Brom substituiertes C₁-C₄-Alkyl steht,
- R¹⁷: für Wasserstoff, jeweils gegebenenfalls durch Fluor, Chlor und/oder Brom substituiertes C₁-C₆-Alkyl, C₂-C₆-Alkenyl oder C₂-C₆-Alkinyl, C₁-C₄-Alkoxy-C₁-C₄-alkyl, Dioxolanyl-C₁-C₄-alkyl, Furyl, Furyl-C₁-C₄-alkyl, Thienyl, Thiazolyl, Piperidinyl, oder gegebenenfalls durch Fluor, Chlor und/oder Brom oder C₁-C₄-Alkyl substituiertes Phenyl steht,
- R¹⁸: für Wasserstoff, jeweils gegebenenfalls durch Fluor, Chlor und/oder Brom substituiertes C₁-C₆-Alkyl, C₂-C₆-Alkenyl oder C₂-C₆-Alkinyl, C₁-C₄-Alkoxy-C₁-C₄-alkyl, Dioxolanyl-C₁-C₄-alkyl, Furyl, Furyl-C₁-C₄-alkyl, Thienyl, Thiazolyl, Piperidinyl, oder gegebenenfalls durch Fluor, Chlor und/oder Brom oder C₁-C₄-Alkyl substituiertes Phenyl,
- R¹⁷ und R¹⁸: auch gemeinsam für jeweils gegebenenfalls durch C₁-C₄-Alkyl, Phenyl, Furyl, einen annellierten Benzolring oder durch zwei Substituenten, die gemeinsam mit dem C-Atom, an das sie gebunden sind, einen 5- oder 6-gliedrigen Carbocyclus bilden, substituiertes C₃-C₆-Alkandiyl oder C₂-C₅-Oxaalkandiyl steht,
- R¹⁹: für Wasserstoff, Cyano, Halogen, oder für jeweils gegebenenfalls durch Fluor, Chlor und/oder Brom substituiertes C₁-C₄-Alkyl, C₃-C₆-Cycloalkyl oder Phenyl steht,
- R²⁰: für Wasserstoff, gegebenenfalls durch Hydroxy, Cyano, Halogen oder C₁-C₄-Alkoxy substituiertes C₁-C₆-Alkyl, C₃-C₆-Cycloalkyl oder Tri-(C₁-C₄-alkyl)-silyl steht,
- R²¹: für Wasserstoff, Cyano, Halogen, oder für jeweils gegebenenfalls durch Fluor, Chlor und/oder Brom substituiertes C₁-C₄-Alkyl, C₃-C₆-Cycloalkyl oder Phenyl steht,
- X¹: für Nitro, Cyano, Halogen, C₁-C₄-Alkyl, C₁-C₄-Halogenalkyl, C₁-C₄-Alkoxy oder C₁-C₄-Halogenalkoxy steht,
- X²: für Wasserstoff, Cyano, Nitro, Halogen, C₁-C₄-Alkyl, C₁-C₄-Halogenalkyl, C₁-C₄-Alkoxy oder C₁-C₄-Halogenalkoxy steht,
- X³: für Wasserstoff, Cyano, Nitro, Halogen, C₁-C₄-Alkyl, C₁-C₄-Halogenalkyl, C₁-C₄-Alkoxy oder C₁-C₄-Halogenalkoxy steht,
und/oder die folgenden durch allgemeine Formeln definierten Verbindungen
der allgemeinen Formel (IId) 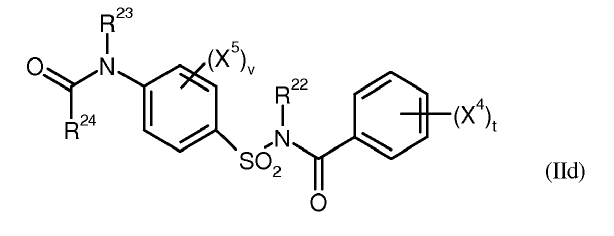 oder der allgemeinen Formel (IIe) 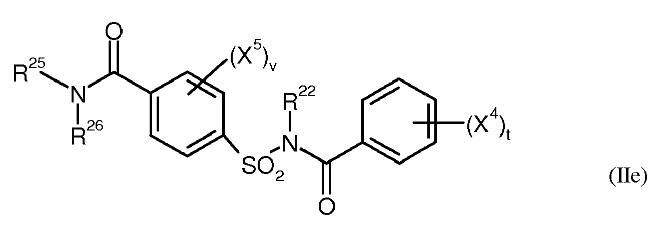 wobei
- t: für eine Zahl 0, 1, 2, 3, 4 oder 5 steht,
- v: für eine Zahl 0, 1, 2, 3, 4 oder 5 steht,
- R²²: für Wasserstoff oder C₁-C₄-Alkyl steht,
- R²³: für Wasserstoff oder C₁-C₄-Alkyl steht,
- R²⁴: für Wasserstoff, jeweils gegebenenfalls durch Cyano, Halogen oder C₁-C₄-Alkoxy substituiertes C₁-C₆-Alkyl, C₁-C₆-Alkoxy, C₁-C₆-Alkylthio, C₁-C₆-Alkylamino oder Di-(C₁-C₄-alkyl)-amino, oder jeweils gegebenenfalls durch Cyano, Halogen oder C₁-C₄-Alkyl substituiertes C₃-C₆-Cycloalkyl, C₃-C₆-Cycloalkyloxy, C₃-C₆-Cycloalkylthio oder C₃-C₆-Cycloalkylamino steht,
- R²⁵: für Wasserstoff, gegebenenfalls durch Cyano, Hydroxy, Halogen oder C₁-C₄-Alkoxy substituiertes C₁-C₆-Alkyl, jeweils gegebenenfalls durch Cyano oder Halogen substituiertes C₃-C₆-Alkenyl oder C₃-C₆-Alkinyl, oder gegebenenfalls durch Cyano, Halogen oder C₁-C₄-Alkyl substituiertes C₃-C₆-Cycloalkyl steht,
- R²⁶: für Wasserstoff, gegebenenfalls durch Cyano, Hydroxy, Halogen oder C₁-C₄-Alkoxy substituiertes C₁-C₆-Alkyl, jeweils gegebenenfalls durch Cyano oder Halogen substituiertes C₃-C₆-Alkenyl oder C₃-C₆-Alkinyl, gegebenenfalls durch Cyano, Halogen oder C₁-C₄-Alkyl substituiertes C₃-C₆-Cycloalkyl, oder gegebenenfalls durch Nitro, Cyano, Halogen, C₁-C₄-Alkyl, C₁-C₄-Halogenalkyl, C₁-C₄-Alkoxy oder C₁-C₄-Halogenalkoxy substituiertes Phenyl steht, oder zusammen mit R²⁵ für jeweils gegebenenfalls durch C₁-C₄-Alkyl substituiertes C₂-C₆-Alkandiyl oder C₂-C₅-Oxaalkandiyl steht,
- X⁴: für Nitro, Cyano, Carboxy, Carbamoyl, Formyl, Sulfamoyl, Hydroxy, Amino, Halogen, C₁-C₄-Alkyl, C₁-C₄-Halogenalkyl, C₁-C₄-Alkoxy oder C₁-C₄-Halogenalkoxy steht, und
- X⁵: für Nitro, Cyano, Carboxy, Carbamoyl, Formyl, Sulfamoyl, Hydroxy, Amino, Halogen, C₁-C₄-Alkyl, C₁-C₄-Halogenalkyl, C₁-C₄-Alkoxy oder C₁-C₄-Halogenalkoxy steht.

Die erfindungsgemäßen Verbindungen sind durch die Formel (I) allgemein definiert. Bevorzugte Substituenten bzw. Bereiche der in der oben und nachstehend erwähnten Formeln aufgeführten Reste werden im folgenden erläutert:
- J: steht bevorzugt für C₃-C₈-Cycloalkyl, welches gegebenenfalls durch Sauerstoff unterbrochen sein kann und welches gegebenenfalls durch Halogen, C₁-C₆-Alkyl, C₁-C₆-Alkoxy, C₁-C₆-Haloalkyl oder gegebenenfalls durch C₁-C₄-Alkyl, C₁-C₄-Alkoxy, Halogen, C₁-C₄-Halogenalkyl, C₁-C₄-Halogenalkoxy substituiertes Phenyl oder C₃-C₆-Cycloalkyl substituiert sein kann,
- X: steht bevorzugt für Wasserstoff, Halogen, C₁-C₆-Alkyl, C₁-C₄-Halogenalkyl, C₁-C₆-Alkoxy oder C₁-C₄-Halogenalkoxy,
- Y: steht bevorzugt für Wasserstoff, C₁-C₆-Alkyl, C₁-C₄-Halogenalkyl, Halogen, C₁-C₆-Alkoxy oder C₁-C₄-Halogenalkoxy,
- m: steht bevorzugt für eine Zahl 1, 2 oder 3
mit der Maßgabe, dass mindestens einer der Reste J, X oder Y in der 2-Position des Phenylrestes steht und dabei ungleich Wasserstoff ist,
- CKE: steht bevorzugt für eine der Gruppen 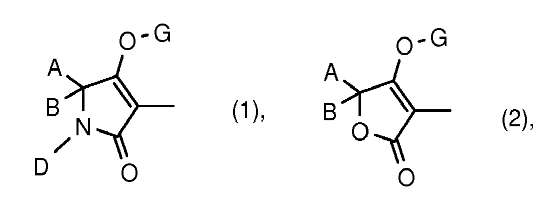 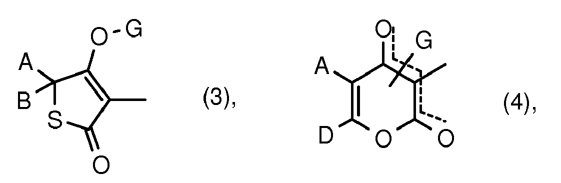 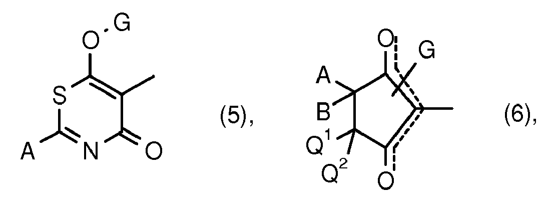 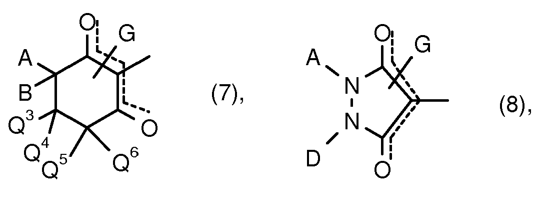 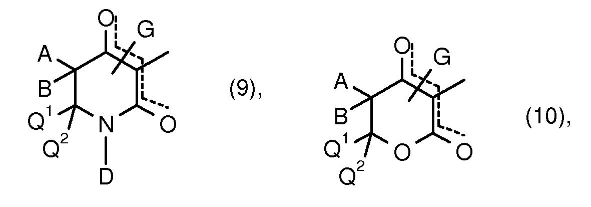
- A: steht bevorzugt für Wasserstoff oder jeweils gegebenenfalls durch Halogen substituiertes C₁-C₁₂-Alkyl, C₃-C₈-Alkenyl, C₁-C₁₀-Alkoxy-C₁-C₈-alkyl, C₁-C₁₀-Alkylthio-C₁-C₆-alkyl, gegebenenfalls durch Halogen, C₁-C₆-Alkyl oder C₁-C₆-Alkoxy substituiertes C₃-C₈-Cyloalkyl, in welchem gegebenenfalls ein oder zwei nicht direkt benachbarte Ringglieder durch Sauerstoff und/oder Schwefel ersetzt sind oder für jeweils gegebenenfalls durch Halogen, C₁-C₆-Alkyl, C₁-C₆-Halogenalkyl, C₁-C₆-Alkoxy, C₁-C₆-Halogen-alkoxy, Cyano oder Nitro substituiertes Phenyl, Naphthyl, Hetaryl mit 5 bis 6 Ringatomen (beispielsweise Furanyl, Pyridyl, Imidazolyl, Triazolyl, Pyrazolyl, Pyrimidyl, Thiazolyl oder Thienyl), Phenyl-C₁-C₆-alkyl oder Naphthyl-C₁-C₆-alkyl,
- B: steht bevorzugt für Wasserstoff, C₁-C₁₂-Alkyl oder C₁-C₈-Alkoxy-C₁-C₆-alkyl oder
- A, B: und das Kohlenstoffatom an das sie gebunden sind, stehen bevorzugt für gesättigtes C₃-C₁₀-Cyloalkyl oder ungesättigtes C₅-C₁₀-Cycloalkyl, worin gegebenenfalls ein Ringglied durch Sauerstoff oder Schwefel ersetzt ist und welche gegebenenfalls einfach oder zweifach durch C₁-C₈-Alkyl, C₃-C₁₀-Cycloalkyl, C₁-C₈-Halogenalkyl, C₁-C₈-Alkoxy, C₁-C₄-Alkoxy-C₁-C₄-alkoxy, C₁-C₈-Alkylthio, Halogen oder Phenyl substituiert sind oder
- A, B: und das Kohlenstoffatom, an das sie gebunden sind, stehen bevorzugt für C₃-C₆-Cyclo-alkyl, welches durch eine gegebenenfalls ein oder zwei nicht direkt benachbarte Sauerstoff- und/oder Schwefelatome enthaltende gegebenenfalls durch C₁-C₄-Alkyl substituierte Alkylendiyl-, oder durch eine Alkylendioxyl- oder durch eine Alkylendithioyl-Gruppe substituiert ist, die mit dem Kohlenstoffatom, an das sie gebunden ist, einen weiteren fünf- bis achtgliedrigen Ring bildet oder
- A, B: und das Kohlenstoffatom, an das sie gebunden sind, stehen bevorzugt für C₃-C₈-Cycloalkyl oder C₅-C₈-Cycloalkenyl, in welchen zwei Substituenten gemeinsam mit den Kohlenstoffatomen, an die sie gebunden sind, für jeweils gegebenenfalls durch C₁-C₆-Alkyl, C₁-C₆-Alkoxy oder Halogen substituiertes C₂-C₆-Alkandiyl, C₂-C₆-Alkendiyl oder C₄-C₆-Alkandiendiyl stehen, worin gegebenenfalls eine Methylengruppe durch Sauerstoff oder Schwefel ersetzt ist,
- D: steht bevorzugt für Wasserstoff, jeweils gegebenenfalls durch Halogen substituiertes C₁-C₁₂-Alkyl, C₃-C₈-Alkenyl, C₃-C₈-Alkinyl, C₁-C₁₀-Alkoxy-C₂-C₈-alkyl, gegebenenfalls durch Halogen, C₁-C₄-Alkyl, C₁-C₄-Alkoxy oder C₁-C₄-Halogenalkyl substituiertes C₃-C₈-Cyloalkyl, in welchem gegebenenfalls ein Ringglied durch Sauerstoff oder Schwefel ersetzt ist oder jeweils gegebenenfalls durch Halogen, C₁-C₆-Alkyl, C₁-C₆-Halogenalkyl, C₁-C₆-Alkoxy, C₁-C₆-Halogenalkoxy, Cyano oder Nitro substituiertes Phenyl, Hetaryl mit 5 oder 6 Ringatomen (beispielsweise Furanyl, Imidazolyl, Pyridyl, Thiazolyl, Pyrazolyl, Pyrimidyl, Pyrrolyl, Thienyl oder Triazolyl), Phenyl-C₁-C₆-alkyl oder Hetaryl-C₁-C₆-alkyl mit 5 oder 6 Ringatomen (beispielsweise Furanyl, Imidazolyl, Pyridyl, Thiazolyl, Pyrazolyl, Pyrimidyl, Pyrrolyl, Thienyl oder Triazolyl) oder
- A und D: stehen gemeinsam bevorzugt für jeweils gegebenenfalls substituiertes C₃-C₆-Alkandiyl oder C₃-C₆-Alkendiyl, worin gegebenenfalls eine Methylengruppe durch eine Carbonylgruppe, Sauerstoff oder Schwefel ersetzt ist und
wobei als Substituenten jeweils in Frage kommen:
Halogen, Hydroxy, Mercapto oder jeweils gegebenenfalls durch Halogen substituiertes C₁-C₁₀-Alkyl, C₁-C₆-Alkoxy, C₁-C₆-Alkylthio, C₃-C₇-Cycloalkyl, Phenyl oder Benzyloxy, oder eine weitere C₃-C₆-Alkandiylgruppierung, C₃-C₆-Alkendiylgruppierung oder eine Butadienylgruppierung, die gegebenenfalls durch C₁-C₆-Alkyl substituiert ist oder in der gegebenenfalls zwei benachbarte Substituenten mit den Kohlenstoffatomen, an die sie gebunden sind, einen weiteren gesättigten oder ungesättigten Cyclus mit 5 oder 6 Ringatomen bilden (im Fall der Verbindung der Formel (I-1) stehen A und D dann gemeinsam mit den Atomen, an die sie gebunden sind beispielsweise für die weiter unten genannten Gruppen AD-1 bis AD-10), der Sauerstoff oder Schwefel enthalten kann, oder worin gegebenenfalls eine der folgenden Gruppen 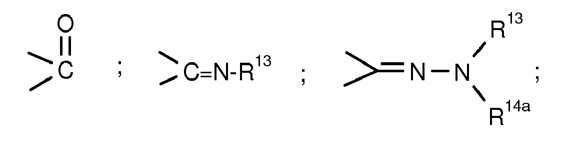 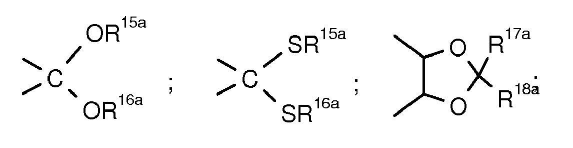 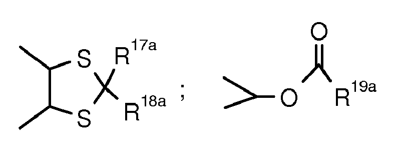 oder 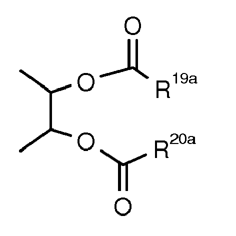 enthalten ist, oder
- A und Q¹: stehen gemeinsam bevorzugt für jeweils gegebenenfalls einfach oder zweifach, gleich oder verschieden durch Halogen, Hydroxy, durch jeweils gegebenenfalls einfach bis dreifach, gleich oder verschieden durch Halogen substituiertes C₁-C₁₀-Alkyl, C₁-C₆-Alkoxy, C₁-C₆-Alkylthio, C₃-C₇-Cycloalkyl oder durch jeweils gegebenenfalls einfach bis dreifach, gleich oder verschieden durch Halogen, C₁-C₆-Alkyl oder C₁-C₆-Alkoxy substituiertes Benzyloxy oder Phenyl substituiertes C₃-C₆-Alkandiyl oder C₄-C₆-Alkendiyl, welches außerdem gegebenenfalls eine der nachstehenden Gruppen 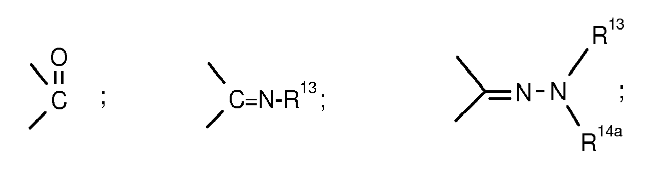 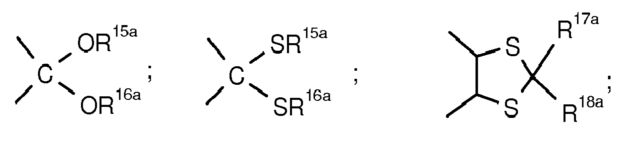 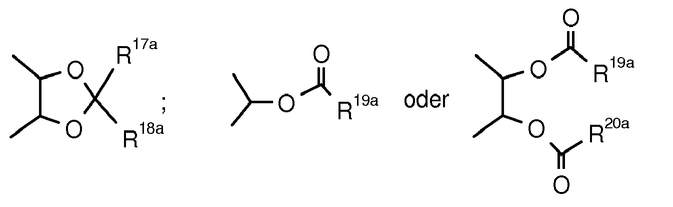 enthält oder durch eine C₁-C₂-Alkandiylgruppe oder durch ein Sauerstoffatom überbrückt ist oder
- D und Q¹: stehen gemeinsam bevorzugt für jeweils gegebenenfalls einfach oder zweifach, gleich oder verschieden durch C₁-C₄-Alkyl, C₁-C₄-Alkoxy substituiertes gegebenenfalls durch ein Sauerstoffatom unterbrochenes C₃-C₆-Alkandiyl oder
- Q¹: steht bevorzugt für Wasserstoff, C₁-C₆-Alkyl, C₁-C₆-Alkoxy-C₁-C₂-alkyl, gegebenenfalls durch Fluor, Chlor, C₁-C₄-Alkyl, C₁-C₂-Halogenalkyl oder C₁-C₄-Alkoxy substituiertes C₃-C₈-Cycloalkyl, worin gegebenenfalls eine Methylengruppe durch Sauerstoff oder Schwefel ersetzt ist oder gegebenenfalls durch Halogen, C₁-C₄-Alkyl, C₁-C₄-Alkoxy, C₁-C₂-Halogenalkyl, C₁-C₂-Halogenalkoxy, Cyano oder Nitro substituiertes Phenyl oder
- Q², Q⁴, Q⁵ und Q⁶: stehen unabhängig voneinander bevorzugt für Wasserstoff oder C₁-C₄-Alkyl,
- Q³: steht bevorzugt für Wasserstoff, C₁-C₆-Alkyl, C₁-C₆-Alkoxy-C₁-C₂-alkyl, C₁-C₆-Alkylthio-C₁-C₂-alkyl, gegebenenfalls durch C₁-C₄-Alkyl oder C₁-C₄-Alkoxy substituiertes C₃-C₈-Cycloalkyl, worin gegebenenfalls eine Methylengruppe durch Sauerstoff oder Schwefel ersetzt ist oder gegebenenfalls durch Halogen, C₁-C₄-Alkyl, C₁-C₄-Alkoxy, C₁-C₂-Halogenalkyl, C₁-C₂-Halogenalkoxy, Cyano oder Nitro substituiertes Phenyl,
- Q¹ und Q²: stehen bevorzugt gemeinsam mit dem Kohlenstoffatom, an das sie gebunden sind, für gegebenenfalls durch C₁-C₆-Alkyl, C₁-C₆-Alkoxy oder C₁-C₂-Halogenalkyl substituiertes C₃-C₇-Cycloalkyl, in welchem gegebenenfalls ein Ringglied durch Sauerstoff oder Schwefel ersetzt ist, oder
- Q³ und Q⁴: stehen bevorzugt gemeinsam mit dem Kohlenstoffatom, an das sie gebunden sind, für einen gegebenenfalls durch C₁-C₄-Alkyl, C₁-C₄-Alkoxy oder C₁-C₂-Halogenalkyl substituierten C₃-C₇-Ring, in welchem gegebenenfalls ein Ringglied durch Sauerstoff oder Schwefel ersetzt ist,
- G: steht bevorzugt für Wasserstoff (a) oder für eine der Gruppen 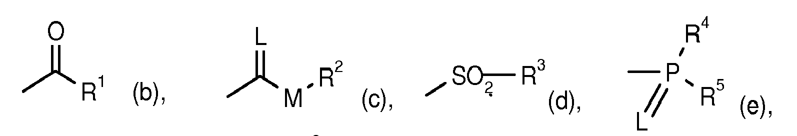 E (f) oder 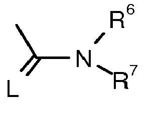 (g), insbesondere für (a), (b), (c) oder (g)
in welchen
E für ein Metallionäquivalent oder ein Ammoniumion steht,
L für Sauerstoff oder Schwefel steht und
M für Sauerstoff oder Schwefel steht,
- R¹: steht bevorzugt für jeweils gegebenenfalls durch Halogen substituiertes C₁-C₂₀-Alkyl, C₂-C₂₀-Alkenyl, C₁-C₈-Alkoxy-C₁-C₈-alkyl, C₁-C₈-Alkylthio-C₁-C₈-alkyl, Poly-C₁-C₈-alkoxy-C₁-C₈-alkyl oder gegebenenfalls durch Halogen, C₁-C₆-Alkyl oder C₁-C₆-Alkoxy substituiertes C₃-C₈-Cycloalkyl, in welchem gegebenenfalls ein oder mehrere (bevorzugt nicht mehr als zwei) nicht direkt benachbarte Ringglieder durch Sauerstoff und/oder Schwefel ersetzt sind,
für gegebenenfalls durch Halogen, Cyano, Nitro, C₁-C₆-Alkyl, C₁-C₆-Alkoxy, C₁-C₆-Halogenalkyl, C₁-C₆-Halogenalkoxy, C₁-C₆-Alkylthio oder C₁-C₆-Alkylsulfonyl substituiertes Phenyl,
für gegebenenfalls durch Halogen, Nitro, Cyano, C₁-C₆-Alkyl, C₁-C₆-Alkoxy, C₁-C₆-Halogenalkyl oder C₁-C₆-Halogenalkoxy substituiertes Phenyl-C₁-C₆-alkyl,
für gegebenenfalls durch Halogen oder C₁-C₆-Alkyl substituiertes 5- oder 6-gliedriges Hetaryl (beispielsweise Pyrazolyl, Thiazolyl, Pyridyl, Pyrimidyl, Furanyl oder Thienyl),
für gegebenenfalls durch Halogen oder C₁-C₆-Alkyl substituiertes Phenoxy-C₁-C₆-alkyl oder
für gegebenenfalls durch Halogen, Amino oder C₁-C₆-Alkyl substituiertes 5- oder 6-gliedriges Hetaryloxy-C₁-C₆-alkyl (beispielsweise Pyridyloxy-C₁-C₆-alkyl, Pyrimidyloxy-C₁-C₆-alkyl oder Thiazolyloxy-C₁-C₆-alkyl),
- R²: steht bevorzugt für jeweils gegebenenfalls durch Halogen substituiertes C₁-C₂₀-Alkyl, C₂-C₂₀-Alkenyl, C₁-C₈-Alkoxy-C₂-C₈-alkyl, Poly-C₁-C₈-alkoxy-C₂-C₈-alkyl,
für gegebenenfalls durch Halogen, C₁-C₆-Alkyl oder C₁-C₆-Alkoxy substituiertes C₃-C₈-Cycloalkyl oder
für jeweils gegebenenfalls durch Halogen, Cyano, Nitro, C₁-C₆-Alkyl, C₁-C₆-Alkoxy, C₁-C₆-Halogenalkyl oder C₁-C₆-Halogenalkoxy substituiertes Phenyl oder Benzyl,
- R³: steht bevorzugt für gegebenenfalls durch Halogen substituiertes C₁-C₈-Alkyl oder für jeweils gegebenenfalls durch Halogen, C₁-C₆-Alkyl, C₁-C₆-Alkoxy, C₁-C₄-Halogenalkyl, C₁-C₄-Halogenalkoxy, Cyano oder Nitro substituiertes Phenyl oder Benzyl,
- R⁴ und R⁵: stehen bevorzugt unabhängig voneinander für jeweils gegebenenfalls durch Halogen substituiertes C₁-C₈-Alkyl, C₁-C₈-Alkoxy, C₁-C₈-Alkylanüno, Di-(C₁-C₈-alkyl)anüno, C₁-C₈-Alkylthio, C₂-C₈-Alkenylthio, C₃-C₇-Cycloalkylthio oder für jeweils gegebenenfalls durch Halogen, Nitro, Cyano, C₁-C₄-Alkoxy, C₁-C₄-Halogenalkoxy, C₁-C₄-Alkylthio, C₁-C₄-Halogenalkylthio, C₁-C₄-Alkyl oder C₁-C₄-Halogenalkyl substituiertes Phenyl, Phenoxy oder Phenylthio,
- R⁶ und R⁷: stehen unabhängig voneinander bevorzugt für Wasserstoff, für jeweils gegebenenfalls durch Halogen substituiertes C₁-C₈-Alkyl, C₃-C₈-Cycloalkyl, C₁-C₈-Alkoxy, C₃-C₈- Alkenyl, C₁-C₈-Alkoxy-C₁-C₈-alkyl, für gegebenenfalls durch Halogen, C₁-C₈-Halogenalkyl, C₁-C₈-Alkyl oder C₁-C₈-Alkoxy substituiertes Phenyl, gegebenenfalls durch Halogen, C₁-C₈-Alkyl, C₁-C₈-Halogenalkyl oder C₁-C₈-Alkoxy substituiertes Benzyl oder zusammen für einen gegebenenfalls durch C₁-C₄-Alkyl substituierten C₃-C₆-Alkylenrest, in welchem gegebenenfalls ein Kohlenstoffatom durch Sauerstoff oder Schwefel ersetzt ist,
- R¹³: steht bevorzugt für Wasserstoff, für jeweils gegebenenfalls durch Halogen substituiertes C₁-C₈-Alkyl oder C₁-C₈-Alkoxy, für gegebenenfalls durch Halogen, C₁-C₄-Alkyl oder C₁-C₄-Alkoxy substituiertes C₃-C₈-Cycloalkyl, in welchem gegebenenfalls eine Methylengruppe durch Sauerstoff oder Schwefel ersetzt ist, oder für jeweils gegebenenfalls durch Halogen, C₁-C₆-Alkyl, C₁-C₆-Alkoxy, C₁-C₄-Halogenalkyl, C₁-C₄-Halogenalkoxy, Nitro oder Cyano substituiertes Phenyl, Phenyl-C₁-C₄-alkyl oder Phenyl-C₁-C₄-alkoxy,
- R^{14a}: steht bevorzugt für Wasserstoff oder C₁-C₈-Alkyl oder
- R¹³ und R^{14a}: stehen gemeinsam bevorzugt für C₄-C₆-Alkandiyl,
- R^{15a} und R^{16a}: sind gleich oder verschieden und stehen bevorzugt für C₁-C₆-Alkyl oder
- R^{15a} und R^{16a}: stehen gemeinsam bevorzugt für einen C₂-C₄-Alkandiylrest, der gegebenenfalls durch C₁-C₆-Alkyl, C₁-C₆-Halogenalkyl oder durch gegebenenfalls durch Halogen, C₁-C₆-Alkyl, C₁-C₄-Halogenalkyl, C₁-C₆-Alkoxy, C₁-C₄-Halogenalkoxy, Nitro oder Cyano substituiertes Phenyl substituiert ist,
- R^{17a} und R^{18a}: stehen unabhängig voneinander bevorzugt für Wasserstoff, für gegebenenfalls durch Halogen substituiertes C₁-C₈-Alkyl oder für gegebenenfalls durch Halogen, C₁-C₆-Alkyl, C₁-C₆-Alkoxy, C₁-C₄-Halogenalkyl, C₁-C₄-Halogenalkoxy, Nitro oder Cyano substituiertes Phenyl oder
- R^{17a} und R^{18a}: stehen gemeinsam mit dem Kohlenstoffatom, an das sie gebunden sind, bevorzugt für eine Carbonylgruppe oder für gegebenenfalls durch Halogen, C₁-C₄-Alkyl oder C₁-C₄-Alkoxy substituiertes C₅-C₇-Cycloalkyl, in dem gegebenenfalls eine Methylengruppe durch Sauerstoff oder Schwefel ersetzt ist,
- R^{19a} und R^{20a}: stehen unabhängig voneinander bevorzugt für C₁-C₁₀-Alkyl, C₂-C₁₀-Alkenyl, C₁-C₁₀-Alkoxy, C₁-C₁₀-Alkylamino, C₃-C₁₀-Alkenylamino, Di-(C₁-C₁₀-alkyl)amino oder Di-(C₃-C₁₀-alkenyl)amino.

In den als bevorzugt genannten Restedefinitionen steht Halogen für Fluor, Chlor, Brom und Jod, insbesondere für Fluor, Chlor und Brom.
- J: steht besonders bevorzugt für gegebenenfalls durch ein Sauerstoffatom unterbrochenes, gegebenenfalls einfach bis zweifach durch Fluor, Chlor, C₁-C₄-Alkyl, C₃-C₆-Cycloalkyl, C₁-C₄-Alkoxy oder C₁-C₂-Halogenalkyl substituiertes C₃-C₆-Cycloalkyl,
- X: steht besonders bevorzugt für Wasserstoff, Fluor, Chlor, Brom, Iod, C₁-C₄-Alkyl, Trifluormethyl, C₁-C₄-Alkoxy, Difluormethoxy oder Trifluormethoxy,
- Y: steht besonders bevorzugt für Wasserstoff, Fluor, Chlor, Brom, Iod, C₁-C₄-Alkyl, Trifluormethyl, C₁-C₄-Alkoxy, Difluormethoxy oder Trifluormethoxy,
- m: steht besonders bevorzugt für eine Zahl 1 oder 2
mit der Maßgabe, dass mindestens einer der Reste J, X oder Y in der 2-Position des Phenylrestes steht und dabei ungleich Wasserstoff ist,
- CKE: steht besonders bevorzugt für eine der Gruppen 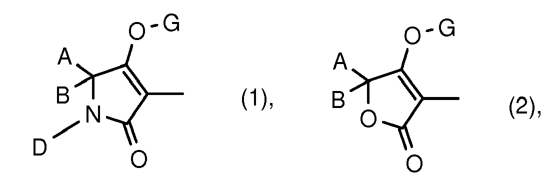 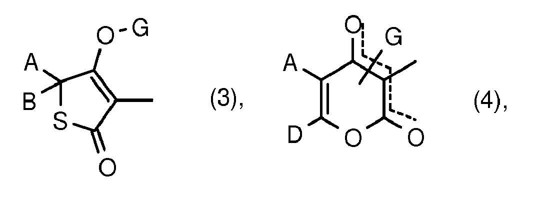 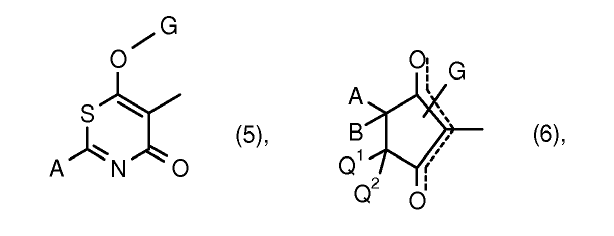 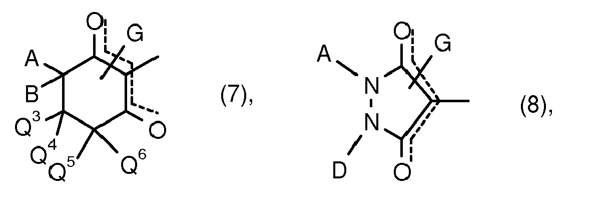 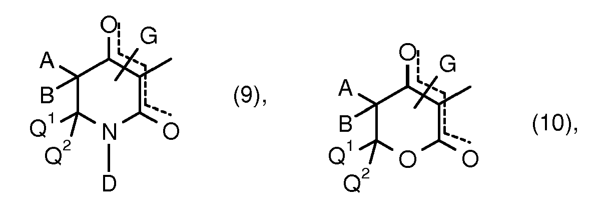
- A: steht besonders bevorzugt für Wasserstoff, jeweils gegebenenfalls einfach bis dreifach durch Fluor oder Chlor substituiertes C₁-C₆-Alkyl, C₁-C₄-Alkoxy-C₁-C₂-alkyl, gegebenenfalls einfach bis zweifach durch C₁-C₂-Alkyl oder C₁-C₂-Alkoxy substituiertes C₃-C₆-Cycloalkyl oder (jedoch nicht im Fall der Verbindungen der Formeln (I-3), (I-4), (I-6) und (I-7)) jeweils gegebenenfalls einfach bis zweifach durch Fluor, Chlor, Brom, C₁-C₄-Alkyl, C₁-C₂-Halogenalkyl, C₁-C₄-Alkoxy, C₁-C₂-Halogenalkoxy, Cyano oder Nitro substituiertes Phenyl oder Benzyl,
- B: steht besonders bevorzugt für Wasserstoff, C₁-C₄-Alkyl oder C₁-C₂-Alkoxy-C₁-C₂-alkyl oder
- A, B: und das Kohlenstoffatom an das sie gebunden sind, stehen besonders bevorzugt für gesättigtes oder ungesättigtes C₅-C₇-Cycloalkyl, worin gegebenenfalls ein Ringglied durch Sauerstoff oder Schwefel ersetzt ist und welches gegebenenfalls einfach bis zweifach durch C₁-C₆-Alkyl, Trifluormethyl, C₁-C₆-Alkoxy oder C₁-C₃-Alkoxy-C₁-C₃-alkoxy substituiert ist mit der Maßgabe, dass dann Q³ besonders bevorzugt für Wasserstoff oder Methyl steht oder
- A, B: und das Kohlenstoffatom, an das sie gebunden sind, stehen besonders bevorzugt für C₅-C₆-Cycloalkyl, welches durch eine gegebenenfalls ein oder zwei nicht direkt benachbarte Sauerstoff- oder Schwefelatome enthaltende gegebenenfalls durch Methyl oder Ethyl substituierte Alkylendiyl- oder durch eine Alkylendioxyl- oder durch eine Alkylendithiol-Gruppe substituiert ist, die mit dem Kohlenstoffatom, an das sie gebunden ist, einen weiteren fünf- oder sechsgliedrigen Ring bildet mit der Maßgabe, dass dann Q³ besonders bevorzugt für Wasserstoff oder Methyl steht, oder
- A, B: und das Kohlenstoffatom, an das sie gebunden sind, stehen besonders bevorzugt für C₃-C₆-Cycloalkyl oder C₅-C₆-Cycloalkenyl, in welchen zwei Substituenten gemeinsam mit den Kohlenstoffatomen, an die sie gebunden sind, für jeweils gegebenenfalls durch C₁-C₂-Alkyl oder C₁-C₂-Alkoxy substituiertes C₂-C₄-Alkandiyl, C₂-C₄-Alkendiyl oder Butadiendiyl stehen, mit der Maßgabe, dass dann Q³ besonders bevorzugt für Wasserstoff oder Methyl steht,
- D: steht besonders bevorzugt für Wasserstoff, für jeweils gegebenenfalls einfach bis dreifach durch Fluor substituiertes C₁-C₆-Alkyl, C₃-C₆-Alkenyl, C₁-C₄-Alkoxy-C₂-C₃-alkyl, für gegebenenfalls einfach bis zweifach durch C₁-C₄-Alkyl, C₁-C₄-Alkoxy oder C₁-C₂-Halogenalkyl substituiertes C₃-C₆-Cycloalkyl, in welchem gegebenenfalls eine Methylengruppe durch Sauerstoff ersetzt ist oder (jedoch nicht im Fall der Verbindungen der Formeln (I-1)) für jeweils gegebenenfalls einfach bis zweifach durch Fluor, Chlor, Brom, C₁-C₄-Alkyl, C₁-C₄-Halogenalkyl, C₁-C₄-Alkoxy oder C₁-C₄-Halogenalkoxy substituiertes Phenyl oder Pyridyl, oder
- A und D: stehen gemeinsam besonders bevorzugt für gegebenenfalls einfach bis zweifach substituiertes C₃-C₅-Alkandiyl, in welchem eine Methylengruppe durch eine Carbonylgruppe (nicht jedoch im Fall der Verbindungen der Formel (I-1)), Sauerstoff oder Schwefel ersetzt sein kann, wobei als Substituenten C₁-C₂-Alkyl oder C₁-C₂-Alkoxy in Frage kommen oder
- A und D: stehen (im Fall der Verbindungen der Formel (I-1)) gemeinsam mit den Atomen, an die sie gebunden sind, für eine der Gruppen AD-1 bis AD-10: 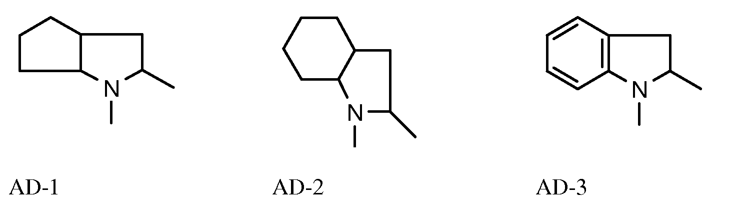 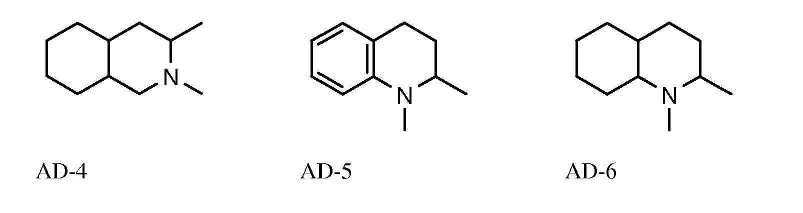 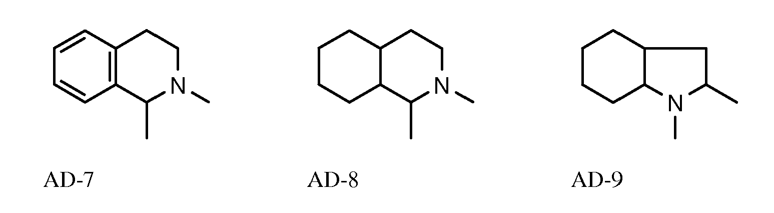 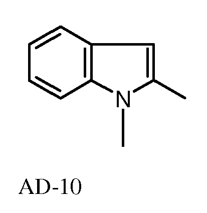 oder
- A und Q¹: stehen gemeinsam besonders bevorzugt für jeweils gegebenenfalls einfach oder zweifach, gleich oder verschieden durch C₁-C₂-Alkyl oder C₁-C₂-Alkoxy substituiertes C₃-C₄-Alkandiyl oder
- D und Q¹: stehen gemeinsam besonders bevorzugt für gegebenenfalls durch ein Sauerstoffatom unterbrochenes C₃-C₄-Alkandiyl, oder
- Q¹: steht besonders bevorzugt für Wasserstoff, C₁-C₄-Alkyl, C₁-C₄-Alkoxy-C₁-C₂-alkyl, oder gegebenenfalls durch Methyl oder Methoxy substituiertes C₃-C₆-Cycloalkyl, worin gegebenenfalls eine Methylengruppe durch Sauerstoff ersetzt ist,
- Q²: steht besonders bevorzugt für Wasserstoff, Methyl oder Ethyl,
- Q⁴ Q⁵ und Q⁶: stehen besonders bevorzugt unabhängig voneinander für Wasserstoff oder C₁-C₃-Alkyl,
- Q³: steht besonders bevorzugt für Wasserstoff, C₁-C₄-Alkyl, oder gegebenenfalls einfach bis zweifach durch Methyl oder Methoxy substituiertes C₃-C₆-Cycloalkyl,
- Q¹ und Q²: stehen besonders bevorzugt gemeinsam mit dem Kohlenstoff, an das sie gebunden sind, für gegebenenfalls durch C₁-C₄-Alkyl oder C₁-C₄-Alkoxy substituiertes gesättigtes C₅-C₆-Cycloalkyl, in welchem gegebenenfalls ein Ringglied durch Sauerstoff ersetzt ist, oder
- Q³ und Q⁴: stehen besonders bevorzugt gemeinsam mit dem Kohlenstoff, an das sie gebunden sind, für einen gegebenenfalls durch C₁-C₂-Alkyl oder C₁-C₂-Alkoxy substituierten gesättigten C₅-C₆-Ring, in welchem gegebenenfalls ein Ringglied durch Sauerstoff oder Schwefel ersetzt ist, mit der Maßgabe, dass dann A besonders bevorzugt für Wasserstoff oder Methyl steht,
- G: steht besonders bevorzugt für Wasserstoff (a) oder für eine der Gruppen 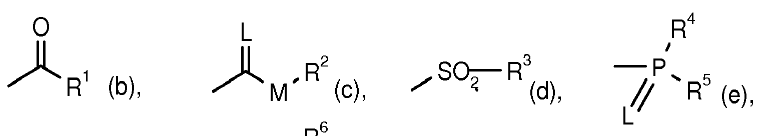 E (f) oder 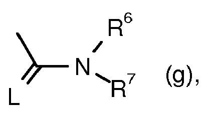 insbesondere für (a), (b) oder (c),
in welchen
E für ein Metallionäquivalent oder ein Ammoniumion steht,
L für Sauerstoff oder Schwefel steht und
M für Sauerstoff oder Schwefel steht,
- R¹: steht besonders bevorzugt für jeweils gegebenenfalls einfach bis dreifach durch Fluor oder Chlor substituiertes C₁-C₈-Alkyl, C₂-C₁₈-Alkenyl, C₁-C₄-Alkoxy-C₁-C₂-alkyl, C₁-C₄-Alkylthio-C₁-C₂-alkyl oder gegebenenfalls einfach bis zweifach durch Fluor, Chlor, C₁-C₂-Alkyl oder C₁-C₂-Alkoxy substituiertes C₃-C₆-Cycloalkyl, in welchem gegebenenfalls ein oder zwei nicht direkt benachbarte Ringglieder durch Sauerstoff ersetzt sind,
für gegebenenfalls einfach bis zweifach durch Fluor, Chlor, Brom, Cyano, Nitro, C₁-C₄-Alkyl, C₁-C₄-Alkoxy, C₁-C₂-Halogenalkyl oder C₁-C₂-Halogenalkoxy substituiertes Phenyl,
- R²: steht besonders bevorzugt für jeweils gegebenenfalls einfach bis dreifach durch Fluor substituiertes C₁-C₈-Alkyl, C₂-C₈-Alkenyl oder C₁-C₄-Alkoxy-C₂-C₄-alkyl,
für gegebenenfalls einfach durch C₁-C₂-Alkyl oder C₁-C₂-Alkoxy substituiertes C₃-C₆-Cycloalkyl oder
für jeweils gegebenenfalls einfach bis zweifach durch Fluor, Chlor, Brom, Cyano, Nitro, C₁-C₄-Alkyl, C₁-C₃-Alkoxy, Trifluormethyl oder Trifluormethoxy substituiertes Phenyl oder Benzyl,
- R³: steht besonders bevorzugt für gegebenenfalls einfach bis dreifach durch Fluor substituiertes C₁-C₆-Alkyl oder für gegebenenfalls einfach durch Fluor, Chlor, Brom, C₁-C₄- Alkyl, C₁-C₄-Alkoxy, Trifluormethyl, Trifluormethoxy, Cyano oder Nitro substituiertes Phenyl,
- R⁴: steht besonders bevorzugt für C₁-C₆-Alkyl, C₁-C₆-Alkoxy, C₁-C₆-Alkylamino, Di-(C₁-C₆-alkyl)amino, C₁-C₆-Alkylthio, C₃-C₄-Alkenylthio, C₃-C₆-Cycloalkylthio oder für jeweils gegebenenfalls einfach durch Fluor, Chlor, Brom, Nitro, Cyano, C₁-C₃-Alkoxy, C₁-C₃-Halogenalkoxy, C₁-C₃-Alkylthio, C₁-C₃-Halogenalkylthio, C₁-C₃-Alkyl oder Trifluormethyl substituiertes Phenyl, Phenoxy oder Phenylthio,
- R⁵: steht besonders bevorzugt für C₁-C₆-Alkoxy oder C₁-C₆-Alkylthio,
- R⁶: steht besonders bevorzugt für Wasserstoff, C₁-C₆-Alkyl, C₃-C₆-Cycloalkyl, C₁-C₆-Alkoxy, C₃-C₆-Alkenyl, C₁-C₆-Alkoxy-C₁-C₄-alkyl, für gegebenenfalls einfach durch Fluor, Chlor, Brom, Trifluormethyl, C₁-C₄-Alkyl oder C₁-C₄-Alkoxy substituiertes Phenyl, für gegebenenfalls einfach durch Fluor, Chlor, Brom, C₁-C₄-Alkyl, Trifluormethyl oder C₁-C₄-Alkoxy substituiertes Benzyl,
- R⁷: steht besonders bevorzugt für C₁-C₆-Alkyl, C₃-C₆-Alkenyl oder C₁-C₆-Alkoxy-C₁-C₄-alkyl,
- R⁶ und R⁷: stehen besonders bevorzugt zusammen für einen gegebenenfalls durch Methyl oder Ethyl substituierten C₄-C₅-Alkylenrest, in welchem gegebenenfalls eine Methylengruppe durch Sauerstoff oder Schwefel ersetzt ist.

In den als besonders bevorzugt genannten Restedefinitionen steht Halogen für Fluor, Chlor und Brom, insbesondere für Fluor und Chlor.
- J: steht ganz besonders bevorzugt für Cyclopropyl, Dicyclopropyl, Cyclobutyl, Cyclopentyl, Cyclohexyl, Oxetanyl, Tetrahydrofurfuryl, Tetrahydropyranyl,
- X: steht ganz besonders bevorzugt für Wasserstoff, Fluor, Chlor, Brom, Methyl, Ethyl, Propyl, Methoxy oder Ethoxy,
- Y: steht ganz besonders bevorzugt für Wasserstoff, Chlor, Brom, Methyl, Ethyl, Propyl, Trifluormethyl, Methoxy, Ethoxy oder Trifluormethoxy,
- m: steht ganz besonders bevorzugt für die Zahl 1 oder 2
mit der Maßgabe, dass mindestens einer der Reste J, X oder Y in der 2-Position des Phenylrestes steht und ungleich Wasserstoff ist.

Ganz besonders bevorzugt stehen dabei die Reste J, X und Y mit ihren ganz besonders bevorzugten Bedeutungen in folgenden Phenylsubstitutionsmustern 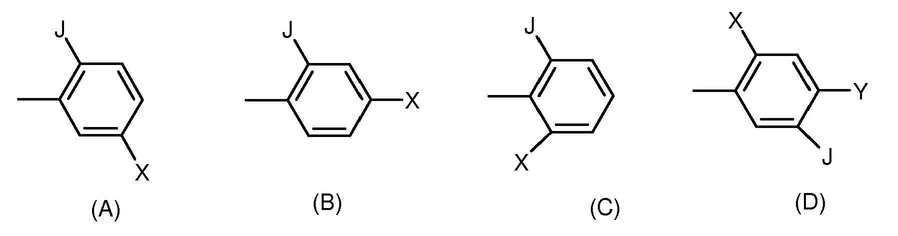 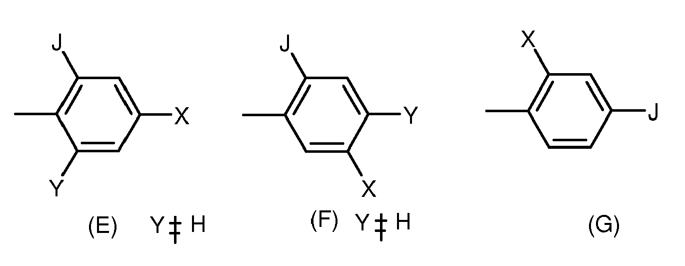 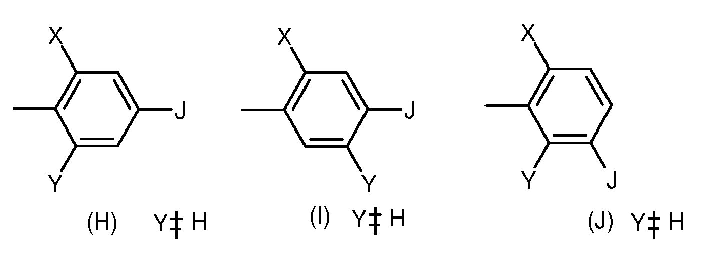 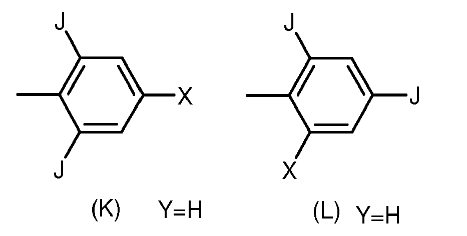 wobei nur im Phenylsubstitutionsmuster (B), (K) und (L) X auch für Wasserstoff stehen darf,
- CKE: steht ganz besonders bevorzugt für eine der Gruppen 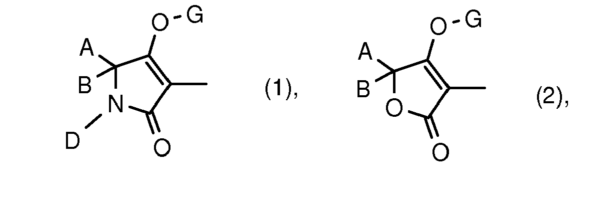 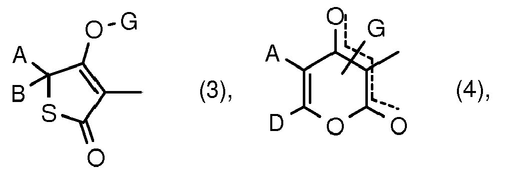 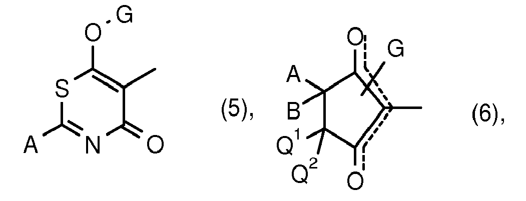 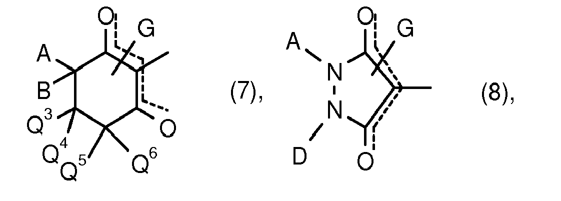 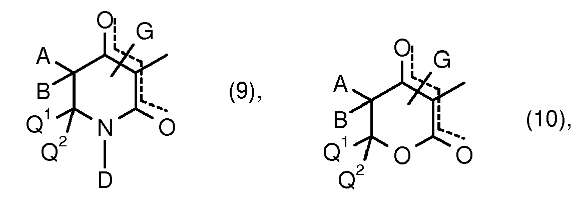
- A: steht ganz besonders bevorzugt für Wasserstoff, jeweils gegebenenfalls einfach bis dreifach durch Fluor substituiertes C₁-C₄-Alkyl oder C₁-C₂-Alkoxy-C₁-C₂-alkyl, für Cyclopropyl, Cyclopentyl oder Cyclohexyl und nur im Fall der Verbindungen der Formel (I-5) für gegebenenfalls einfach bis zweifach durch Fluor, Chlor, Brom, Methyl, Ethyl, n-Propyl, iso-Propyl, Methoxy, Ethoxy, Trifluormethyl, Trifluormethoxy, Cyano oder Nitro substituiertes Phenyl,
- B: steht ganz besonders bevorzugt für Wasserstoff, Methyl oder Ethyl oder
- A, B: und das Kohlenstoffatom an das sie gebunden sind, stehen ganz besonders bevorzugt für gesättigtes C₅-C₆-Cycloalkyl, in welchem gegebenenfalls ein Ringglied durch Sauerstoff oder Schwefel ersetzt ist und welches gegebenenfalls einfach durch Methyl, Ethyl, Propyl, Isopropyl, Trifluormethyl, Methoxy, Ethoxy, Propoxy, Methoxyethoxy, Butoxy, Methoxymethyl oder Ethoxyethoxy substituiert ist, mit der Maßgabe, dass dann Q³ ganz besonders bevorzugt für Wasserstoff steht oder
- A, B: und das Kohlenstoffatom, an das sie gebunden sind, stehen ganz besonders bevorzugt für C₆-Cycloalkyl, welches gegebenenfalls durch mit zwei nicht direkt benachbarten Sauerstoffatomen enthaltende Alkylendioxyl-Gruppe substituiert ist, mit der Maßgabe, dass dann Q³ ganz besonders bevorzugt für Wasserstoff steht oder
- A, B: und das Kohlenstoffatom, an das sie gebunden sind, stehen ganz besonders bevorzugt für C₅-C₆-Cycloalkyl oder C₅-C₆-Cycloalkenyl, worin zwei Substituenten gemeinsam mit den Kohlenstoffatomen, an die sie gebunden sind, für C₂-C₄-Alkandiyl oder C₂-C₄-Alkendiyl oder Butadiendiyl stehen, mit der Maßgabe, dass dann Q³ ganz besonders bevorzugt für Wasserstoff steht,
- D: steht ganz besonders bevorzugt für Wasserstoff, für jeweils gegebenenfalls einfach bis dreifach durch Fluor substituiertes C₁-C₄-Alkyl, C₃-C₄-Alkenyl, C₁-C₄-Alkoxy-C₂-C₃-alkyl, für Cyclopropyl, Cyclopentyl oder Cyclohexyl oder (jedoch nicht im Fall der Verbindungen der Formeln (I-1)) für jeweils gegebenenfalls einfach durch Fluor, Chlor, Methyl, Ethyl, n-Propyl, iso-Propyl, Methoxy, Ethoxy oder Trifluormethyl substituiertes Phenyl oder Pyridyl,
oder
- A und D: stehen gemeinsam ganz besonders bevorzugt für gegebenenfalls einfach durch Methyl oder Methoxy substituiertes C₃-C₅-Alkandiyl, worin gegebenenfalls ein Kohlenstoffatom durch Sauerstoff oder Schwefel ersetzt ist oder für die Gruppe AD-1,
- A und Q¹: stehen gemeinsam ganz besonders bevorzugt für gegebenenfalls einfach oder zweifach durch Methyl oder Methoxy substituiertes C₃-C₄-Alkandiyl oder
- D und Q¹: stehen gemeinsam ganz besonders bevorzugt für C₃-C₄-Alkandiyl,
- Q¹: steht ganz besonders bevorzugt für Wasserstoff, Methyl, Ethyl, Propyl, iso-Propyl, Cyclopropyl, Cyclopentyl oder Cyclohexyl,
- Q²: steht ganz besonders bevorzugt für Wasserstoff oder Methyl,
- Q⁴ Q⁵ und Q⁶: stehen ganz besonders bevorzugt unabhängig voneinander für Wasserstoff oder Methyl,
- Q³: steht ganz besonders bevorzugt für Wasserstoff, Methyl, Ethyl oder Propyl, oder
- Q¹ und Q²: stehen ganz besonders bevorzugt gemeinsam mit dem Kohlenstoff, an den sie gebunden sind, für gegebenenfalls durch Methyl, Ethyl, Propyl, iso-Propyl, Methoxy, Ethoxy, Propoxy oder Butoxy substituiertes gesättigtes C₅-C₆-Cycloalkyl, in welchem gegebenenfalls ein Ringglied durch Sauerstoff ersetzt ist, oder
- Q³ und Q⁴: stehen ganz besonders bevorzugt gemeinsam mit dem Kohlenstoff, an den sie gebunden sind, für einen gegebenenfalls einfach durch Methyl oder Methoxy substituierten gesättigten C₅-C₆-Ring, mit der Maßgabe, dass dann A ganz besonders bevorzugt für Wasserstoff steht,
- G: steht ganz besonders bevorzugt für Wasserstoff (a) oder für eine der Gruppen 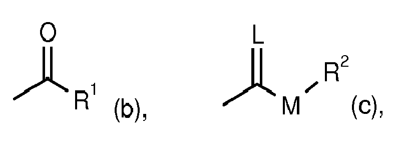 -SO₂-R³ (d) oder E (f),
in welchen
L für Sauerstoff oder Schwefel steht,
M für Sauerstoff oder Schwefel steht und
E für ein Ammoniumion steht,
- R¹: steht ganz besonders bevorzugt für jeweils gegebenenfalls einfach durch Chlor substituiertes C₁-C₆-Alkyl, C₂-C₁₇-Alkenyl, C₁-C₂-Alkoxy-C₁-alkyl, C₁-C₂-Alkylthio-C₁-alkyl oder jeweils gegebenenfalls einfach durch Fluor, Chlor, Methyl oder Methoxy substituiertes Cyclopropyl oder Cyclohexyl,
für gegebenenfalls einfach durch Fluor, Chlor, Brom, Cyano, Nitro, Methyl, Methoxy, Trifluormethyl oder Trifluormethoxy substituiertes Phenyl,
- R²: steht ganz besonders bevorzugt für jeweils gegebenenfalls einfach durch Fluor substituiertes C₁-C₈-Alkyl, C₂-C₆-Alkenyl oder C₁-C₄-Alkoxy-C₂-C₃-alkyl, Phenyl oder Benzyl,
- R³: steht ganz besonders bevorzugt für C₁-C₈-Alkyl.
- J: steht hervorgehoben für Cyclopropyl,
- X: steht hervorgehoben für Chlor, Methyl oder Ethyl,
- Y: steht hervorgehoben für Chlor, Methyl, Ethyl oder Wasserstoff,
- m: steht hervorgehoben für die Zahl 1 oder 2,
mit der Maßgabe, dass mindestens einer der Reste J, X oder Y in der 2-Position des Phenylrestes steht und ungleich Wasserstoff ist.

Insbesondere bevorzugt stehen dabei die Reste J, X und Y mit ihren hervorgehobenen Bedeutungen in folgenden Phenylsubstitutionsmustern 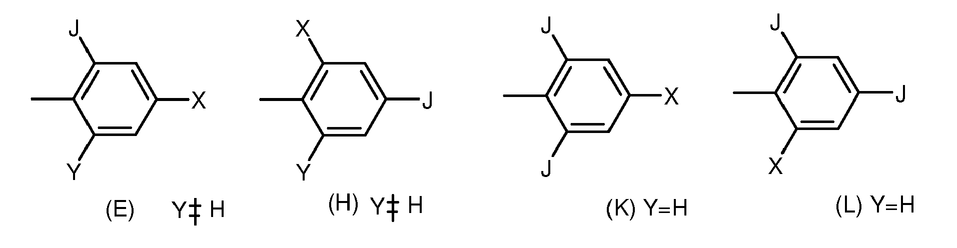
- CKE: steht hervorgehoben für eine der Gruppen 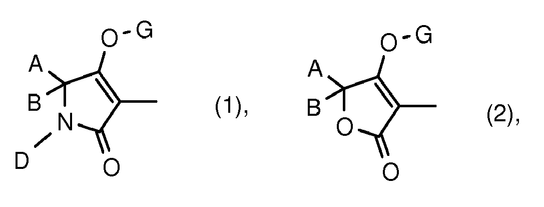 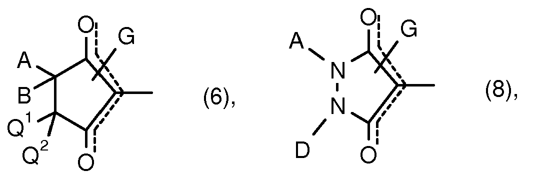
- A: steht hervorgehoben für C₁-C₄-Alkyl oder Cyclopropyl,
- B: steht hervorgehoben für Wasserstoff oder Methyl oder
- A, B: und das Kohlenstoffatom, an das sie gebunden sind, stehen hervorgehoben für gesättigtes C₅-C₆-Cycloalkyl, in welchem gegebenenfalls ein Ringglied durch Sauerstoff ersetzt ist und welches gegebenenfalls einfach durch Methoxy, Ethoxy, Butoxy oder Methoxymethyl substituiert ist,
- A, B: und das Kohlenstoffatom, an das sie gebunden sind, stehen hervorgehoben für C₆-Cycloalkyl, welches gegebenenfalls durch mit zwei nicht direkt benachbarten Sauerstoffatomen enthaltende C₂-C₃-Alkylendioxyl-Gruppe substituiert ist,
- D: steht hervorgehoben für Wasserstoff oder
- A und D: stehen gemeinsam hervorgehoben für C₃-C₅-Alkandiyl,
- A und Q¹: stehen gemeinsam hervorgehoben für C₃-C₄-Alkandiyl,
- Q²: steht hervorgehoben für Wasserstoff,
- G: steht hervorgehoben für Wasserstoff (a) oder für eine der Gruppen 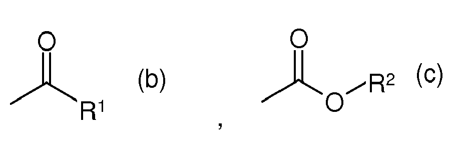 oder SO₂-R³(d),
- R¹: steht hervorgehoben für C₁-C₆-Alkyl oder einfach durch Chlor substituiertes Phenyl,
- R²: steht hervorgehoben für C₁-C₈-Alkyl,
- R³: steht hervorgehoben für C₁-C₈-Alkyl.

Die oben aufgeführten allgemeinen oder in Vorzugsbereichen aufgeführten Restedefinitionen bzw. Erläuterungen können untereinander, also auch zwischen den jeweiligen Bereichen und Vorzugsbereichen beliebig kombiniert werden. Sie gelten für die Endprodukte sowie für die Vor- und Zwischenprodukte entsprechend.

Erfindungsgemäß bevorzugt werden die Verbindungen der Formel (I), in welchen eine Kombination der vorstehend als bevorzugt (vorzugsweise) aufgeführten Bedeutungen vorliegt.

Erfindungsgemäß besonders bevorzugt werden die Verbindungen der Formel (I), in welchen eine Kombination der vorstehend als besonders bevorzugt aufgeführten Bedeutungen vorliegt.

Erfindungsgemäß ganz besonders bevorzugt werden die Verbindungen der Formel (I), in welchen eine Kombination der vorstehend als ganz besonders bevorzugt aufgeführten Bedeutungen vorliegt.

Erfindungsgemäß hervorgehoben werden die Verbindungen der Formel (I), in welchen eine Kombination der vorstehend als hervorgehoben aufgeführten Bedeutungen vorliegt.

Gesättigte oder ungesättigte Kohlenwasserstoffreste wie Alkyl, Alkandiyl oder Alkenyl können, auch in Verbindung mit Heteroatomen, wie z.B. in Alkoxy, soweit möglich, jeweils geradkettig oder verzweigt sein.

Gegebenenfalls substituierte Reste können, sofern nichts anderes angegeben ist, einfach oder mehrfach substituiert sein, wobei bei Mehrfachsubstitutionen die Substituenten gleich oder verschieden sein können.

Im einzelnen seien außer den bei den Herstellungsbeispielen genannten Verbindungen die folgenden Verbindungen der Formel (I-1-a) genannt:

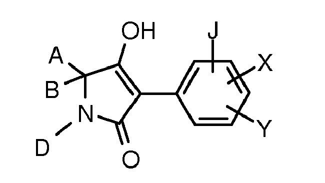

**Tabelle 1:**

| J = 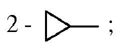 X = H; Y= H. | | |
|---|---|---|
| A | B | D |
| CH₃ | H | H |
| C₂H₅ | H | H |
| C₃H₇ | H | H |
| i-C₃H₇ | H | H |
| C₄H₉ | H | H |
| i-C₄H₉ | H | H |
| s-C₄H₉ | H | H |
| t-C₄H₉ | H | H |
| CH₃ | CH₃ | H |
| C₂H₅ | CH₃ | H |
| C₃H₇ | CH₃ | H |
| i-C₃H₇ | CH₃ | H |
| C₄H₉ | CH₃ | H |
| i-C₄H₉ | CH₃ | H |
| s-C₄H₉ | CH₃ | H |
| t-C₄H₉ | CH₃ | H |
| C₂H₅ | C₂H₅ | H |
| C₃H₇ | C₃H₇ | H |
| 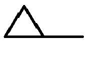 | CH₃ | H |
| 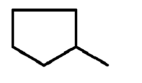 | CH₃ | H |
| 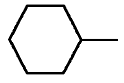 | CH₃ | H |
| -(CH₂)₂- | | H |
| -(CH₂)₄- | | H |
| -(CH₂)₅- | | H |
| -(CH₂)₆- | | H |
| -(CH₂)₇- | | H |
| -(CH₂)₂-O-(CH₂)₂- | | H |
| -CH₂-O-(CH₂)₃- | | H |
| -(CH₂)₂-S-(CH₂)₂- | | H |
| -CH₂-CHCH₃-(CH₂)₃- | | H |
| -CH₂-CHOCH₃-(CH₂)₃- | | H |
| -CH₂-CHOC₂H₅-(CH₂)₃- | | H |
| -CH₂-CHOC₃H₇-(CH₂)₃- | | H |
| -CH₂-CHOC₄H₉-(CH₂)₃- | | H |
| -CH₂-CHO-(CH₂)₂-OCH₃-(CH₂)₃- | | H |
| 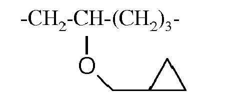 | | H |
| -(CH₂)₂-CHCH₃-(CH₂)₂- | | H |
| -(CH₂)₂-CHC₂H₅-(CH₂)₂- | | H |
| -(CH₂)₂-CHC₃H₇-(CH₂)₂- | | H |
| -(CH₂)₂-CHi-C₃H₇-(CH₂)₂- | | H |
| -(CH₂)₂-CHOCH₃-(CH₂)₂- | | H |
| -(CH₂)₂-CHOC₂H₅-(CH₂)₂- | | H |
| -(CH₂)₂-CHOC₃H₇-(CH₂)₂- | | H |
| -(CH₂)₂-CHO-i-C₃H₇-(CH₂)₂- | | H |
| -(CH₂)₂-C(CH₃)₂-(CH₂)₂- | | H |
| -CH₂-(CHCH₃)₂-(CH₂)₂- | | H |
| 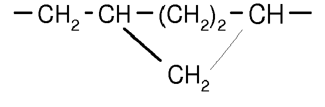 | | H |
| 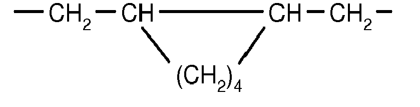 | | H |
| 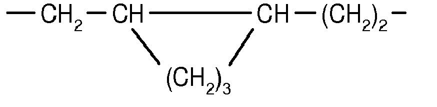 | | H |
| 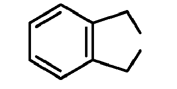 | | H |
| 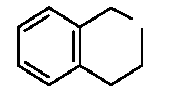 | | H |
| 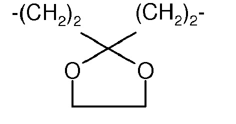 | | H |
| 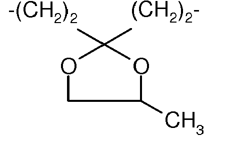 | | H |
| 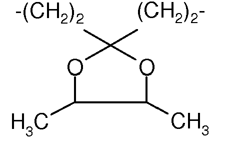 | | H |
| 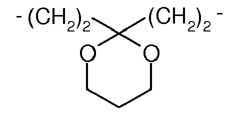 | | H |
| 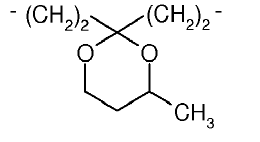 | | H |
| 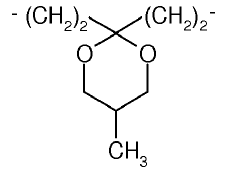 | | H |
| 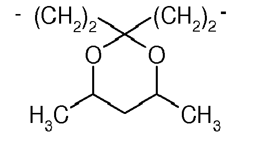 | | H |
| 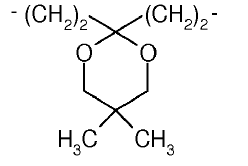 | | H |
| 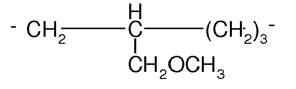 | | H |
| 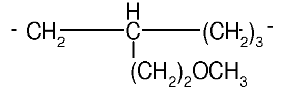 | | H |
| 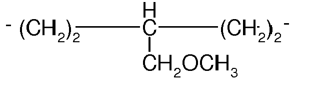 | | H |
| 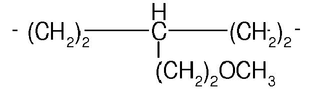 | | H |
| 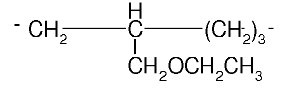 | | H |
| 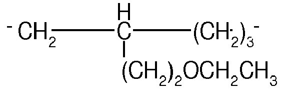 | | H |
| 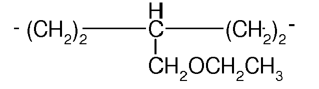 | | H |
| 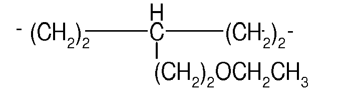 | | H |

| A | D | B |
|---|---|---|
| -(CH₂)₃- | | H |
| -(CH₂)₄- | | H |
| -CH₂-CHCH₃-CH₂- | | H |
| -CH₂-CH₂-CHCH₃- | | H |
| -CH₂-CHCH₃-CHCH₃- | | H |
| -CH₂-CH(OCH₃)-CH₂- | | H |
| -CH₂-CH=CH-CH₂- | | H |
| 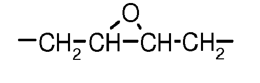 | | H |
| -CH₂-S-CH₂- | | H |
| -CH₂-S-(CH₂)₂- | | H |
| -(CH₂)₂-S-CH₂- | | H |
| 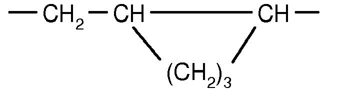 | | H |
| H | CH₃ | H |
| H | C₂H₅ | H |
| H | C₃H₇ | H |
| H | i-C₃H₇ | H |
| H | 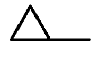 | H |
| H | 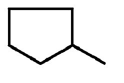 | H |
| H | 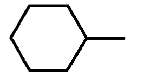 | H |
| CH₃ | CH₃ | H |
| CH₃ | C₂H₅ | H |
| CH₃ | C₃H₇ | H |
| CH₃ | i-C₃H₇ | H |
| CH₃ | 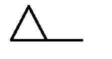 | H |
| CH₃ | 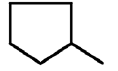 | H |
| CH₃ | 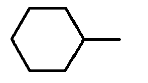 | H |
| C₂H₅ | CH₃ | H |
| C₂H₅ | C₂H₅ | H |

| | |
|---|---|
| **Tabelle 2:** | A, B und D wie in Tabelle 1 angegeben |
| | J= 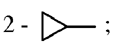 X = 4-CH₃; Y = H |
| **Tabelle 3:** | A, B und D wie in Tabelle 1 angegeben |
| | J= 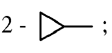 X = 6- CH₃; Y = H. |
| **Tabelle 4:** | A, B und D wie in Tabelle 1 angegeben |
| | J= 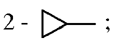 X = 6-C₂H₅; Y = H. |
| **Tabelle 5:** | A, B und D wie in Tabelle 1 angegeben |
| | X = 2-CH₃; Y = H; J = 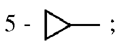 |
| **Tabelle 6:** | A, B und D wie in Tabelle 1 angegeben |
| | X = 2-CH₃; Y = 4-CH₃; J = 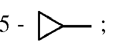 |
| **Tabelle 7:** | A, B und D wie in Tabelle 1 angegeben |
| | J= 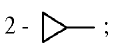 X = 4-CH₃; Y = 6-CH₃. |
| **Tabelle 8:** | A, B und D wie in Tabelle 1 angegeben |
| | J= 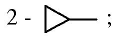 X = 6-C₂H₅; Y = 4-CH₃. |
| **Tabelle 9:** | A, B und D wie in Tabelle 1 angegeben |
| | J= 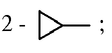 X = 6-CH₃; Y = 4-Cl. |
| **Tabelle 10:** | A, B und D wie in Tabelle 1 angegeben |
| | J= 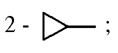 X = 6-C₂H₅; Y = 4-Cl. |
| **Tabelle 11:** | A, B und D wie in Tabelle 1 angegeben |
| | J= 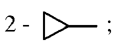 X = 6-Cl; Y = 4-CH₃. |
| **Tabelle 12:** | A, B und D wie in Tabelle 1 angegeben |
| | J= 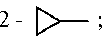 X = 5-CH₃; Y = 4-CH₃. |
| **Tabelle 13:** | A, B und D wie in Tabelle 1 angegeben |
| | X = 2-CH₃; J = 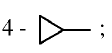 Y=H. |
| **Tabelle 14:** | A, B und D wie in Tabelle 1 angegeben |
| | X = 2-C₂H₅; J = 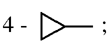 Y=H. |
| **Tabelle 15:** | A, B und D wie in Tabelle 1 angegeben |
| | X = 2-CH₃; J = 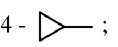 Y = 6-CH₃. |
| **Tabelle 16:** | A, B und D wie in Tabelle 1 angegeben |
| | X = 2-C₂H₅; J = 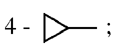 Y = 6- CH₃. |
| **Tabelle 17:** | A, B und D wie in Tabelle 1 angegeben |
| | X = 2-C₂H₅; J = 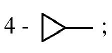 Y = 6-C₂H₅. |
| **Tabelle 18:** | A, B und D wie in Tabelle 1 angegeben |
| | X = 2-Cl; J = 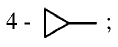 Y = 6-CH₃. |
| **Tabelle 19:** | A, B und D wie in Tabelle 1 angegeben |
| | X = 2-Cl; J = 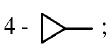 Y = 6-C₂H₅. |
| **Tabelle 20:** | A, B und D wie in Tabelle 1 angegeben |
| | X = 2-CH₃; J = 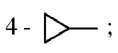 Y = 5-CH₃. |
| **Tabelle 21:** | A, B und D wie in Tabelle 1 angegeben |
| | X = 2-CH₃; J = 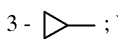 Y = 6-CH₃. |
| **Tabelle 22:** | A, B und D wie in Tabelle 1 angegeben |
| | J= 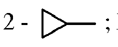 X = 5-CH₃; Y = H. |
| **Tabelle 22a:** | A und B wie in Tabelle 1 angegeben |
| | J= 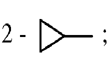 x = 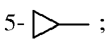 Y=H. |

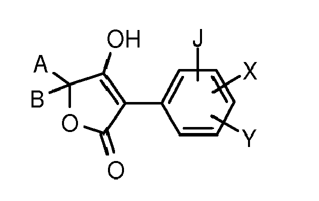

| | |
|---|---|
| **Tabelle 23** | J = 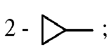 X = H; Y= H. |

| A | B |
|---|---|
| CH₃ | H |
| C₂H₅ | H |
| C₃H₇ | H |
| i-C₃H₇ | H |
| C₄H₉ | H |
| i-C₄H₉ | H |
| s-C₄H₉ | H |
| t-C₄H₉ | H |
| CH₃ | CH₃ |
| C₂H₅ | CH₃ |
| C₃H₇ | CH₃ |
| i-C₃H₇ | CH₃ |
| C₄H₉ | CH₃ |
| i-C₄H₉ | CH₃ |
| s-C₄H₉ | CH₃ |
| t-C₄H₉ | CH₃ |
| C₂H₅ | C₂H₅ |
| C₃H₇ | C₃H₇ |
| 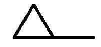 | CH₃ |
| 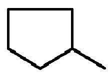 | CH₃ |
| 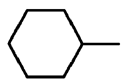 | CH₃ |
| -(CH₂)₂- | |
| -(CH₂)₄- | |
| -(CH₂)₅- | |
| -(CH₂)₆- | |
| -(CH₂)₇- | |
| -(CH₂)₂-O-(CH₂)₂- | |
| -CH₂-O-(CH₂)₃- | |
| -(CH₂)₂-S-(CH₂)₂- | |
| -CH₂-CHCH₃-(CH₂)₃- | |
| -CH₂-CHOCH₃-(CH₂)₃- | |
| -CH₂-CHOC₂H₅-(CH₂)₃- | |
| -CH₂-CHOC₃H₇-(CH₂)₃- | |
| -CH₂-CHOC₄H₉-(CH₂)₃- | |
| -CH₂-CHO-(CH₂)₂-OCH₃-(CH₂)₃- | |
| 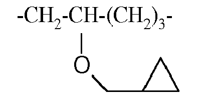 | |
| -(CH₂)₂-CHCH₃-(CH₂)₂- | |
| -(CH₂)₂-CHC₂H₅-(CH₂)₂- | |
| -(CH₂)₂-CHC₃H₇-(CH₂)₂- | |
| -(CH₂)₂-CHi-C₃H₇-(CH₂)₂- | |
| -(CH₂)₂-CHOCH₃-(CH₂)₂- | |
| -(CH₂)₂-CHOC₂H₅-(CH₂)₂- | |
| -(CH₂)₂-CHOC₃H₇-(CH₂)₂- | |
| -(CH₂)₂-CHO-i-C₃H₇-(CH₂)₂- | |
| -(CH₂)₂-C(CH₃)₂-(CH₂)₂- | |
| -CH₂-(CHCH₃)₂-(CH₂)₂- | |
| 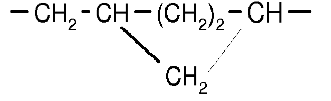 | |
| 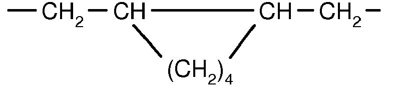 | |
| 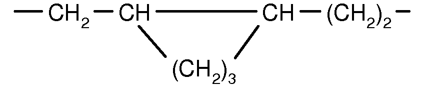 | |
| 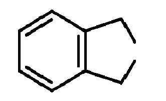 | |
| 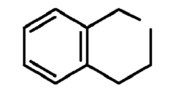 | |
| 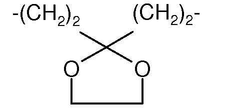 | |
| 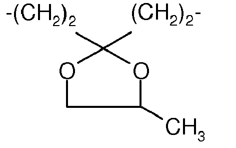 | |
| 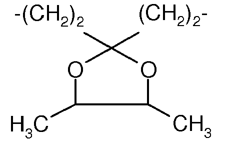 | |
| 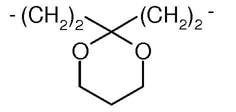 | |
| 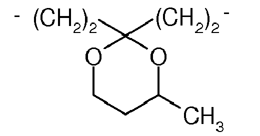 | |
| 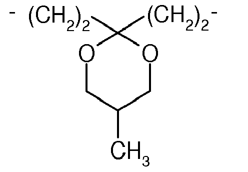 | |
| 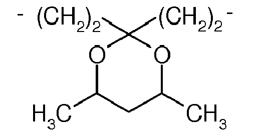 | |
| 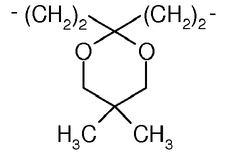 | |
| 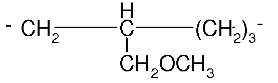 | |
| 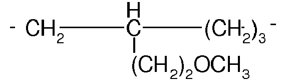 | |
| 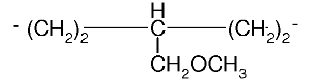 | |
| 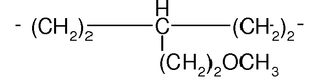 | |
| 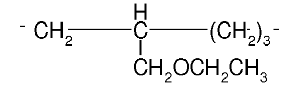 | |
| 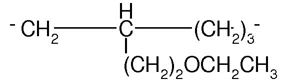 | |
| 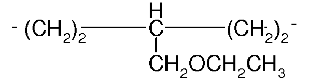 | |
| 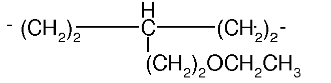 | |

| | |
|---|---|
| **Tabelle 24:** | A und B wie in Tabelle 23 angegeben |
| | J= 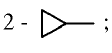 X = 4-CH₃; Y = H |
| **Tabelle 25:** | A und B wie in Tabelle 23 angegeben |
| | J= 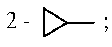 X = 6- CH₃; Y = H. |
| **Tabelle 26:** | A und B wie in Tabelle 23 angegeben |
| | J= 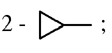 X = 6-C₂H₅; Y = H. |
| **Tabelle 27:** | A und B wie in Tabelle 23 angegeben |
| | X = 2-CH₃; Y = H; J = 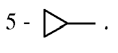 |
| **Tabelle 28:** | A und B wie in Tabelle 23 angegeben |
| | X = 2-CH₃; Y = 4-CH₃; J = 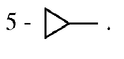 |
| **Tabelle 29:** | A und B wie in Tabelle 23 angegeben |
| | J = 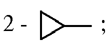 X = 4-CH₃; Y = 6-CH₃. |
| **Tabelle 30:** | A und B wie in Tabelle 23 angegeben |
| | J = 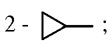 X = 6-C₂H₅; Y = 4-CH₃. |
| **Tabelle 31:** | A und B wie in Tabelle 23 angegeben |
| | J= 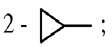 X = 6-CH₃; Y = 4-Cl. |
| **Tabelle 32:** | A und B wie in Tabelle 23 angegeben |
| | J= 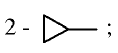 X = 6-C₂H₅; Y = 4-Cl. |
| **Tabelle 33:** | A und B wie in Tabelle 23 angegeben |
| | J= 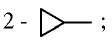 X = 6-Cl; Y = 4-CH₃. |
| **Tabelle 34:** | A und B wie in Tabelle 23 angegeben |
| | J= 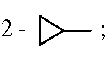 X = 5-CH₃; Y = 4-CH₃. |
| **Tabelle 35:** | A und B wie in Tabelle 23 angegeben |
| | X = 2-CH₃; J = 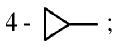 Y=H. |
| **Tabelle 36:** | A und B wie in Tabelle 23 angegeben |
| | X = 2-C₂H₅; J = 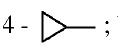 Y=H. |
| **Tabelle 37:** | A und B wie in Tabelle 23 angegeben |
| | X = 2-CH₃; J = 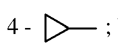 Y = 6-CH₃. |
| **Tabelle 38:** | A und B wie in Tabelle 23 angegeben |
| | X = 2-C₂H₅; J = 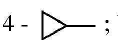 Y = 6- CH₃. |
| **Tabelle 39:** | A und B wie in Tabelle 23 angegeben |
| | X = 2-C₂H₅; J = 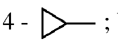 Y = 6-C₂H₅. |
| **Tabelle 40:** | A und B wie in Tabelle 23 angegeben |
| | X = 2-Cl; J = 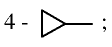 Y = 6-CH₃. |
| **Tabelle 41:** | A und B wie in Tabelle 23 angegeben |
| | X = 2-Cl; J = 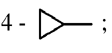 Y = 6-C₂H₅. |
| **Tabelle 42:** | A und B wie in Tabelle 23 angegeben |
| | X = 2-CH₃; J = 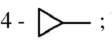 Y = 5-CH₃. |
| **Tabelle 43:** | A und B wie in Tabelle 23 angegeben |
| | X = 2-CH₃; J = 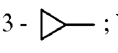 Y = 6-CH₃. |
| **Tabelle 44:** | A und B wie in Tabelle 23 angegeben |
| | J = 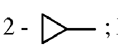 X = 5-CH₃; Y = H. |
| **Tabelle 45:** | A und B wie in Tabelle 23 angegeben |
| | J= 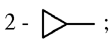 X = 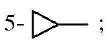 Y=H. |

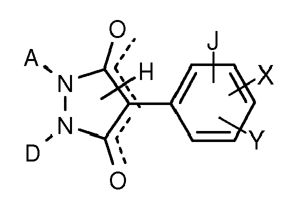

| | |
|---|---|
| **Tabelle 46:** | J = 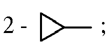 X = H; Y = H. |

| **A** | **D** |
|---|---|
| CH₃ | CH₃ |
| CH₃ | -(CH₂)₂OH |
| CH₃ | -(CH₂)₂OCH₃ |
| CH₃ | -(CH₂)₂-O-(CH₂)₂-OCH₃ |
| -(CH₂)₂-O-CH₃ | -(CH₂)₂-O-CH₃ |
| -(CH₂)₂-O-(CH₂)₂-OCH₃ | -(CH₂)₂-O-(CH₂)₂-OCH₃ |
| -(CH₂)₃- | |
| -(CH₂)₄- | |
| -(CH2)₂-O-(CH₂)₂- | |
| 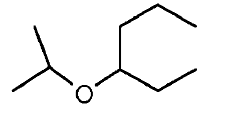 | |
| 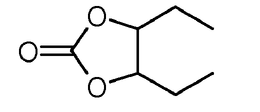 | |
| 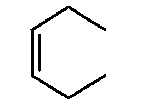 | |
| 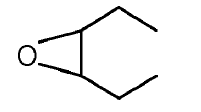 | |
| 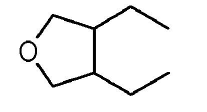 | |
| 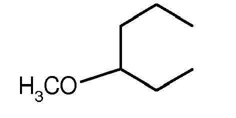 | |
| 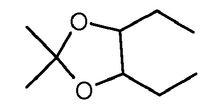 | |
| 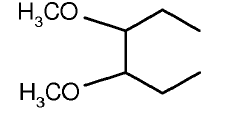 | |
| 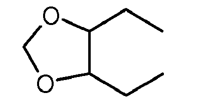 | |
| 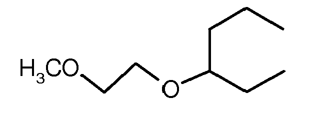 | |

| | |
|---|---|
| **Tabelle 47:** | A und D wie in Tabelle 46 angegeben |
| | J= 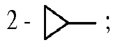 X = 4-CH₃; Y = H |
| **Tabelle 48:** | A und D wie in Tabelle 46 angegeben |
| | J= 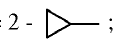 X = 6- CH₃; Y = H. |
| **Tabelle 49:** | A und D wie in Tabelle 46 angegeben |
| | J= 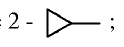 X = 6-C₂H₅; Y = H. |
| **Tabelle 50:** | A und D wie in Tabelle 46 angegeben |
| | X = 2-CH₃; Y = H; J = 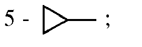 |
| **Tabelle 51:** | A und D wie in Tabelle 46 angegeben |
| | X = 2-CH₃; Y = 4-CH₃; J = 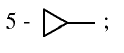 |
| **Tabelle 52:** | A und D wie in Tabelle 46 angegeben |
| | J= 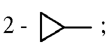 X = 4-CH₃; Y = 6-CH₃. |
| **Tabelle 53:** | A und D wie in Tabelle 46 angegeben |
| | J= 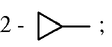 X = 6-C₂H₅; Y = 4-CH₃. |
| **Tabelle 54:** | A und D wie in Tabelle 46 angegeben |
| | J= 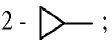 X = 6-CH₃; Y = 4-Cl. |
| **Tabelle 55:** | A und D wie in Tabelle 46 angegeben |
| | J= 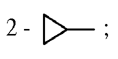 X = 6-C₂H₅; Y = 4-Cl. |
| **Tabelle 56:** | A und D wie in Tabelle 46 angegeben |
| | J= 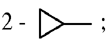 X = 6-Cl; Y = 4-CH₃. |
| **Tabelle 57:** | A und D wie in Tabelle 46 angegeben |
| | J= 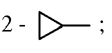 X = 5-CH₃; Y = 4-CH₃. |
| **Tabelle 58:** | A und D wie in Tabelle 46 angegeben |
| | X = 2-CH₃; J = 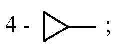 Y=H. |
| **Tabelle 59:** | A und D wie in Tabelle 46 angegeben |
| | X = 2-C₂H₅; J = 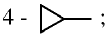 Y=H. |
| **Tabelle 60:** | A und D wie in Tabelle 46 angegeben |
| | X = 2-CH₃; J = 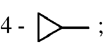 Y = 6-CH₃. |
| **Tabelle 61:** | A und D wie in Tabelle 46 angegeben |
| | X = 2-C₂H₅; J = 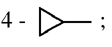 Y = 6- CH₃. |
| **Tabelle 62:** | A und D wie in Tabelle 46 angegeben |
| | X = 2-C₂H₅; J = 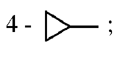 Y = 6-C₂H₅. |
| **Tabelle 63:** | A und D wie in Tabelle 46 angegeben |
| | X = 2-Cl; J = 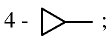 Y = 6-CH₃. |
| **Tabelle 64:** | A und D wie in Tabelle 46 angegeben |
| | X = 2-Cl; J = 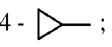 Y = 6-C₂H₅. |
| **Tabelle 65:** | A und D wie in Tabelle 46 angegeben |
| | X = 2-CH₃; J = 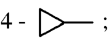 Y = 5-CH₃. |
| **Tabelle 66:** | A und D wie in Tabelle 46 angegeben |
| | X = 2-CH₃; J = 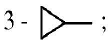 Y = 6-CH₃. |
| **Tabelle 67:** | A und D wie in Tabelle 46 angegeben |
| | J= 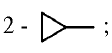 X = 5-CH₃; Y = H. |
| **Tabelle 68:** | A und D wie in Tabelle 46 angegeben |
| | J= 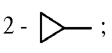 X = 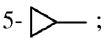 Y=H. |

Bevorzugte Bedeutungen der oben in Zusammenhang mit den die Kulturpflanzen-Verträglichkeit verbessernden Verbindungen ("Herbizid-Safenern") der Formeln (IIa), (IIb), (IIc), (IId) und (IIe) aufgeführten Gruppen werden im Folgenden definiert.
- m: steht bevorzugt für die Zahlen 0, 1, 2, 3 oder 4.
- A¹: steht bevorzugt für eine der nachstehend skizzierten divalenten heterocyclischen Gruppierungen 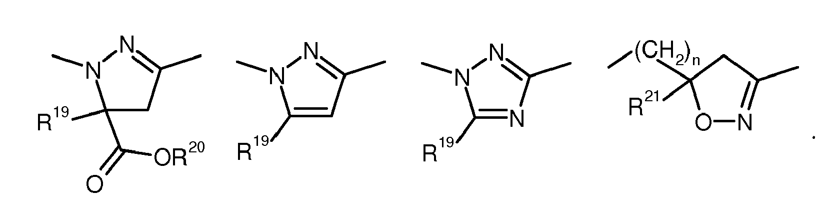
- n: steht bevorzugt für die Zahlen 0, 1, 2, 3 oder 4.
- A²: steht bevorzugt für jeweils gegebenenfalls durch Methyl, Ethyl, Methoxycarbonyl oder Ethoxy-carbonyl substituiertes Methylen oder Ethylen.
- R¹⁴: steht bevorzugt für Hydroxy, Mercapto, Amino, Methoxy, Ethoxy, n- oder i-Propoxy, n-, i-, s- oder t-Butoxy, Methylthio, Ethylthio, n- oder i-Propylthio, n-, i-, s- oder t-Butylthio, Methylamino, Ethylamino, n- oder i-Propylamino, n-, i-, s- oder t-Butylamino, Dimethylamino oder Diethylamino.
- R¹⁵: steht bevorzugt für Hydroxy, Mercapto, Amino, Methoxy, Ethoxy, n- oder i-Propoxy, n-, i-, s- oder t-Butoxy, 1-Methylhexaloxy, Allyloxy, 1-Allyloxymethyl-ethoxy, Methylthio, Ethylthio, n- oder i-Propylthio, n-, i-, s- oder t-Butylthio, Methylamino, Ethylamino, n- oder i-Propylamino, n-, i-, s- oder t-Butylamino, Dimethylamino oder Diethylamino.
- R¹⁶: steht bevorzugt für jeweils gegebenenfalls durch Fluor, Chlor und/oder Brom substituiertes Methyl, Ethyl, n- oder i-Propyl.
- R¹⁷: steht bevorzugt für Wasserstoff, jeweils gegebenenfalls durch Fluor und/oder Chlor substituiertes Methyl, Ethyl, n- oder i-Propyl, n-, i-, s- oder t-Butyl, Propenyl, Butenyl, Propinyl oder Butinyl, Methoxymethyl, Ethoxymethyl, Methoxyethyl, Ethoxyethyl, Dioxolanylmethyl, Furyl, Furylmethyl, Thienyl, Thiazolyl, Piperidinyl, oder gegebenenfalls durch Fluor, Chlor, Methyl, Ethyl, n- oder i-Propyl, n-, i-, s- oder t-Butyl substituiertes Phenyl.
- R¹⁸: steht bevorzugt für Wasserstoff, jeweils gegebenenfalls durch Fluor und/oder Chlor substituiertes Methyl, Ethyl, n- oder i-Propyl, n-, i-, s- oder t-Butyl, Propenyl, Butenyl, Propinyl oder Butinyl, Methoxymethyl, Ethoxymethyl, Methoxyethyl, Ethoxyethyl, Dioxolanylmethyl, Furyl, Furylmethyl, Thienyl, Thiazolyl, Piperidinyl, oder gegebenenfalls durch Fluor, Chlor, Methyl, Ethyl, n- oder i-Propyl, n-, i-, s- oder t-Butyl substituiertes Phenyl, oder zusammen mit R¹⁷ für einen der Reste -CH₂-O-CH₂-CH₂- und -CH₂-CH₂-O-CH₂-CH₂-, die gegebenenfalls substituiert sind durch Methyl, Ethyl, Furyl, Phenyl, einen annellierten Benzolring oder durch zwei Substituenten, die gemeinsam mit dem C-Atom, an das sie gebunden sind, einen 5- oder 6-gliedrigen Carbocyclus bilden.
- R¹⁹: steht bevorzugt für Wasserstoff, Cyano, Fluor, Chlor, Brom, oder für jeweils gegebenenfalls durch Fluor, Chlor und/oder Brom substituiertes Methyl, Ethyl, n- oder i-Propyl, Cyclopropyl, Cyclobutyl, Cyclopentyl, Cyclohexyl oder Phenyl.
- R²⁰: steht bevorzugt für Wasserstoff, gegebenenfalls durch Hydroxy, Cyano, Fluor, Chlor, Methoxy, Ethoxy, n- oder i-Propoxy substituiertes Methyl, Ethyl, n- oder i-Propyl, n-, i-, s- oder t-Butyl.
- R²¹: steht bevorzugt für Wasserstoff, Cyano, Fluor, Chlor, Brom, oder für jeweils gegebenenfalls durch Fluor, Chlor und/oder Brom substituiertes Methyl, Ethyl, n- oder i-Propyl, n-, i-, s- oder t-Butyl, Cyclopropyl, Cyclobutyl, Cyclopentyl, Cyclohexyl oder Phenyl.
- X¹: steht bevorzugt für Nitro, Cyano, Fluor, Chlor, Brom, Methyl, Ethyl, n- oder i-Propyl, n-, i-, s- oder t-Butyl, Difluormethyl, Dichlormethyl, Trifluormethyl, Trichlormethyl, Chlordifluormethyl, Fluordichlormethyl, Methoxy, Ethoxy, n- oder i-Propoxy, Difluormethoxy oder Trifluormethoxy.
- X²: steht bevorzugt für Wasserstoff, Nitro, Cyano, Fluor, Chlor, Brom, Methyl, Ethyl, n- oder i-Propyl, n-, i-, s- oder t-Butyl, Difluormethyl, Dichlormethyl, Trifluormethyl, Trichlormethyl, Chlordifluormethyl, Fluordichlormethyl, Methoxy, Ethoxy, n- oder i-Propoxy, Difluormethoxy oder Trifluormethoxy.
- X³: steht bevorzugt für Wasserstoff, Nitro, Cyano, Fluor, Chlor, Brom, Methyl, Ethyl, n- oder i-Propyl, n-, i-, s- oder t-Butyl, Difluormethyl, Dichlormethyl, Trifluormethyl, Trichlormethyl, Chlordifluormethyl, Fluordichlormethyl, Methoxy, Ethoxy, n- oder i-Propoxy, Difluormethoxy oder Trifluormethoxy.
- t: steht bevorzugt für die Zahlen 0, 1, 2, 3 oder 4.
- v: steht bevorzugt für die Zahlen 0, 1, 2 oder 3.
- R²²: steht bevorzugt für Wasserstoff, Methyl, Ethyl, n- oder i-Propyl.
- R²³: steht bevorzugt für Wasserstoff, Methyl, Ethyl, n- oder i-Propyl.
- R²⁴: steht bevorzugt für Wasserstoff, jeweils gegebenenfalls durch Cyano, Fluor, Chlor, Methoxy, Ethoxy, n- oder i-Propoxy substituiertes Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, n- oder i-Propylthio, n-, i-, s- oder t-Butylthio, , Methylamino, Ethylamino, n- oder i-Propylamino, n-, i-, s- oder t-Butylamino, Dimethylamino oder Diethylamino, oder jeweils gegebenenfalls durch Cyano, Fluor, Chlor, Brom, Methyl, Ethyl, n- oder i-Propyl substituiertes Cyclopropyl, Cyclobutyl, Cyclopentyl, Cyclohexyl, Cyclopropyloxy, Cyclobutyloxy, Cyclopentyloxy, Cyclohexyloxy, Cyclopropylthio, Cyclobutylthio, Cyclopentylthio, Cyclohexylthio, Cyclopropylamino, Cyclobutylamino, Cyclopentylamino oder Cyclohexylamino.
- R²⁵: steht bevorzugt für Wasserstoff, jeweils gegebenenfalls durch Cyano, Hydroxy, Fluor, Chlor, Methoxy, Ethoxy, n- oder i-Propoxy substituiertes Methyl, Ethyl, n- oder i-Propyl, n-, i- oder s-Butyl, jeweils gegebenenfalls durch Cyano, Fluor, Chlor oder Brom substituiertes Propenyl, Butenyl, Propinyl oder Butinyl, oder jeweils gegebenenfalls durch Cyano, Fluor, Chlor, Brom, Methyl, Ethyl, n- oder i-Propyl substituiertes Cyclopropyl, Cyclobutyl, Cyclopentyl oder Cyclohexyl.
- R²⁶: steht bevorzugt für Wasserstoff, jeweils gegebenenfalls durch Cyano, Hydroxy, Fluor, Chlor, Methoxy, Ethoxy, n- oder i-Propoxy substituiertes Methyl, Ethyl, n- oder i-Propyl, n-, i- oder s-Butyl, jeweils gegebenenfalls durch Cyano, Fluor, Chlor oder Brom substituiertes Propenyl, Butenyl, Propinyl oder Butinyl, jeweils gegebenenfalls durch Cyano, Fluor, Chlor, Brom, Methyl, Ethyl, n- oder i-Propyl substituiertes Cyclopropyl, Cyclobutyl, Cyclopentyl oder Cyclohexyl, oder gegebenenfalls durch Nitro, Cyano, Fluor, Chlor, Brom, Methyl, Ethyl, n-oder i-Propyl, n-, i-, s- oder t-Butyl, Trifluormethyl, Methoxy, Ethoxy, n- oder i-Propoxy, Difluormethoxy oder Trifluormethoxy substituiertes Phenyl, oder zusammen mit R²⁵ für jeweils gegebenenfalls durch Methyl oder Ethyl substituiertes Butan-1,4-diyl (Trimethylen), Pentan-1,5-diyl, 1-Oxa-butan-1,4-diyl oder 3-Oxa-pentan-1,5-diyl.
- X⁴: steht bevorzugt für Nitro, Cyano, Carboxy, Carbamoyl, Formyl, Sulfamoyl, Hydroxy, Amino, Fluor, Chlor, Brom, Methyl, Ethyl, n- oder i-Propyl, n-, i-, s- oder t-Butyl, Trifluormethyl, Methoxy, Ethoxy, n- oder i-Propoxy, Difluormethoxy oder Trifluormethoxy.
- X⁵: steht bevorzugt für Nitro, Cyano, Carboxy, Carbamoyl, Formyl, Sulfamoyl, Hydroxy, Amino, Fluor, Chlor, Brom, Methyl, Ethyl, n- oder i-Propyl, n-, i-, s- oder t-Butyl, Trifluormethyl, Methoxy, Ethoxy, n- oder i-Propoxy, Difluormethoxy oder Trifluormethoxy.

Beispiele für die als erfindungsgemäße Herbizid-Safener ganz besonders bevorzugten Verbindungen der Formel (IIa) sind in der nachstehenden Tabelle aufgeführt.

**Tabelle Beispiele für die Verbindungen der Formel (IIa)**

| | | | |
|---|---|---|---|
| 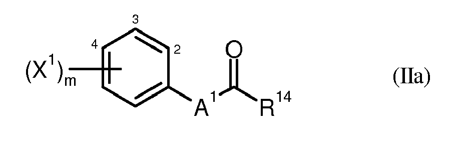 | | | |

| **Beispiel-Nr.** | **(Positionen) (X¹)ₘ** | **A¹** | **R¹⁴** |
|---|---|---|---|
| IIa-1 | (2) Cl, (4) Cl | 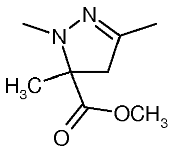 | OCH₃ |
| IIa-2 | (2) Cl, (4) Cl | 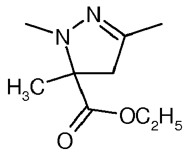 | OCH₃ |
| IIa-3 | (2) Cl, (4) Cl | 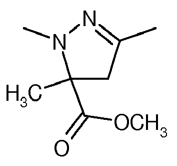 | OC₂H₅ |
| IIa-4 | (2) Cl, (4) Cl | 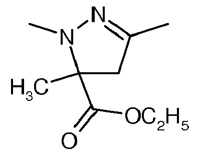 | OC₂H₅ |
| IIa-5 | (2) Cl | 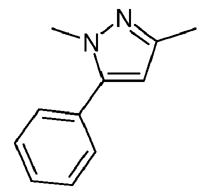 | OCH₃ |
| IIa-6 | (2) Cl, (4) Cl | 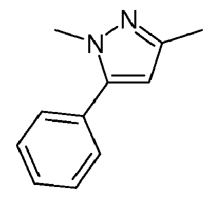 | OCH₃ |
| IIa-7 | (2) F | 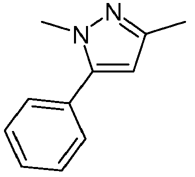 | OCH₃ |
| IIa-8 | (2) F | 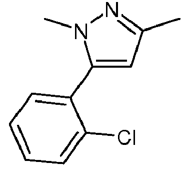 | OCH₃ |
| IIa-9 | (2) Cl, (4) Cl | 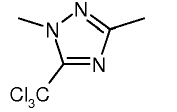 | OC₂H₅ |
| IIa-10 | (2) Cl, (4) CF₃ | 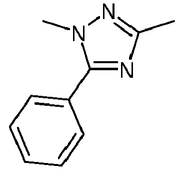 | OCH₃ |
| IIa-11 | (2) Cl | 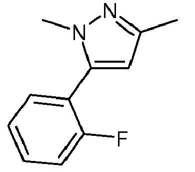 | OCH₃ |
| IIa-12 | - | 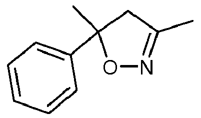 | OC₂H₅ |
| IIa-13 | (2) Cl, (4) Cl | 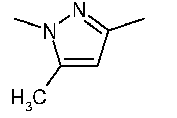 | OC₂H₅ |
| IIa-14 | (2) Cl, (4) Cl | 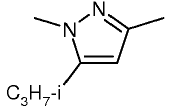 | OC₂H₅ |
| IIa-15 | (2) Cl, (4) Cl | 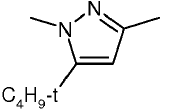 | OC₂H₅ |
| IIa-16 | (2) Cl, (4) Cl | 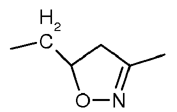 | OC₂H₅ |
| IIa-17 | (2) Cl, (4) Cl | 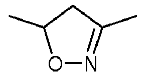 | OC₂H₅ |
| IIa-18 | - | 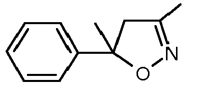 | OH |

Beispiele für die als erfindungsgemäße Herbizid-Safener ganz besonders bevorzugten Verbindungen der Formel (IIb) sind in der nachstehenden Tabelle aufgeführt.

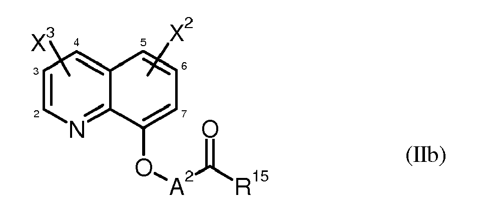

**Tabelle Beispiele für die Verbindungen der Formel (IIb)**

| **Beispiel-Nr.** | **(Position) X²** | **(Position) X³** | **A²** | **R¹⁵** |
|---|---|---|---|---|
| IIb-1 | (5) Cl | - | CH₂ | OH |
| IIb-2 | (5) Cl | - | CH₂ | OCH₃ |
| IIb-3 | (5) Cl | - | CH₂ | OC₂H₅ |
| IIb-4 | (5) Cl | - | CH₂ | OC₃H₇-n |
| IIb-5 | (5) Cl | - | CH₂ | OC₃H₇-i |
| IIb-6 | (5) Cl | - | CH₂ | OC₄H₉-n |
| IIb-7 | (5) Cl | - | CH₂ | OCH(CH₃)C₅H₁₁-n |
| IIb-8 | (5) Cl | (2) F | CH₂ | OH |
| IIb-9 | (5) Cl | (2) Cl | CH₂ | OH |
| IIb-10 | (5) Cl | - | CH₂ | OCH₂CH=CH₂ |
| IIb-11 | (5) Cl | - | CH₂ | OC₄H₉-i |
| IIb-12 | (5) Cl | - | CH₂ | 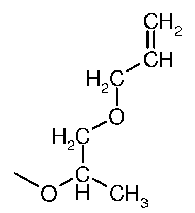 |
| IIb-13 | (5) Cl | - | 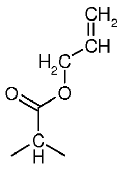 | OCH₂CH=CH₂ |
| IIb-14 | (5) Cl | - | 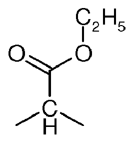 | OC₂H₅ |
| IIb-15 | (5) Cl | - | 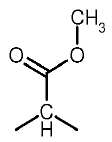 | OCH₃ |

Beispiele für die als erfindungsgemäße Herbizid-Safener ganz besonders bevorzugten Verbindungen der Formel (IIc) sind in der nachstehenden Tabelle aufgeführt.

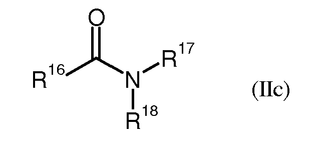

**Tabelle Beispiele für die Verbindungen der Formel (IIc)**

| **Beispiel-Nr.** | **R¹⁶** | **N(R¹⁷,R¹⁸)** |
|---|---|---|
| IIc-1 | CHCl₂ | N(CH₂CH=CH₂)₂ |
| IIc-2 | CHCl₂ | 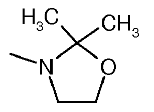 |
| IIc-3 | CHCl₂ | 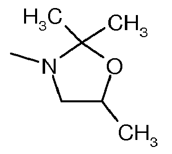 |
| IIc-4 | CHCl₂ | 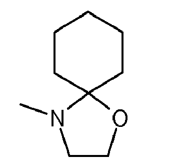 |
| IIc-5 | CHCl₂ | 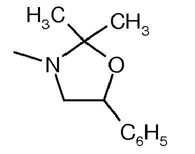 |
| IIc-6 | CHCl₂ | 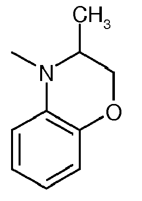 |
| IIc-7 | CHCl₂ | 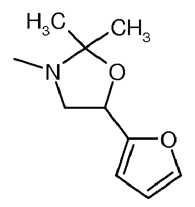 |

Beispiele für die als erfindungsgemäße Herbizid-Safener ganz besonders bevorzugten Verbindungen der Formel (IId) sind in der nachstehenden Tabelle aufgeführt.

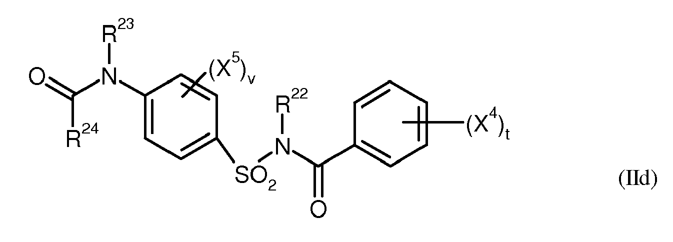

**Tabelle Beispiele für die Verbindungen der Formel (IId)**

| **Beispiel-Nr.** | **R²²** | **R²³** | **R²⁴** | **(Positionen) (X⁴)ₜ** | **(Positionen) (X⁵)ᵥ** |
|---|---|---|---|---|---|
| IId-1 | H | H | CH₃ | (2) OCH₃ | - |
| IId-2 | H | H | C₂H₅ | (2) OCH₃ | - |
| IId-3 | H | H | C₃H₇-n | (2) OCH₃ | - |
| IId-4 | H | H | C₃H₇-i | (2) OCH₃ | - |
| IId-5 | H | H | 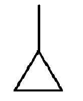 | (2) OCH₃ | - |
| IId-6 | H | H | CH₃ | (2) OCH₃ (5) CH₃ | - |
| IId-7 | H | H | C₂H₅ | (2) OCH₃ (5) CH₃ | - |
| IId-8 | H | H | C₃H₇-n | (2) OCH₃ (5) CH₃ | - |
| IId-9 | H | H | C₃H₇-i | (2) OCH₃ (5) CH₃ | - |
| IId-10 | H | H | 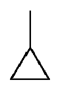 | (2) OCH₃ (5) CH₃ | - |
| IId-11 | H | H | OCH₃ | (2) OCH₃ (5) CH₃ | - |
| IId-12 | H | H | OC₂H₅ | (2) OCH₃ (5) CH₃ | - |
| IId-13 | H | H | OC₃H₇-i | (2) OCH₃ (5) CH₃ | - |
| IId-14 | H | H | SCH₃ | (2) OCH₃ (5) CH₃ | - |
| IId-15 | H | H | SC₂H₅ | (2) OCH₃ (5) CH₃ | - |
| IId-16 | H | H | SC₃H₇-i | (2) OCH₃ (5) CH₃ | - |
| IId-17 | H | H | NHCH₃ | (2) OCH₃ (5) CH₃ | - |
| IId-18 | H | H | NHC₂H₅ | (2) OCH₃ (5) CH₃ | - |
| IId-19 | H | H | NHC₃H₇-i | (2) OCH₃ (5) CH₃ | - |
| IId-20 | H | H | 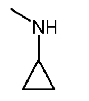 | (2) OCH₃ (5) CH₃ | - |
| IId-21 | H | H | NHCH₃ | (2) OCH₃ | - |
| IId-22 | H | H | NHC₃H₇-i | (2) OCH₃ | - |
| IId-23 | H | H | N(CH₃)₂ | (2) OCH₃ | - |
| IId-24 | H | H | N(CH₃)₂ | (3) CH₃ (4) CH₃ | - |
| IId-25 | H | H | CH₂-O-CH₃ | (2) OCH3 | - |

Beispiele für die als erfindungsgemäße Herbizid-Safener ganz besonders bevorzugten Verbindungen der Formel (IIe) sind in der nachstehenden Tabelle aufgeführt.

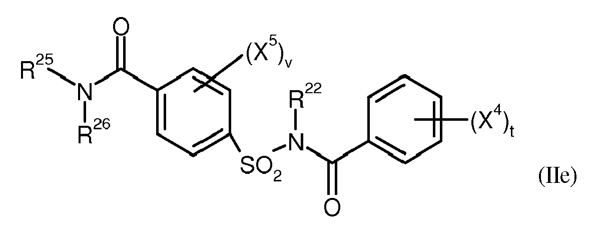

**Tabelle Beispiele für die Verbindungen der Formel (IIe)**

| **Beispiel-Nr.** | **R²²** | **R²⁵** | **R²⁶** | **(Positionen) (X⁴)ₜ** | **(Positionen) (X⁵)ᵥ** |
|---|---|---|---|---|---|
| IIe-1 | H | H | CH₃ | (2) OCH₃ | - |
| IIe-2 | H | H | C₂H₅ | (2) OCH₃ | - |
| IIe-3 | H | H | C₃H₇-n | (2) OCH₃ | - |
| IIe-4 | H | H | C₃H₇-i | (2) OCH₃ | - |
| IIe-5 | H | H | 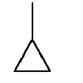 | (2) OCH₃ | - |
| IIe-6 | H | CH₃ | CH₃ | (2) OCH₃ | - |
| IIe-7 | H | H | CH₃ | (2) OCH₃ (5) CH₃ | - |
| IIe-8 | H | H | C₂H₅ | (2) OCH₃ (5) CH₃ | - |
| IIe-9 | H | H | C₃H₇-n | (2) OCH₃ (5) CH₃ | - |
| IIe-10 | H | H | C₃H₇-i | (2) OCH₃ (5) CH₃ | - |
| IIe-11 | H | H | 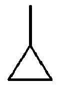 | (2) OCH₃ (5) CH₃ | - |
| IIe-12 | H | CH₃ | CH₃ | (2) OCH₃ (5) CH₃ | - |

Als die die Kulturpflanzen-Verträglichkeit verbessernde Verbindung [Komponente (b')] sind Cloquintocet-mexyl, Fenchlorazol-ethyl, Isoxadifen-ethyl, Mefenpyr-diethyl, Furilazole, Fenclorim, Cumyluron, Dymron, Dimepiperate und die Verbindungen IIe-5 und IIe-11 am meisten bevorzugt, wobei Cloquintocet-mexyl und Mefenpyr-diethyl besonders hervorgehoben seien.

Die als Safener erfindungsgemäß zu verwendenden Verbindungen der allgemeinen Formel (IIa) sind bekannt und/oder können nach an sich bekannten Verfahren hergestellt werden (vgl. WO-A-91/07874, WO-A-95/07897).

Die als Safener erfindungsgemäß zu verwendenden Verbindungen der allgemeinen Formel (IIb) sind bekannt und/oder können nach an sich bekannten Verfahren hergestellt werden (vgl. EP-A-191736).

Die als Safener erfindungsgemäß zu verwendenden Verbindungen der allgemeinen Formel (IIc) sind bekannt und/oder können nach an sich bekannten Verfahren hergestellt werden (vgl. DE-A-2218097, DE-A-2350547).

Die als Safener erfindungsgemäß zu verwendenden Verbindungen der allgemeinen Formel (IId) sind bekannt und/oder können nach an sich bekannten Verfahren hergestellt werden (vgl. DE-A-19621522/US-A-6235680).

Die als Safener erfindungsgemäß zu verwendenden Verbindungen der allgemeinen Formel (IIe) sind bekannt und können nach an sich bekannten Verfahren hergestellt werden (vgl. WO-A-99/66795/US-A-6251827).

Beispiele für die erfindungsgemäßen selektiv herbiziden Kombinationen aus jeweils einem Wirkstoff der Formel (I) und jeweils einem der oben definierten Safener sind in der nachstehenden Tabelle aufgeführt.

**Tabelle Beispiele für die erfindungsgemäßen Kombinationen**

| **Wirkstoffe der Formel (I)** | **Safener** |
|---|---|
| I-1 | Cloquintocet-mexyl |
| I-1 | Fenchlorazole-ethyl |
| I-1 | Isoxadifen-ethyl |
| I-1 | Mefenpyr-diethyl |
| I-1 | Furilazole |
| I-1 | Fenclorim |
| I-1 | Cumyluron |
| I-1 | Daimuron /Dymron |
| I-1 | Dimepiperate |
| I-1 | IIe-11 |
| I-1 | IIe-5 |
| I-2 | Cloquintocet-mexyl |
| I-2 | Fenchlorazole-ethyl |
| I-2 | Isoxadifen-ethyl |
| I-2 | Mefenpyr-diethyl |
| I-2 | Furilazole |
| I-2 | Fenclorim |
| I-2 | Cumyluron |
| I-2 | Daimuron /Dymron |
| I-2 | Dimepiperate |
| I-2 | IIe-11 |
| I-2 | IIe-5 |
| I-3 | Cloquintocet-mexyl |
| I-3 | Fenchlorazole-ethyl |
| I-3 | Isoxadifen-ethyl |
| I-3 | Mefenpyr-diethyl |
| I-3 | Furilazole |
| I-3 | Fenclorim |
| I-3 | Cumyluron |
| I-3 | Daimuron /Dymron |
| I-3 | Dimepiperate |
| I-3 | IIe-5 |
| I-3 | IIe-11 |
| I-4 | Cloquintocet-mexyl |
| I-4 | Fenchlorazole-ethyl |
| I-4 | Isoxadifen-ethyl |
| I-4 | Mefenpyr-diethyl |
| I-4 | Furilazole |
| I-4 | Fenclorim |
| I-4 | Cumyluron |
| I-4 | Daimuron /Dymron |
| I-4 | Dimepiperate |
| I-4 | IIe-11 |
| I-4 | IIe-5 |
| I-5 | Cloquintocet-mexyl |
| I-5 | Fenchlorazole-ethyl |
| I-5 | Isoxadifen-ethyl |
| I-5 | Mefenpyr-diethyl |
| I-5 | Furilazole |
| I-5 | Fenclorim |
| I-5 | Cumyluron |
| I-5 | Daimuron /Dymron |
| I-5 | Dimepiperate |
| I-5 | IIe-5 |
| I-5 | IIe-11 |
| I-6 | Cloquintocet-mexyl |
| I-6 | Fenchlorazole-ethyl |
| I-6 | Isoxadifen-ethyl |
| I-6 | Mefenpyr-diethyl |
| I-6 | Furilazole |
| I-6 | Fenclorim |
| I-6 | Cumyluron |
| I-6 | Daimuron /Dymron |
| I-6 | Dimepiperate |
| I-6 | IIe-5 |
| I-6 | IIe-11 |
| I-7 | Cloquintocet-mexyl |
| I-7 | Fenchlorazole-ethyl |
| I-7 | Isoxadifen-ethyl |
| I-7 | Mefenpyr-diethyl |
| I-7 | Furilazole |
| I-7 | Fenclorim |
| I-7 | Cumyluron |
| I-7 | Daimuron /Dymron |
| I-7 | Dimepiperate |
| I-7 | IIe-5 |
| I-7 | IIe-11 |
| I-8 | Cloquintocet-mexyl |
| I-8 | Fenchlorazole-ethyl |
| I-8 | Isoxadifen-ethyl |
| I-8 | Mefenpyr-diethyl |
| I-8 | Furilazole |
| I-8 | Fenclorim |
| I-8 | Cumyluron |
| I-8 | Daimuron /Dymron |
| I-8 | Dimepiperate |
| I-8 | IIe-5 |
| I-8 | IIe-11 |
| I-9 | Cloquintocet-mexyl |
| I-9 | Fenchlorazole-ethyl |
| I-9 | Isoxadifen-ethyl |
| I-9 | Mefenpyr-diethyl |
| I-9 | Furilazole |
| I-9 | Fenclorim |
| I-9 | Cumyluron |
| I-9 | Daimuron /Dymron |
| I-9 | Dimepiperate |
| I-9 | IIe-5 |
| I-9 | IIe-11 |
| I-10 | Cloquintocet-mexyl |
| I-10 | Fenchlorazole-ethyl |
| I-10 | Isoxadifen-ethyl |
| I-10 | Mefenpyr-diethyl |
| I-10 | Furilazole |
| I-10 | Fenclorim |
| I-10 | Cumyluron |
| I-10 | Daimuron /Dymron |
| I-10 | Dimepiperate |
| I-10 | IIe-5 |
| I-10 | IIe-11 |

Es wurde nun überraschend gefunden, dass die oben definierten Wirkstoffkombinationen aus Cycloalkylphenyl substituierten cyclischen Ketoenolen der allgemeinen Formel (I) und Safenern (Antidots) aus der oben aufgeführten Gruppe (b') bei sehr guter Nutzpflanzen-Verträglichkeit eine besonders hohe herbizide Wirksamkeit aufweisen und in verschiedenen Kulturen, insbesondere in Getreide (vor allem Weizen), aber auch in Soja, Kartoffeln, Mais und Reis zur selektiven Unkrautbekämpfung verwendet werden können.

Dabei ist es als überraschend anzusehen, dass aus einer Vielzahl von bekannten Safenern oder Antidots, die befähigt sind, die schädigende Wirkung eines Herbizids auf die Kulturpflanzen zu antagonisieren, gerade die oben aufgeführten Verbindungen der Gruppe (b') geeignet sind, die schädigende Wirkung von cycloalkylphenyl substituierten cyclischen Ketoenolen auf die Kulturpflanzen annähernd vollständig aufzuheben, ohne dabei die herbizide Wirksamkeit gegenüber den Unkräutern maßgeblich zu beeinträchtigen.

Hervorgehoben sei hierbei die besonders vorteilhafte Wirkung der besonders und am meisten bevorzugten Kombinationspartner aus der Gruppe (b'), insbesondere hinsichtlich der Schonung von Getreidepflanzen, wie z.B. Weizen, Gerste und Roggen, aber auch Mais und Reis, als Kulturpflanzen.

In der Literatur wurde bereits beschrieben, dass sich die Wirkung verschiedener Wirkstoffe durch Zugabe von Ammoniumsalzen steigern lässt. Dabei handelt es sich jedoch um als Detergens wirkende Salze (z.B. WO 95/017817) bzw. Salze mit längeren Alkyl- und / oder Arylsubstituenten, die permeabilisierend wirken oder die Löslichkeit des Wirkstoffs erhöhen (z.B. EP-A 0 453 086, EP-A 0 664 081, FR-A 2 600 494, US 4 844 734, US 5 462 912, US 5 538 937, US-A 03/0224939, US-A 05/0009880, US-A 05/0096386). Weiterhin beschreibt der Stand der Technik die Wirkung nur für bestimmte Wirkstoffe und / oder bestimmte Anwendungen der entsprechenden Mittel. In wieder anderen Fällen handelt es sich um Salze von Sulfonsäuren, bei denen die Säuren selber paralysierend auf Insekten wirken (US 2 842 476). Eine Wirkungssteigerung z.B. durch Ammoniumsulfat ist beispielsweise für die Herbizide Glyphosat und Phosphinothricin beschrieben (US 6 645 914, EP-A2 0 036 106). Eine entsprechende Wirkung bei Insektiziden wird durch diesen Stand der Technik weder offenbart noch nahegelegt.

Auch der Einsatz von Ammoniumsulfat als Formulierhilfsmittel ist für bestimmte Wirkstoffe und Anwendungen beschrieben (WO 92/16108), es dient dort aber zur Stabilisierung der Formulierung, nicht zur Wirkungssteigerung.

Es wurde nun völlig überraschend gefunden, dass sich die Wirkung von Insektiziden und/oder Akariziden und/oder Herbiziden aus der Klasse der cycloalkyl-phenylsubstituierten, spirocyclischen Ketoenole durch den Zusatz von Ammonium- oder Phosphoniumsalzen zur Anwendungslösung oder durch den Einbau dieser Salze in eine Formulierung enthaltend cycloalkyl-phenylsubstituierte, cyclische Ketoenole, deutlich steigern lässt. Gegenstand der vorliegenden Erfindung ist also die Verwendung von Ammonium- oder Phosphoniumsalzen zur Wirkungssteigerung von Pflanzenschutzmitteln, die insektizid und / oder akarizid wirksame cycloalkyl-phenylsubstituierte, cyclische Ketoenole als Wirkstoff enthalten. Gegenstand der Erfindung sind ebenfalls Mittel, die insektizid wirksame cycloalkyl-phenylsubstituierte, cyclische Ketoenole und die Wirkung steigernde Ammonium- oder Phosphoniumsalze enthalten und zwar sowohl formulierte Wirkstoffe als auch anwendungsfertige Mittel (Spritzbrühen). Gegenstand der Erfindung ist schließlich weiterhin die Verwendung dieser Mittel zur Bekämpfung von Schadinsekten und/oder Spinnmilben und/oder unerwünschten Pflanzenwuchs.

Die Verbindungen der Formel (I) besitzen eine breite insektizide und/oder akarizide und/oder herbizide Wirkung, die Wirkung und/oder Pflanzenverträglichkeit lässt im Einzelnen aber zu wünschen übrig.

Die Wirkstoffe können in den erfindungsgemäßen Zusammensetzungen in einem breiten Konzentrationsbereich eingesetzt werden. Die Konzentration der Wirkstoffe in der Formulierung beträgt dabei üblicherweise 0,1 - 50 Gew.-%.

Ammonium- und Phosphoniumsalze, die erfindungsgemäß die Wirkung von Pflanzenschutzmitteln enthaltend Fettsäure-Biosynthese-Inhibitoren steigern, werden durch Formel (III') definiert 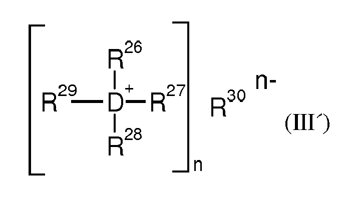 in welcher
- D: für Stickstoff oder Phosphor steht,
- D: bevorzugt für Stickstoff steht,
- R²⁶, R²⁷, R²⁸ und R²⁹: unabhängig voneinander für Wasserstoff oder jeweils gegebenenfalls substituiertes C₁-C₈-Alkyl oder einfach oder mehrfach ungesättigtes, gegebenenfalls substituiertes C₁-C₈-Alkylen stehen, wobei die Substituenten aus Halogen, Nitro und Cyano ausgewählt sein können,
- R^{26'}, R²⁷, R²⁸ und R²⁹: bevorzugt unabhängig voneinander für Wasserstoff oder jeweils gegebenenfalls substituiertes C₁-C₄-Alkyl stehen, wobei die Substituenten aus Halogen, Nitro und Cyano ausgewählt sein können,
- R^{26'}, R²⁷, R²⁸ und R²⁹: besonders bevorzugt unabhängig voneinander für Wasserstoff, Methyl, Ethyl, n-Propyl, i-Propyl, n-Butyl, i-Butyl, s-Butyl oder t-Butyl stehen,
- R^{26'}, R²⁷, R²⁸ und R²⁹: ganz besonders bevorzugt für Wasserstoff stehen,
- n: für 1, 2, 3 oder 4 steht,
- n: bevorzugt für 1 oder 2 steht,
- R³⁰: für ein anorganisches oder organisches Anion steht,
- R³⁰: bevorzugt für Hydrogencarbonat, Tetraborat, Fluorid, Bromid, Jodid, Chlorid, Monohydrogenphosphat, Dihydrogenphosphat, Hydrogensulfat, Tartrat, Sulfat, Nitrat, Thiosulfat, Thiocyanat, Formiat, Laktat, Acetat, Propionat, Butyrat, Pentanoat oder Oxalat steht,
- R³⁰: besonders bevorzugt für Laktat, Sulfat, Nitrat, Thiosulfat, Thiocyanat, Oxalat oder Formiat steht.
- R³⁰: ganz besonders bevorzugt für Sulfat steht.

Erfindungsgemäß hervorgehobene Kombinationen von Wirkstoff, Salz und Penetrationsförderer sind in folgender Tabelle aufgeführt. "Penetrationsförderer gemäß Test" bedeutet dabei, dass jede Verbindung geeignet ist, die in dem Test für die Kutikelpenetration (Baur et al., 1997, Pesticide Science 51, 131-152) als Penetrationsförderer wirkt.

Die Ammonium- und Phosphoniumsalze der Formel (III') können in einem breiten Konzentrationsbereich zur Steigerung der Wirkung von Pflanzenschutzmitteln enthaltend Ketoenole eingesetzt werden. Im Allgemeinen werden die Ammonium- oder Phosphoniumsalze im anwendungsfertigen Pflanzenschutzmittel in einer Konzentration von 0,5 bis 80 mmol/l, bevorzugt 0,75 bis 37,5 mmol/l besonders bevorzugt 1,5 bis 25 mmol/l eingesetzt. Im Fall eines formulierten Produktes wird die Ammonium- und/oder Phosphoniumsalzkonzentration in der Formulierung so gewählt, dass sie nach Verdünnung der Formulierung auf die gewünschte Wirkstoffkonzentration in diesen angegebenen allgemeinen, bevorzugten oder besonders bevorzugten Bereichen liegt. Die Konzentration des Salzes in der Formulierung beträgt dabei üblicherweise 1 - 50 Gew.-%.

In einer bevorzugten Ausführungsform der Erfindung wird den Pflanzenschutzmitteln zur Wirkungssteigerung nicht nur ein Ammonium- und/oder Phosphoniumsalz, sondern zusätzlich ein Penetrationsförderer zugegeben. Es ist als völlig überraschend zu bezeichnen, dass selbst in diesen Fällen eine noch weiter gehende Wirkungssteigerung zu beobachten ist. Gegenstand der vorliegenden Erfindung ist also ebenfalls die Verwendung einer Kombination von Penetrationsförderer und Ammonium- und/oder Phosphoniumsalzen zur Wirkungssteigerung von Pflanzenschutzmitteln, die insektizid wirksame, cycloalkyl-phenylsubstituierte cyclische Ketoenole als Wirkstoff enthalten. Gegenstand der Erfindung sind ebenfalls Mittel, die insektizid wirksame cycloalkyl-phenylsubstituierte cyclische Ketoenole, Penetrationsförderer und Ammonium- und/oder Phosphoniumsalze enthalten und zwar sowohl formulierte Wirkstoffe als auch anwendungsfertige Mittel (Spritzbrühen). Gegenstand der Erfindung ist schließlich weiterhin die Verwendung dieser Mittel zur Bekämpfung von Schadinsekten.

Als Penetrationsförderer kommen im vorliegenden Zusammenhang alle diejenigen Substanzen in Betracht, die üblicherweise eingesetzt werden, um das Eindringen von agrochemischen Wirkstoffen in Pflanzen zu verbessern. Penetrationsförderer werden in diesem Zusammenhang dadurch definiert, dass sie aus der wässerigen Spritzbrühe und/oder aus dem Spritzbelag in die Kutikula der Pflanze eindringen und dadurch die Stoffbeweglichkeit (Mobilität) von Wirkstoffen in der Kutikula erhöhen können. Die in der Literatur (Baur et al., 1997, Pesticide Science 51, 131-152) beschriebene Methode kann zur Bestimmung dieser Eigenschaft eingesetzt werden.

Als Penetrationsförderer kommen beispielsweise Alkanol-alkoxylate in Betracht. Erfindungsgemäße Penetrationsförderer sind Alkanol-alkoxylate der Formel (IV')

R-O-(-AO)ᵥ-R' (IV')

in welcher
- R: für geradkettiges oder verzweigtes Alkyl mit 4 bis 20 Kohlenstoffatomen steht,
- R^{'}: für Wasserstoff, Methyl, Ethyl, n-Propyl, i-Propyl, n-Butyl, i-Butyl, t-Butyl, n-Pentyl oder n-Hexyl steht,
- AO: für einen Ethylenoxid-Rest, einen Propylenoxid-Rest, einen Butylenoxid-Rest oder für Gemische aus Ethylenoxid- und Propylenoxid-Resten oder Butylenoxid-Resten steht und
- v: für Zahlen von 2 bis 30 steht.

Eine bevorzugte Gruppe von Penetrationsförderern sind Alkanolalkoxylate der Formel

R-O-(-EO-)ₙ-R' (IV'-a)

in welcher
- R: die oben angegebene Bedeutung hat,
- R': die oben angegebene Bedeutung hat,
- EO: für -CH₂-CH₂-O- steht und
- n: für Zahlen von 2 bis 20 steht.

Eine weitere bevorzugte Gruppe von Penetrationsförderern sind Alkanol-alkoxylate der Formel

R-O-(-EO-)ₚ-(-PO-)_{q}-R' (IV'-b)

in welcher
- R: die oben angegebene Bedeutung hat,
- R': die oben angegebene Bedeutung hat,
- EO: für -CH₂-CH₂-O- steht,
- PO: für 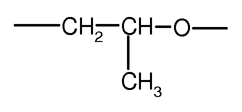 steht,
- p: für Zahlen von 1 bis 10 steht und
- q: für Zahlen von 1 bis 10 steht.

Eine weitere bevorzugte Gruppe von Penetrationsförderern sind Alkanol-Alkoxylate der Formel

R-O-(-PO-)ᵣ-(EO-)ₛ-R' (IV'-c)

in welcher
- R: die oben angegebene Bedeutung hat,
- R': die oben angegebene Bedeutung hat,
- EO: für -CH₂-CH₂-O- steht,
- PO: für 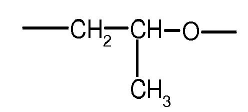 steht,
- r: für Zahlen von 1 bis 10 steht und
- s: für Zahlen von 1 bis 10 steht.

Eine weitere bevorzugte Gruppe von Penetrationsförderern sind Alkanol-alkoxylate der Formel

R-O-(-EO-)ₚ-(-BO-)_{q}-R' (IV'-d)

in welcher
- R und R^{'}: die oben angegebenen Bedeutungen haben,
- EO: für CH₂-CH₂-O- steht,
- BO: für 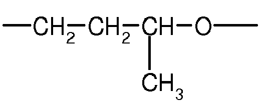 steht,
- p: für Zahlen von 1 bis 10 steht und
- q: für Zahlen von 1 bis 10 steht.

Eine weitere bevorzugte Gruppe von Penetrationsförderern sind Alkanol-alkoxylate der Formel

R-O-(-BO-)ᵣ-(-EO-)ₛ-R' (IV'-e)

in welcher
- R und R: die oben angegebenen Bedeutungen haben,
- BO: für 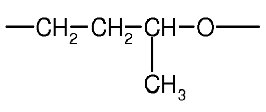 steht,
- EO: für CH₂-CH₂-O- steht,
- r: für Zahlen von 1 bis 10 steht und
- s: für Zahlen von 1 bis 10 steht.

Eine weitere bevorzugte Gruppe von Penetrationsförderern sind Alkanol-Alkoxylate der Formel

CH₃-(CH₂)ₜ-CH₂-O-(-CH₂-CH₂-O-)ᵤ-R' (IV'-f)

in welcher
- R': die oben angegebene Bedeutung hat,
- t: für Zahlen von 8 bis 13 steht
- u: für Zahlen von 6 bis 17 steht.

In den zuvor angegebenen Formeln steht
- R: vorzugsweise für Butyl, i-Butyl, n-Pentyl, i-Pentyl, Neopentyl, n-Hexyl, i-Hexyl, n-Octyl, i-Octyl, 2-Ethyl-hexyl, Nonyl, i-Nonyl, Decyl, n-Dodecyl, i-Dodecyl, Lauryl, Myristyl, i-Tridecyl, Trimethyl-nonyl, Palmityl, Stearyl oder Eicosyl.

Als Beispiel für ein Alkanol-Alkoxylat der Formel (IV-c) sei 2-Ethyl-hexyl-alkoxylat der Formel 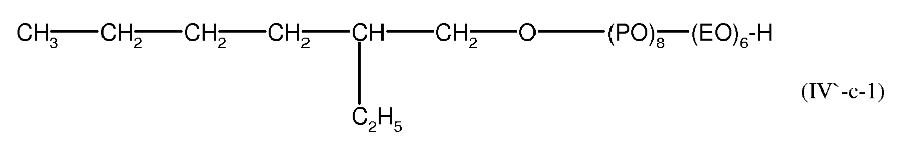 in welcher
- EO: für -CH₂-CH₂-O- steht,
- PO: für 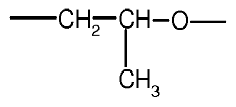 steht und
die Zahlen 8 und 6 Durchschnittswerte darstellen, genannt.

Als Beispiel für ein Alkanol-Alkoxylat der Formel (IV-d) sei die Formel

CH₃-(CH₂)₁₀-O-(-EO-)₆-(-BO-)₂-CH₃ (IV'-d-1)

in welcher
- EO: für CH₂-CH₂-O- steht,
- BO: für 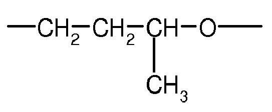 steht und
die Zahlen 10, 6 und 2 Durchschnittswerte darstellen, genannt.

Besonders bevorzugte Alkanol-Alkoxylate der Formel (IV'-f) sind Verbindungen dieser Formel, in denen
- t: für Zahlen von 9 bis 12 und
- u: für Zahlen von 7 bis 9
steht.

Ganz besonders bevorzugt genannt sei Alkanol-Alkoxylat der Formel (IV'-f-1)

CH₃-(CH₂)ₜ-CH₂-O-(-CH₂-CH₂-O-)ᵤ-H (IV'-f-1)

in welcher
- t: für den Durchschnittswert 10,5 steht und
- u: für den Durchschnittswert 8,4 steht.

Die Alkanol-Alkoxylate sind durch die obigen Formeln allgemein definiert. Bei diesen Substanzen handelt es sich um Gemische von Stoffen des angegebenen Typs mit unterschiedlichen Kettenlängen. Für die Indices errechnen sich deshalb Durchschnittswerte, die auch von ganzen Zahlen abweichen können.

Die Alkanol-Alkoxylate der angegebenen Formeln sind bekannt und sind teilweise kommerziell erhältlich oder lassen sich nach bekannten Methoden herstellen (vgl. WO 98/35 553, WO 00/35 278 und EP-A 0 681 865).

Als Penetrationsförderer kommen beispielsweise auch Substanzen in Betracht, die die Verfügbarkeit der Verbindungen der Formel (I) im Spritzbelag fördern. Dazu gehören beispielsweise mineralische oder vegetabile Öle. Als Öle kommen alle üblicherweise in agrochemischen Mitteln einsetzbaren mineralischen oder vegetabilen - gegebenenfalls modifizierte - Öle in Frage. Beispielhaft genannt seien Sonnenblumenöl, Rapsöl, Olivenöl, Rizinusöl, Rüböl, Maiskernöl, Baumwollsaatöl und Sojabohnenöl oder die Ester der genannten Öle. Bevorzugt sind Rapsöl, Sonnenblumenöl und deren Methyl- oder Ethylester.

Die Konzentration an Penetrationsförderer kann in den erfindungsgemäßen Mitteln in einem weiten Bereich variiert werden. Bei einem formulierten Pflanzenschutzmittel liegt sie im allgemeinen bei 1 bis 95 Gew.-%, bevorzugt bei 1 bis 55 Gew.-%, besonders bevorzugt bei 15 - 40 Gew.-%. In den anwendungsfertigen Mitteln (Spritzbrühen) liegen die Konzentration im allgemeinen zwischen 0,1 und 10 g/l, bevorzugt zwischen 0,5 und 5 g/l.

Erfindungsgemäße Pflanzenschutzmittel können auch weitere Komponente, beispielsweise Tenside bzw. Dispergierhilfsmittel oder Emulgatoren enthalten.

Als nicht-ionische Tenside bzw. Dispergierhilfsmittel kommen alle üblicherweise in agrochemischen Mitteln einsetzbaren Stoffe dieses Typs in Betracht. Vorzugsweise genannt seien Polyethylenoxid-polypropylenoxid-Blockcopolymere, Polyethylenglykolether von linearen Alkoholen, Umsetzungsprodukte von Fettsäuren mit Ethylenoxid und/oder Propylenoxid, ferner Polyvinylalkohol, Polyvinylpyrrolidon, Mischpolymerisate aus Polyvinylalkohol und Polyvinylpyrrolidon sowie Copolymerisate aus (Meth)acrylsäure und (Meth)acrylsäureestern, weiterhin Alkylethoxylate und Alkylarylethoxylate, die gegebenenfalls phosphatiert und gegebenenfalls mit Basen neutralisiert sein können, wobei Sorbitolethoxylate beispielhaft genannt seien, sowie Polyoxyalkylenamin-Derivate.

Als anionische Tenside kommen alle üblicherweise in agrochemischen Mitteln einsetzbaren Substanzen dieses Typs in Frage. Bevorzugt sind Alkalimetall- und Erdalkalimetall-Salze von Alkylsulfonsäuren oder Alkylarylsulfonsäuren.

Eine weitere bevorzugte Gruppe von anionischen Tensiden bzw. Dispergierhilfsmitteln sind in Pflanzenöl wenig lösliche Salze von Polystyrolsulfonsäuren, Salze von Polyvinylsulfonsäuren, Salze von Naphthalinsulfonsäure-Formaldehyd-Kondensationsprodukten, Salze von Kondensationsprodukten aus Naphthalinsulfonsäure, Phenolsulfonsäure und Formaldehyd sowie Salze von Ligninsulfonsäure.

Als Zusatzstoffe, die in den erfindungsgemäßen Formulierungen enthalten sein können, kommen Emulgatoren, schaumhemmende Mittel, Konservierungsmittel, Antioxydantien, Farbstoffe und inerte Füllmaterialien in Betracht.

Bevorzugte Emulgatoren sind ethoxylierte Nonylphenole, Umsetzungsprodukte von Alkylphenolen mit Ethylenoxid und/oder Propylenoxid, ethoxylierte Arylalkylphenole, weiterhin ethoxylierte und propoxylierte Arylalkylphenole, sowie sulfatierte oder phosphatierte Arylalkylethoxylate bzw. -ethoxy-propoxylate, wobei Sorbitan-Derivate, wie Polyethylenoxid-Sorbitan-Fettsäureester und Sorbitan-Fettsäureester, beispielhaft genannt seien.

Verwendet man beispielsweise gemäß Verfahren (A) N-(2,6-Dimethyl-4-cyclopropyl-phenylacetyl)-1-amino-cyclohexan-carbonsäureethylester als Ausgangsstoff, so kann der Verlauf des erfindungsgemäßen Verfahrens durch folgendes Reaktionsschema wiedergegeben werden:

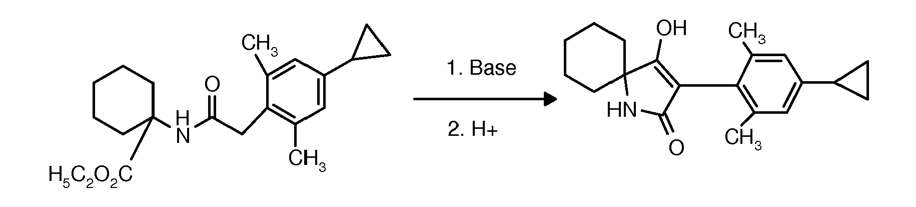

Verwendet man beispielsweise gemäß Verfahren (B) O-(2,6-Dimethyl-4-cyclopropyl-phenylacetyl)-2-hydroxyisobuttersäureethylester, so kann der Verlauf des erfindungsgemäßen Verfahrens durch folgendes Reaktionsschema wiedergegeben werden:

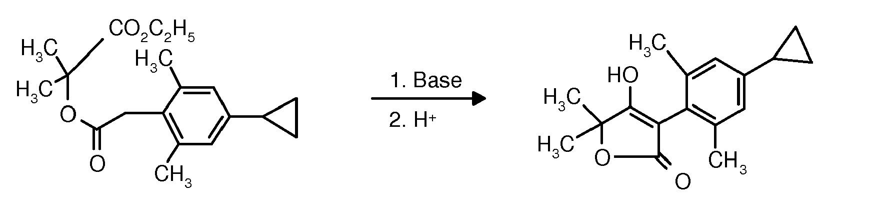

Verwendet man beispielsweise gemäß Verfahren (C) 2-(2,6-Dimethyl-4-cyclopropyl-phenyl)-4-(4-methoxy)-benzylmercapto-4-methyl-3-oxo-valeriansäure-ethylester, so kann der Verlauf des erfindungsgemäßen Verfahrens durch folgendes Reaktionsschema wiedergegeben werden:

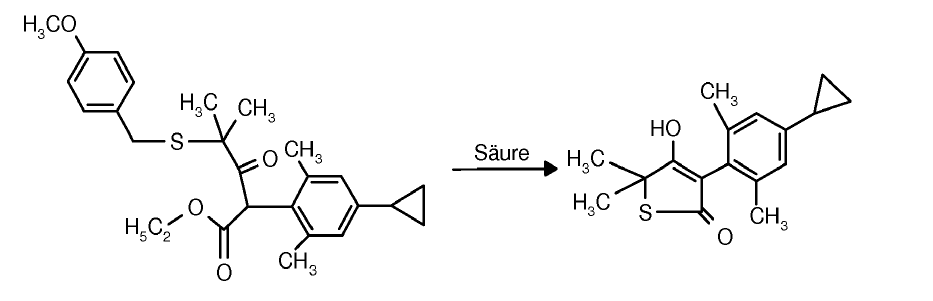

Verwendet man beispielsweise gemäß Verfahren (D) (Chlorcarbonyl)-2-[(2,6-Dimethyl-4-cyclopropyl-phenyl)]-keten und Aceton als Ausgangsverbindungen, so kann der Verlauf des erfindungsgemäßen Verfahrens durch das folgende Reaktionsschema wiedergegeben werden:

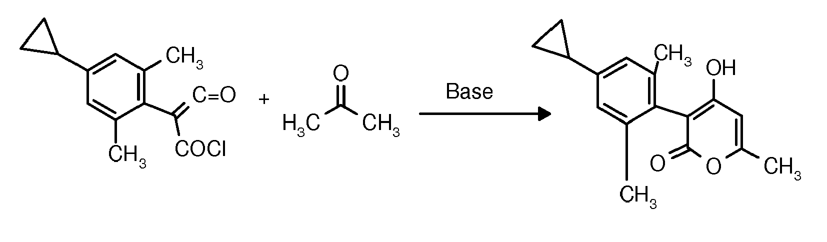

Verwendet man beispielsweise gemäß Verfahren (E) (Chlorcarbonyl)-2-(2,6-Dimethyl-4-cyclopropyl-phenyl)-keten und Thiobenzamid als Ausgangsverbindungen, so kann der Verlauf des erfindungsgemäßen Verfahrens durch das folgende Reaktionsschema wiedergegeben werden:

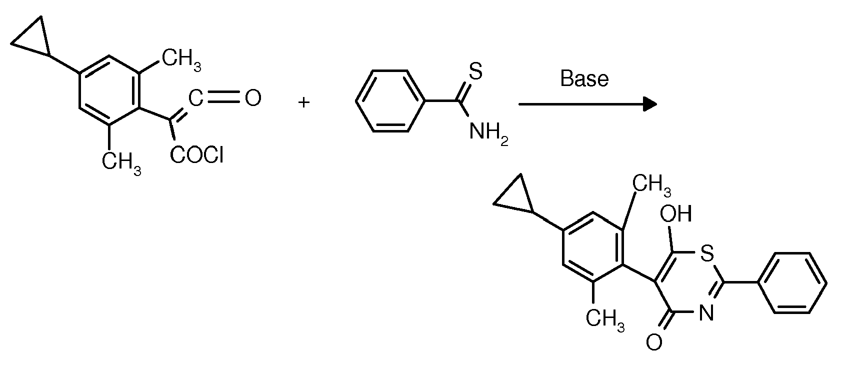

Verwendet man beispielsweise gemäß Verfahren (F) 5-(2,6-Dimethyl-4-cyclopropyl-phenyl)-2,3-trimethylen-4-oxo-valeriansäureethylester, so kann der Verlauf des erfindungsgemäßen Verfahrens durch folgendes Reaktionsschema wiedergegeben werden:

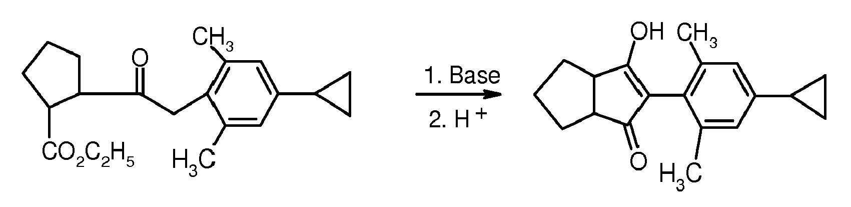

Verwendet man beispielsweise gemäß Verfahren (G) 5-[(2,6-Dimethyl-4-cyclopropyl-)-phenyl]-2-methyl-5-oxo-hexansäure-ethylester, so kann der Verlauf des erfindungsgemäßen Verfahrens durch folgendes Reaktionsschema wiedergegeben werden:

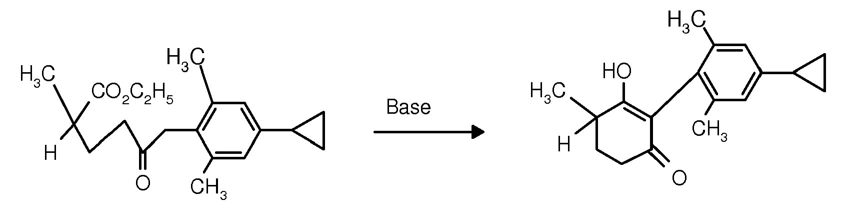

Verwendet man beispielsweise gemäß Verfahren (Hα) Hexahydropyridazin und (Chlorcarbonyl)-2-[(2,6-dimethyl-4-cyclopropyl-)-phenyl]-keten als Ausgangsverbindungen, so kann der Reaktionsverlauf des erfindungsgemäßen Verfahrens durch folgendes Reaktionsschema wiedergegeben werden:

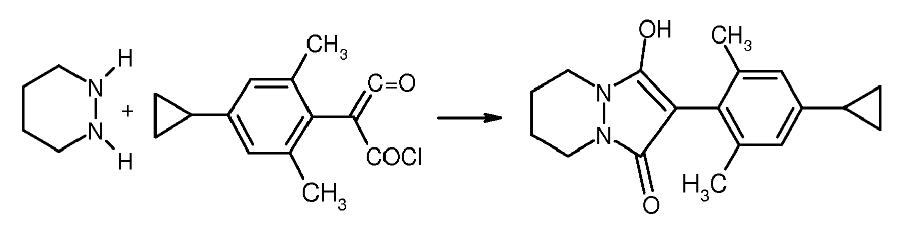

Verwendet man beispielsweise gemäß Verfahren (Hβ) Hexahydropyridazin und 2-(2,6-Dimethyl-4-cyclopropyl-)-phenylmalonsäuredimethylester als Ausgangsprodukte, so kann der Verlauf des erfindungsgemäßen Verfahrens durch folgendes Reaktionsschema wiedergegeben werden:

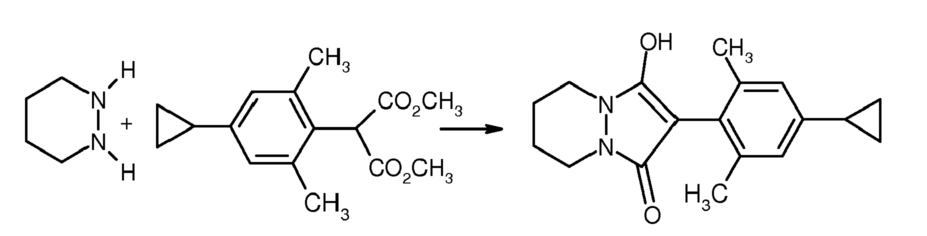

Verwendet man beispielsweise gemäß Verfahren (Hγ) 1-Ethoxycarbonyl-2-[(2,6-dimethyl-4-cyclopropyl)-phenylacetyl]-hexahydropyridazin als Ausgangsprodukt, so kann der Reaktionsverlauf durch folgendes Schema wiedergegeben werden:

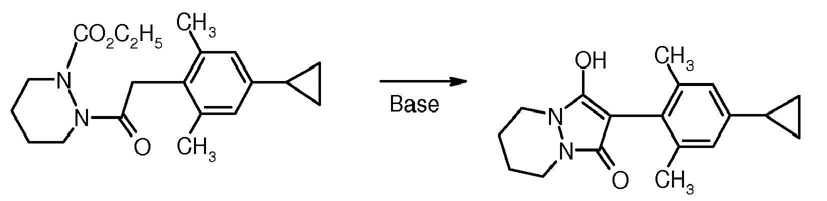

Verwendet man beispielsweise gemäß Verfahren (I) N-(2,6-Dimethyl-4-cyclopropyl-phenylacetyl)-1-amino-cyclohexan-carbonsäureethylester als Ausgangsstoff, so kann der Verlauf des erfindungsgemäßen Verfahrens durch folgendes Reaktionsschema wiedergegeben werden:

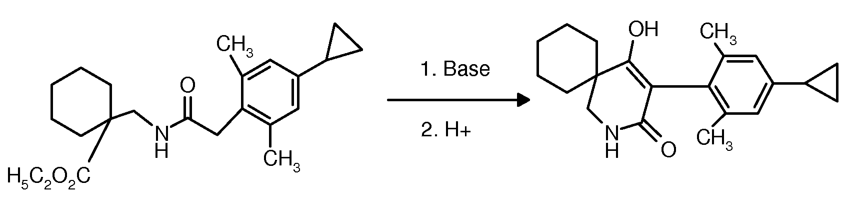

Verwendet man beispielsweise gemäß Verfahren (J) O-(2,6-Dimethyl-4-cyclopropyl-phenylacetyl)-3-hydroxy-2,2-dimethyl-propion-säureethylester, so kann der Verlauf des erfindungsgemäßen Verfahrens durch folgendes Reaktionsschema wiedergegeben werden:

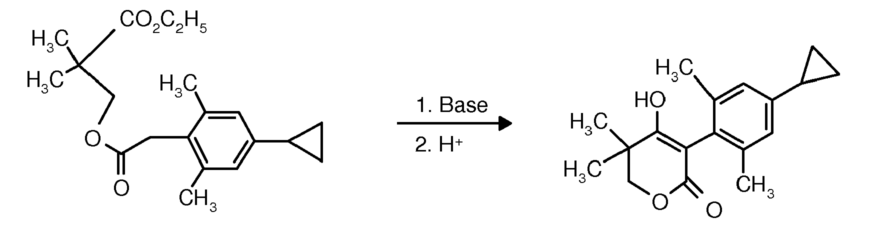

Verwendet man beispielsweise gemäß Verfahren (Kα) 3-(2-Methyl-4-cyclopropyl-6-ethyl-phenyl)-5,5-dimethylpyrrolidin-2,4-dion und Pivaloylchlorid als Ausgangsstoffe, so kann der Verlauf des erfindungsgemäßen Verfahrens durch folgendes Reaktionsschema wiedergegeben werden:

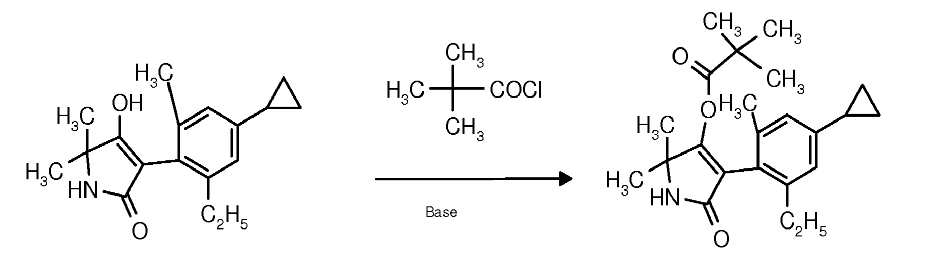

Verwendet man beispielsweise gemäß Verfahren (Kβ) 3-(2,6-Dimethyl-4-cyclopropyl-phenyl)-5,5-dimethylpyrrolidin-2,4-dion und Acetanhydrid als Ausgangsverbindungen, so kann der Verlauf des erfindungsgemäßen Verfahrens durch folgendes Reaktionsschema wiedergegeben werden:

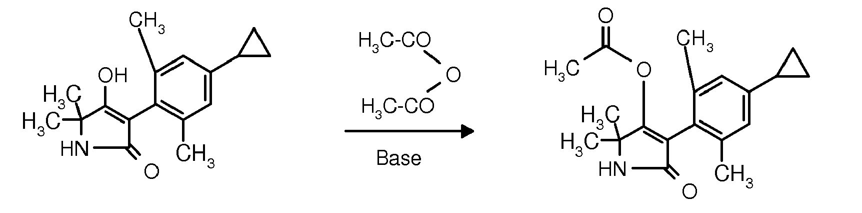

Verwendet man beispielsweise gemäß Verfahren (J) 8-[(2,6-Dimethyl-4-cyclopropyl)-phenyl]-1-aza-bicyclo-(4,3,0^{1,6})-nonan-7,9-dion und Chlorameisensäureethylester als Ausgangsverbindungen, so kann der Verlauf des erfindungsgemäßen Verfahrens durch folgendes Reaktionsschema wiedergegeben werden:

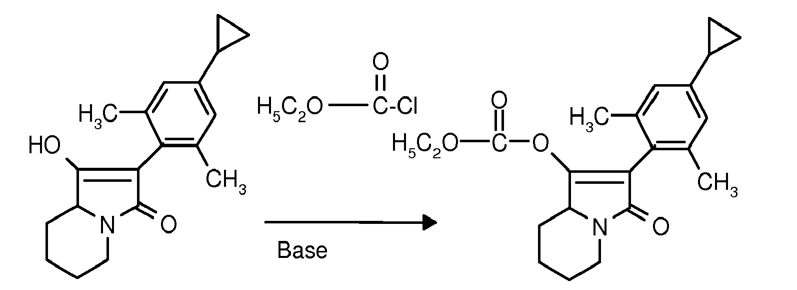

Verwendet man beispielsweise gemäß Verfahren (M), 3-(2,6-Dimethyl-4-cyclopropyl-phenyl)-4-hydroxy-5-methyl-6-(3-pyridyl)-pyron und Chlormonothioameisensäuremethylester als Ausgangsprodukte, so kann der Reaktionsverlauf folgendermaßen wiedergegeben werden:

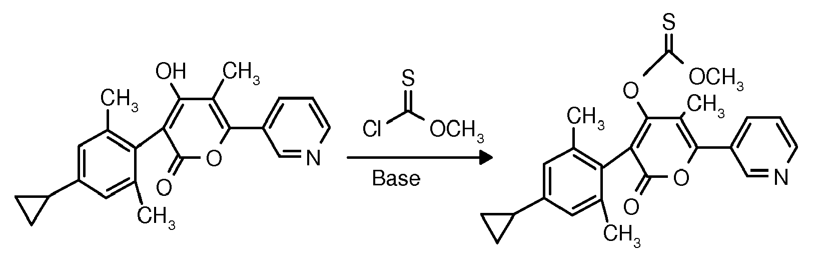

Verwendet man beispielsweise gemäß Verfahren (N) 3-(2,6-Dimethyl-4-cyclopropyl-phenyl)-5,5-pentamethylen-pyrrolidin-2,4-dion und Methansulfonsäurechlorid als Ausgangsprodukt, so kann der Reaktionsverlauf durch folgendes Reaktionsschema wiedergegeben werden:

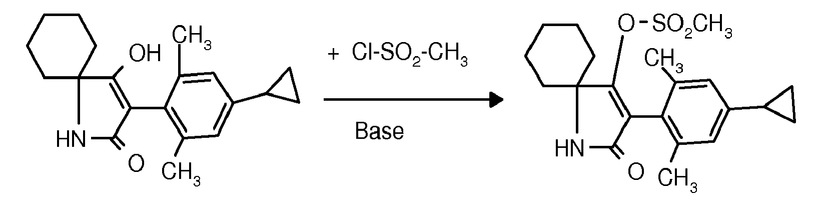

Verwendet man beispielsweise gemäß Verfahren (O) 3-(2,6-Dimethyl-4-cyclopropyl-phenyl)-4-hydroxy-5,5-dimethyl-pyrrolidin-2,4-dion und Methanthio-phosphonsäurechlorid-(2,2,2-trifluorethylester) als Ausgangsprodukte, so kann der Reaktionsverlauf durch folgendes Reaktionsschema wiedergegeben werden:

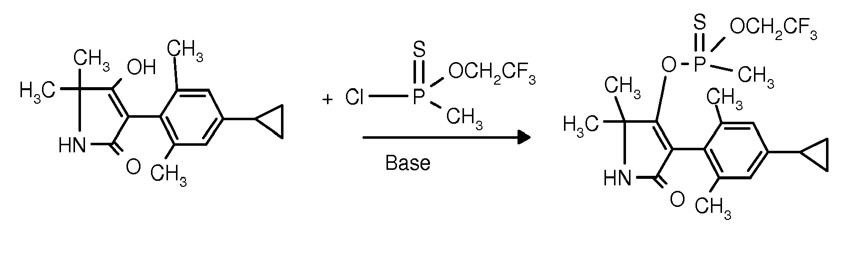

Verwendet man beispielsweise gemäß Verfahren (P) 3-(2-Ethyl-4-cyclopropyl-6-methyl-phenyl]-5-cyclopropyl-5-methyl-pyrrolidin-2,4-dion und NaOH als Komponenten, so kann der Verlauf des erfindungsgemäßen Verfahrens durch folgendes Reaktionsschema wiedergegeben werden:

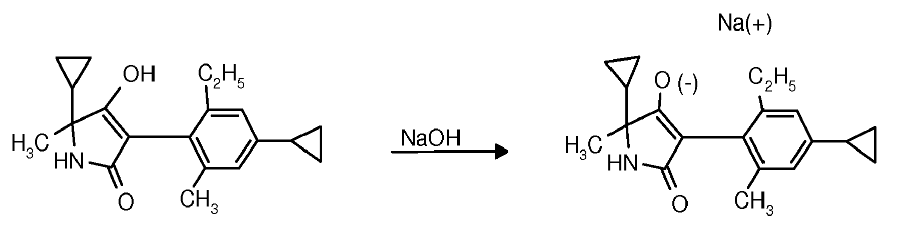

Verwendet man beispielsweise gemäß Verfahren (Q) Variante α 3-(2,6-Dimethyl-cyclopropyl-phenyl)-4-hydroxy-5,5-tetramethylen-Δ³-dihydro-furan-2-on und Ethylisocyanat als Ausgangsprodukte, so kann der Reaktionsverlauf durch folgendes Reaktionsschema wiedergegeben werden:

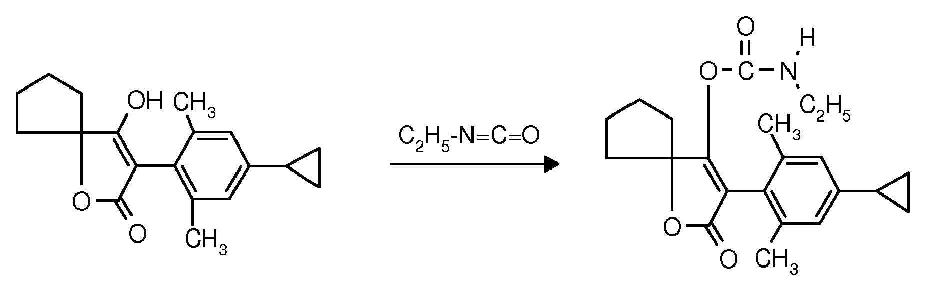

Verwendet man beispielsweise gemäß Verfahren (Q) Variante β 3-(2-Methyl-4-cyclopropyl-6-ethyl-phenyl)-5-methyl-pyrrolidin-2,4-dion und Dimethylcarbamidsäurechlorid als Ausgangsprodukte, so kann der Reaktionsverlauf durch folgendes Schema wiedergegeben werden:

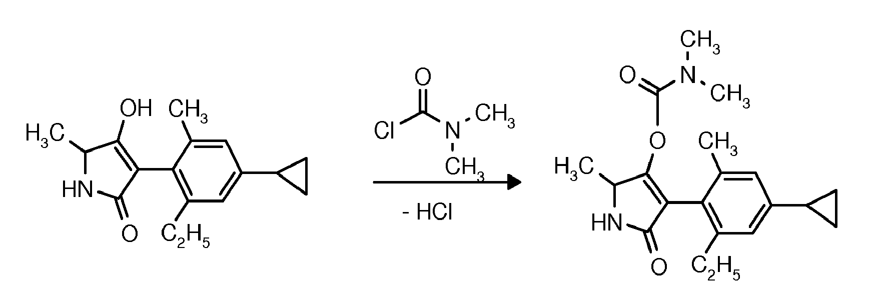

Verwendet man beispielsweise gemäß Verfahren (R) 3-(4-Brom-2,6-dimethyl-phenyl)-5,5-dimethyl-pyrrolidin-2,4-dion und Cyclopropyl-boronsäure als Ausgangsstoffe, so kann der Reaktionsverlauf durch folgendes Schema wiedergegeben werden:

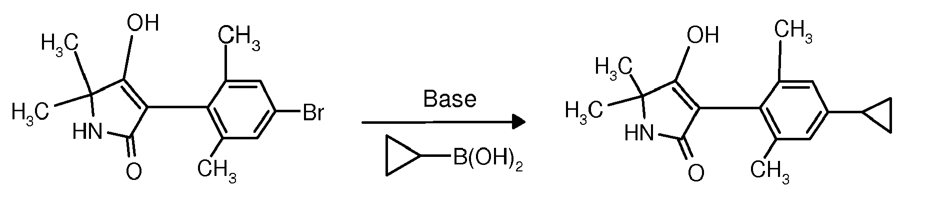

Die beim erfindungsgemäßen Verfahren (a) als Ausgangsstoffe benötigten Verbindungen der Formel (II) 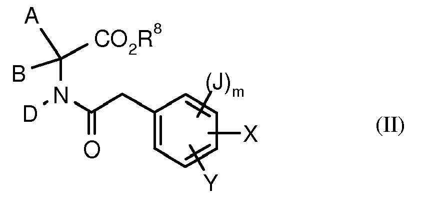 in welcher
A, B, D, J, m, X, Y und R⁸ die oben angegebenen Bedeutungen haben,
sind neu.

Man erhält die Acylaminosäureester der Formel (II) beispielsweise, wenn man Aminosäurederivate der Formel (XXVI) 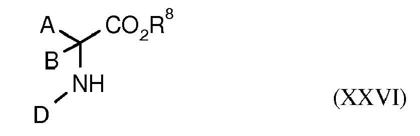 in welcher
A, B, R⁸ und D die oben angegebenen Bedeutungen haben,
mit substituierten Phenylessigsäurederivaten Formel (XXVII) 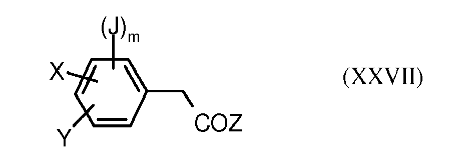 in welcher
J, m, X und Y die oben angegebenen Bedeutungen haben und
- Z: für eine durch Carbonsäureaktivierungsreagenzien wie Carbonyldiimidazol, Carbonyldiimide (wie z.B. Dicyclohexylcarbondiimid), Phosphorylierungsreagenzen (wie z.B. POCl₃, BOP-Cl), Halogenierungsmittel z.B. Thionylchlorid, Oxalylchlorid, Phosgen oder Chlorameisensäsureester eingeführte Abgangsgruppe steht,
acyliert (Chem. Reviews 52, 237-416 (1953); Bhattacharya, Indian J. Chem. 6, 341-5, 1968)
oder wenn man Acylaminosäuren der Formel (XXVIII) 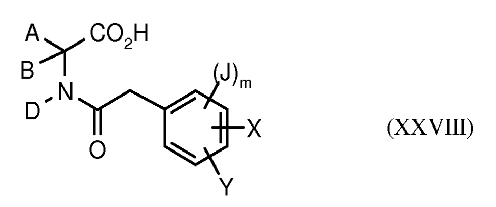 in welcher
A, B, D, J, m, X und Y die oben angegebenen Bedeutungen haben,
verestert (Chem. Ind. (London) 1568 (1968)).

Die Verbindungen der Formel (XXVIII) 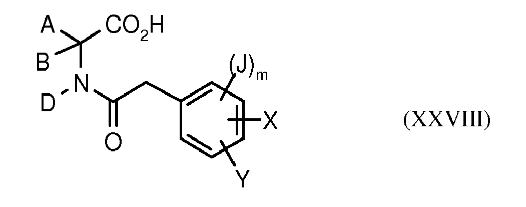 in welcher
A, B, D, J, m, X und Y die oben angegebenen Bedeutungen haben,
sind neu.

Man erhält die Verbindungen der Formel (XXVIII), wenn man Aminosäuren der Formel (XXIX) 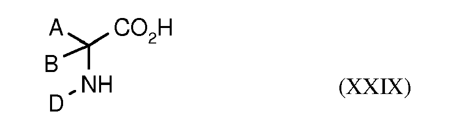 in welcher
- A, B und D: die oben angegebenen Bedeutungen haben,
mit substituierten Phenylessigsäurederivaten der Formel (XXVII) 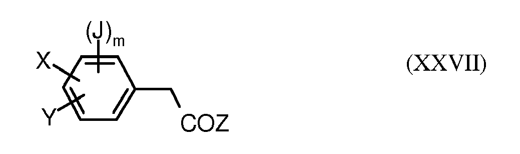 in welcher
- J, m, X und Y: die oben angegebenen Bedeutungen haben und
- Z: die oben angegebene Bedeutung hat,
beispielsweise nach Schotten-Baumann acyliert (Organikum, VEB Deutscher Verlag der Wissenschaften, Berlin 1977, S. 505).

Die Verbindungen der Formel (XXVII) sind neu. Sie lassen sich nach im Prinzip bekannten Verfahren und wie aus den Beispielen ersichtlich darstellen (s. z.B. H. Henecka, Houben-Weyl, Methoden der Organischen Chemie, Bd. 8, S. 467-469 (1952)).

Man erhält die Verbindungen der Formel (XXVII) beispielsweise, indem man substituierte Phenylessigsäuren der Formel (XXX) 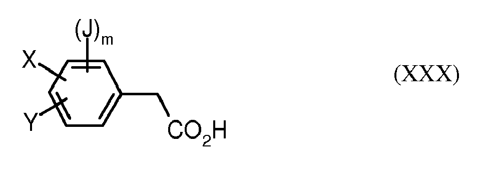 in welcher
- J, m, X und Y: die oben angegebene Bedeutung haben,
mit Halogenierungsmitteln (z.B. Thionylchlorid, Thionylbromid, Oxalylchlorid, Phosgen, Phosphortrichlorid, Phosphortribromid oder Phosphorpentachlorid), Phosphonylierungsreagenzien wie (z.B. POCl₃, BOP-Cl), Carbonyldiimidazol, Carbonyldiimide (z.B. Dicyclohexylcarbonyldiimid) gegebenenfalls in Gegenwart eines Verdünnungsmittels (z.B. gegebenenfalls chlorierten aliphatischen oder aromatischen Kohlenwasserstoffen wie Toluol oder Methylenchlorid oder Ethern, z.B. Tetrahydrofuran, Dioxan, Methyl-tert.-butylether) bei Temperaturen von -20°C bis 150°C, bevorzugt von -10°C bis 100°C, umsetzt.

Die Verbindungen der Formel (XXVI) und (XXIX) sind teilweise aus der eingangs zitierten Patentliteratur bekannt und/oder lassen sich nach bekannten Verfahren darstellen (siehe z.B. Compagnon, Miocque Ann. Chim. (Paris) [14] 5, S. 11-22, 23-27 (1970)).

Die substituierten cyclischen Aminocarbonsäuren der Formel (XXIX), in der A und B einen Ring bilden, sind im Allgemeinen nach der Bucherer-Bergs-Synthese oder nach der Strecker-Synthese erhältlich und fallen dabei jeweils in unterschiedlichen Isomerenformen an. So erhält man unter den Bedingungen der Bucherer-Bergs-Synthese vorwiegend die Isomeren (im folgenden der Einfachheit halber als β bezeichnet), in welchen die Reste R und die Carboxylgruppe äquatorial stehen, während nach den Bedingungen der Strecker-Synthese vorwiegend die Isomeren (im folgenden der Einfachheit halber als α bezeichnet) anfallen, bei denen die Aminogruppe und die Reste R äquatorial stehen.

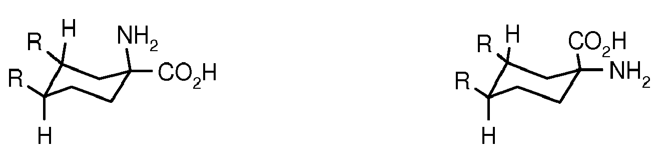

| | |
|---|---|
| Bucherer-Bergs-Synthese | Strecker-Synthese |
| (β-Isomeres) | (α-Isomeres) |

(L. Munday, J. Chem. Soc. 4372 (1961); J.T. Eward, C. Jitrangeri, Can. J. Chem. 53, 3339 (1975).

Weiterhin lassen sich die bei dem obigen Verfahren (A) verwendeten Ausgangsstoffe der Formel (II) 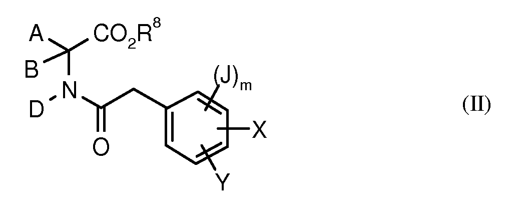 in welcher
- A, B, D, J, m, X, Y und R⁸: die oben angegebenen Bedeutungen haben,
herstellen, wenn man Aminonitrile der Formel (XXXI) 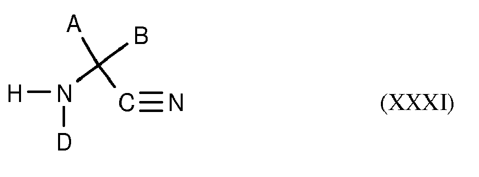 in welcher
- A, B und D: die oben angegebenen Bedeutungen haben,
mit substituierten Phenylessigsäurederivaten der Formel (XXVII) 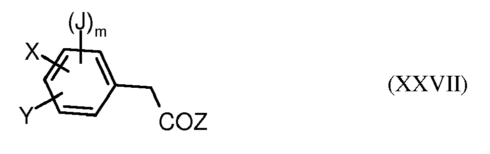 in welcher
- J, m, X, Y und Z: die oben angegebenen Bedeutungen haben,
zu Verbindungen der Formel (XXXII) 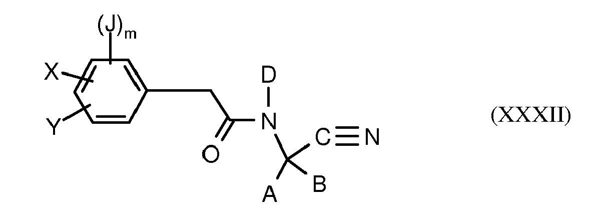 in welcher
A, B, D, J, m, X und Y die oben angegebenen Bedeutungen haben,
umsetzt,
und diese anschließend einer sauren Alkoholyse unterwirft.

Die Verbindungen der Formel (XXXII) sind ebenfalls neu.

Die bei dem erfindungsgemäßen Verfahren (B) als Ausgangstoffe benötigten Verbindungen der Formel (III) 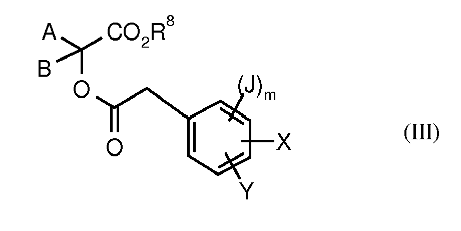 in welcher
A, B, J, m, X, Y und R⁸die oben angegebenen Bedeutungen haben,
sind neu.

Sie lassen sich nach im Prinzip bekannten Methoden herstellen.

So erhält man die Verbindungen der Formel (III) beispielsweise, wenn man 2-Hydroxycarbonsäureester der Formel (XXXIII-A) 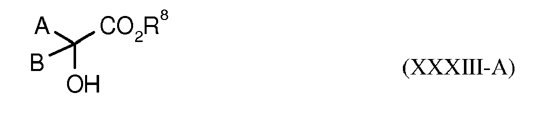 in welcher
- A, B und R⁸: die oben angegebenen Bedeutungen haben,
mit substituierten Phenylessigsäurederivaten der Formel (XXVII) 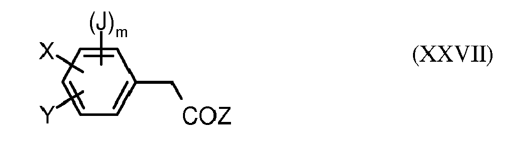 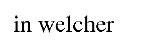
- J, m, X, Y und Z: die oben angegebenen Bedeutungen haben,
acyliert (Chem. Reviews 52, 237-416 (1953)).

Weiterhin erhält man Verbindungen der Formel (III), wenn man
substituierte Phenylessigsäuren der Formel (XXX) 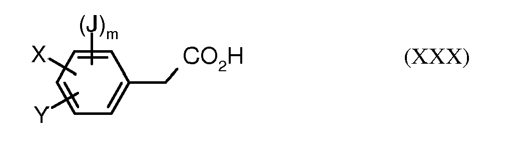 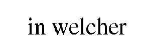
- J, m, X und Y: die oben angegebenen Bedeutungen haben,
mit α-Halogencarbonsäureestern der Formel (XXXIII-B) 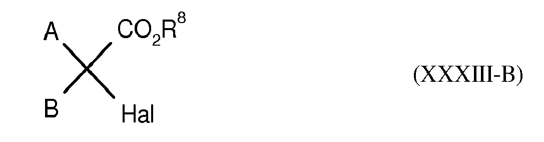 in welcher
- A, B und R⁸: die oben angegebenen Bedeutungen haben und
- Hal: für Chlor oder Brom steht,
alkyliert.

Die Verbindungen der Formel (XXXIII) sind teilweise käuflich, teilweise bekannt, aber auch teilweise neu.

Die Verbindungen der Formel (XXXIII-B) sind käuflich.

Die Verbindungen der Formel (XXX) sind neu.

Beispielsweise erhält man die Verbindungen der Formel (XXX), 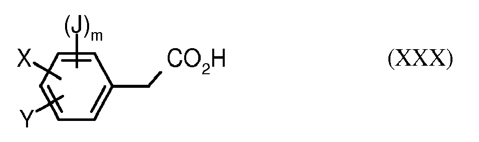 in welcher
- J, m, X und Y: die oben angegebenen Bedeutungen haben,
wenn man Phenylessigsäureester der Formel (XXXIV) 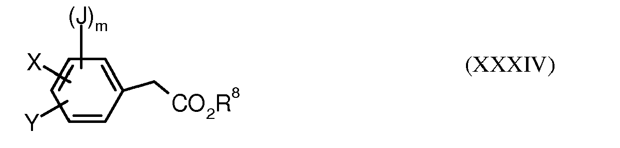 in welcher
- J, m, X, Y und R⁸: die oben angegebene Bedeutung haben,
in Gegenwart von Säuren oder Basen, in Gegenwart eines Lösungsmittels unter allgemein bekannten Standardbedingungen verseift.

Die Verbindungen der Formel (XXXIV) sind neu.

Die Verbindungen der Formel (XXXIV) 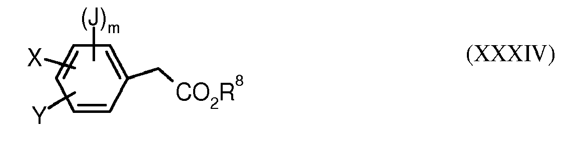 in welcher
- J, m, X, Y und R⁸: die oben angegebene Bedeutung haben,
erhält man nach dem in den Beispielen beschriebenen Verfahren analog dem Verfahren (R),
wenn man Phenylessigsäureester der Formel (XXXIV-a) 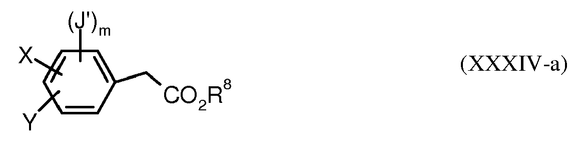 in welcher
- R⁸, m, X und Y: die oben angegebene Bedeutung haben
und J' für Brom oder Iod steht,
in Gegenwart von kupplungsfähigen Cycloalkylboronsäurederivaten z.B. Cyclopropan-boronsäure in Gegenwart einer Base und gegebenenfalls in Gegenwart eines Katalysators (bevorzugt Palladiumsalze und einen Komplexbildner wie z.B. Palladiumacetat/Tricyclohexylphosphin) umsetzt.

Die Phenylessigsäureester der Formel (XXXIV-a) sind prinzipiell beispielsweise aus den Offenlegungen WO 96/35 664, WO 97/02243, WO 97/01535, WO 98/05638 und DE-A-10 301 804 bekannt und lassen sich nach den dort beschriebenen Verfahren herstellen.

Die bei dem obigen Verfahren (C) als Ausgangsstoffe benötigten Verbindungen der Formel (IV) 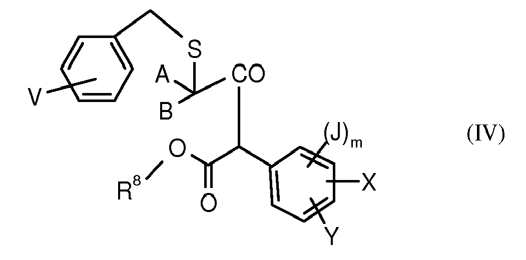 in welcher
- A, B, J, m, V, X, Y und R⁸: die oben angegebenen Bedeutungen haben,
sind neu.

Sie lassen sich nach im Prinzip bekannten Methoden herstellen.

Man erhält die Verbindungen der Formel (IV) beispielsweise, wenn man
substituierte Phenylessigsäureester der Formel (XXXIV) 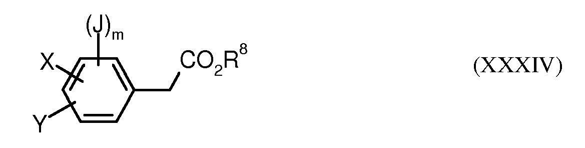 in welcher
- J, m, X, Y und R⁸: die oben angegebenen Bedeutungen haben,
mit 2-Benzylthio-carbonsäurehalogeniden der Formel (XXXV) 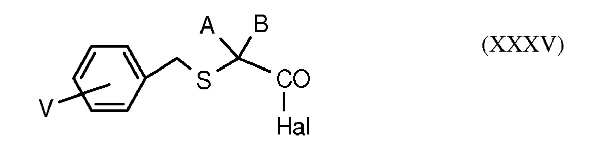 in welcher
- A, B und V: die oben angegebenen Bedeutungen haben und
- Hal: für Halogen (insbesondere Chlor oder Brom) steht,
in Gegenwart von starken Basen acyliert (siehe z.B. M.S. Chambers, E.J. Thomas, D.J. Williams, J. Chem. Soc. Chem. Commun., (1987), 1228).

Die Benzylthio-carbonsäurehalogenide der Formel (XXXV) sind teilweise bekannt und/oder lassen sich nach bekannten Verfahren herstellen (J. Antibiotics (1983), 26, 1589).

Die bei den obigen Verfahren (D), (E) und (H-α) als Ausgangsstoffe benötigten Halogencarbonylketene der Formel (VI) sind neu. Sie lassen sich nach im Prinzip bekannten Methoden herstellen (vgl. beispielsweise Org. Prep. Proced. Int., 7, (4), 155-158, 1975 und DE 1 945 703). So erhält man z.B. die Verbindungen der Formel (VI) 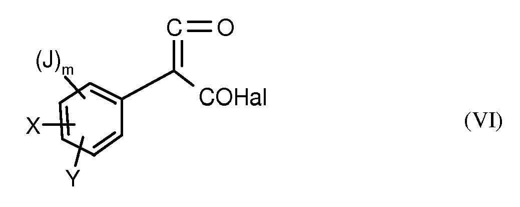 in welcher
- J, m, X und Y: die oben angegebenen Bedeutungen haben und
- Hal: für Chlor oder Brom steht,
wenn man
substituierte Phenylmalonsäuren der Formel (XXXVI) 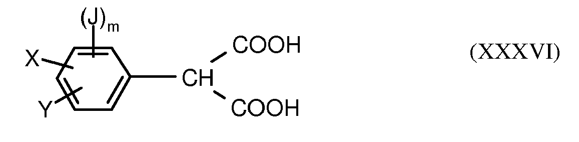 in welcher
- J, m, X und Y: die oben angegebenen Bedeutungen haben,
mit Säurehalogeniden, wie beispielsweise Thionylchlorid, Phosphor(V)chlorid, Phosphor(III)-chlorid, Oxalylchlorid, Phosgen oder Thionylbromid gegebenenfalls in Gegenwart von Katalysatoren, wie beispielsweise Dimethylformamid, Methyl-Sterylformamid oder Triphenylphosphin und gegebenenfalls in Gegenwart von Basen wie z.B. Pyridin oder Triethylamin, umsetzt.

Die substituierten Phenylmalonsäuren der Formel (XXXVI) sind neu. Sie lassen sich in einfacher Weise nach bekannten Verfahren herstellen (vgl. z.B. Organikum, VEB Deutscher Verlag der Wissenschaften, Berlin 1977, S. 517 ff, EP-A-528 156, WO 96/35 664, WO 97/02 243, WO 97/01535, WO 97/36868 und WO 98/05638).

So erhält man Phenylmalonsäuren der Formel (XXXVI) 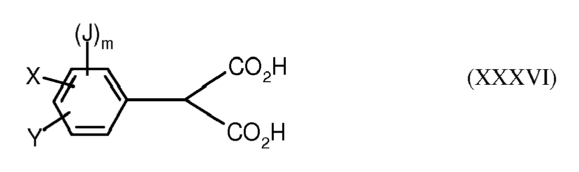 in welcher
- J, m, X und Y: die oben angegebenen Bedeutungen haben,
wenn man Phenylmalonsäureester der Formel (XI) 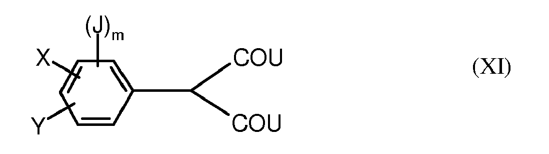 in welcher
- J, m, X und Y: die oben angegebene Bedeutung haben,
- und U: für OR⁸ steht,
wobei R⁸ die oben angegebene Bedeutung hat,
zunächst in Gegenwart einer Base und einem Lösungsmittel verseift und anschließend vorsichtig ansäuert (s. beispielsweise EP-A-528 156, WO 96/35 664, WO 97/02 243).

Die Malonsäureester der Formel (XI) 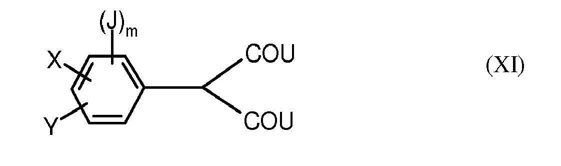 in welcher
- J, m, X und Y: die oben angegebene Bedeutung haben,
- und U: für OR⁸ steht,
wobei R⁸ die oben angegebene Bedeutung hat
sind neu.

Sie lassen sich nach allgemein bekannten Methoden der Organischen Chemie darstellen (vgl. z.B. Tetrahedron Lett. 27, 2763 (1986), Organikum VEB Deutscher Verlag der Wissenschaften, Berlin 1977, S. 587 ff., WO 96/35664, WO 97/02243, WO 97/01535, WO 97/36868, WO 98/05638 und WO 99/47525).

Die für das erfindungsgemäße Verfahren (D) als Ausgangsstoffe benötigten Carbonylverbindungen der Formel (V) 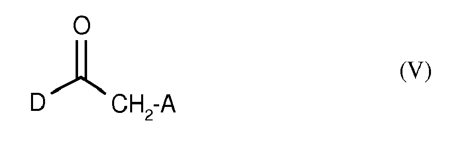 in welcher
- A und D: die oben angegebenen Bedeutungen haben,
oder deren Silylenolether der Formel (Va) 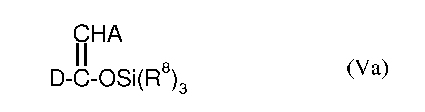 in welcher
- A, D und R⁸: die oben angegebenen Bedeutungen haben,
sind käufliche, allgemeine bekannte oder nach bekannten Verfahren zugängliche Verbindungen.

Die Herstellung der zur Durchführung des erfindungsgemäßen Verfahrens (E) als Ausgangsstoffe benötigten Ketensäurechloride der Formel (VI) wurden bereits oben beschrieben. Die zur Durchführung des erfindungsgemäßen Verfahrens (E) benötigten Thioamide der Formel (VII) 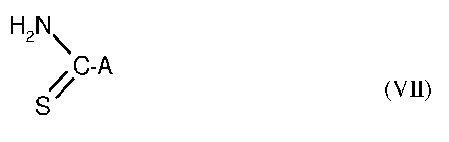 in welcher
- A: die oben angegebene Bedeutung hat,
sind allgemein in der Organischen Chemie bekannte Verbindungen.

Die bei dem obigen Verfahren (F) als Ausgangsstoffe benötigten Verbindungen der Formel (VIII) 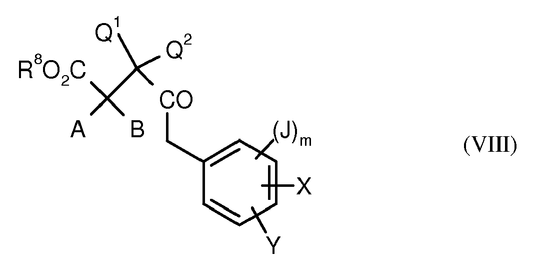 in welcher
- A, B, J, m, Q¹, Q², X, Y und R⁸: die oben angegebene Bedeutung haben,
sind neu.
Sie lassen sich nach im Prinzip bekannten Methoden herstellen.

Man erhält die 5-Aryl-4-ketocarbonsäureester der Formel (VIII) beispielsweise, wenn man 5-Aryl-4-ketocarbonsäuren der Formel (XXXVII) 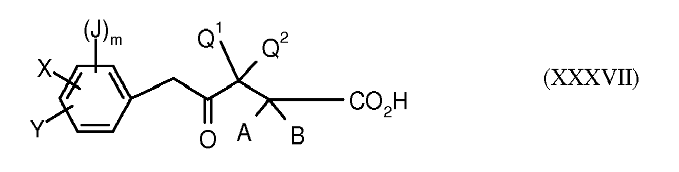 in welcher
- J, m, X, Y, A, B, Q¹ und Q²: die oben angegebene Bedeutung haben,
verestert (vgl. z.B. Organikum, 15. Auflage, Berlin, 1977, Seite 499) oder alkyliert (siehe Herstellungsbeispiel).

Die 5-Aryl-4-ketocarbonsäuren der Formel (XXXVII) 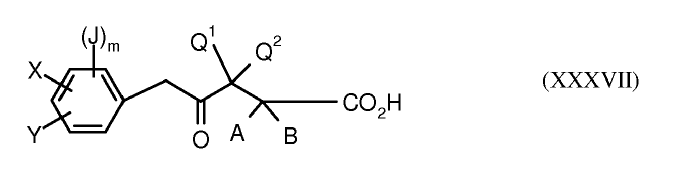 in welcher
A, B, J, m, Q¹, Q², X und Y die oben angegebene Bedeutung haben,
sind neu, lassen sich aber nach im Prinzip bekannten Methoden herstellen (WO 96/01 798, WO 97/14667, WO 98/39281).

Man erhält die 5-Aryl-4-ketocarbonsäuren der Formel (XXXVII) beispielsweise, wenn man 2-Phenyl-3-oxo-adipinsäureester der Formel (XXXVIII) 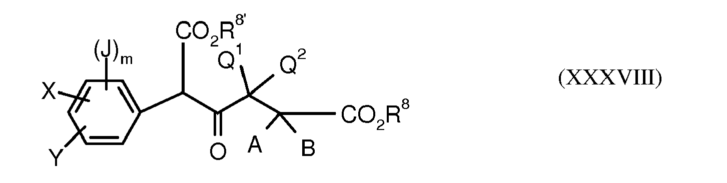 in welcher
- A, B, J, m, Q¹, Q², X und Y: die oben angegebene Bedeutung haben und
- R⁸ und R⁸': für Alkyl (insbesondere C₁-C₈-Alkyl) stehen und
bei Einsatz der Verbindung der Formel (XL-a) R⁸ für Wasserstoff steht,
gegebenenfalls in Gegenwart eines Verdünnungsmittels und gegebenenfalls in Gegenwart einer Base oder Säure decarboxyliert (vgl. z.B. Organikum, 15. Auflage, Berlin, 1977, Seite 519 bis 521, WO 96/01798, WO 97/14667, WO 98/39281).

Die Verbindungen der Formel (XXXVIII) 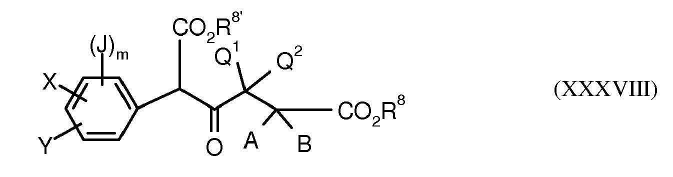 in welcher
- A, B, J, m, Q¹, Q², X, Y, R⁸, R⁸': die oben angegebene Bedeutung haben und
bei Einsatz der Verbindung der Formel (XL-a) R⁸ für Wasserstoff steht
sind neu.

Man erhält die Verbindungen der Formel (XXXVIII) beispielsweise,
wenn man Dicarbonsäurehalbesterchloride der Formel (XXXIX), 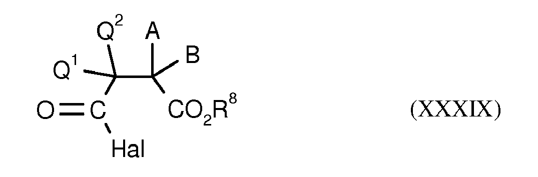 in welcher
- A, B, Q¹, Q² und R⁸: die oben angegebene Bedeutung haben und
- Hal: für Chlor oder Brom steht,
oder Carbonsäureanhydride der Formel (XL-a) 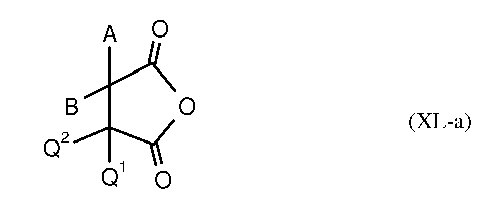 in welcher
A, B, Q¹ und Q² die oben angegebene Bedeutung haben,
mit einem Phenylessigsäureester der Formel (XXXIV) 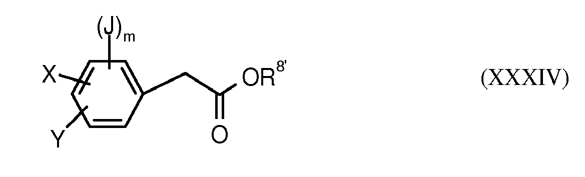 in welcher
- J, m, X, Y und R⁸': die oben angegebene Bedeutung haben,
in Gegenwart eines Verdünnungsmittels und in Gegenwart einer Base acyliert (vgl. z.B. M.S. Chambers, E. J. Thomas, D.J. Williams, J. Chem. Soc. Chem. Commun., (1987), 1228, vgl. auch die Herstellungsbeispiele).

Die Verbindungen der Formeln (XXXIX) und (XL-a) sind teilweise bekannte Verbindungen der Organischen Chemie und/oder lassen sich nach im Prinzip bekannten Methoden in einfacher Weise herstellen.

Die bei dem obigen Verfahren (G) als Ausgangsstoffe benötigten Verbindungen der Formel (IX) 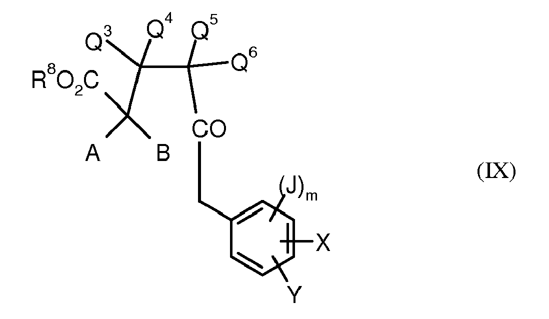 in welcher
- A, B, J, m, Q³, Q⁴, Q⁵, Q⁶ X, Y und R⁸: die oben angegebene Bedeutung haben,
sind neu.

Sie lassen sich nach im Prinzip bekannten Methoden herstellen.

Man erhält die 6-Aryl-5-ketocarbonsäureester der Formel (IX) beispielsweise, wenn man 6-Aryl-5-ketocarbonsäuren der Formel (XLI) 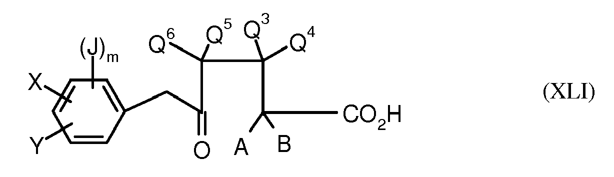 in welcher
- A, B, J, m, Q³, Q⁴, Q⁵, Q⁶ X und Y: die oben angegebene Bedeutung haben,
verestert, (vgl. z.B. Organikum, 15. Auflage, Berlin, 1977, Seite 499, WO 99/43649, WO 99/48869).

Die 6-Aryl-5-ketocarbonsäuren der Formel (XLI) 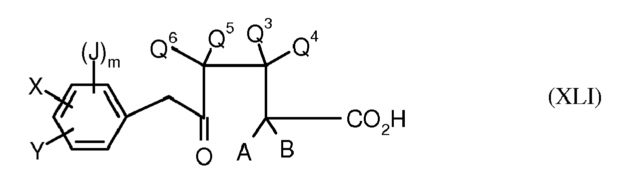 in welcher
- A, B, J, m, Q³, Q⁴, Q⁵, Q⁶ X und Y: die oben angegebene Bedeutung haben,
sind neu. Sie lassen sich nach im Prinzip bekannten Methoden herstellen (WO 99/43649, WO 99/48869), beispielsweise wenn man
substituierte 2-Phenyl-3-oxo-heptandisäureester der Formel (XLII) 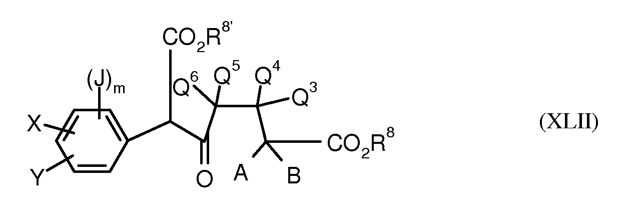 in welcher
- A, B, J, m, Q³, Q⁴, Q⁵, Q⁶ X und Y: die oben angegebene Bedeutung haben und
- R⁸ und R⁸': für Alkyl (bevorzugt C₁-C₆-Alkyl), stehen, und
bei Einsatz der Verbindung der Formel (XL-b) R⁸ für Wasserstoff steht,
gegebenenfalls in Gegenwart eines Verdünnungsmittels und gegebenenfalls in Gegenwart einer Base oder Säure verseift und decarboxyliert (vgl. z.B. Organikum, 15. Auflage, Berlin, 1977, Seite 519 bis 521, WO 99/43649, WO 99/48869).

Die Verbindungen der Formel (XLII) 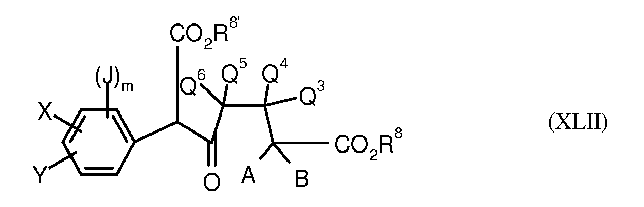 in welcher
A, B, J, m, Q³, Q⁴, Q⁵, Q⁶ X, Y, R⁸ und R⁸' die oben angegebene Bedeutung haben,
sind neu und erhältlich,
wenn man Dicarbonsäureester der Formel (XLIII), 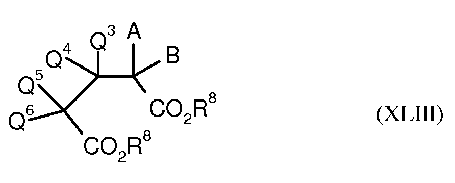 in welcher
- A B, Q³ Q⁴ Q⁵, Q⁶ und R⁸: die oben angegebene Bedeutung haben,
oder Carbonsäureanhydride der Formel (XL-b) 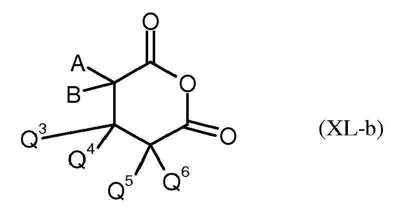
- in welcher A, B, Q³, Q⁴, Q⁵, Q⁶: die oben angegebene Bedeutung haben
mit einem substituierten Phenylessigsäureester der Formel (XXXIV)
in welcher
- J, m, X, Y und R⁸': die oben angegebene Bedeutung haben,
in Gegenwart eines Verdünnungsmittels und in Gegenwart einer Base kondensiert.

Die Verbindungen der Formel (XLIII) sind teilweise bekannt und/oder lassen sich nach bekannten Verfahren darstellen.

Die für das erfindungsgemäße Verfahren (H-α) und (H-β) als Ausgangsstoffe benötigten Hydrazine der Formel (X)

A-NH-NH-D (X)

in welcher
- A und D: die oben angegebenen Bedeutungen haben,
sind teilweise bekannt und/oder nach literaturbekannten Methoden herstellbar (vgl. beispielsweise Liebigs Ann. Chem. 585, 6 (1954); Reaktionen der organischen Synthese, C. Ferri, Seite 212, 513; Georg Thieme Verlag Stuttgart, 1978; Liebigs Ann. Chem. 443, 242 (1925); Chem. Ber. 98, 2551 (1965), EP-A-508 126, WO 92/16510, WO 99/47 525, WO 01/17 972).

Die für das erfindungsgemäße Verfahren (H-γ) benötigten Verbindungen der Formel (XII) 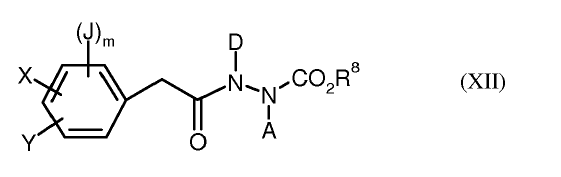 in welcher
- A, D, J, m, X, Y und R⁸: die oben angegebene Bedeutung haben,
sind neu.

Man erhält die Acylcarbazate der Formel (XII) beispielsweise, wenn man Carbazate der Formel (XLIV) 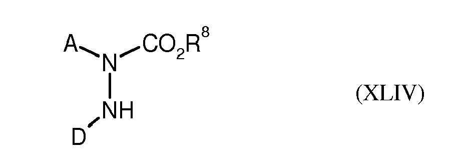 in welcher
- A, R⁸ und D: die oben angegebenen Bedeutungen haben,
mit substituierten Phenylessigsäurederivaten der Formel (XXVII) 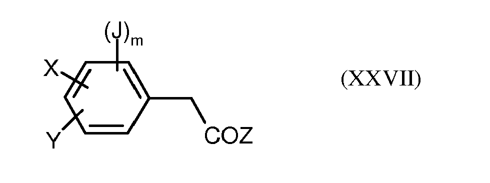 in welcher
- J, m, X, Y und Z: die oben angegebenen Bedeutungen haben
acyliert (Chem. Reviews 52, 237-416 (1953); Bhattacharya, Indian J. Chem. 6, 341-5, 1968).

Die Carbazate der Formel (XLIV) sind teilweise käufliche und teilweise bekannte Verbindungen oder lassen sich nach im Prinzip bekannten Verfahren der organischen Chemie herstellen.

Die Verbindungen der Formel (XXVII) wurden bereits bei den Vorstufen für das Verfahren (A) und (B) beschrieben.

Die beim erfindungsgemäßen Verfahren (I) als Ausgangsstoffe benötigten Verbindungen der Formel (XIII) 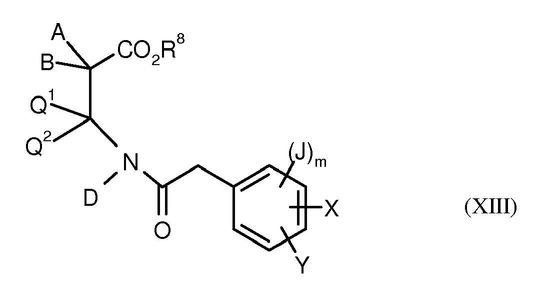 in welcher

A, B, D, J, m, Q¹, Q², X, Y und R⁸ die oben angegebenen Bedeutungen haben, sind neu.

Man erhält die Acylaminosäureester der Formel (XIII) beispielsweise, wenn man Aminosäurederivate der Formel (XLV) 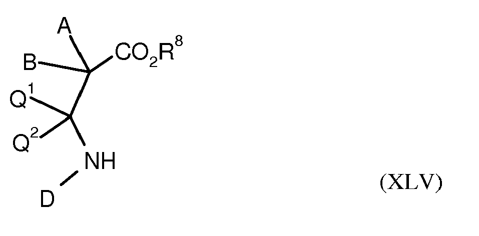 in welcher
- A, B, Q¹, Q², R⁸ und D: die oben angegebenen Bedeutungen haben,
mit substituierten Hetarylessigsäurederivaten der Formel (XXVII) 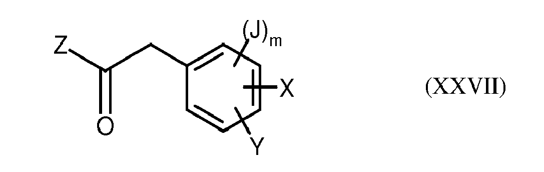 in welcher
- J, m, X, Y und Z: die oben angegebene Bedeutung haben
acyliert (Chem. Reviews 52, 237-416 (1953); Bhattacharya, Indian J. Chem. 6, 341-5, 1968)
oder wenn man Acylaminosäuren der Formel (LXVI) 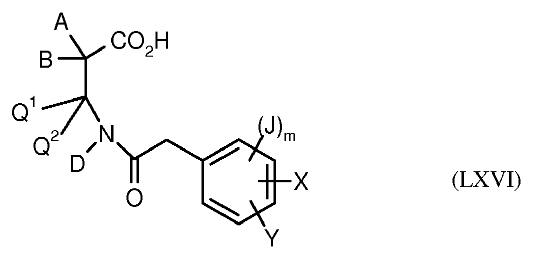 in welcher
A, B, J, m, D, Q¹, Q², X und Y die oben angegebenen Bedeutungen haben,
verestert (Chem. Ind. (London) 1568 (1968)).

Die Verbindungen der Formel (LXVI) 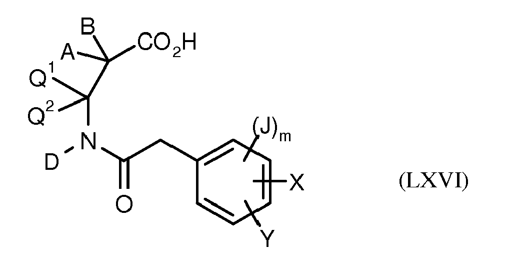 in welcher
A, B, D, J, m, Q¹, Q², X und Y die oben angegebenen Bedeutungen haben,
sind neu.

Man erhält die Verbindungen der Formel (XLVI), wenn man β-Aminosäuren der Formel (XLVII) 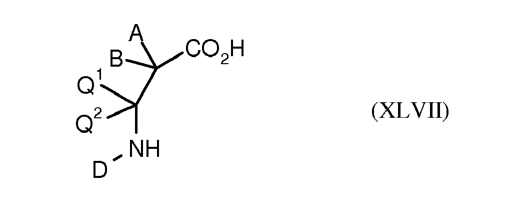 in welcher
- A, B, Q¹, Q² und D: die oben angegebenen Bedeutungen haben,
mit substituierten Phenylessigsäurederivaten der Formel (XXVII) 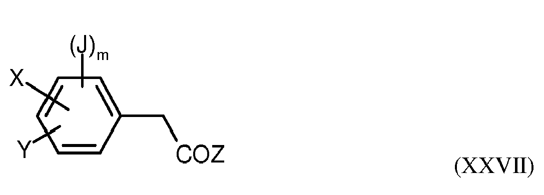 in welcher
- J, m, X, Y und Z: die oben angegebenen Bedeutungen haben
beispielsweise nach Schotten-Baumann acyliert (Organikum, VEB Deutscher Verlag der Wissenschaften, Berlin 1977, S. 505).

Die Verbindungen der Formel (XXVII) sind neu. Sie lassen sich nach im Prinzip bekannten Verfahren darstellen (s. z.B. H. Henecka, Houben-Weyl, Methoden der Organischen Chemie, Bd. 8, S. 467-469 (1952), WO 97/02243, WO 99/43699) oder werden mit den oben angegebenen Reagenzien in situ erzeugt.

Die Verbindungen der Formel (XLV) und (XLVII) sind teilweise aus WO 01/79204 bekannt oder lassen sich nach dem dort genannten im Prinzip bekannten Verfahren herstellen.

Die beim erfindungsgemäßen Verfahren (J) als Ausgangsstoffe benötigten Verbindungen der Formel (XIV) 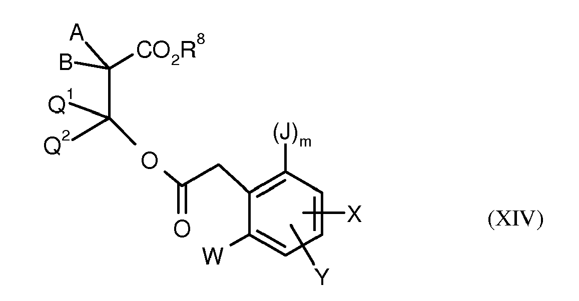 in welcher
- A, B, J, m, Q¹, Q², X, Y und R⁸: die oben angegebenen Bedeutungen haben,
sind neu.

Man erhält die Acylhydroxycarbonsäureester der Formel (XIV) beispielsweise, wenn man Hydroxycarbonsäureester der Formel (XLVIII) 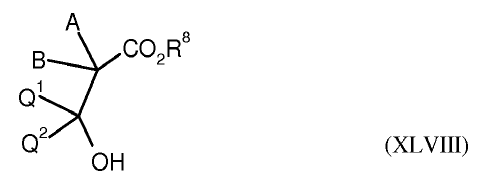 in welcher
- A, B, Q¹, Q² und R⁸: die oben angegebenen Bedeutungen haben,
mit substituierten Phenylessigsäurederivaten der Formel (XXVII) 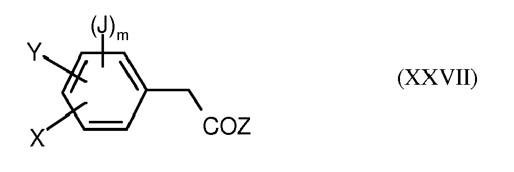 in welcher
- J, m, X, Y und Z: die oben angegebenen Bedeutungen haben
acyliert (s. Herstellungsbeispiele der Formel (II)).

Die Verbindungen der Formel (XLVIII) sind teilweise aus WO 01/98288 bekannt oder lassen sich nach im Prinzip bekannten Verfahren z.B. durch Reformatskij-Synthese herstellen (Organikum, VEB Deutscher Verlag der Wissenschaften, Berlin 1990, 18. Aufl., S. 501 ff.).

Die Verbindungen der Formel (XXVII) sind neu. Sie lassen sich nach im Prinzip bekannten Verfahren darstellen (s. z.B. H. Henecka, Houben-Weyl, Methoden der Organischen Chemie, Bd. 8, S. 467-469 (1952), WO 97/02243, WO 99/43649).

Die zur Durchführung der erfindungsgemäßen Verfahren (K), (L), (M), (N), (O), (P), (Q) und (R) außerdem als Ausgangsstoffe benötigten Säurehalogenide der Formel (XV), Carbonsäureanhydride der Formel (XVI), Chlorameisensäureester oder Chlorameisensäurethioester der Formel (XVII), Chlormonothioameisensäureester oder Chlordithioameisensäureester der Formel (XVIII), Sulfonsäurechloride der Formel (XIX), Phosphorverbindungen der Formel (XX) und Metallhydroxide, Metallalkoxide oder Amine der Formel (XXI) und (XXII) und Isocyanate der Formel (XXIII) und Carbamidsäurechloride der Formel (XXIV) sowie Cycloalkylboronsäurederivate (XXV) sind allgemein bekannte Verbindungen der Organischen bzw. Anorganischen Chemie.

Die Verbindungen der Formeln (V), (VII), (X), (XXVI), (XXIX), (XXXI), (XXXIII-A), (XXXIII-B), (XXXV), (XXXIX), (XL-a), (XL-b), (XLIII), (XLIV), (XLV), (XLVII) und (XLVIII) sind darüber hinaus aus den eingangs zitierten Patentanmeldungen bekannt und/oder lassen sich nach den dort angegebenen Methoden herstellen.

Das Verfahren (A) ist **dadurch gekennzeichnet, dass** man Verbindungen der Formel (II), in welcher A, B, D, J, m, X, Y und R⁸ die oben angegebenen Bedeutungen haben, in Gegenwart einer Base einer intramolekularen Kondensation unterwirft.

Als Verdünnungsmittel können bei dem erfindungsgemäßen Verfahren (A) alle inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid und N-Methyl-pyrrolidon, sowie Alkohole wie Methanol, Ethanol, Propanol, Iso-Propanol, Butanol, Iso-Butanol und tert.-Butanol.

Als Base (Deprotonierungsmittel) können bei der Durchführung des erfindungsgemäßen Verfahrens (A) alle üblichen Protonenakzeptoren eingesetzt werden. Vorzugsweise verwendbar sind Alkalimetall- und Erdalkalimetalloxide, -hydroxide und -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 (= Methyltrialkyl(C₈-C₁₀)ammoniumchlorid) oder TDA 1 (= Tris-(methoxyethoxyethyl)-amin) eingesetzt werden können. Weiterhin können Alkalimetalle wie Natrium oder Kalium verwendet werden. Ferner sind Alkalimetall- und Erdalkalimetallamide und -hydride, wie Natriumamid, Natriumhydrid und Calciumhydrid, und außerdem auch Alkalimetallalkoholate, wie Natrium-methylat, Natrium-ethylat und Kalium-tert.-butylat einsetzbar.

Die Reaktionstemperaturen können bei der Durchführung des erfindungsgemäßen Verfahrens (A) innerhalb eines größeren Bereiches variiert werden. Im Allgemeinen arbeitet man bei Temperaturen zwischen 0°C und 250°C, vorzugsweise zwischen 50°C und 150°C.

Das erfindungsgemäße Verfahren (A) wird im Allgemeinen unter Normaldruck durchgeführt.

Bei der Durchführung des erfindungsgemäßen Verfahrens (A) setzt man die Reaktionskomponenten der Formel (II) und die deprotonierenden Basen im Allgemeinen in etwa doppeläquimolaren Mengen ein. Es ist jedoch auch möglich, die eine oder andere Komponente in einem größeren Überschuss (bis zu 3 Mol) zu verwenden.

Das Verfahren (B) ist **dadurch gekennzeichnet, dass** Verbindungen der Formel (III), in welcher A, B, J, m, X, Y und R⁸ die oben angegebenen Bedeutungen haben, in Gegenwart eines Verdünnungsmittels und in Gegenwart einer Base einer intramolekularen Kondensation unterwirft.

Als Verdünnungsmittel können bei dem erfindungsgemäßen Verfahren (B) alle inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid und N-Methyl-pyrrolidon. Weiterhin können Alkohole wie Methanol, Ethanol, Propanol, Iso-Propanol, Butanol, Iso-Butanol und tert.-Butanol eingesetzt werden.

Als Base (Deprotonierungsmittel) können bei der Durchführung des erfindungsgemäßen Verfahrens (B) alle üblichen Protonenakzeptoren eingesetzt werden. Vorzugsweise verwendbar sind Alkalimetall- und Erdalkalimetalloxide, -hydroxide und -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 (= Methyltrialkyl(C₈-C₁₀)ammoniumchlorid) oder TDA 1 (= Tris-(methoxyethoxyethyl)-amin) eingesetzt werden können. Weiterhin können Alkalimetalle wie Natrium oder Kalium verwendet werden. Ferner sind Alkalimetall- und Erdalkalimetallamide und -hydride, wie Natriumamid, Natriumhydrid und Calciumhydrid, und außerdem auch Alkalimetallalkoholate, wie Natrium-methylat, Natrium-ethylat und Kalium-tert.-butylat einsetzbar.

Die Reaktionstemperaturen können bei der Durchführung des erfindungsgemäßen Verfahrens (B) innerhalb eines größeren Bereiches variiert werden. Im Allgemeinen arbeitet man bei Temperaturen zwischen 0°C und 250°C, vorzugsweise zwischen 50°C und 150°C.

Das erfindungsgemäße Verfahren (B) wird im Allgemeinen unter Normaldruck durchgeführt.

Bei der Durchführung des erfindungsgemäßen Verfahrens (B) setzt man die Reaktionskomponenten der Formel (III) und die deprotonierenden Basen im Allgemeinen in etwa äquimolaren Mengen ein. Es ist jedoch auch möglich, die eine oder andere Komponente in einem größeren Überschuss (bis zu 3 Mol) zu verwenden.

Das Verfahren (C) ist **dadurch gekennzeichnet, dass** man Verbindungen der Formel (IV) in welcher A, B, V, J, m, X, Y und R⁸ die oben angegebene Bedeutung haben, in Gegenwart einer Säure und gegebenenfalls in Gegenwart eines Verdünnungsmittels intramolekular cyclisiert.

Als Verdünnungsmittel können bei dem erfindungsgemäßen Verfahren (C) alle inerten organischen Solventien eingesetzt werden. Vorzugsweise verwendbar sind Kohlenwasserstoffe, wie Toluol und Xylol, ferner halogenierte Kohlenwasserstoffe wie Dichlormethan, Chloroform, Ethylenchlorid, Chlorbenzol, Dichlorbenzol, außerdem polare Lösungsmittel, wie Dimethylsulfoxid, Sulfolan, Dimethylformamid und N-Methyl-pyrrolidon. Weiterhin können Alkohole wie Methanol, Ethanol, Propanol, iso-Propanol, Butanol, Isobutanol, tert.-Butanol eingesetzt werden.

Gegebenenfalls kann auch die eingesetzte Säure als Verdünnungsmittel dienen.

Als Säure können bei dem erfindungsgemäßen Verfahren (C) alle üblichen anorganischen und organischen Säuren eingesetzt werden wie z.B. Halogenwasserstoffsäuren, Schwefelsäure, Alkyl-, Aryl- und Haloalkylsulfonsäuren, insbesondere halogenierte Alkylcarbonsäuren wie z.B. Trifluoressigsäure.

Die Reaktionstemperaturen können bei der Durchführung des erfindungsgemäßen Verfahrens (C) innerhalb eines größeren Bereiches variiert werden. Im Allgemeinen arbeitet man bei Temperaturen zwischen 0°C und 250°C, vorzugsweise zwischen 50°C und 150°C.

Das erfindungsgemäße Verfahren (C) wird im Allgemeinen unter Normaldruck durchgeführt.

Bei der Durchführung des erfindungsgemäßen Verfahrens (C) setzt man die Reaktionskomponenten der Formeln (IV) und die Säure z.B. in äquimolaren Mengen ein. Es ist jedoch gegebenenfalls auch möglich, die Säure als Lösungsmittel oder als Katalysator zu verwenden.

Das erfindungsgemäße Verfahren (D) ist **dadurch gekennzeichnet, dass** man Carbonylverbindungen der Formel (V) oder deren Enolether der Formel (V-a) mit Ketensäurehalogeniden der Formel (VI) in Gegenwart eines Verdünnungsmittels und gegebenenfalls in Gegenwart eines Säureakzeptors umsetzt.

Als Verdünnungsmittel können bei dem erfindungsgemäßen Verfahren (D) alle inerten organischen Solventien eingesetzt werden. Vorzugsweise verwendbar sind gegebenenfalls halogenierte Kohlenwasserstoffe, wie Toluol, Xylol, Mesitylen, Chlorbenzol und Dichlorbenzol, ferner Ether, wie Dibutylether, Glykoldimethylether Diglykoldimethylether und Diphenylether, außerdem polare Lösungsmittel, wie Dimethylsulfoxid, Sulfolan, Dimethylformamid oder N-Methyl-pyrrolidon.

Als Säureakzeptoren können bei der Durchführung der erfindungsgemäßen Verfahrensvariante (D) alle üblichen Säureakzeptoren verwendet werden.

Vorzugsweise verwendbar sind tertiäre Amine, wie Triethylamin, Pyridin, Diazabicyclooctan (DABCO), Diazabicycloundecan (DBU), Diazabicyclononen (DBN), Hünig-Base und N,N-Dimethyl-anilin.

Die Reaktionstemperaturen können bei der Durchführung der erfindungsgemäßen Verfahrensvariante (D) innerhalb eines größeren Bereiches variiert werden. Zweckmäßigerweise arbeitet man bei Temperaturen zwischen 0°C und 250°C, vorzugsweise zwischen 50°C und 220°C.

Das erfindungsgemäße Verfahren (D) wird zweckmäßigerweise unter Normaldruck durchgeführt.

Bei der Durchführung des erfindungsgemäßen Verfahrens (D) setzt man die Reaktionskomponenten der Formeln (V) und (VI), in welchen A, D, J, m, X und Y die oben angegebenen Bedeutungen haben und Hal für Halogen steht, und gegebenenfalls die Säureakzeptoren im Allgemeinen in etwa äquimolaren Mengen ein. Es ist jedoch auch möglich, die eine oder andere Komponente in einem größeren Überschuss (bis zu 5 Mol) zu verwenden.

Das erfindungsgemäße Verfahren (E) ist **dadurch gekennzeichnet, dass** man Thioamide der Formel (VII) mit Ketensäurehalogeniden der Formel (VI) in Gegenwart eines Verdünnungsmittels und gegebenenfalls in Gegenwart eines Säureakzeptors umsetzt.

Als Verdünnungsmittel können bei der erfindungsgemäßen Verfahrensvariante (E) alle inerten organischen Solventien eingesetzt werden. Vorzugsweise verwendbar sind Kohlenwasserstoffe, wie Toluol und Xylol, ferner Ether, wie Dibutylether, Glykoldimethylether und Diglykoldimethylether, außerdem polare Lösungsmittel, wie Dimethylsulfoxid, Sulfolan, Dimethylformamid und N-Methyl-pyrrolidon.

Als Säureakzeptoren können bei der Durchführung des erfindungsgemäßen Verfahrens (E) alle üblichen Säureakzeptoren verwendet werden.

Vorzugsweise verwendbar sind tertiäre Amine, wie Triethylamin, Pyridin, Diazabicyclooctan (DABCO), Diazabicycloundecan (DBU), Diazabicyclononen (DBN), Hünig-Base und N,N-Dimethyl-anilin.

Die Reaktionstemperaturen können bei der Durchführung des erfindungsgemäßen Verfahrens (E) innerhalb eines größeren Bereiches variiert werden. Zweckmäßigerweise arbeitet man bei Temperaturen zwischen 0°C und 250°C, vorzugsweise zwischen 20°C und 220°C.

Das erfindungsgemäße Verfahren (E) wird zweckmäßigerweise unter Normaldruck durchgeführt.

Bei der Durchführung des erfindungsgemäßen Verfahrens (E) setzt man die Reaktionskomponenten der Formeln (VII) und (VI), in welchen A, J, m, X und Y die oben angegebenen Bedeutungen haben und Hal für Halogen steht und gegebenenfalls die Säureakzeptoren im Allgemeinen in etwa äquimolaren Mengen ein. Es ist jedoch auch möglich, die eine oder andere Komponente in einem größeren Überschuss (bis zu 5 Mol) zu verwenden.

Das Verfahren (F) ist **dadurch gekennzeichnet, dass** man Verbindungen der Formel (VIII), in welcher A, B, J, m, Q¹, Q², X, Y und R⁸ die oben angegebene Bedeutung haben, in Gegenwart einer Base einer intramolekularen Kondensation unterwirft.

Als Verdünnungsmittel können bei dem erfindungsgemäßen Verfahren (F) alle gegenüber den Reaktionsteilnehmern inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid und N-Methyl-pyrrolidon. Weiterhin können Alkohole wie Methanol, Ethanol, Propanol, iso-Propanol, Butanol, Isobutanol, tert.-Butanol eingesetzt werden.

Als Basen (Deprotonierungsmittel) können bei der Durchführung des erfindungsgemäßen Verfahrens (F) alle üblichen Protonenakzeptoren eingesetzt werden. Vorzugsweise verwendbar sind Alkalimetall- und Erdalkalimetalloxide, -hydroxide und -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 (Methyltrialkyl(C₈-C₁₀)ammoniumchlorid) oder TDA 1 (Tris-(methoxyethoxyethyl)-amin) eingesetzt werden können. Weiterhin können Alkalimetalle wie Natrium oder Kalium verwendet werden. Ferner sind Alkalimetall- und Erdalkalimetallamide und -hydride, wie Natriumamid, Natriumhydrid und Calciumhydrid, und außerdem auch Alkalimetall-alkoholate, wie Natrium-methylat, Natrium-ethylat und Kalium-tert.-butylat einsetzbar.

Die Reaktionstemperaturen können bei der Durchführung des erfindungsgemäßen Verfahrens (F) innerhalb eines größeren Bereiches variiert werden. Im Allgemeinen arbeitet man bei Temperaturen zwischen -75°C und 250°C, vorzugsweise zwischen -50°C und 150°C.

Das erfindungsgemäße Verfahren (F) wird im Allgemeinen unter Normaldruck durchgeführt.

Bei der Durchführung des erfindungsgemäßen Verfahrens (F) setzt man die Reaktionskomponenten der Formel (VIII) und die deprotonierenden Basen im Allgemeinen in etwa äquimolaren Mengen ein. Es ist jedoch auch möglich, die eine oder andere Komponente in einem größeren Überschuss (bis zu 3 Mol) zu verwenden.

Das Verfahren (G) ist **dadurch gekennzeichnet, dass** man Verbindungen der Formel (IX), in welcher A, B, Q³, Q⁴, Q⁵, Q⁶ J, m, X, Y und R⁸ die oben angegebene Bedeutung haben, in Gegenwart von Basen einer intramolekularen Kondensation unterwirft.

Als Verdünnungsmittel können bei dem erfindungsgemäßen Verfahren (G) alle gegenüber den Reaktionsteilnehmern inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid und N-Methyl-pyrrolidon. Weiterhin können Alkohole wie Methanol, Ethanol, Propanol, iso-Propanol, Butanol, Isobutanol, tert.-Butanol eingesetzt werden.

Als Basen (Deprotonierungsmittel) können bei der Durchführung des erfindungsgemäßen Verfahrens (G) alle üblichen Protonenakzeptoren eingesetzt werden.

Vorzugsweise verwendbar sind Alkalimetall- und Erdalkalimetalloxide, -hydroxide und -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 (Methyltrialkyl(C₈-C₁₀)ammoniumchlorid) oder TDA 1 (Tris-(methoxyethoxyethyl)-amin) eingesetzt werden können. Weiterhin können Alkalimetalle wie Natrium oder Kalium verwendet werden. Ferner sind Alkalimetall- und Erdalkalimetallamide und -hydride, wie Natriumamid, Natriumhydrid und Calciumhydrid, und außerdem auch Alkalimetall-alkoholate, wie Natrium-methylat, Natrium-ethylat und Kalium-tert.-butylat einsetzbar.

Die Reaktionstemperaturen können bei der Durchführung des erfindungsgemäßen Verfahrens (G) innerhalb eines größeren Bereiches variiert werden. Im Allgemeinen arbeitet man bei Temperaturen zwischen 0°C und 250°C, vorzugsweise zwischen 50°C und 150°C.

Das erfindungsgemäße Verfahren (G) wird im Allgemeinen unter Normaldruck durchgeführt.

Bei der Durchführung des erfindungsgemäßen Verfahrens (G) setzt man die Reaktionskomponenten der Formel (IX) und die deprotonierenden Basen im Allgemeinen in etwa äquimolaren Mengen ein. Es ist jedoch auch möglich, die eine oder andere Komponente in einem größeren Überschuss (bis zu 3 Mol) zu verwenden.

Das erfindungsgemäße Verfahren (H-α) ist **dadurch gekennzeichnet, dass** man Hydrazine der Formel (X) oder Salze dieser Verbindungen mit Ketensäurehalogeniden der Formel (VI) in Gegenwart eines Verdünnungsmittels und gegebenenfalls in Gegenwart eines Säureakzeptors umsetzt.

Als Verdünnungsmittel können bei dem erfindungsgemäßen Verfahren (H-α) alle inerten organischen Solventien eingesetzt werden. Vorzugsweise verwendbar sind gegebenenfalls chlorierte Kohlenwasserstoffe, wie beispielsweise Mesitylen, Chlorbenzol und Dichlorbenzol, Toluol, Xylol, ferner Ether, wie Dibutylether, Glykoldimethylether, Diglykoldimethylether und Diphenylethan, außerdem polare Lösungsmittel, wie Dimethylsulfoxid, Sulfolan, Dimethylformamid oder N-Methyl-pyrrolidon.

Als Säureakzeptoren können bei der Durchführung der erfindungsgemäßen Verfahrensvariante (H-α) alle üblichen Säureakzeptoren verwendet werden.

Vorzugsweise verwendbar sind tertiäre Amine, wie Triethylamin, Pyridin, Diazabicyclooctan (DABCO), Diazabicycloundecan (DBU), Diazabicyclononen (DBN), Hünig-Base und N,N-Dimethyl-anilin.

Die Reaktionstemperaturen können bei der Durchführung der erfindungsgemäßen Verfahrensvariante (H-α) innerhalb eines größeren Bereiches variiert werden. Zweckmäßigerweise arbeitet man bei Temperaturen zwischen 0°C und 250°C, vorzugsweise zwischen 50°C und 220°C.

Das erfindungsgemäße Verfahren (H-α) wird zweckmäßigerweise unter Normaldruck durchgeführt.

Bei der Durchführung des erfindungsgemäßen Verfahrens (H-α) setzt man die Reaktionskomponenten der Formeln (VI) und (X), in welchen A, D, J, m, X und Y die oben angegebenen Bedeutungen haben und Hal für Halogen steht, und gegebenenfalls die Säureakzeptoren im Allgemeinen in etwa äquimolaren Mengen ein. Es ist jedoch auch möglich, die eine oder andere Komponente in einem größeren Überschuss (bis zu 5 Mol) zu verwenden.

Das Verfahren (H-β) ist **dadurch gekennzeichnet, dass** man Hydrazine der Formel (X) oder Salze dieser Verbindung, in welcher A und D die oben angegebenen Bedeutungen haben, mit Malonestern oder Malonsäureamiden der Formel (XI), in welcher U, J, m, X, Y und R⁸ die oben angegebene Bedeutung haben, in Gegenwart einer Base einer Kondensation unterwirft.

Als Verdünnungsmittel können bei dem erfindungsgemäßen Verfahren (H-β) alle inerten organischen Solventien eingesetzt werden. Vorzugsweise verwendbar sind gegebenenfalls halogenierte Kohlenwasserstoffe, wie Toluol, Xylol, Mesitylen, Chlorbenzol und Dichlorbenzol, ferner Ether, wie Dibutylether, Tetrahydrofuran, Dioxan, Diphenylether, Glykoldimethylether und Diglykoldimethylether, außerdem polare Lösungsmittel, wie Dimethylsulfoxid, Sulfolan, Dimethylformamid, Dimethylacetamid und N-Methyl-pyrrolidon, sowie Alkohole wie Methanol, Ethanol, Propanol, Iso-Propanol, Butanol, Iso-Butanol und tert.-Butanol.

Als Base (Deprotonierungsmittel) können bei der Durchführung des erfindungsgemäßen Verfahrens (H-β) alle üblichen Protonenakzeptoren eingesetzt werden. Vorzugsweise verwendbar sind Alkalimetall- und Erdalkalimetalloxide, -hydroxide und -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 (= Methyltrialkyl(C₈-C₁₀)ammoniumchlorid) oder TDA 1 (= Tris-(methoxyethoxyethyl)-amin) eingesetzt werden können. Weiterhin können Alkalimetalle wie Natrium oder Kalium verwendet werden. Ferner sind Alkalimetall- und Erdalkalimetallamide und -hydride, wie Natriumamid, Natriumhydrid und Calciumhydrid, und außerdem auch Alkalimetallalkoholate, wie Natrium-methylat, Natrium-ethylat und Kalium-tert.-butylat einsetzbar.

Verwendbar sind auch tertiäre Amine, wie Triethylamin, Pyridin, Diazabicyclooctan (DABCO), Diazabicycloundecan (DBU), Diazabicyclononen (DBN), Hünig-Base und N,N-Dimethyl-anilin.

Die Reaktionstemperaturen können bei der Durchführung des erfindungsgemäßen Verfahrens (H-β) innerhalb eines größeren Bereiches variiert werden. Im Allgemeinen arbeitet man bei Temperaturen zwischen 0°C und 280°C, vorzugsweise zwischen 50°C und 180°C.

Das erfindungsgemäße Verfahren (H-β) wird im Allgemeinen unter Normaldruck durchgeführt.

Bei der Durchführung des erfindungsgemäßen Verfahrens (H-β) setzt man die Reaktionskomponenten der Formeln (XI) und (X) im Allgemeinen in etwa äquimolaren Mengen ein. Es ist jedoch auch möglich, die eine oder andere Komponente in einem größeren Überschuss (bis zu 3 mol) zu verwenden.

Das Verfahren (H-γ) ist **dadurch gekennzeichnet, dass** man Verbindungen der Formel (XII), in welcher A, D, J, m, X, Y und R⁸ die oben angegebenen Bedeutungen haben, in Gegenwart einer Base einer intramolekularen Kondensation unterwirft.

Als Verdünnungsmittel können bei dem erfindungsgemäßen Verfahren (H-γ) alle inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid und N-Methyl-pyrrolidon, sowie Alkohole wie Methanol, Ethanol, Propanol, Iso-Propanol, Butanol, Iso-Butanol und tert.-Butanol.

Als Base (Deprotonierungsmittel) können bei der Durchführung des erfindungsgemäßen Verfahrens (H-γ) alle üblichen Protonenakzeptoren eingesetzt werden. Vorzugsweise verwendbar sind Alkalimetall- und Erdalkalimetalloxide, -hydroxide und -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 (= Methyltrialkyl(C₈-C₁₀)ammoniumchlorid) oder TDA 1 (= Tris-(methoxyethoxyethyl)-amin) eingesetzt werden können. Weiterhin können Alkalimetalle wie Natrium oder Kalium verwendet werden. Ferner sind Alkalimetall- und Erdalkalimetallamide und -hydride, wie Natriumamid, Natriumhydrid und Calciumhydrid, und außerdem auch Alkalimetallalkoholate, wie Natrium-methylat, Natrium-ethylat und Kalium-tert.-butylat einsetzbar.

Die Reaktionstemperaturen können bei der Durchführung des erfindungsgemäßen Verfahrens (H-γ) innerhalb eines größeren Bereiches variiert werden. Im Allgemeinen arbeitet man bei Temperaturen zwischen 0°C und 250°C, vorzugsweise zwischen 50°C und 150°C.

Das erfindungsgemäße Verfahren (H-γ) wird im Allgemeinen unter Normaldruck durchgeführt.

Bei der Durchführung des erfindungsgemäßen Verfahrens (H-γ) setzt man die Reaktionskomponenten der Formel (XII) und die deprotonierenden Basen im Allgemeinen in etwa doppeltäquimolaren Mengen ein. Es ist jedoch auch möglich, die eine oder andere Komponente in einem größeren Überschuss (bis zu 3 Mol) zu verwenden.

Das Verfahren (I) ist **dadurch gekennzeichnet, dass** man Verbindungen der Formel (XIII), in welcher A, B, D, J, m, Q¹, Q², X, Y und R⁸ die oben angegebenen Bedeutungen haben, in Gegenwart einer Base einer intramolekularen Kondensation unterwirft.

Als Verdünnungsmittel können bei dem erfindungsgemäßen Verfahren (I) alle inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid, Dimethylacetamid und N-Methyl-pyrrolidon, sowie Alkohole wie Methanol, Ethanol, Propanol, Iso-Propanol, Butanol, Iso-Butanol und tert.-Butanol.

Als Base (Deprotonierungsmittel) können bei der Durchführung des erfindungsgemäßen Verfahrens (I) alle üblichen Protonenakzeptoren eingesetzt werden. Vorzugsweise verwendbar sind Alkalimetall- und Erdalkalimetalloxide, -hydroxide und -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 (= Methyltrialkyl(C₈-C₁₀)ammoniumchlorid) oder TDA 1 (= Tris-(methoxyethoxyethyl)-amin) eingesetzt werden können. Weiterhin können Alkalimetalle wie Natrium oder Kalium verwendet werden. Ferner sind Alkalimetall- und Erdalkalimetallamide und -hydride, wie Natriumamid, Natriumhydrid und Calciumhydrid, und außerdem auch Alkalimetallalkoholate, wie Natrium-methylat, Natrium-ethylat und Kalium-tert.-butylat einsetzbar.

Die Reaktionstemperaturen können bei der Durchführung des erfindungsgemäßen Verfahrens (I) innerhalb eines größeren Bereiches variiert werden. Im allgemeinen arbeitet man bei Temperaturen zwischen -80°C und 180°C, vorzugsweise zwischen -50°C und 120°C.

Das erfindungsgemäße Verfahren (I) wird im allgemeinen unter Normaldruck durchgeführt.

Bei der Durchführung des erfindungsgemäßen Verfahrens (I) setzt man die Reaktionskomponenten der Formel (XIII) und die deprotonierenden Basen im allgemeinen in etwa doppeltäquimolaren Mengen ein. Es ist jedoch auch möglich, die eine oder andere Komponente in einem größeren Überschuss (bis zu 3 Mol) zu verwenden.

Das Verfahren (J) ist **dadurch gekennzeichnet, dass** man Verbindungen der Formel (XIV), in welcher A, B, Q¹, Q², J, m, X, Y und R⁸ die oben angegebenen Bedeutungen haben, in Gegenwart einer Base einer intramolekularen Kondensation unterwirft.

Als Verdünnungsmittel können bei dem erfindungsgemäßen Verfahren (J) alle inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid und N-Methyl-pyrrolidon, sowie Alkohole wie Methanol, Ethanol, Propanol, iso-Propanol, Butanol, iso-Butanol und tert-Butanol.

Als Base (Deprotonierungsmittel) können bei der Durchführung des erfindungsgemäßen Verfahrens (J) alle üblichen Protonenakzeptoren eingesetzt werden. Vorzugsweise verwendbar sind Alkalimetall- und Erdalkalimetalloxide, -hydroxide und -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 (= Methyltrialkyl(C₈-C₁₀)ammoniumchlorid) oder TDA 1 (= Tris-(methoxyethoxyethyl)-amin) eingesetzt werden können. Weiterhin können Alkalimetalle wie Natrium oder Kalium verwendet werden. Ferner sind Alkalimetall- und Erdalkalimetallamide und -hydride, wie Natriumamid, Natriumhydrid und Calciumhydrid, und außerdem auch Alkalimetallalkoholate, wie Natrium-methylat, Natrium-ethylat und Kalium-tert-butylat einsetzbar.

Die Reaktionstemperaturen können bei der Durchführung des erfindungsgemäßen Verfahrens (J) innerhalb eines größeren Bereiches variiert werden. Im allgemeinen arbeitet man bei Temperaturen zwischen 0°C und 250°C, vorzugsweise zwischen 50°C und 150°C.

Das erfindungsgemäße Verfahren (J) wird im allgemeinen unter Normaldruck durchgeführt.

Bei der Durchführung des erfindungsgemäßen Verfahrens (J) setzt man die Reaktionskomponenten der Formel (XIV) und die deprotonierenden Basen im allgemeinen in etwa doppeltäquimolaren Mengen ein. Es ist jedoch auch möglich, die eine oder andere Komponente in einem größeren Überschuss (bis zu 3 Mol) zu verwenden.

Das Verfahren (K-α) ist **dadurch gekennzeichnet, dass** man Verbindungen der Formeln (I-1-a) bis (I-10-a) jeweils mit Carbonsäurehalogeniden der Formel (XV) gegebenenfalls in Gegenwart eines Verdünnungsmittels und gegebenenfalls in Gegenwart eines Säurebindemittels umsetzt.

Als Verdünnungsmittel können bei dem erfindungsgemäßen Verfahren (K-α) alle gegenüber den Säurehalogeniden inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenstoff, Chlorbenzol und o-Dichlorbenzol, außerdem Ketone, wie Aceton und Methylisopropylketon, weiterhin Ether, wie Diethylether, Tetrahydrofuran und Dioxan, darüber hinaus Carbonsäureester, wie Ethylacetat, und auch stark polare Solventien, wie Dimethylsulfoxid und Sulfolan. Wenn die Hydrolysestabilität des Säurehalogenids es zulässt, kann die Umsetzung auch in Gegenwart von Wasser durchgeführt werden.

Als Säurebindemittel kommen bei der Umsetzung nach dem erfindungsgemäßen Verfahren (K-α) alle üblichen Säureakzeptoren in Betracht. Vorzugsweise verwendbar sind tertiäre Amine, wie Triethylamin, Pyridin, Diazabicyclooctan (DABCO), Diazabicycloundecen (DBU), Diazabicyclononen (DBN), Hünig-Base und N,N-Dimethyl-anilin, ferner Erdalkalimetalloxide, wie Magnesium- und Calciumoxid, außerdem Alkali- und Erdalkali-metall-carbonate, wie Natriumcarbonat, Kaliumcarbonat und Calciumcarbonat sowie Alkalihydroxide wie Natriumhydroxid und Kaliumhydroxid.

Die Reaktionstemperaturen können bei dem erfindungsgemäßen Verfahren (K-α) innerhalb eines größeren Bereiches variiert werden. Im Allgemeinen arbeitet man bei Temperaturen zwischen - 20°C und +150°C, vorzugsweise zwischen 0°C und 100°C.

Bei der Durchführung des erfindungsgemäßen Verfahrens (K-α) werden die Ausgangsstoffe der Formeln (I-1-a) bis (1-10-a) und das Carbonsäurehalogenid der Formel (XV) im Allgemeinen jeweils in angenähert äquivalenten Mengen verwendet. Es ist jedoch auch möglich, das Carbonsäurehalogenid in einem größeren Überschuss (bis zu 5 Mol) einzusetzen. Die Aufarbeitung erfolgt nach üblichen Methoden.

Das Verfahren (K-β) ist **dadurch gekennzeichnet, dass** man Verbindungen der Formeln (I-1-a) bis (I-10-a) mit Carbonsäureanhydriden der Formel (XVI) gegebenenfalls in Gegenwart eines Verdünnungsmittels und gegebenenfalls in Gegenwart eines Säurebindemittels umsetzt.

Als Verdünnungsmittel können bei dem erfindungsgemäßen Verfahren (K-β) vorzugsweise diejenigen Verdünnungsmittel verwendet werden, die auch bei der Verwendung von Säurehalogeniden vorzugsweise in Betracht kommen. Im übrigen kann auch ein im Überschuss eingesetztes Carbonsäureanhydrid gleichzeitig als Verdünnungsmittel fungieren.

Als gegebenenfalls zugesetzte Säurebindemittel kommen beim Verfahren (K-β) vorzugsweise diejenigen Säurebindemittel in Frage, die auch bei der Verwendung von Säurehalogeniden vorzugsweise in Betracht kommen.

Die Reaktionstemperaturen können bei dem erfindungsgemäßen Verfahren (K-β) innerhalb eines größeren Bereiches variiert werden. Im Allgemeinen arbeitet man bei Temperaturen zwischen -20°C und +150°C, vorzugsweise zwischen 0°C und 100°C.

Bei der Durchführung des erfindungsgemäßen Verfahrens (K-β) werden die Ausgangsstoffe der Formeln (I-1-a) bis (I-10-a) und das Carbonsäureanhydrid der Formel (XVI) im Allgemeinen in jeweils angenähert äquivalenten Mengen verwendet. Es ist jedoch auch möglich, das Carbonsäureanhydrid in einem größeren Überschuss (bis zu 5 Mol) einzusetzen. Die Aufarbeitung erfolgt nach üblichen Methoden.

Im Allgemeinen geht man so vor, dass man Verdünnungsmittel und im Überschuss vorhandenes Carbonsäureanhydrid sowie die entstehende Carbonsäure durch Destillation oder durch Waschen mit einem organischen Lösungsmittel oder mit Wasser entfernt.

Das Verfahren (L) ist **dadurch gekennzeichnet, dass** man Verbindungen der Formeln (I-1-a) bis (I-10-a) jeweils mit Chlorameisensäureestern oder Chlorameisensäurethiolestern der Formel (XVII) gegebenenfalls in Gegenwart eines Verdünnungsmittels und gegebenenfalls in Gegenwart eines Säurebindemittels umsetzt.

Als Säurebindemittel kommen bei der Umsetzung nach dem erfindungsgemäßen Verfahren (L) alle üblichen Säureakzeptoren in Betracht. Vorzugsweise verwendbar sind tertiäre Amine, wie Triethylamin, Pyridin, DABCO, DBU, DBA, Hünig-Base und N,N-Dimethyl-anilin, ferner Erdalkalimetalloxide, wie Magnesium- und Calciumoxid, außerdem Alkali- und Erdalkalimetallcarbonate, wie Natriumcarbonat, Kaliumcarbonat und Calciumcarbonat sowie Alkalihydroxide wie Natriumhydroxid und Kaliumhydroxid.

Als Verdünnungsmittel können bei dem erfindungsgemäßen Verfahren (L) alle gegenüber den Chlorameisensäureestern bzw. Chlorameisensäurethiolestern inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenwasserstoff, Chlorbenzol und o-Dichlorbenzol, außerdem Ketone, wie Aceton und Methylisopropylketon, weiterhin Ether, wie Diethylether, Tetrahydrofuran und Dioxan, darüber hinaus Carbonsäureester, wie Ethylacetat, und auch stark polare Solventien, wie Dimethylsulfoxid und Sulfolan.

Die Reaktionstemperaturen können bei der Durchführung des erfindungsgemäßen Verfahrens (L) innerhalb eines größeren Bereiches variiert werden. Arbeitet man in Gegenwart eines Verdünnungsmittels und eines Säurebindemittels, so liegen die Reaktionstemperaturen im Allgemeinen zwischen -20°C und +100°C, vorzugsweise zwischen 0°C und 50°C.

Das erfindungsgemäße Verfahren (L) wird im Allgemeinen unter Normaldruck durchgeführt.

Bei der Durchführung des erfindungsgemäßen Verfahrens (L) werden die Ausgangsstoffe der Formeln (I-1-a) bis (I-10-a) und der entsprechende Chlorameisensäureester bzw. Chlorameisensäurethiolester der Formel (XVII) im Allgemeinen jeweils in angenähert äquivalenten Mengen verwendet. Es ist jedoch auch möglich, die eine oder andere Komponente in einem größeren Überschuss (bis zu 2 Mol) einzusetzen. Die Aufarbeitung erfolgt nach üblichen Methoden. Im Allgemeinen geht man so vor, dass man ausgefallene Salze entfernt und das verbleibende Reaktionsgemisch durch Abziehen des Verdünnungsmittels einengt.

Das erfindungsgemäße Verfahren (M) ist **dadurch gekennzeichnet, dass** man Verbindungen der Formeln (I-1-a) bis (I-10-a) jeweils mit Verbindungen der Formel (XVIII) in Gegenwart eines Verdünnungsmittels und gegebenenfalls in Gegenwart eines Säurebindemittels umsetzt.

Beim Herstellungsverfahren (M) setzt man pro Mol Ausgangsverbindung der Formeln (I-1-a) bis (I-10-a) ca. 1 Mol Chlormonothioameisensäureester bzw. Chlordithioameisensäureester der Formel (XVIII) bei 0 bis 120°C, vorzugsweise bei 20 bis 60°C um.

Als gegebenenfalls zugesetzte Verdünnungsmittel kommen alle inerten polaren organischen Lösungsmittel in Frage, wie Ether, Amide, Sulfone, Sulfoxide, aber auch Halogenalkane. Vorzugsweise werden Dimethylsulfoxid, Tetrahydrofuran, Dimethylformamid oder Methylenchlorid eingesetzt.

Stellt man in einer bevorzugten Ausführungsform durch Zusatz von starken Deprotonierungsmitteln wie z.B. Natriumhydrid oder Kaliumtertiärbutylat das Enolatsalz der Verbindungen (I-1-a) bis (I-10-a) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Pyridin, Triethylamin aufgeführt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Das erfindungsgemäße Verfahren (N) ist **dadurch gekennzeichnet, dass** man Verbindungen der Formeln (I-1-a) bis (I-10-a) jeweils mit Sulfonsäurechloriden der Formel (XIX) gegebenenfalls in Gegenwart eines Verdünnungsmittels und gegebenenfalls in Gegenwart eines Säurebindemittels umsetzt.

Beim Herstellungsverfahren (N) setzt man pro Mol Ausgangsverbindung der Formel (I-1-a bis I-10-a) ca. 1 Mol Sulfonsäurechlorid der Formel (XIX) bei -20 bis 150°C, vorzugsweise bei 20 bis 70°C um.

Als gegebenenfalls zugesetzte Verdünnungsmittel kommen alle inerten polaren organischen Lösungsmittel in Frage wie Ether, Amide, Nitrile, Sulfone, Sulfoxide oder halogenierte Kohlenwasserstoffe wie Methylenchlorid.

Vorzugsweise werden Dimethylsulfoxid, Tetrahydrofuran, Dimethylformamid, Methylenchlorid eingesetzt.

Stellt man in einer bevorzugten Ausführungsform durch Zusatz von starken Deprotonierungsmitteln (wie z.B. Natriumhydrid oder Kaliumtertiärbutylat) das Enolatsalz der Verbindungen (I-1-a) bis (I-10-a) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Pyridin, Triethylamin aufgeführt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Das erfindungsgemäße Verfahren (O) ist **dadurch gekennzeichnet, dass** man Verbindungen der Formeln (I-1-a) bis (I-10-a) jeweils mit Phosphorverbindungen der Formel (XX) gegebenenfalls in Gegenwart eines Verdünnungsmittels und gegebenenfalls in Gegenwart eines Säurebindemittels umsetzt.

Beim Herstellungsverfahren (O) setzt man zum Erhalt von Verbindungen der Formeln (I-1-e) bis (I-10-e) auf 1 Mol der Verbindungen (I-1-a) bis (I-10-a), 1 bis 2, vorzugsweise 1 bis 1,3 Mol der Phosphorverbindung der Formel (XX) bei Temperaturen zwischen -40°C und 150°C, vorzugsweise zwischen -10 und 110°C um.

Als gegebenenfalls zugesetzte Verdünnungsmittel kommen alle inerten, polaren organischen Lösungsmittel in Frage wie Ether, Amide, Nitrile, Alkohole, Sulfide, Sulfone, Sulfoxide etc.

Vorzugsweise werden Acetonitril, Dimethylsulfoxid, Tetrahydrofuran, Dimethylformamid, Methylenchlorid eingesetzt.

Als gegebenenfalls zugesetzte Säurebindemittel kommen übliche anorganische oder organische Basen in Frage wie Hydroxide, Carbonate oder Amine. Beispielhaft seien Natriumhydroxid, Natriumcarbonat, Kaliumcarbonat, Pyridin, Triethylamin aufgeführt.

Die Umsetzung kann bei Normaldruck oder unter erhöhtem Druck durchgeführt werden, vorzugsweise wird bei Normaldruck gearbeitet. Die Aufarbeitung geschieht nach üblichen Methoden der organischen Chemie. Die Reinigung der anfallenden Endprodukte geschieht vorzugsweise durch Kristallisation, chromatographische Reinigung oder durch sogenanntes "Andestillieren", d.h. Entfernung der flüchtigen Bestandteile im Vakuum.

Das Verfahren (P) ist **dadurch gekennzeichnet, dass** man Verbindungen der Formeln (I-1-a) bis (I-10-a) mit Metallhydroxiden bzw. Metallalkoxiden der Formel (XXI) oder Aminen der Formel (XXII), gegebenenfalls in Gegenwart eines Verdünnungsmittels, umsetzt.

Als Verdünnungsmittel können bei dem erfindungsgemäßen Verfahren (P) vorzugsweise Ether wie Tetrahydrofuran, Dioxan, Diethylether oder aber Alkohole wie Methanol, Ethanol, Isopropanol, aber auch Wasser eingesetzt werden.

Das erfindungsgemäße Verfahren (P) wird im Allgemeinen unter Normaldruck durchgeführt.

Die Reaktionstemperaturen liegen im Allgemeinen zwischen -20°C und 100°C, vorzugsweise zwischen 0°C und 50°C.

Das erfindungsgemäße Verfahren (Q) ist **dadurch gekennzeichnet, dass** man Verbindungen der Formeln (I-1-a) bis (I-10-a) jeweils mit (Q-α) Verbindungen der Formel (XXIII) gegebenenfalls in Gegenwart eines Verdünnungsmittels und gegebenenfalls in Gegenwart eines Katalysators oder (Q-β) mit Verbindungen der Formel (XXIV) gegebenenfalls in Gegenwart eines Verdünnungsmittels und gegebenenfalls in Gegenwart eines Säurebindemittels umsetzt.

Bei Herstellungsverfahren (Q-α) setzt man pro Mol Ausgangsverbindung der Formeln (I-1-a) bis (I-10-a) ca. 1 Mol Isocyanat der Formel (XXIII) bei 0 bis 100°C, vorzugsweise bei 20 bis 50°C um.

Als gegebenenfalls zugesetzte Verdünnungsmittel kommen alle inerten organischen Lösungsmittel in Frage, wie Ether, Amide, Nitrile, Sulfone, Sulfoxide.

Gegebenenfalls können Katalysatoren zur Beschleunigung der Reaktion zugesetzt werden. Als Katalysatoren können sehr vorteilhaft zinnorganische Verbindungen, wie z.B. Dibutylzinndilaurat eingesetzt werden. Es wird vorzugsweise bei Normaldruck gearbeitet.

Beim Herstellungsverfahren (Q-β) setzt man pro Mol Ausgangsverbindung der Formeln (I-1-a) bis (I-10-a) ca. 1 Mol Carbamidsäurechlorid der Formel (XXIV) bei -20 bis 150°C, vorzugsweise bei 0 bis 70°C um.

Als gegebenenfalls zugesetzte Verdünnungsmittel kommen alle inerten polaren organischen Lösungsmittel in Frage wie Ether, Amide, Sulfone, Sulfoxide oder halogenierte Kohlenwasserstoffe.

Vorzugsweise werden Dimethylsulfoxid, Tetrahydrofuran, Dimethylformamid oder Methylenchlorid eingesetzt.

Stellt man in einer bevorzugten Ausführungsform durch Zusatz von starken Deprotonierungsmitteln (wie z.B. Natriumhydrid oder Kaliumtertiärbutylat) das Enolatsalz der Verbindung (I-1-a) bis (I-10-a) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Triethylamin oder Pyridin genannt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Zur Durchführung des erfindungsgemäßen Verfahrens (R) sind Palladium(0)-Komplexe als Katalysator geeignet. Bevorzugt wird beispielsweise Tetrakis-(triphenylphosphin)palladium. Gegebenenfalls können auch Palladium(II)-Verbindungen eingesetzt werden, beispielsweise PdCl₂, Pd(OAC)₂. Bei der Verwendung von Palladium(II)-Verbindungen werden in der Regel Phosphine als Komplexbildner wie beispielsweise Tricyclohexylphosphin eingesetzt.

Als Säureakzeptoren zur Durchführung des erfindungsgemäßen Verfahrens (R) kommen anorganische oder organische Basen in Frage. Hierzu gehören vorzugsweise Erdalkalimetall- oder Alkalimetallhydroxide, -acetate, -carbonate oder -hydrogencarbonate, wie beispielsweise Natrium-, Kalium-, Barium- oder Ammoniumhydroxid, Natrium-, Kalium-, Calcium- oder Ammoniumacetat, Natrium-, Kalium-, Cäsium- oder Ammoniumcarbonat, Natriumhydrogen- oder Kaliumhydrogencarbonat, Alkalifluoride, wie beispielsweise Cäsiumfluorid, Alkaliphosphate wie z.B. Kalium-dihydrogenphosphat sowie tertiäre Amine, wie Trimethylamin, Triethylamin, Tributylamin, N,N-Dimethylanilin, N,N-Dimethylbenzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Als Verdünnungsmittel zur Durchführung des erfindungsgemäßen Verfahrens (R) kommen Wasser, organische Lösungsmittel und beliebige Mischungen davon in Betracht. Beispielhaft seien genannt: aliphatische, alicyclische oder aromatische Kohlenwasserstoffe, wie beispielsweise Petrolether, Hexan, Heptan, Cyclohexan, Methylcyclohexan, Benzol, Toluol, Xylol oder Dicalin; halogenierte Kohlenwasserstoffe, wie beispielsweise Chlorbenzol, Dichlorbenzol, Methylenchlorid, Chloroform, Tetrachlormethan, Dichlor-, Trichlorethan oder Tetrachlorethylen; Ether, wie Diethyl-, Diisopropyl-, Methyl-t-butyl-, Methyl-t-amylether, Dioxan, Tetrahydrofuren, 1,2-Dimethoxyethan, 1,2-Diethoxyethan, Diethylenglykoldimethylether oder Anisol; Alkohole, wie Methanol, Ethanol, n- oder i-Propanol, n-, iso-, sek.- oder tert.-Butanol, Ethandiol, Propan-1,2-diol, Ethoxyethanol, Methoxyethanol, Diethylenglykolmonomethylether, Diethylglykolmonomethylether; Wasser.

Die Reaktionstemperatur kann bei dem erfindungsgemäßen Verfahren (R) innerhalb eines größeren Bereiches variiert werden. Im allgemeinen arbeitet man bei Temperaturen zwischen 0°C und +140°C, bevorzugt zwischen 50°C und +100°C.

Bei der Durchführung des erfindungsgemäßen Verfahrens (R) werden die Boronsäuren der Formel (XXV), in welcher J die oben angegebene Bedeutung hat und Verbindungen der Formel (I-1') bis (I-10'), in welchen A, B, D, G, m, Q1, Q2, Q3, Q4, Q5, Q6, X, Y, und J' die oben angegebene Bedeutung haben, im molaren Verhältnis 1:1 bis 3:1, vorzugsweise 1:1 bis 2:1 eingesetzt. Vom Katalysator setzt man im allgemeinen 0,005 bis 0,5 Mol, vorzugsweise 0,01 Mol bis 0,1 Mol pro Mol der Verbindungen der Formeln (I-1') bis (I-10') ein. Die Base setzt man im allgemeinen in einem Überschuss ein.

Die erfindungsgemäßen Wirkstoffe/Wirkstoffkombinationen eignen sich bei guter Pflanzenverträglichkeit, günstiger Warmblütertoxizität und guter Umweltverträglichkeit zum Schutz von Pflanzen und Pflanzenorganen, zur Steigerung der Ernteerträge, Verbesserung der Qualität des Erntegutes und zur Bekämpfung von tierischen Schädlingen, insbesondere Insekten, Spinnentieren, Helminthen, Nematoden und Mollusken, die in der Landwirtschaft, im Gartenbau, bei der Tierzucht, in Forsten, in Gärten und Freizeiteinrichtungen, im Vorrats- und Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören:

Aus der Ordnung der Anoplura (Phthiraptera) z.B. Damalinia spp., Haematopinus spp., Linognathus spp., Pediculus spp., Trichodectes spp.

Aus der Klasse der Arachnida z.B. Acarus siro, Aceria sheldoni, Aculops spp., Aculus spp., Amblyomma spp., Argas spp., Boophilus spp., Brevipalpus spp., Bryobia praetiosa, Chorioptes spp., Dermanyssus gallinae, Eotetranychus spp., Epitrimerus pyri, Eutetranychus spp., Eriophyes spp., Hemitarsonemus spp., Hyalomma spp., Ixodes spp., Latrodectus mactans, Metatetranychus spp., Oligonychus spp., Ornithodoros spp., Panonychus spp., Phyllocoptruta oleivora, Polyphagotarsonemus latus, Psoroptes spp., Rhipicephalus spp., Rhizoglyphus spp., Sarcoptes spp., Scorpio maurus, Stenotarsonemus spp., Tarsonemus spp., Tetranychus spp., Vasates lycopersici.

Aus der Klasse der Bivalva z.B. Dreissena spp.

Aus der Ordnung der Chilopoda z.B. Geophilus spp., Scutigera spp.

Aus der Ordnung der Coleoptera z.B. Acanthoscelides obtectus, Adoretus spp., Agelastica alni, Agriotes spp., Amphimallon solstitialis, Anobium punctatum, Anoplophora spp., Anthonomus spp., Anthrenus spp., Apogonia spp., Atomaria spp., Attagenus spp., Bruchidius obtectus, Bruchus spp., Ceuthorhynchus spp., Cleonus mendicus, Conoderus spp., Cosmopolites spp., Costelytra zealandica, Curculio spp., Cryptorhynchus lapathi, Dermestes spp., Diabrotica spp., Epilachna spp., Faustinus cubae, Gibbium psylloides, Heteronychus arator, Hylamorpha elegans, Hylotrupes bajulus, Hypera postica, Hypothenemus spp., Lachnosterna consanguinea, Leptinotarsa decemlineata, Lissorhoptrus oryzophilus, Lixus spp., Lyctus spp., Meligethes aeneus, Melolontha melolontha, Migdolus spp., Monochamus spp., Naupactus xanthographus, Niptus hololeucus, Oryctes rhinoceros, Oryzaephilus surinamensis, Otiorrhynchus sulcatus, Oxycetonia jucunda, Phaedon cochleariae, Phyllophaga spp., Popillia japonica, Premnotrypes spp., Psylliodes chrysocephala, Ptinus spp., Rhizobius ventralis, Rhizopertha dominica, Sitophilus spp., Sphenophorus spp., Sternechus spp., Symphyletes spp., Tenebrio molitor, Tribolium spp., Trogoderma spp., Tychius spp., Xylotrechus spp., Zabrus spp.

Aus der Ordnung der Collembola z.B. Onychiurus armatus.

Aus der Ordnung der Dermaptera z.B. Forficula auricularia.

Aus der Ordnung der Diplopoda z.B. Blaniulus guttulatus.

Aus der Ordnung der Diptera z.B. Aedes spp., Anopheles spp., Bibio hortulanus, Calliphora erythrocephala, Ceratitis capitata, Chrysomyia spp., Cochliomyia spp., Cordylobia anthropophaga, Culex spp., Cuterebra spp., Dacus oleae, Dermatobia hominis, Drosophila spp., Fannia spp., Gastrophilus spp., Hylemyia spp., Hyppobosca spp., Hypoderma spp., Liriomyza spp.. Lucilia spp., Musca spp., Nezara spp., Oestrus spp., Oscinella frit, Pegomyia hyoscyami, Phorbia spp., Stomoxys spp., Tabanus spp., Tannia spp., Tipula paludosa.

Aus der Klasse der Gastropoda z.B. Arion spp., Biomphalaria spp., Bulinus spp., Deroceras spp., Galba spp., Lymnaea spp., Oncomelania spp., Succinea spp.

Aus der Klasse der Helminthen z.B. Ancylostoma duodenale, Ancylostoma ceylanicum, Acylostoma braziliensis, Ancylostoma spp., Ascaris lubricoides, Ascaris spp., Brugia malayi, Brugia timori, Bunostomum spp., Chabertia spp., Clonorchis spp., Cooperia spp., Dicrocoelium spp, Dictyocaulus filaria, Diphyllobothrium latum, Dracunculus medinensis, Echinococcus granulosus, Echinococcus multilocularis, Enterobius vermicularis, Faciola spp., Haemonchus spp., Heterakis spp., Hymenolepis nana, Hyostrongulus spp., Loa Loa, Nematodirus spp., Oesophagostomum spp., Opisthorchis spp., Onchocerca volvulus, Ostertagia spp., Paragonimus spp., Schistosomen spp, Strongyloides fuelleborni, Strongyloides stercoralis, Stronyloides spp., Taenia saginata, Taenia solium, Trichinella spiralis, Trichinella nativa, Trichinella britovi, Trichinella nelsoni, Trichinella pseudopsiralis, Trichostrongulus spp., Trichuris trichuria, Wuchereria bancrofti.

Weiterhin lassen sich Protozoen, wie Eimeria, bekämpfen.

Aus der Ordnung der Heteroptera z.B. Anasa tristis, Antestiopsis spp., Blissus spp., Calocoris spp., Campylomma livida, Cavelerius spp., Cimex spp., Creontiades dilutus, Dasynus piperis, Dichelops furcatus, Diconocoris hewetti, Dysdercus spp., Euschistus spp., Eurygaster spp., Heliopeltis spp., Horcias nobilellus, Leptocorisa spp., Leptoglossus phyllopus, Lygus spp., Macropes excavatus, Miridae, Nezara spp., Oebalus spp., Pentomidae, Piesma quadrata, Piezodorus spp., Psallus seriatus, Pseudacysta persea, Rhodnius spp., Sahlbergella singularis, Scotinophora spp., Stephanitis nashi, Tibraca spp., Triatoma spp.

Aus der Ordnung der Homoptera z.B. Acyrthosipon spp., Aeneolamia spp., Agonoscena spp., Aleurodes spp., Aleurolobus barodensis, Aleurothrixus spp., Amrasca spp., Anuraphis cardui, Aonidiella spp., Aphanostigma piri, Aphis spp., Arboridia apicalis, Aspidiella spp., Aspidiotus spp., Atanus spp., Aulacorthum solani, Bemisia spp., Brachycaudus helichrysii, Brachycolus spp., Brevicoryne brassicae, Calligypona marginata, Carneocephala fulgida, Ceratovacuna lanigera, Cercopidae, Ceroplastes spp., Chaetosiphon fragaefolii, Chionaspis tegalensis, Chlorita onukii, Chromaphis juglandicola, Chrysomphalus ficus, Cicadulina mbila, Coccomytilus halli, Coccus spp., Cryptomyzus ribis, Dalbulus spp., Dialeurodes spp., Diaphorina spp., Diaspis spp., Drosicha spp., Dysaphis spp., Dysmicoccus spp., Empoasca spp., Eriosoma spp., Erythroneura spp., Euscelis bilobatus, Geococcus coffeae, Homalodisca coagulata, Hyalopterus arundinis, Icerya spp., Idiocerus spp., Idioscopus spp., Laodelphax striatellus, Lecanium spp., Lepidosaphes spp., Lipaphis erysimi, Macrosiphum spp., Mahanarva fimbriolata, Melanaphis sacchari, Metcalfiella spp., Metopolophium dirhodum, Monellia costalis, Monelliopsis pecanis, Myzus spp., Nasonovia ribisnigri, Nephotettix spp., Nilaparvata lugens, Oncometopia spp., Orthezia praelonga, Parabemisia myricae, Paratrioza spp., Parlatoria spp., Pemphigus spp., Peregrinus maidis, Phenacoccus spp., Phloeomyzus passerinii, Phorodon humuli, Phylloxera spp., Pinnaspis aspidistrae, Planococcus spp., Protopulvinaria pyriformis, Pseudaulacaspis pentagona, Pseudococcus spp., Psylla spp., Pteromalus spp., Pyrilla spp., Quadraspidiotus spp., Quesada gigas, Rastrococcus spp., Rhopalosiphum spp., Saissetia spp., Scaphoides titanus, Schizaphis graminum, Selenaspidus articulatus, Sogata spp., Sogatella furcifera, Sogatodes spp., Stictocephala festina, Tenalaphara malayensis, Tinocallis caryaefoliae, Tomaspis spp., Toxoptera spp., Trialeurodes vaporariorum, Trioza spp., Typhlocyba spp., Unaspis spp., Viteus vitifolii.

Aus der Ordnung der Hymenoptera z.B. Diprion spp., Hoplocampa spp., Lasius spp., Monomorium pharaonis, Vespa spp.

Aus der Ordnung der Isopoda z.B. Armadillidium vulgare, Oniscus asellus, Porcellio scaber.

Aus der Ordnung der Isoptera z.B. Reticulitermes spp.

Aus der Ordnung der Lepidoptera z.B. Acronicta major, Aedia leucomelas, Agrotis spp., Alabama argillacea, Anticarsia spp., Barathra brassicae, Bucculatrix thurberiella, Bupalus piniarius, Cacoecia podana, Capua reticulana, Carpocapsa pomonella, Cheimatobia brumata, Chilo spp., Choristoneura fumiferana, Clysia ambiguella, Cnaphalocerus spp., Earias insulana, Ephestia kuehniella, Euproctis chrysorrhoea, Euxoa spp., Feltia spp., Galleria mellonella, Helicoverpa spp., Heliothis spp., Hofmannophila pseudospretella, Homona magnanima, Hyponomeuta padella, Laphygma spp., Lithocolletis blancardella, Lithophane antennata, Loxagrotis albicosta, Lymantria spp., Malacosoma neustria, Mamestra brassicae, Mocis repanda, Mythimna separata, Oria spp., Oulema oryzae, Panolis flammea, Pectinophora gossypiella, Phyllocnistis citrella, Pieris spp., Plutella xylostella, Prodenia spp., Pseudaletia spp., Pseudoplusia includens, Pyrausta nubilalis, Spodoptera spp., Thermesia gemmatalis, Tinea pellionella, Tineola bisselliella, Tortrix viridana, Trichoplusia spp.

Aus der Ordnung der Orthoptera z.B. Acheta domesticus, Blatta orientalis, Blattella germanica, Gryllotalpa spp., Leucophaea maderae, Locusta spp., Melanoplus spp., Periplaneta americana, Schistocerca gregaria.

Aus der Ordnung der Siphonaptera z.B. Ceratophyllus spp., Xenopsylla cheopis.

Aus der Ordnung der Symphyla z.B. Scutigerella immaculata.

Aus der Ordnung der Thysanoptera z.B. Baliothrips biformis, Enneothrips flavens, Frankliniella spp., Heliothrips spp., Hercinothrips femoralis, Rhipiphorothrips cruentatus, Scirtothrips spp., Taeniothrips cardamoni, Thrips spp.

Aus der Ordnung der Thysanura z.B. Lepisma saccharina.

Zu den pflanzenparasitären Nematoden gehören z.B. Aphelenchoides spp., Bursaphelenchus spp., Ditylenchus dipsaci, Globodera spp., Heterodera spp., Longidorus spp., Meloidogyne spp., Pratylenchus spp., Radopholus similis, Trichodorus spp., Tylenchulus semipenetrans, Xiphinema spp.

Die erfindungsgemäßen Verbindungen/Wirkstoffkombinationen können gegebenenfalls in bestimmten Konzentrationen bzw. Aufwandmengen auch als Herbizide, Safener, Wachstumsregulatoren oder Mittel zur Verbesserung der Pflanzeneigenschaften, oder als Mikrobizide, beispielsweise als Fungizide, Antimykotika, Bakterizide, Virizide (einschließlich Mittel gegen Viroide) oder als Mittel gegen MLO (Mycoplasma-like-organism) und RLO (Rickettsia-like-organism) verwendet werden. Sie lassen sich gegebenenfalls auch als Zwischen- oder Vorprodukte für die Synthese weiterer Wirkstoffe einsetzen.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Spross, Blatt, Blüte und Wurzel verstanden werden, wobei beispielhaft Blätter, Nadeln, Stängel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den Wirkstoffen/Wirkstoffkombinationen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen, Injizieren und bei Vermehrungsmaterial, insbesondere bei Samen, weiterhin durch ein- oder mehrschichtiges Umhüllen.

Die Wirkstoffe/Wirkstoffkombinationen können in die üblichen Formulierungen überführt werden, wie Lösungen, Emulsionen, Spritzpulver, wasser- und ölbasierte Suspensionen, Pulver, Stäubemittel, Pasten, lösliche Pulver, lösliche Granulate, Streugranulate, Suspensions-Emulsions-Konzentrate, Wirkstoff-imprägnierte Naturstoffe, Wirkstoff-imprägnierte synthetische Stoffe, Düngemittel sowie Feinstverkapselungen in polymeren Stoffen.

Diese Formulierungen werden in bekannter Weise hergestellt, z.B. durch Vermischen der Wirkstoffe/Wirkstoffkombinationen mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylsulfoxid, sowie Wasser.

Als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstängeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Einweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im Allgemeinen zwischen 0,1 und 95 Gew.-% Wirkstoff, vorzugsweise zwischen 0,5 und 90 %.

Der erfindungsgemäße Wirkstoff/Wirkstoffkombinationen kann in seinen handelsüblichen Formulierungen sowie in den aus diesen Formulierungen bereiteten Anwendungsformen in Mischung mit anderen Wirkstoffen wie Insektiziden, Lockstoffen, Sterilantien, Bakteriziden, Akariziden, Nematiziden, Fungiziden, wachstumsregulierenden Stoffen, Herbiziden, Safenern, Düngemitteln oder Semiochemicals vorliegen.

### Besonders günstige Mischpartner sind z.B. die folgenden:

### Fungizide:

Inhibitoren der Nucleinsäure Synthese
   Benalaxyl, Benalaxyl-M, Bupirimat, Chiralaxyl, Clozylacon, Dimethirimol, Ethirimol, Furalaxyl, Hymexazol, Metalaxyl, Metalaxyl-M, Ofurace, Oxadixyl, Oxolinsäure
Inhibitoren der Mitose und Zellteilung
   Benomyl, Carbendazim, Diethofencarb, Fuberidazole, Pencycuron, Thiabendazol, Thiophanat-methyl, Zoxamid
Inhibitoren der Atmungskette Komplex I
   Diflumetorim
Inhibitoren der Atmungskette Komplex II
   Boscalid, Carboxin, Fenfuram, Flutolanil, Furametpyr, Mepronil, Oxycarboxin, Penthiopyrad, Thifluzamid
Inhibitoren der Atmungskette Komplex III
   Azoxystrobin, Cyazofamid, Dimoxystrobin, Enestrobin, Famoxadon, Fenamidon, Fluoxastrobin, Kresoximmethyl, Metominostrobin, Orysastrobin, Pyraclostrobin, Picoxystrobin, Trifloxystrobin
Entkoppler
   Dinocap, Fluazinam
Inhibitoren der ATP Produktion
   Fentinacetat, Fentinchlorid, Fentinhydroxid, Silthiofam
Inhibitoren der Aminosäure- und Proteinbiosynthese
   Andoprim, Blasticidin-S, Cyprodinil, Kasugamycin, Kasugamycinhydrochlorid Hydrat, Mepanipyrim, Pyrimethanil
Inhibitoren der Signal-Transduktion
   Fenpiclonil, Fludioxonil, Quinoxyfen
Inhibitoren der Fett- und Membran Synthese
   Chlozolinat, Iprodion, Procymidon, Vinclozolin
   Ampropylfos, Kalium-Ampropylfos, Edifenphos, Iprobenfos (IBP), Isoprothiolan, Pyrazophos
   Tolclofos-methyl, Biphenyl
   Iodocarb, Propamocarb, Propamocarb hydrochlorid
Inhibitoren der Ergosterol Biosynthese
   Fenhexamid,
   Azaconazol, Bitertanol, Bromuconazol, Cyproconazol, Diclobutrazol, Difenoconazol, Diniconazol, Diniconazol-M, Epoxiconazol, Etaconazol, Fenbuconazol, Fluquinconazol, Flusilazol, Flutriafol, Furconazol, Furconazol-cis, Hexaconazol, Imibenconazol, Ipconazol, Metconazol, Myclobutanil, Paclobutrazol, Penconazol, Propiconazol, Prothioconazol, Simeconazol, Tebuconazol, Tetraconazol, Triadimefon, Triadimenol, Triticonazol, Uniconazol, Voriconazol, Imazalil, Imazalilsulfat, Oxpoconazol, Fenarimol, Flurprimidol, Nuarimol, Pyrifenox, Triforin, Pefurazoat, Prochloraz, Triflumizol, Viniconazol,
   Aldimorph, Dodemorph, Dodemorphacetat, Fenpropimorph, Tridemorph, Fenpropidin, Spiroxamin,
   Naftifin, Pyributicarb, Terbinafin
Inhibitoren der Zellwand Synthese
   Benthiavalicarb, Bialaphos, Dimethomorph, Flumorph, Iprovalicarb, Polyoxins, Polyoxorim, Validamycin A
Inhibitoren der Melanin Biosynthese
   Capropamid, Diclocymet, Fenoxanil, Phtalid, Pyroquilon, Tricyclazol
Resistenzinduktion
   Acibenzolar-S-methyl, Probenazol, Tiadinil
Multisite
   Captafol, Captan, Chlorothalonil, Kupfersalze wie: Kupferhydroxid, Kupfernaphthenat, Kupferoxychlorid, Kupfersulfat, Kupferoxid, Oxin-Kupfer und Bordeaux Mischung, Dichlofluanid, Dithianon, Dodin, Dodin freie Base, Ferbam, Folpet, Fluorofolpet, Guazatin, Guazatinacetat, Iminoctadin, Iminoctadinalbesilat, Iminoctadintriacetat, Mankupfer, Mancozeb, Maneb, Metiram, Metiram Zink, Propineb, Schwefel und Schwefelpräparate enthaltend Calciumpolysulphid, Thiram, Tolylfluanid, Zineb, Ziram
Unbekannter Mechanismus
   Amibromdol, Benthiazol, Bethoxazin, Capsimycin, Carvon, Chinomethionat, Chloropicrin, Cufraneb, Cyflufenamid, Cymoxanil, Dazomet, Debacarb, Diclomezine, Dichlorophen, Dicloran, Difenzoquat, Difenzoquat Methylsulphat, Diphenylamin, Ethaboxam, Ferimzon, flumetover, Flusulfamid, Fluopicolid, Fluoroimid, Hexachlorobenzol, 8-Hydroxychinolinsulfat, Irumamycin, Methasulphocarb, Metrafenon, Methyl Isothiocyanat, Mildiomycin, Natamycin, Nickel dimethyldithiocarbamat, Nitrothal-isopropyl, Octhilinon, Oxamocarb, Oxyfenthiin, Pentachlorophenol und Salze, 2-Phenylphenol und Salze, Piperalin, Propanosin -Natrium, Proquinazid, Pyrrolnitrin, Quintozen, Tecloftalam, Tecnazen, Triazoxid, Trichlamid, Zarilamid und 2,3,5,6-Tetrachlor-4-(methylsulfonyl)-pyridin, N-(4-Chlor-2-nitrophenyl)-N-ethyl-4-methyl-benzenesulfonamid, 2-Amino-4-methyl-N-phenyl-5-thiazolecarboxamid, 2-Chlor-N-(2,3-dihydro-1,1,3-trimethyl-1H-inden-4-yl)-3-pyridincarboxamid, 3-[5-(4-Chlorphenyl)-2,3-dimethylisoxazolidin-3-yl]pyridin, cis-1-(4-Chlorphenyl)-2-(1H-1,2,4-triazol-1-yl)-cycloheptanol, 2,4-Dihydro-5-methoxy-2-methyl-4-[[[[1-[3-(trifluoromethyl)-phenyl]-ethyliden]-amino]-oxy]-methyl]-phenyl]-3H-1,2,3-triazol-3-on (185336-79-2), Methyl 1-(2,3-dihydro-2,2-dimethyl-1H-inden-1-yl)-1H-imidazole-5-carboxylat, 3,4,5-Trichlor-2,6-pyridindicarbonitril, Methyl 2-[[[cyclopropyl[(4-methoxyphenyl) imino]methyl]thio]methyl]-.alpha.-(methoxymethylen)- benzacetat, 4-Chlor-alpha-propinyloxy-N-[2-[3-methoxy-4-(2-propinyloxy)phenyl]ethyl]-benzacetamide, (2S)-N-[2-[4-[[3-(4-chlorophenyl)-2-propinyl]oxy]-3-methoxyphenyl]ethyl]-3-methyl-2-[(methylsulfonyl)amino]-butanamid, 5-Chlor-7-(4-methylpiperidin-1-yl)-6-(2,4,6-trifluorophenyl)-[1,2,4]triazolo[1,5-a]pyrimidin, 5-Chlor-6-(2,4,6-trifluorophenyl)-N-[(1R)-1,2,2-trimethyl-propyl][1,2,4]triazolo[1,5-a]pyrimidin-7-amin, 5-Chlor-N-[(1R)-1,2-dimethylpropyl]-6-(2,4,6-trifluorophenyl) [1,2,4]triazolo[1,5-a]pyrimidin-7-amine, N-[1-(5-Brom-3-chloro-pyridin-2-yl)ethyl]-2,4-dichloronicotinamid, N-(5-Brom-3-chlorpyridin-2-yl)methyl-2,4-dichlornicotinamid, 2-Butoxy-6-iod-3-propyl-benzopyranon-4-on, N-{(Z)-[(cyclopropyl-methoxy) imino][6-(difluormethoxy)-2,3-difluorphenyl]methyl}-2-benzacetamid, N-(3-Ethyl-3,5,5-trimethyl-cyclohexyl)-3-formylamino-2-hydroxy-benzamid, 2-[[[[1-[3(1Fluor-2-phenylethyl)oxy] phenyl] ethyliden]amino]oxy]methyl]-alpha-(methoxyimino)-N-methyl-alphaE-benzacetamid, N-{2-[3-Chlor-5-(trifluormethyl)pyridin-2-yl]ethyl}-2-(trifluoromethyl)-benzamid, N-(3',4'-dichlor-5-fluorbiphenyl-2-yl)-3-(difluormethyl)-1-methyl-1H-pyrazol-4-carboxamid, N-(6-Methoxy-3-pyridinyl)-cyclopropan carboxamid, 1-[(4-Methoxyphenoxy)-methyl]-2,2-dimethylpropyl-1H-imidazol-1- carbonsäure, O-[1-[(4-Methoxyphenoxy)-methyl]-2,2-dimethylpropyl]-1H-imidazol- 1- carbothioic acid, 2-(2-{[6-(3-Chlor-2-methyl-phenoxy)-5-fluorpyrimidin-4-yl]oxy}phenyl)-2-(methoxyimino)-N-methylacetamid

### Bakterizide:

Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Acetylcholinesterase (AChE) Inhibitoren
   Carbamate,
   zum Beispiel Alanycarb, Aldicarb, Aldoxycarb, Allyxycarb, Aminocarb, Bendiocarb, Benfuracarb, Bufencarb, Butacarb, Butocarboxim, Butoxycarboxim, Carbaryl, Carbofuran, Carbosulfan, Cloethocarb, Dimetilan, Ethiofencarb, Fenobucarb, Fenothiocarb, Formetanate, Furathiocarb, Isoprocarb, Metam-sodium, Methiocarb, Methomyl, Metolcarb, Oxamyl, Pirimicarb, Promecarb, Propoxur, Thiodicarb, Thiofanox, Trimethacarb, XMC, Xylylcarb, Triazamate
   Organophosphate,
   zum Beispiel Acephate, Azamethiphos, Azinphos (-methyl, -ethyl), Bromophos-ethyl, Bromfenvinfos (-methyl), Butathiofos, Cadusafos, Carbophenothion, Chlorethoxyfos, Chlorfenvinphos, Chlormephos, Chlorpyrifos (-methyl/-ethyl), Coumaphos, Cyanofenphos, Cyanophos, Chlorfenvinphos, Demeton-S-methyl, Demeton-S-methylsulphon, Dialifos, Diazinon, Dichlofenthion, Dichlorvos/DDVP, Dicrotophos, Dimethoate, Dimethylvinphos, Dioxabenzofos, Disulfoton, EPN, Ethion, Ethoprophos, Etrimfos, Famphur, Fenamiphos, Fenitrothion, Fensulfothion, Fenthion, Flupyrazofos, Fonofos, Formothion, Fosmethilan, Fosthiazate, Heptenophos, Iodofenphos, Iprobenfos, Isazofos, Isofenphos, Isopropyl O-salicylate, Isoxathion, Malathion, Mecarbam, Methacrifos, Methamidophos, Methidathion, Mevinphos, Monocrotophos, Naled, Omethoate, Oxydemeton-methyl, Parathion (-methyl/-ethyl), Phenthoate, Phorate, Phosalone, Phosmet, Phosphamidon, Phosphocarb, Phoxim, Pirimiphos (-methyl/-ethyl), Profenofos, Propaphos, Propetamphos, Prothiofos, Prothoate, Pyraclofos, Pyridaphenthion, Pyridathion, Quinalphos, Sebufos, Sulfotep, Sulprofos, Tebupirimfos, Temephos, Terbufos, Tetrachlorvinphos, Thiometon, Triazophos, Triclorfon, Vamidothion
Natrium-Kanal-Modulatoren / Spannungsabhängige Natrium-Kanal-Blocker
   Pyrethroide,
   zum Beispiel Acrinathrin, Allethrin (d-cis-trans, d-trans), Beta-Cyfluthrin, Bifenthrin, Bioallethrin, Bioallethrin-S-cyclopentyl-isomer, Bioethanomethrin, Biopermethrin, Bioresmethrin, Chlovaporthrin, Cis-Cypermethrin, Cis-Resmethrin, Cis-Permethrin, Clocythrin, Cycloprothrin, Cyfluthrin, Cyhalothrin, Cypermethrin (alpha-, beta-, theta-, zeta-), Cyphenothrin, Deltamethrin, Empenthrin (1R-isomer), Esfenvalerate, Etofenprox, Fenfluthrin, Fenpropathrin, Fenpyrithrin, Fenvalerate, Flubrocythrinate, Flucythrinate, Flufenprox, Flumethrin, Fluvalinate, Fubfenprox, Gamma-Cyhalothrin, Imiprothrin, Kadethrin, Lambda- Cyhalothrin, Metofluthrin, Permethrin (cis-, trans-), Phenothrin (1R-trans isomer), Prallethrin, Profluthrin, Protrifenbute, Pyresmethrin, Resmethrin, RU 15525, Silafluofen, Tau-Fluvalinate, Tefluthrin, Terallethrin, Tetramethrin (-1R- isomer), Tralomethrin, Transfluthrin, ZXI 8901, Pyrethrins (pyrethrum)
   DDT
   Oxadiazine,
   zum Beispiel Indoxacarb
   Semicarbazone,
   zum Beispiel Metaflumizone (BAS 3201)
Acetylcholin-Rezeptor-Agonisten/-Antagonisten
   Chloronicotinyle,
   zum Beispiel Acetamiprid, Clothianidin, Dinotefuran, Imidacloprid, Nitenpyram, Nithiazine, Thiacloprid, Thiamethoxam
   Nicotine, Bensultap, Cartap
Acetylcholin-Rezeptor-Modulatoren
   Spinosyne,
   zum Beispiel Spinosad
GABA-gesteuerte Chlorid-Kanal-Antagonisten
   Organochlorine,
   zum Beispiel Camphechlor, Chlordane, Endosulfan, Gamma-HCH, HCH, Heptachlor, Lindane, Methoxychlor
   Fiprole,
   zum Beispiel Acetoprole, Ethiprole, Fipronil, Pyrafluprole, Pyriprole, Vaniliprole
Chlorid-Kanal-Aktivatoren
   Mectine,
   zum Beispiel Avermectin, Emamectin, Emamectin-benzoate, Ivermectin, Milbemycin
Juvenilhormon-Mimetika,
   zum Beispiel Diofenolan, Epofenonane, Fenoxycarb, Hydroprene, Kinoprene, Methoprene, Pyriproxifen, Triprene
Ecdysonagonisten/disruptoren
   Diacylhydrazine,
   zum Beispiel Chromafenozide, Halofenozide, Methoxyfenozide, Tebufenozide
Inhibitoren der Chitinbiosynthese
   Benzoylharnstoffe,
   zum Beispiel Bistrifluron, Chlofluazuron, Diflubenzuron, Fluazuron, Flucycloxuron, Flufenoxuron, Hexaflumuron, Lufenuron, Novaluron, Noviflumuron, Penfluron, Teflubenzuron, Triflumuron
   Buprofezin
   Cyromazine
Inhibitoren der oxidativen Phosphorylierung, ATP-Disruptoren
   Diafenthiuron
   Organozinnverbindungen,
   zum Beispiel Azocyclotin, Cyhexatin, Fenbutatin-oxide
Entkoppler der oxidativen Phoshorylierung durch Unterbrechung des H-Protongradienten
   Pyrrole,
   zum Beispiel Chlorfenapyr
   Dinitrophenole,
   zum Beispiel Binapacyrl, Dinobuton, Dinocap, DNOC
Seite-I-Elektronentransportinhibitoren
   METI's,
   zum Beispiel Fenazaquin, Fenpyroximate, Pyrimidifen, Pyridaben, Tebufenpyrad, Tolfenpyrad
   Hydramethylnon
   Dicofol
Seite-II-Elektronentransportinhibitoren
   Rotenone
Seite-III-Elektronentransportinhibitoren
   Acequinocyl, Fluacrypyrim
Mikrobielle Disruptoren der Insektendarmmembran
   Bacillus thuringiensis-Stämme
Inhibitoren der Fettsynthese
   Tetronsäuren,
   zum Beispiel Spirodiclofen, Spiromesifen
   Tetramsäuren,
   zum Beispiel Spirotetramat
Carboxamide,
   zum Beispiel Flonicamid
Oktopaminerge Agonisten,
   zum Beispiel Amitraz
Inhibitoren der Magnesium-stimulierten ATPase,
   Propargite
Ryanodinrezeptor-Effektoren
   a) Benzoesäuredicarboxamide,
      zum Beispiel Flubendiamide
   b) Anthranilamide, z.B.
      Rynaxapyr (3-bromo-N-{4-chloro-2-methyl-6-[(methylamino)carbonyl]phenyl}-1-(3-chloropyridin-2-yl)-1H-pyrazole-5-carboxamide)
Nereistoxin-Analoge,
   zum Beispiel Thiocyclam hydrogen oxalate, Thiosultap-sodium
Biologika, Hormone oder Pheromone
   Azadirachtin, Bacillus spec., Beauveria spec., Codlemone, Metarrhizium spec., Paecilomyces spec., Thuringiensin, Verticillium spec.
Wirkstoffe mit unbekannten oder nicht spezifischen Wirkmechanismen
   Begasungsmittel,
   zum Beispiel Aluminium phosphide, Methyl bromide, Sulfuryl fluoride
   Fraßhemmer,
   zum Beispiel Cryolite, Flonicamid, Pymetrozine
   Milbenwachstumsinhibitoren,
   zum Beispiel Clofentezine, Etoxazole, Hexythiazox
   Amidoflumet, Benclothiaz, Benzoximate, Bifenazate, Bromopropylate, Buprofezin, Chinomethionat, Chlordimeform, Chlorobenzilate, Chloropicrin, Clothiazoben, Cycloprene, Cyflumetofen, Dicyclanil, Fenoxacrim, Fentrifanil, Flubenzimine, Flufenerim, Flutenzin, Gossyplure, Hydramethylnone, Japonilure, Metoxadiazone, Petroleum, Piperonyl butoxide, Potassium oleate, Pyridalyl, Sulfluramid, Tetradifon, Tetrasul, Tri-arathene,Verbutin

Auch eine Mischung mit anderen bekannten Wirkstoffen, wie Herbiziden, Düngemitteln, Wachstumsregulatoren, Safenern, Semiochemicals, oder auch mit Mitteln zur Verbesserung der Pflanzeneigenschaften ist möglich.

Die erfindungsgemäßen Wirkstoffe/Wirkstoffkombinationen können ferner beim Einsatz als Insektizide in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muss.

Die erfindungsgemäßen Wirkstoffe/Wirkstoffkombinationen können ferner beim Einsatz als Insektizide in ihren handelsüblichen Formulierungen sowie in den aus diesen Formulierungen bereiteten Anwendungsformen in Mischungen mit Hemmstoffen vorliegen, die einen Abbau des Wirkstoffes nach Anwendung in der Umgebung der Pflanze, auf der Oberfläche von Pflanzenteilen oder in pflanzlichen Geweben vermindern.

Der Wirkstoffgehalt der aus den handelsüblichen Formulierungen bereiteten Anwendungsformen kann in weiten Bereichen variieren. Die Wirkstoffkonzentration der Anwendungsformen kann von 0,00000001 bis zu 95 Gew.-% Wirkstoff, vorzugsweise zwischen 0,00001 und 1 Gew.-% liegen.

Die Anwendung geschieht in einer den Anwendungsformen angepassten üblichen Weise.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetic Modified Organisms) und deren Teile behandelt. Die Begriffe "Teile" bzw. "Teile von Pflanzen" oder "Pflanzenteile" wurden oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Bio- und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Zuckerrüben, Tomaten, Erbsen und andere Gemüsesorten, Baumwolle, Tabak, Raps, sowie Obstpflanzen (mit den Früchten Äpfel, Birnen, Zitrusfrüchten und Weintrauben) erwähnt, wobei Mais, Soja, Kartoffel, Baumwolle, Tabak und Raps besonders hervorgehoben werden. Als Eigenschaften ("Traits") werden besonders hervorgehoben die erhöhte Abwehr der Pflanzen gegen Insekten, Spinnentiere, Nematoden und Schnecken durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden (im folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD® (z.B. Mais, Baumwolle, Soja), KnockOut® (z.B. Mais), StarLink® (z.B. Mais), Bollgard® (Baumwolle), Nucotn® (Baumwolle) und NewLeaf® (Kartoffel) vertrieben werden. Als Beispiele für Herbizid-tolerante Pflanzen seien Maissorten, Baumwollsorten und Sojasorten genannt, die unter den Handelsbezeichnungen Roundup Ready® (Toleranz gegen Glyphosate z.B. Mais, Baumwolle, Soja), Liberty Link® (Toleranz gegen Phosphinotricin, z.B. Raps), IMI® (Toleranz gegen Imidazolinone) und STS® (Toleranz gegen Sulfonylharnstoffe z.B. Mais) vertrieben werden. Als Herbizid- resistente (konventionell auf Herbizid-Toleranz gezüchtete) Pflanzen seien auch die unter der Bezeichnung Clearfield® vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft erfindungsgemäß mit den Verbindungen der allgemeinen Formel I bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Die erfindungsgemäßen Wirkstoffe/Wirkstoffkombinationen wirken nicht nur gegen Pflanzen-, Hygiene- und Vorratsschädlinge, sondern auch auf dem veterinärmedizinischen Sektor gegen tierische Parasiten (Ekto- und Endoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:

Aus der Ordnung der Anoplurida z.B. Haematopinus spp., Linognathus spp., Pediculus spp., Phtirus spp., Solenopotes spp..

Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp., Felicola spp..

Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp..

Aus der Ordnung der Siphonapterida z.B. Pulex spp., Ctenocephalides spp., Xenopsylla spp., Ceratophyllus spp..

Aus der Ordnung der Heteropterida z.B. Cimex spp., Triatoma spp., Rhodnius spp., Panstrongylus spp..

Aus der Ordnung der Blattarida z.B. Blatta orientalis, Periplaneta americana, Blattela germanica, Supella spp..

Aus der Unterklasse der Acari (Acarina) und den Ordnungen der Meta- sowie Mesostigmata z.B. Argas spp., Ornithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia spp., Pneumonyssus spp., Sternostoma spp., Varroa spp..

Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Die erfindungsgemäßen Wirkstoffe/Wirkstoffkombinationen der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so dass durch den Einsatz der erfindungsgemäßen Wirkstoffe eine wirtschaftlichere und einfachere Tierhaltung möglich ist.

Die Anwendung der erfindungsgemäßen Wirkstoffe/Wirkstoffkombinationen geschieht im Veterinärsektor und bei der Tierhaltung in bekannter Weise durch enterale Verabreichung in Form von beispielsweise Tabletten, Kapseln, Tränken, Drenchen, Granulaten, Pasten, Boli, des feedthrough-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffe der Formel (I) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, daß die erfindungsgemäßen Verbindungen/Wirkstoffkombinationen eine hohe insektizide Wirkung gegen Insekten zeigen, die technische Materialien zerstören.

Beispielhaft und vorzugsweise - ohne jedoch zu limitieren - seien die folgenden Insekten genannt:

Käfer wie Hylotrupes bajulus, Chlorophorus pilosis, Anobium punctatum, Xestobium rufovillosum, Ptilinus pecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus;

Hautflügler wie Sirex juvencus, Urocerus gigas, Urocerus gigas taignus, Urocerus augur;

Termiten wie Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus;

Borstenschwänze wie Lepisma saccharina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz, Holzverarbeitungsprodukte und Anstrichmittel.

Ganz besonders bevorzugt handelt es sich bei dem vor Insektenbefall zu schützenden Material um Holz und Holzverarbeitungsprodukte.

Unter Holz und Holzverarbeitungsprodukten, welche durch das erfindungsgemäße Mittel bzw. dieses enthaltende Mischungen geschützt werden kann, ist beispielhaft zu verstehen:

Bauholz, Holzbalken, Eisenbahnschwellen, Brückenteile, Bootsstege, Holzfahrzeuge, Kisten, Paletten, Container, Telefonmasten, Holzverkleidungen, Holzfenster und -türen, Sperrholz, Spanplatten, Tischlerarbeiten oder Holzprodukte, die ganz allgemein beim Hausbau oder in der Bautischlerei Verwendung finden.

Die Wirkstoffe können als solche, in Form von Konzentraten oder allgemein üblichen Formulierungen wie Pulver, Granulate, Lösungen, Suspensionen, Emulsionen oder Pasten angewendet werden.

Die genannten Formulierungen können in an sich bekannter Weise hergestellt werden, z.B. durch Vermischen der Wirkstoffe mit mindestens einem Lösungs- bzw. Verdünnungsmittel, Emulgator, Dispergier- und/oder Binde- oder Fixiermittels, Wasser-Repellent, gegebenenfalls Sikkative und UV-Stabilisatoren und gegebenenfalls Farbstoffen und Pigmenten sowie weiteren Verarbeitungshilfsmitteln.

Die zum Schutz von Holz und Holzwerkstoffen verwendeten insektiziden Mittel oder Konzentrate enthalten den erfindungsgemäßen Wirkstoff in einer Konzentration von 0,0001 bis 95 Gew.-%, insbesondere 0,001 bis 60 Gew.-%.

Die Menge der eingesetzten Mittel bzw. Konzentrate ist von der Art und dem Vorkommen der Insekten und von dem Medium abhängig. Die optimale Einsatzmenge kann bei der Anwendung jeweils durch Testreihen ermittelt werden. Im Allgemeinen ist es jedoch ausreichend 0,0001 bis 20 Gew.-%, vorzugsweise 0,001 bis 10 Gew.-%, des Wirkstoffs, bezogen auf das zu schützende Material, einzusetzen.

Als Lösungs- und/oder Verdünnungsmittel dient ein organisch-chemisches Lösungsmittel oder Lösungsmittelgemisch und/oder ein öliges oder ölartiges schwer flüchtiges organisch-chemisches Lösungsmittel oder Lösungsmittelgemisch und/oder ein polares organisch-chemisches Lösungsmittel oder Lösungsmittelgemisch und/oder Wasser und gegebenenfalls einen Emulgator und/oder Netzmittel.

Als organisch-chemische Lösungsmittel werden vorzugsweise ölige oder ölartige Lösungsmittel mit einer Verdunstungszahl über 35 und einem Flammpunkt oberhalb 30°C, vorzugsweise oberhalb 45°C, eingesetzt. Als derartige schwerflüchtige, wasserunlösliche, ölige und ölartige Lösungsmittel werden entsprechende Mineralöle oder deren Aromatenfraktionen oder mineralölhaltige Lösungsmittelgemische, vorzugsweise Testbenzin, Petroleum und/oder Alkylbenzol verwendet.

Vorteilhaft gelangen Mineralöle mit einem Siedebereich von 170 bis 220°C, Testbenzin mit einem Siedebereich von 170 bis 220°C, Spindelöl mit einem Siedebereich von 250 bis 350°C, Petroleum bzw. Aromaten vom Siedebereich von 160 bis 280°C, Terpentinöl und dgl. zum Einsatz.

In einer bevorzugten Ausführungsform werden flüssige aliphatische Kohlenwasserstoffe mit einem Siedebereich von 180 bis 210°C oder hochsiedende Gemische von aromatischen und aliphatischen Kohlenwasserstoffen mit einem Siedebereich von 180 bis 220°C und/oder Spindeöl und/oder Monochlornaphthalin, vorzugsweise α-Monochlornaphthalin, verwendet.

Die organischen schwerflüchtigen öligen oder ölartigen Lösungsmittel mit einer Verdunstungszahl über 35 und einem Flammpunkt oberhalb 30°C, vorzugsweise oberhalb 45°C, können teilweise durch leicht oder mittelflüchtige organisch-chemische Lösungsmittel ersetzt werden, mit der Maßgabe, dass das Lösungsmittelgemisch ebenfalls eine Verdunstungszahl über 35 und einen Flammpunkt oberhalb 30°C, vorzugsweise oberhalb 45°C, aufweist und dass das Insektizid-Fungizid-Gemisch in diesem Lösungsmittelgemisch löslich oder emulgierbar ist.

Nach einer bevorzugten Ausführungsform wird ein Teil des organisch-chemischen Lösungsmittel oder Lösungsmittelgemisches oder ein aliphatisches polares organisch-chemisches Lösungsmittel oder Lösungsmittelgemisch ersetzt. Vorzugsweise gelangen Hydroxyl- und/oder Ester- und/oder Ethergruppen enthaltende aliphatische organisch-chemische Lösungsmittel wie beispielsweise Glycolether, Ester oder dgl. zur Anwendung.

Als organisch-chemische Bindemittel werden im Rahmen der vorliegenden Erfindung die an sich bekannten wasserverdünnbaren und/oder in den eingesetzten organisch-chemischen Lösungsmitteln löslichen oder dispergier- bzw. emulgierbaren Kunstharze und/oder bindende trocknende Öle, insbesondere Bindemittel bestehend aus oder enthaltend ein Acrylatharz, ein Vinylharz, z.B. Polyvinylacetat, Polyesterharz, Polykondensations- oder Polyadditionsharz, Polyurethanharz, Alkydharz bzw. modifiziertes Alkydharz, Phenolharz, Kohlenwasserstoffharz wie Inden-Cumaronharz, Siliconharz, trocknende pflanzliche und/oder trocknende Öle und/oder physikalisch trocknende Bindemittel auf der Basis eines Natur- und/oder Kunstharzes verwendet.

Das als Bindemittel verwendete Kunstharz kann in Form einer Emulsion, Dispersion oder Lösung, eingesetzt werden. Als Bindemittel können auch Bitumen oder bituminöse Substanzen bis zu 10 Gew.-%, verwendet werden. Zusätzlich können an sich bekannte Farbstoffe, Pigmente, wasserabweisende Mittel, Geruchskorrigentien und Inhibitoren bzw. Korrosionsschutzmittel und dgl. eingesetzt werden.

Bevorzugt ist gemäß der Erfindung als organisch-chemische Bindemittel mindestens ein Alkydharz bzw. modifiziertes Alkydharz und/oder ein trocknendes pflanzliches Öl im Mittel oder im Konzentrat enthalten. Bevorzugt werden gemäß der Erfindung Alkydharze mit einem Ölgehalt von mehr als 45 Gew.-%, vorzugsweise 50 bis 68 Gew.-%, verwendet.

Das erwähnte Bindemittel kann ganz oder teilweise durch ein Fixierungsmittel(gemisch) oder ein Weichmacher(gemisch) ersetzt werden. Diese Zusätze sollen einer Verflüchtigung der Wirkstoffe sowie einer Kristallisation bzw. Ausfällem vorbeugen. Vorzugsweise ersetzen sie 0,01 bis 30 % des Bindemittels (bezogen auf 100 % des eingesetzten Bindemittels).

Die Weichmacher stammen aus den chemischen Klassen der Phthalsäureester wie Dibutyl-, Dioctyl- oder Benzylbutylphthalat, Phosphorsäureester wie Tributylphosphat, Adipinsäureester wie Di-(2-ethylhexyl)-adipat, Stearate wie Butylstearat oder Amylstearat, Oleate wie Butyloleat, Glycerinether oder höhermolekulare Glykolether, Glycerinester sowie p-Toluolsulfonsäureester.

Fixierungsmittel basieren chemisch auf Polyvinylalkylethern wie z.B. Polyvinylmethylether oder Ketonen wie Benzophenon, Ethylenbenzophenon.

Als Lösungs- bzw. Verdünnungsmittel kommt insbesondere auch Wasser in Frage, gegebenenfalls in Mischung mit einem oder mehreren der oben genannten organisch-chemischen Lösungs- bzw. Verdünnungsmittel, Emulgatoren und Dispergatoren.

Ein besonders effektiver Holzschutz wird durch großtechnische Imprägnierverfahren, z.B. Vakuum, Doppelvakuum oder Druckverfahren, erzielt.

Die anwendungsfertigen Mittel können gegebenenfalls noch weitere Insektizide und gegebenenfalls noch ein oder mehrere Fungizide enthalten.

Als zusätzliche Zumischpartner kommen vorzugsweise die in der WO 94/29 268 genannten Insektizide und Fungizide in Frage. Die in diesem Dokument genannten Verbindungen sind ausdrücklicher Bestandteil der vorliegenden Anmeldung.

Als ganz besonders bevorzugte Zumischpartner können Insektizide, wie Chlorpyriphos, Phoxim, Silafluofin, Alphamethrin, Cyfluthrin, Cypermethrin, Deltamethrin, Permethrin, Imidacloprid, NI-25, Flufenoxuron, Hexaflumuron, Transfluthrin, Thiacloprid, Methoxyphenoxid, Triflumuron, Chlothianidin, Spinosad, Tefluthrin,
sowie Fungizide wie Epoxyconazole, Hexaconazole, Azaconazole, Propiconazole, Tebuconazole, Cyproconazole, Metconazole, Imazalil, Dichlorfluanid, Tolylfluanid, 3-Iod-2-propinyl-butylcarbamat, N-Octyl-isothiazolin-3-on und 4,5-Dichlor-N-octylisothiazolin-3-on, sein.

Zugleich können die erfindungsgemäßen Verbindungen zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, eingesetzt werden.

Bewuchs durch sessile Oligochaeten, wie Kalkröhrenwürmer sowie durch Muscheln und Arten der Gruppe Ledamorpha (Entenmuscheln), wie verschiedene Lepas- und Scalpellum-Arten, oder durch Arten der Gruppe Balanomorpha (Seepocken), wie Balanus- oder Pollicipes-Species, erhöht den Reibungswiderstand von Schiffen und führt in der Folge durch erhöhten Energieverbrauch und darüber hinaus durch häufige Trockendockaufenthalte zu einer deutlichen Steigerung der Betriebskosten.

Neben dem Bewuchs durch Algen, beispielsweise Ectocarpus sp. und Ceramium sp., kommt insbesondere dem Bewuchs durch sessile Entomostraken-Gruppen, welche unter dem Namen Cirripedia (Rankenflusskrebse) zusammengefasst werden, besondere Bedeutung zu.

Es wurde nun überraschenderweise gefunden, dass die erfindungsgemäßen Verbindungen allein oder in Kombination mit anderen Wirkstoffen, eine hervorragende Antifouling (Antibewuchs)-Wirkung aufweisen.

Durch Einsatz von erfindungsgemäßen Verbindungen allein oder in Kombination mit anderen Wirkstoffen, kann auf den Einsatz von Schwermetallen wie z.B. in Bis(trialkylzinn)-sulfiden, Tri-n-butylzinnlaurat, Tri-n-butylzinnchlorid, Kupfer(I)-oxid, Triethylzinnchlorid, Tri-n-butyl(2-phenyl-4-chlorphenoxy)-zinn, Tributylzinnoxid, Molybdändisulfid, Antimonoxid, polymerem Butyltitanat, Phenyl-(bispyridin)-wismutchlorid, Tri-n-butylzinnfluorid, Manganethylenbisthiocarbamat, Zinkdimethyldithiocarbamat, Zinkethylenbisthiocarbamat, Zink- und Kupfersalze von 2-Pyridinthiol-1-oxid, Bisdimethyldithiocarbamoylzinkethylenbisthiocarbamat, Zinkoxid, Kupfer(I)-ethylen-bisdithiocarbamat, Kupferthiocyanat, Kupfernaphthenat und Tributylzinnhalogeniden verzichtet werden oder die Konzentration dieser Verbindungen entscheidend reduziert werden.

Die anwendungsfertigen Antifoulingfarben können gegebenenfalls noch andere Wirkstoffe, vorzugsweise Algizide, Fungizide, Herbizide, Molluskizide bzw. andere Antifouling-Wirkstoffe enthalten.

Als Kombinationspartner für die erfindungsgemäßen Antifouling-Mittel eignen sich vorzugsweise:
Algizide wie
   2-*tert*.-Butylamino-4-cyclopropylamino-6-methylthio-1,3,5-triazin, Dichlorophen, Diuron, Endothal, Fentinacetat, Isoproturon, Methabenzthiazuron, Oxyfluorfen, Quinoclamine und Terbutryn;
Fungizide wie
   Benzo[*b*]thiophencarbonsäurecyclohexylamid-S,S-dioxid, Dichlofluanid, Fluorfolpet, 3-Iod-2-propinyl-butylcarbamat, Tolylfluanid und Azole wie
   Azaconazole, Cyproconazole, Epoxyconazole, Hexaconazole, Metconazole, Propiconazole und Tebuconazole;
Molluskizide wie
Fentinacetat, Metaldehyd, Methiocarb, Niclosamid, Thiodicarb und Trimethacarb, Fe-chelate;
   oder herkömmliche Antifouling-Wirkstoffe wie
   4,5-Dichlor-2-octyl-4-isothiazolin-3-on, Diiodmethylparatrylsulfon, 2-(N,N-Dimethylthiocarbamoylthio)-5-nitrothiazyl, Kalium-, Kupfer-, Natrium- und Zinksalze von 2-Pyridinthiol-1-oxid, Pyridin-triphenylboran, Tetrabutyldistannoxan, 2,3,5,6-Tetrachlor-4-(methylsulfonyl)-pyridin, 2,4,5,6-Tetrachloroisophthalonitril, Tetramethylthiuramdisulfid und 2,4,6-Trichlorphenylmaleinimid.

Die verwendeten Antifouling-Mittel enthalten die erfindungsgemäßen Wirkstoff der erfindungsgemäßen Verbindungen in einer Konzentration von 0,001 bis 50 Gew.-%, insbesondere von 0,01 bis 20 Gew.-%.

Die erfindungsgemäßen Antifouling-Mittel enthalten desweiteren die üblichen Bestandteile wie z.B. in Ungerer, Chem. Ind. 1985, 37, 730-732 und Williams, Antifouling Marine Coatings, Noyes, Park Ridge, 1973 beschrieben.

Antifouling-Anstrichmittel enthalten neben den algiziden, fungiziden, molluskiziden und erfindungsgemäßen insektiziden Wirkstoffen insbesondere Bindemittel.

Beispiele für anerkannte Bindemittel sind Polyvinylchlorid in einem Lösungsmittelsystem, chlorierter Kautschuk in einem Lösungsmittelsystem, Acrylharze in einem Lösungsmittelsystem insbesondere in einem wässrigen System, Vinylchlorid/Vinylacetat-Copolymersysteme in Form wässriger Dispersionen oder in Form von organischen Lösungsmittelsystemen, Butadien/Styrol/Acrylnitril-Kautschuke, trocknende Öle, wie Leinsamenöl, Harzester oder modifizierte Hartharze in Kombination mit Teer oder Bitumina, Asphalt sowie Epoxyverbindungen, geringe Mengen Chlorkautschuk, chloriertes Polypropylen und Vinylharze.

Gegebenenfalls enthalten Anstrichmittel auch anorganische Pigmente, organische Pigmente oder Farbstoffe, welche vorzugsweise in Seewasser unlöslich sind. Ferner können Anstrichmittel Materialien, wie Kolophonium enthalten, um eine gesteuerte Freisetzung der Wirkstoffe zu ermöglichen. Die Anstriche können ferner Weichmacher, die rheologischen Eigenschaften beeinflussende Modifizierungsmittel sowie andere herkömmliche Bestandteile enthalten. Auch in Self-Polishing-Antifouling-Systemen können die erfindungsgemäßen Verbindungen oder die oben genannten Mischungen eingearbeitet werden.

Die Wirkstoffe eignen sich auch zur Bekämpfung von tierischen Schädlingen, insbesondere von Insekten, Spinnentieren und Milben, die in geschlossenen Räumen, wie beispielsweise Wohnungen, Fabrikhallen, Büros, Fahrzeugkabinen u.ä. vorkommen. Sie können zur Bekämpfung dieser Schädlinge allein oder in Kombination mit anderen Wirk- und Hilfsstoffen in Haushaltsinsektizid-Produkten verwendet werden. Sie sind gegen sensible und resistente Arten sowie gegen alle Entwicklungsstadien wirksam. Zu diesen Schädlingen gehören:

Aus der Ordnung der Scorpionidea z.B. Buthus occitanus.

Aus der Ordnung der Acarina z.B. Argas persicus, Argas reflexus, Bryobia ssp., Dermanyssus gallinae, Glyciphagus domesticus, Ornithodorus moubat, Rhipicephalus sanguineus, Trombicula alfreddugesi, Neutrombicula autumnalis, Dermatophagoides pteronissimus, Dermatophagoides forinae.

Aus der Ordnung der Araneae z.B. Aviculariidae, Araneidae.

Aus der Ordnung der Opiliones z.B. Pseudoscorpiones chelifer, Pseudoscorpiones cheiridium, Opiliones phalangium.

Aus der Ordnung der Isopoda z.B. Oniscus asellus, Porcellio scaber.

Aus der Ordnung der Diplopoda z.B. Blaniulus guttulatus, Polydesmus spp.

Aus der Ordnung der Chilopoda z.B. Geophilus spp.

Aus der Ordnung der Zygentoma z.B. Ctenolepisma spp., Lepisma saccharina, Lepismodes inquilinus.

Aus der Ordnung der Blattaria z.B. Blatta orientalies, Blattella germanica, Blattella asahinai, Leucophaea maderae, Panchlora spp., Parcoblatta spp., Periplaneta australasiae, Periplaneta americana, Periplaneta brunnea, Periplaneta fuliginosa, Supella longipalpa.

Aus der Ordnung der Saltatoria z.B. Acheta domesticus.

Aus der Ordnung der Dermaptera z.B. Forficula auricularia.

Aus der Ordnung der Isoptera z.B. Kalotermes spp., Reticulitermes spp.

Aus der Ordnung der Psocoptera z.B. Lepinatus spp., Liposcelis spp.

Aus der Ordnung der Coloptera z.B. Anthrenus spp., Attagenus spp., Dermestes spp., Latheticus oryzae, Necrobia spp., Ptinus spp., Rhizopertha dominica, Sitophilus granarius, Sitophilus oryzae, Sitophilus zeamais, Stegobium paniceum.

Aus der Ordnung der Diptera z.B. Aedes aegypti, Aedes albopictus, Aedes taeniorhynchus, Anopheles spp., Calliphora erythrocephala, Chrysozona pluvialis, Culex quinquefasciatus, Culex pipiens, Culex tarsalis, Drosophila spp., Fannia canicularis, Musca domestica, Phlebotomus spp., Sarcophaga carnaria, Simulium spp., Stomoxys calcitrans, Tipula paludosa.

Aus der Ordnung der Lepidoptera z.B. Achroia grisella, Galleria mellonella, Plodia interpunctella, Tinea cloacella, Tinea pellionella, Tineola bisselliella.

Aus der Ordnung der Siphonaptera z.B. Ctenocephalides canis, Ctenocephalides felis, Pulex irritans, Tunga penetrans, Xenopsylla cheopis.

Aus der Ordnung der Hymenoptera z.B. Camponotus herculeanus, Lasius fuliginosus, Lasius niger, Lasius umbratus, Monomorium pharaonis, Paravespula spp., Tetramorium caespitum.

Aus der Ordnung der Anoplura z.B. Pediculus humanus capitis, Pediculus humanus corporis, Phthirus pubis.

Aus der Ordnung der Heteroptera z.B. Cimex hemipterus, Cimex lectularius, Rhodinus prolixus, Triatoma infestans.

Die Anwendung im Bereich der Haushaltsinsektizide erfolgt allein oder in Kombination mit anderen geeigneten Wirkstoffen wie Phosphorsäureestern, Carbamaten, Pyrethroiden, Neonicotinoiden, Wachstumsregulatoren oder Wirkstoffen aus anderen bekannten Insektizidklassen.

Die Anwendung erfolgt in Aerosolen, drucklosen Sprühmitteln, z.B. Pump- und Zerstäubersprays, Nebelautomaten, Foggern, Schäumen, Gelen, Verdampferprodukten mit Verdampferplättchen aus Cellulose oder Kunststoff, Flüssigverdampfern, Gel- und Membranverdampfern, propellergetriebenen Verdampfern, energielosen bzw. passiven Verdampfungssystemen, Mottenpapieren, Mottensäckchen und Mottengelen, als Granulate oder Stäube, in Streuködern oder Köderstationen.

Die erfindungsgemäßen Wirkstoffe/Wirkstoffkombinationen können auch als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe/Wirkstoffkombinationen können z.B. bei den folgenden Pflanzen verwendet werden:

Dikotyle Unkräuter der Gattungen: Abutilon, Amaranthus, Ambrosia, Anoda, Anthemis, Aphanes, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, Ipomoea, Kochia, Lamium, Lepidium, Lindernia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola, Xanthium.

Dikotyle Kulturen der Gattungen: Arachis, Beta, Brassica, Cucumis, Cucurbita, Helianthus, Daucus, Glycine, Gossypium, Ipomoea, Lactuca, Linum, Lycopersicon, Nicotiana, Phaseolus, Pisum, Solanum, Vicia.

Monokotyle Unkräuter der Gattungen: Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria, Sorghum.

Monokotyle Kulturen der Gattungen: Allium, Ananas, Asparagus, Avena, Hordeum, Oryza, Panicum, Saccharum, Secale, Sorghum, Triticale, Triticum, Zea.

Die Verwendung der erfindungsgemäßen Wirkstoffe/Wirkstoffkombinationen ist jedoch keineswegs auf diese Gattungen beschränkt, sondern erstreckt sich in gleicher Weise auch auf andere Pflanzen.

Die erfindungsgemäßen Wirkstoffe/Wirkstoffkombinationen eignen sich in Abhängigkeit von der Konzentration zur Totalunkrautbekämpfung, z.B. auf Industrie- und Gleisanlagen und auf Wegen und Plätzen mit und ohne Baumbewuchs. Ebenso können die erfindungsgemäßen Wirkstoffe zur Unkrautbekämpfung in Dauerkulturen, z.B. Forst, Ziergehölz-, Obst-, Wein-, Citrus-, Nuss-, Bananen-, Kaffee-, Tee-, Gummi-, Ölpalm-, Kakao-, Beerenfrucht- und Hopfenanlagen, auf Zier- und Sportrasen und Weideflächen sowie zur selektiven Unkrautbekämpfung in einjährigen Kulturen eingesetzt werden.

Die erfindungsgemäßen Verbindungen der Formel (I)/Wirkstoffkombinationen zeigen starke herbizide Wirksamkeit und ein breites Wirkungsspektrum bei Anwendung auf dem Boden und auf oberirdische Pflanzenteile. Sie eignen sich in gewissem Umfang auch zur selektiven Bekämpfung von monokotylen und dikotylen Unkräutern in monokotylen und dikotylen Kulturen, sowohl im Vorauflauf- als auch im Nachauflauf-Verfahren.

Die erfindungsgemäßen Wirkstoffe/Wirkstoffkombinationen können in bestimmten Konzentrationen bzw. Aufwandmengen auch zur Bekämpfung von tierischen Schädlingen und pilzlichen oder bakteriellen Pflanzenkrankheiten verwendet werden. Sie lassen sich gegebenenfalls auch als Zwischen- oder Vorprodukte für die Synthese weiterer Wirkstoffe einsetzen.

Die Wirkstoffe/Wirkstoffkombinationen können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstängeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im Allgemeinen zwischen 0,1 und 95 Gewichtsprozent Wirkstoff, vorzugsweise zwischen 0,5 und 90 %.

Die erfindungsgemäßen Wirkstoffe/Wirkstoffkombinationen können als solche oder in ihren Formulierungen auch in Mischung mit bekannten Herbiziden und/oder mit Stoffen, welche die Kulturpflanzen-Verträglichkeit verbessern ("Safenern") zur Unkrautbekämpfung verwendet werden, wobei Fertigformulierungen oder Tankmischungen möglich sind. Es sind also auch Mischungen mit Unkrautbekämpfungsmitteln möglich, welche ein oder mehrere bekannte Herbizide und einen Safener enthalten.

Für die Mischungen kommen bekannte Herbizide infrage, beispielsweise

Acetochlor, Acifluorfen (-sodium), Aclonifen, Alachlor, Alloxydim (-sodium), Ametryne, Amicarbazone, Amidochlor, Amidosulfuron, Aminopyralid, Anilofos, Asulam, Atrazine, Azafenidin, Azimsulfuron, Beflubutamid, Benazolin (-ethyl), Benfuresate, Bensulfuron (-methyl), Bentazon, Benzcarbazone, Benzfendizone, Benzobicyclon, Benzofenap, Benzoylprop (-ethyl), Bialaphos, Bifenox, Bispyribac (-sodium), Bromobutide, Bromofenoxim, Bromoxynil, Butachlor, Butafenacil (-allyl), Butroxydim, Butylate, Cafenstrole, Caloxydim, Carbetamide, Carfentrazone (-ethyl), Chlomethoxyfen, Chloramben, Chloridazon, Chlorimuron (-ethyl), Chlornitrofen, Chlorsulfuron, Chlortoluron, Cinidon (-ethyl), Cinmethylin, Cinosulfuron, Clefoxydim, Clethodim, Clodinafop (-propargyl), Clomazone, Clomeprop, Clopyralid, Clopyrasulfuron (-methyl), Cloransulam (-methyl), Cumyluron, Cyanazine, Cybutryne, Cycloate, Cyclosulfamuron, Cycloxydim, Cyhalofop (-butyl), 2,4-D, 2,4-DB, Desmedipham, Diallate, Dicamba, Dichlorprop (-P), Diclofop (-methyl), Diclosulam, Diethatyl (-ethyl), Difenzoquat, Diflufenican, Diflufenzopyr, Dimefuron, Dimepiperate, Dimethachlor, Dimethametryn, Dimethenamid, Dimexyflam, Dinitramine, Diphenamid, Diquat, Dithiopyr, Diuron, Dymron, Epropodan, EPTC, Esprocarb, Ethalfluralin, Ethametsulfuron (-methyl), Ethofumesate, Ethoxyfen, Ethoxysulfuron, Etobenzanid, Fenoxaprop (-P-ethyl), Fentrazamide, Flamprop (-isopropyl, -isopropyl-L, -methyl), Flazasulfuron, Florasulam, Fluazifop (-P-butyl), Fluazolate, Flucarbazone (-sodium), Flufenacet, Flumetsulam, Flumiclorac (-pentyl), Flumioxazin, Flumipropyn, Flumetsulam, Fluometuron, Fluorochloridone, Fluoroglycofen (-ethyl), Flupoxam, Flupropacil, Flurpyrsulfuron (-methyl, -sodium), Flurenol (-butyl), Fluridone, Fluroxypyr (-butoxypropyl, -meptyl), Flurprimidol, Flurtamone, Fluthiacet (-methyl), Fluthiamide, Fomesafen, Foramsulfuron, Glufosinate (-ammonium), Glyphosate (-isopropylammonium), Halosafen, Haloxyfop (-ethoxyethyl, -P-methyl), Hexazinone, HOK-201, Imazamethabenz -methyl), Imazamethapyr, Imazamox, Imazapic, Imazapyr, Imazaquin, Imazethapyr, Imazosulfuron, Iodosulfuron (-methyl, -sodium), Ioxynil, Isopropalin, Isoproturon, Isouron, Isoxaben, Isoxachlortole, Isoxaflutole, Isoxapyrifop, Lactofen, Lenacil, Linuron, MCPA, Mecoprop, Mefenacet, Mesosulfurone, Mesotrione, Metamifop, Metamitron, Metazachlor, Methabenzthiazuron, Metobenzuron, Metobromuron, (alpha-) Metolachlor, Metosulam, Metoxuron, Metribuzin, Metsulfuron (-methyl), Molinate, Monolinuron, Naproanilide, Napropamide, Neburon, Nicosulfuron, Norflurazon, Orbencarb, Orthosulfamuron, Oryzalin, Oxadiargyl, Oxadiazon, Oxasulfuron, Oxaziclomefone, Oxyfluorfen, Paraquat, Pelargonsäure, Pendimethalin, Pendralin, Penoxsulam, Pentoxazone, Phenmedipham, Picolinafen, Pinoxaden, Piperophos, Pretilachlor, Primisulfuron (-methyl), Profluazol, Prometryn, Propachlor, Propanil, Propaquizafop, Propisochlor, Propoxycarbazone (-sodium), Propyzamide, Prosulfocarb, Prosulfuron, Pyraflufen (-ethyl), Pyrasulfotole, Pyrazogyl, Pyrazolate, Pyrazosulfuron (-ethyl), Pyrazoxyfen, Pyribenzoxim, Pyributicarb, Pyridate, Pyridatol, Pyriftalid, Pyriminobac (-methyl), Pyrimisulfan, Pyrithiobac (-sodium), Pyroxsulam, Pyroxasulfone, Quinchlorac, Quinmerac, Quinoclamine, Quizalofop (-P-ethyl, -P-tefuryl), Rimsulfuron, Sethoxydim, Simazine, Simetryn, Sulcotrione, Sulfentrazone, Sulfometuron (-methyl), Sulfosate, Sulfosulfuron, Tebutam, Tebuthiuron, Tembotrione, Tepraloxydim, Terbuthylazine, Terbutryn, Thenylchlor, Thiafluamide, Thiazopyr, Thidiazimin, Thiencarbazone-methyl, Thifensulfuron (-methyl), Thiobencarb, Tiocarbazil, Topramezone, Tralkoxydim, Triallate, Triasulfuron, Tribenuron (-methyl), Triclopyr, Tridiphane, Trifluralin, Trifloxysulfuron, Triflusulfuron (-methyl), Tritosulfuron und

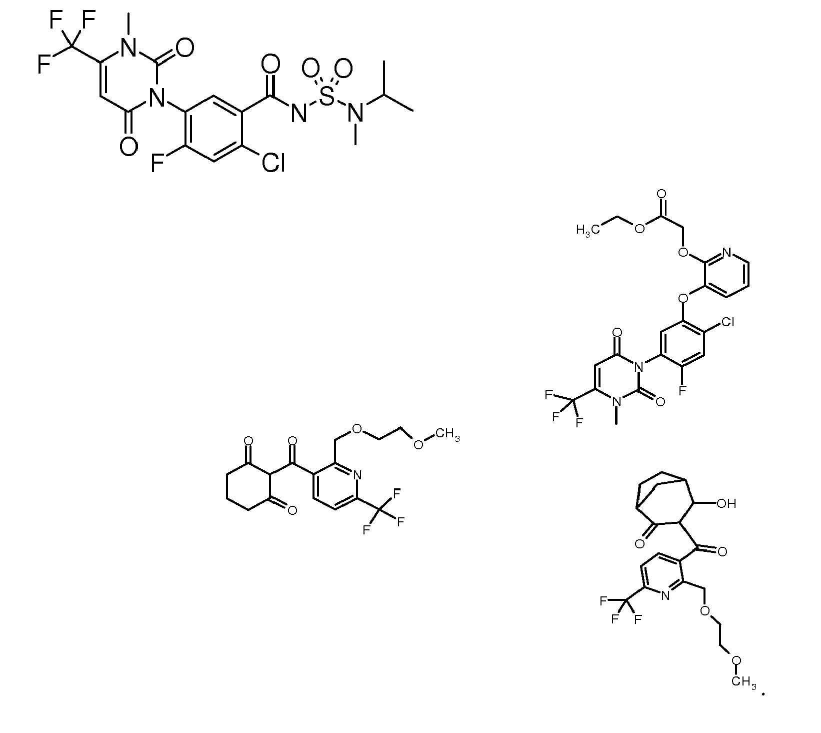

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe/Wirkstoffkombinationen können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe/Wirkstoffkombinationen können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im Allgemeinen liegen die Aufwandmengen zwischen 1 g und 10 kg Wirkstoff pro Hektar Bodenfläche, vorzugsweise zwischen 5 g und 5 kg pro ha.

Der vorteilhafte Effekt der Kulturpflanzen-Verträglichkeit der erfindungsgemäßen Wirkstoffkombinationen ist bei bestimmten Konzentrationsverhältnissen besonders stark ausgeprägt. Jedoch können die Gewichtsverhältnisse der Wirkstoffe in den Wirkstoffkombinationen in relativ großen Bereichen variiert werden. Im allgemeinen entfallen auf 1 Gewichtsteil Wirkstoff der Formel (I) 0,001 bis 1000 Gewichtsteile, vorzugsweise 0,01 bis 100 Gewichtsteile, besonders bevorzugt 0,05 bis 20 Gewichtsteile einer der oben unter (b') genannten, die Kulturpflanzen Verträglichkeit verbessernden Verbindungen (Antidots/Safener).
Die erfindungsgemäßen Wirkstoffkombinationen werden im allgemeinen in Form von Fertigformulierungen zur Anwendung gebracht. Die in den Wirkstoffkombinationen enthaltenen Wirkstoffe können aber auch in Einzelformulierungen bei der Anwendung gemischt, d.h. in Form von Tankmischungen zur Anwendung gebracht werden.

Für bestimmte Anwendungszwecke, insbesondere im Nachauflauf-Verfahren, kann es ferner vorteilhaft sein, in die Formulierungen als weitere Zusatzstoffe pflanzenverträgliche mineralische oder vegetabilische Öle (z.B. das Handelspräparat "Rako Binol") oder Ammoniumsalze wie z.B. Ammoniumsulfat oder Ammoniumrhodanid aufzunehmen.

Die neuen Wirkstoffkombinationen können als solche, in Form ihrer Formulierungen oder der daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Stäuben oder Streuen.

Die Aufwandmengen der erfindungsgemäßen Wirkstoffkombinationen können in einem gewissen Bereich variiert werden; sie hängen u.a. vom Wetter und von den Bodenfaktoren ab. Im allgemeinen liegen die Aufwandmengen zwischen 0,001 und 5 kg pro ha, vorzugsweise zwischen 0,005 und 2 kg pro ha, besonders bevorzugt zwischen 0,01 und 0,5 kg pro ha.

Die erfindungsgemäßen Wirkstoffkombinationen können vor und nach dem Auflaufen der Pflanzen appliziert werden, also im Vorauflauf und Nachauflauf-Verfahren.

Die erfindungsgemäß einzusetzenden Safener können je nach ihren Eigenschaften zur Vorbehandlung des Saatgutes der Kulturpflanze (Beizung der Samen) verwendet werden oder vor der Saat in die Saatfurchen eingebracht oder vor dem Herbizid separat angewendet werden oder zusammen mit dem Herbizid vor oder nach dem Ablaufen der Pflanzen angewendet werden.

Als Beispiele der Pflanzen werden die wichtigen Kulturpflanzen, wie Getreide (Weizen, Gerste, Reis), Mais, Soja, Kartoffel, Baumwolle, Raps, Rüben, Zuckerrohr sowie Obstpflanzen (mit den Früchten Äpfel, Birnen, Zitrusfrüchten und Weintrauben) erwähnt, wobei Getreide, Mais, Soja, Kartoffel, Baumwolle und Raps besonders hervorgehoben werden.

Die Bezeichnung "Wirkstoffe" schließt immer auch die hier genannten Wirkstoffkombinationen mit ein.

### Herstellungsbeispiele

### Beispiel I-1-a-1

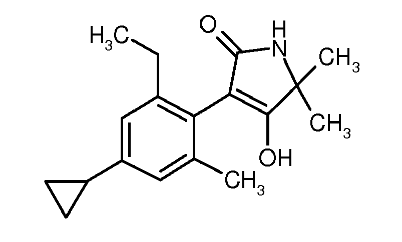

0,88 g Kalium-tert.-butylat werden in 10 ml N,N-Dimethylformamid vorgelegt, bei 60°C 1 g der Verbindung gemäß Beispiel II-1 in 5 ml N,N-Dimethylformamid gelöst über 30 min zugetropft und 4 h bei 60°C gerührt. Das Gemisch in 100 ml Wasser aufnehmen, mit IN HCl auf pH 1 stellen, einengen, nochmals in Wasser aufnehmen und einengen. Den Rückstand in 10 ml Wasser aufnehmen und mit Dichlormethan extrahieren, mit Natriumsulfat trocknen und einengen. Anschließend in 4 ml Essigsäureethylester verrühren und den Rückstand absaugen. Man erhält 0,3 g eines farblosen Feststoffs (36 % Ausbeute der Theorie) mit Fp.: 218°C.

In Analogie zu Beispiel (I-1-a-1) und gemäß den allgemeinen Angaben zur Herstellung erhält man folgende Verbindungen der Formel (I-1-a)

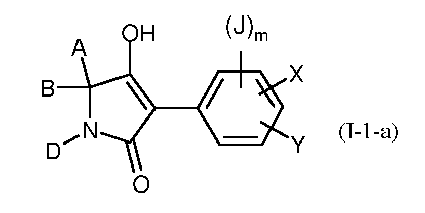

| **Bsp.-Nr,** | **m** | **J** | **X** | **Y** | **D** | **A** | **B** | **Fp °C** | **Isomer** |
|---|---|---|---|---|---|---|---|---|---|
| I-1-a-2 | 1 | 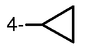 | 2-C₂H₅ | 6-CH₃ | H | -(CH₂)₂-CHOCH₃-(CH₂)₂- | | 222 | β |
| I-1-a-3 | 1 | 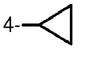 | 2 -C₂H₅ | 6-CH₃ | H | -CH₂-CHOC₄H₉-(CH₂)₃- | | 203 | β |
| I-1-a-4 | 1 | 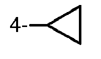 | 2 -CH₃ | 6- CH₃ | H | -(CH₂)₂-CHOCH₃-(CH₂)₂- | | 264 | β |
| I-1-a-5 | 1 | 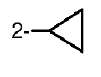 | 6 -C₂H₅ | 4- CH₃ | H | CH₃ | CH₃ | 262 | - |
| I-1-a-6 | 1 | 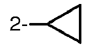 | 6 -C₂H₅ | 4- CH₃ | H | -(CH₂)₂-CHOCH₃-(CH₂)₂- | | 245 | β |
| I-1-a-7 | 1 | 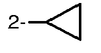 | 4 -CH₃ | 6- CH₃ | H | -(CH₂)₂-CHOCH₃-(CH₂)₂- | | 113 | β |
| I-1-a-8 | 1 | 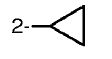 | 4 -CH₃ | 6- CH₃ | H | CH₃ | CH₃ | 100 | - |
| I-1-a-9 | 1 | 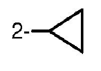 | 4- CH₃ | 6- CH₃ | H | -(CH₂)₂-CHOC₂H₅-(CH₂)₃- | | 109 | β |
| I-1-a-10 | 1 | 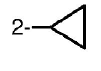 | 6-Cl | 4- CH₃ | H | CH₃ | CH₃ | *1.33 ppm (s, 6H, C(CH₃)₂), 0.75 (m, 2H, CH₂ (Cyclopropyl)), 0.60 und 0.51 ppm (je m, 1H, CH₂(Cyclopropyl)) | - |
| I-1-a-11 | 1 | 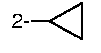 | 6-Cl | 4- CH₃ | H | -(CH₂)₂-CHOCH₃-(CH₂)₂- | | *3.26 ppm (s, 3H, O-CH₃), 0.75 (m, 2H, CH₂ (Cyclopropyl)), 0.59 und 0.51 ppm (je m, 1H, CH₂(Cyclopropyl)) | β |
| I-1-a-12 | 1 | 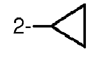 | 6-Cl | 4- CH₃ | H | -CH₂-CHOC₄H₉-(CH₂)₃- | | *3.58 ppm (m, 2H, OC^{H}2), 0.80 (m, 2H, CH₂ (Cyclopropyl)), 0.67 und 0.53 ppm (je m, 1H, CH₂(Cyclopropyl)) | β |
| I-1-a-13 | 1 | 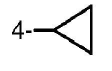 | 2-CH₃ | 6-CH₃ | H | -(CH₂)₂-O-CH₂)₂- | | 263 | - |
| I-1-a-14 | 1 | 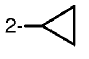 | 6-Cl | 4-CH₃ | H | -CH₂-CHOC₂H₅-(CH₂)₃- | | *3.45 ppm (m, 2H, OCH₂), 0.75 (m, 2H, CH₂ (Cyclopropyl)), 0.59 und 0.51 ppm (je m, 1H, CH₂(Cyclopropyl)) | β |
| I-1-a-15 | 2 | 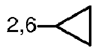 | 4- CH₃ | H | H | -(CH₂)₂-CHOCH₃-(CH₂)₂- | | 258-260 | β |
| I-1-a-16 | 1 | 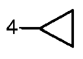 | 2-CH₃ | 6-CH₃ | H | 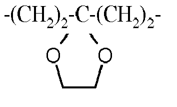 | | 283 | β |
| I-1-a-17 | 1 | 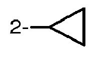 | 4- CH₃ | 6-CH₃ | H | -CH₂-CHOC₄H₉-(CH₂)₃- | | *3.55 ppm (m, 2H, OCH₂), 0.75 (m, 2H, CH₂ (Cyclopropyl)), 0.64 und 0.49 ppm (je m, 1H, CH₂(Cyclopropyl)) | β |
| I-1-a-18 | 1 | 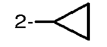 | 6-C₂H₅ | 4-CH₃ | -(CH₂)₃- | | H | 224-226 | - |
| I-1-a-19 | 2 | 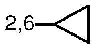 | 4- CH₃ | H | H | CH₃ | CH₃ | 248-250 | - |
| I-1-a-20 | 1 | 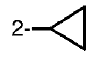 | 6-CH₃ | 4-Cl | H | -(CH₂)₂-CHOCH₃-(CH₂)₂- | | *3.41 ppm (s, 3H, O-CH₃), 0.88 (m, 2H, CH₂ (Cyclopropyl)), 0.72 und 0.51 ppm (je m, 1H, CH₂(Cyclopropyl)) | β |
| I-1-a-21 | 2 | 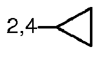 | 6-C₂H₅ | H | H | CH₃ | CH₃ | 216-219 | - |
| I-1-a-22 | 2 | 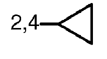 | 6-C₂H₅ | H | H | -(CH₂)₂-CHOCH₃-(CH₂)₂- | | 245-248 | β |
| I-1-a-23 | 2 | 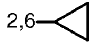 | 4- CH₃ | H | H | 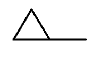 | CH₃ | 222-225 | - |
| I-1-a-24 | 1 | 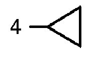 | 2-CH₃ | 6-CH₃ | H | -CH₂-CHOCH₃-(CH₂)₂- | | 202-205 | cis |
| I-1-a-25 | 1 | 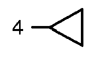 | 2-CH₃ | 6-CH₃ | H | -CH₂-CHOCH₃-(CH₂)₂- | | 80-90 | trans |
| I-1-a-26 | 1 | 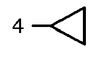 | 2-CH₃ | 6-CH₃ | H | -(CH₂)₂-CH-(CH₂)₂-CH₂OCH₃ | | 251 | β |

### Beispiel I-1-b-1

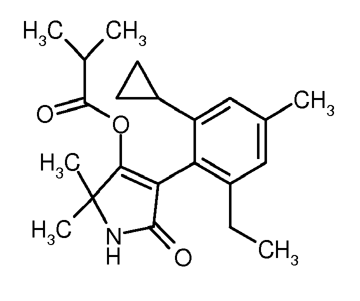

0,14 g der Verbindung gemäß Beispiel I-1-a-5 werden in 8 ml Essigsäureethylester vorgelegt, 0,05 g Triethylamin und 1,5 mg 4-N,N'-Dimethylaminopyridin zugegeben und auf 60°C erhitzt. Eine Lösung von 0,05 g Isobuttersäurechlorid in 2 ml Essigsäureethylester werden in 7 Portionen innerhalb von 60 min zugegeben und 6 h bei 60°C gerührt. Nach Stehenlassen über Nacht wird mit halbkonzentrierter Natriumchloridlösung versetzt, die organische Phase abgetrennt und säulenchromatographisch (Gradient EtOAc/n-Heptan 1:9 nach Essigsäureethylester/n-Heptan 100:0) an Kieselgel gereinigt. Man erhält 90 mg eines farblosen Feststoffes (60 % Ausbeute der Theorie). Fp.: 151°C

In Analogie zu Beispiel (I-1-b-1) und gemäß den allgemeinen Angaben zur Herstellung erhält man folgende Verbindungen der Formel (I-1-b)

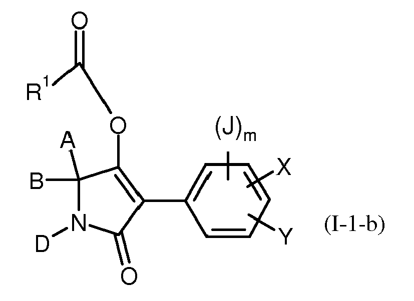

| **Bsp.-Nr.** | **m** | **J** | **X** | **Y** | **D** | **A** | **B** | **R¹** | **Fp °C** | **Isomer** |
|---|---|---|---|---|---|---|---|---|---|---|
| I-1-b-2 | 1 | 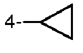 | 2-C₂H₅ | 6- CH₃ | H | -CH₂-CHOC₄H₉-(CH₂)₃- | | i-C₃H₇ | 173 | β |
| I-1-b-3 | 1 | 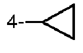 | 2-C₂H₅ | 6- CH₃ | H | -(CH₂)₂-CHOCH₃-(CH₂)₂- | | i-C₃H₇ | *0.99(dd, 6H,CH(CH₃)₂) 2.18(s,3H, Ar-CH₃) 3.21(m,1H, CHOCH₃) | β |
| I-1-b-4 | 1 | 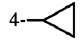 | 2-CH₃ | 6- CH₃ | H | -(CH₂)₂-CHOCH₃-(CH₂)₂- | | i-C₃H₇ | 194-196 | β |
| I-1-b-5 | 1 | 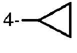 | 2-CH₃ | 6- CH₃ | H | -CH₂-CHOC₂H₅-(CH2)₃- | | i-C₃H₇ | 199-202 | β |
| I-1-b-6 | 1 | 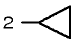 | 6-C₂H₅ | 4-CH₃ | H | -(CH₂)₂-CHOCH₃-(CH₂)₂- | | i-C₃H₇ | 190 | β |
| I-1-b-7 | 2 | 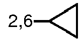 | 4-CH₃ | H | H | 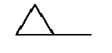 | CH₃ | i-C₃H₇ | 162-163 | - |
| I-1-b-8 | 1 | 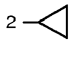 | 4-CH₃ | 6-CH₃ | H | CH₃ | CH₃ | 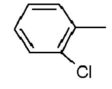 | 154-155 | - |
| I-1-b-9 | 2 | 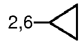 | 4-CH₃ | H | H | CH₃ | CH₃ | i-C₃H₇ | 171 | - |
| 1-1-b-10 | 1 | 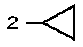 | 4-CH₃ | 6-CH₃ | H | -CH₂-CHOC₄H₉-(CH₂)₃- | | CH₃ | *3.47 ppm (m, 2H, OCH₂), 2.04 ppm( s, 3H, COCH₃), 0.85 - 0.50 ppm (Multiplett breit, 4H, CH₂ (Cyclopropyl)) | β |
| I-1-b-11 | 2 | 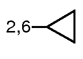 | 4-CH₃ | H | H | -(CH₂)₂-CHOCH₃-(CH₂)₂- | | i-C₃H₇ | 220-222 | β |
| I-1-b-12 | 1 | 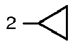 | 6-Cl | 4-CH₃ | H | -(CH₂)₂-CHOCH₃-(CH₂)₂- | | i-C₃H₇ | 186-187 | β |
| I-1-b-13 | 2 | 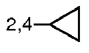 | 6-CH₃ | H | H | CH₃ | CH₃ | i-C₃H₇ | 176-177 | - |
| I-1-b-14 | 2 | 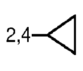 | 6-C₂H₅ | H | H | -(CH₂)₂-CHOCH₃-(CH₂)₂- | | i-C₃H₇ | 190-192 | β |
| I-1-b-15 | 1 | 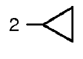 | 6-C₂H₅ | 4-CH₃ | -(CH₂)₃- | | H | i-C₃H₇ | * 4.75 ppm (m, 1H, CH-N (Brückenkopf)), 1.10 ppm (m, 9H, Ar-CH2)-**CH**₃ und CH(**CH**₃)₂), 0.95 - 0.54 ppm (Signalhaufen breit, 4H, CH₂ (Cyclopropyl)) | - |

| | | | | | | | | | | |
|---|---|---|---|---|---|---|---|---|---|---|
| *¹H-NMR (400 MHz, CDCl₃): Verschiebungen δ in ppm | | | | | | | | | | |

### Beispiel I-1-c-1

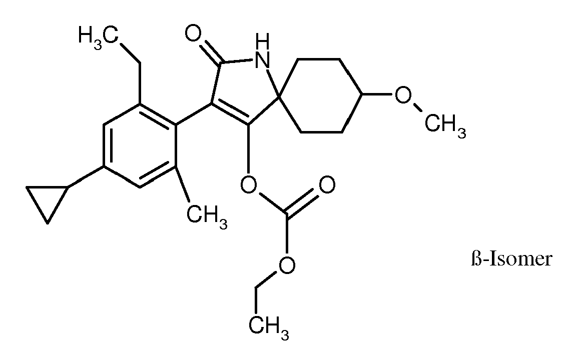

0,15 g der Verbindung gemäß Beispiel I-1-a-2 und 0,05 g Triethylamin werden in 8 ml Dichlormethan vorgelegt, 5 min bei RT gerührt, 0,05 g Chlorameisensäure-ethylester zugegeben und über Nacht bei RT gerührt. Es werden 5 ml 10 % Na₂CO₃-Lösung zugegeben, 10 min bei RT gerührt, über Extraktionskartuschen getrennt, einrotiert und säulenchromatographisch an Kieselgel mit 1/1 Essigsäureethylester/n-Heptan gereinigt. Man erhält 68 g eines Öls (38 % Ausbeute d. Theorie).
¹H-NMR (400 MHz, CDCl₃): δ = 2.18 (s, 3H; Ar-CH₃), 3.23 (m, 1H, CHOCH₃), 4.01 (q, 2H, OCH₂CH₃) ppm.

In Analogie zu Beispiel (I-1-c-1) und gemäß den allgemeinen Angaben zur Herstellung erhält man folgende Verbindungen der Formel (I-1-c)

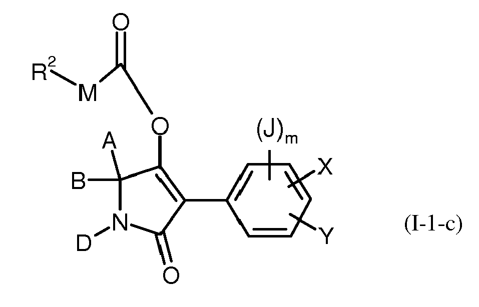

| **Bsp.-Nr.** | **m** | **J** | **X** | **Y** | **D** | **A** | **B** | **M** | **R²** | **Fp°C** | **Isomer** |
|---|---|---|---|---|---|---|---|---|---|---|---|
| I-1-c-2 | 1 | 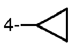 | 2-C₂H₅ | 6- CH₃ | H | -CH₂-CHOC₄H₉-(CH₂)₂- | | O | C₂H₅ | *2.46(m,2H, Ar-CH₂) 3.38(m,1H,CHO-CH₂) 4.01(q,2H,O-CH₂-CH₃) | β |
| I-1-c-3 | 1 | 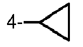 | 2-CH₃ | 6- CH₃ | H | -(CH₂)₂-CHOCH₃-(CH₂)₂- | | O | C₂H₅ | 200-202 | β |
| I-1-c-4 | 1 | 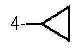 | 2-CH₃ | 6- CH₃ | H | -CH₂-CHOC₂H₅-(CH₂)₃- | | O | C₂H₅ | 190-192 | β |
| I-1-c-5 | 1 | 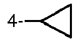 | 2-CH₃ | 6- CH₃ | H | -CH₂-CHOCH₃-(CH₂)₂- | | O | C₂H₅ | 114-116 | cis |
| I-1-c-6 | 1 | 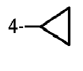 | 2-CH₃ | 6- CH₃ | H | -CH₂-CHOCH₃-(CH₂)₂- | | O | C₂H₅ | 177 | trans |
| I-1-c-7 | 1 | 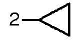 | 4-CH₃ | 6- CH₃ | H | -(CH₂)₂-CHOCH₃-(CH₂)₂- | | O | C₂H₅ | *4.01 ppm (q, 2H, O-CH₂), 3.38 ppm (s, 3H, OCH₃), 0.83 0.75, 0.66, 0.56 ppm (je m, 1H, CH₂ (Cyclopropyl)) | β |
| I-1-c-8 | 1 | 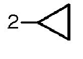 | 4-CH₃ | 6- CH₃ | H | -CH₂-CHOC₂H₅-(CH₂)₃- | | O | C₂H₅ | *4.01 ppm (q, 2H, O-CH₂), 3.53 ppm (s, 2H, OCH₂), 0.86 - 0.5 ppm (Signalhaufen, zusammen 4H, CH₂ (Cyclopropyl)) | β |
| I-1-c-9 | 1 | 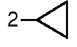 | 6-Cl | 4- CH₃ | H | -(CH₂)₂-CHOCH₃-(CH₂)₂- | | O | C₂H₅ | 199-202 | β |
| I-1-c-10 | 1 | 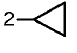 | 4- CH₃ | 6- CH₃ | H | CH₃ | CH₃ | O | C₂H₅ | *4.03 ppm (q, 2H, O-CH2), 1.51 ppm (s, 6H, C(CH₃)₂), 0.81 0.74, 0.64, 0.55 ppm (je m, 1H, CH₂ (Cyclopropyl)) | |
| I-1-c-11 | 1 | 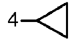 | 2-CH₃ | 6- CH₃ | H | -(CH₂)₂-O-(CH₂)₂- | | O | C₂H₅ | 210 | - |
| I-1-c-12 | 1 | 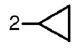 | 6-Cl | 4- CH₃ | H | -CH₂-CHOC₂H₅-(CH₂)₃- | | O | C₂H₅ | 188-189 | β |
| I-1-c-13 | 2 | 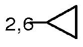 | 4- CH₃ | H | H | CH₃ | CH₃ | O | C₂H₅ | *4.05 ppm (q, 2H, OCH₂), 1.51 ppm( s, 6H, C(CH₃)₂), 0.79 ppm (m, 4H, CH₂ (Cyclopropyl)), 0.70 und 0.59 ppm (je m, 2H, CH₂(Cyclopropyl)) | - |
| I-1-c-14 | 1 | 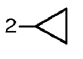 | 4- CH₃ | 6- CH₃ | H | -CH₂-CHOC₂H₅-(CH₂)₃- | | O | C₂H₅ | 236-237 | β |
| I-1-c-15 | 1 | 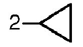 | 6-Cl | 4- CH₃ | H | -CH₂-CHOC₂H₅-(CH₂)₃- | | O | CH₃ | 230-231 | β |
| I-1-c-16 | 1 | 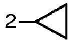 | 4- CH₃ | 6- CH₃ | H | -CH₂-CHOC₄H₉-(CH₂)₃- | | O | C₂H₅ | 173 | β |
| I-1-c-17 | 1 | 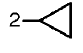 | 4- CH₃ | 6- CH₃ | H | -CH₂-CHOC₄H₉-(CH₂)₃- | | O | CH₃ | 180-183 | β |
| I-1-c-18 | 2 | 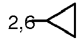 | 4- CH₃ | H | H | 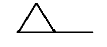 | CH₃ | O | C₂H₅ | 155-156 | - |
| I-1-c-19 | 2 | 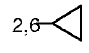 | 4- CH₃ | H | H | -(CH₂)₂-CHOCH₃-(CH₂)₂- | | O | C₂H₅ | 205-208 | β |
| I-1-c-20 | 1 | 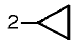 | 4- Cl | 6- CH₃ | H | -(CH₂)₂-CHOCH₃-(CH₂)₂- | | O | C₂H₅ | 176-177 | β |
| I-1-c-21 | 2 | 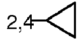 | 6-C₂H₅ | H | H | CH₃ | CH₃ | O | C₂H₅ | 167-168 | - |
| I-1-c-22 | 2 | 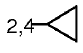 | 6-C₂H₅ | H | H | -(CH₂)₂-CHOCH₃-(CH₂)₂- | | O | C₂H₅ | *4.05 ppm (q, 2H, O-CH₂), 3.40 ppm (s, 3H, O-CH₃), 0.81 (m, 4H, CH₂ (Cyclopropyl)), 0.67, 0.60 ppm (je m, 2H, CH₂ (Cyclopropyl)) | β |
| I-1-c-23 | 2 | 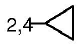 | 6-C₂H₅ | H | H | -(CH₂)₂-CHOCH₃-(CH₂)₂- | | O | CH₃ | *3.63 ppm (s, 2H, COO-CH₃), 3.40 ppm (s, 3H, O-CH₃), 0.92 (m, 2H, CH₂ (Cyclopropyl)), 0.82 - 0.55 ppm (Signalhaufen breit, 6H, CH₂ (Cyclopropyl)) | β |
| I-1-c-24 | 1 | 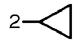 | 6-C₂H₅ | 4- CH₃ | -(CH₂)₃ | | H | O | C₂H₅ | *4.75 ppm (m, 1H, CH-N (Brückenkopf)), 4.19 ppm (dq, 2H, O-CH₂), 0.95 - 0.54 ppm (Signalhaufen breit, 4H, CH₂ (Cyclopropyl)) | - |
| I-1-c-25 | 1 | 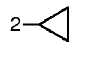 | 6-C₂H₅ | 4- CH₃ | H | CH₃ | CH₃ | O | CH₃ | *3.63 ppm (s, 2H, O-CH₃), 1.51 ppm (s, 6H, (CH₃)₂), 0.78 (m, 2H, CH₂ (Cyclopropyl)), 0.67, 0.59 ppm (je m, 1H, CH₂ (Cyclopropyl)) | - |

| | | | | | | | | | | | |
|---|---|---|---|---|---|---|---|---|---|---|---|
| *¹H-NMR (400 MHz, CDCl₃): Verschiebungen δ in ppm | | | | | | | | | | | |

### Beispiel I-1-d-1

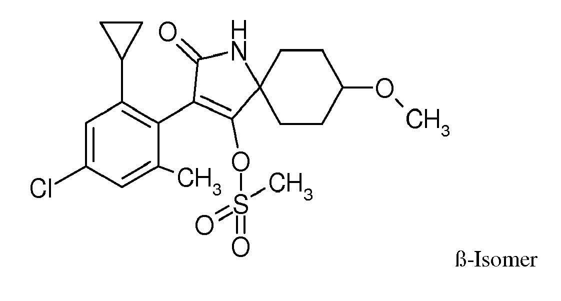

0,15g (0,415mmol) der Verbindung gemäß Beispiel I-1-a-20, 0,07ml Triethylamin und 1,5mg DMAP in 10ml Chloroform vorlegen, 10min bei Raumtemperatur rühren, und 0,04ml Methansulfonsäurechlorid zugeben. Man rührt ca. 18h bei Raumtemperatur. Nach beendigter Reaktion wird der Ansatz auf 5ml 5%ige Natriumhydrogencarbonat-Lösung geben, es wird 10min. bei Raumtemperatur gerührt. Es wird die organische Phase abgetrennt, über Na₂SO₄ getrocknet und das Lösungsmittel im Vakuum einrotiert. Es erfolgt säulenchromatographische Reinigung (Essigsäureethylester / n-Heptan 1: 1).
Ausbeute: 0,082g (45% d. Theorie)
¹H-NMR (400MHz, CDCl₃): δ = 0.67, 0.89 ppm (2m, je 2H, CH₂-Cyclopropyl), 2.57 ppm (s, 3H, SO₂-CH₃), 3.40 ppm (s, 3H, O-CH₃).

In Analogie zu Beispiel (I-1-d-1) erhält man **Beispiel (I-1-d-2):** 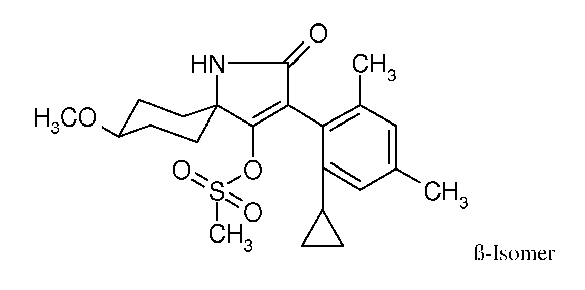 ¹H-NMR (400MHz, CDCl₃): δ = 3.40 ppm (s, 3H, O-CH₃), 2.47 ppm (s, 3H, SO₂-CH₃), 0.87, 0.81, 0.67, 0.62 ppm (je m, 1H, CH₂ (Cyclopropyl)).

### Beispiel II-1

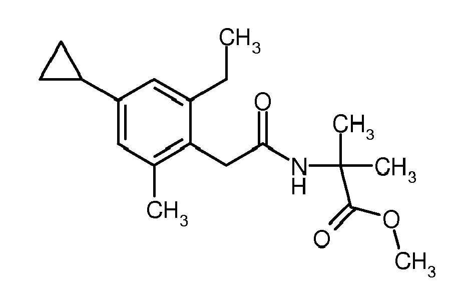

1 g der Verbindung gemäß Beispiel (XXX-2) werden in 30 ml Dichlormethan gelöst, 2 Tropfen N,N-Dimethylformamid und 0,76 g Oxalylchlorid hinzugegeben und 2 h bis zum Ende der Gasentwicklung unter Rückfluss zum Sieden erhitzt, eingeengt, zweimal in je 20 ml Dichlormethan aufgenommen, erneut eingeengt und in 20 ml Dichlormethan aufgenommen (Lösung 1).

0,7 g 1-Aminoisobuttersäuremethylester-Hydrochlorid werden in 30 ml Dichlormethan vorgelegt, 0,97 g Triethylamin zugegeben, 15 min bei Raumtemperatur gerührt, anschließend Lösung 1 in 30 min zugetropft, über Nacht bei Raumtemperatur gerührt, 30 ml Wasser hinzugegeben, 20 min gerührt, die org. Phase abgetrennt, getrocknet und chromatographisch an Kieselgel (Gradient Essigsäureethylester/Heptan 5:95 nach 100:0) gereinigt. Man erhält 1,05 g eines farblosen Feststoffs (72 % Ausbeute der Theorie). Fp.: 127°C

In Analogie zu Beispiel (II-1) und gemäß den allgemeinen Angaben zur Herstellung erhält man folgende Verbindungen der Formel (II)

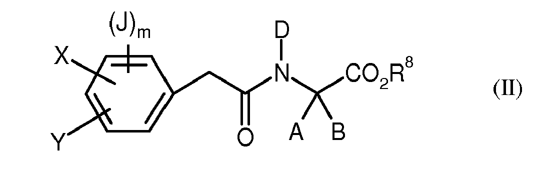

| **Bsp.-Nr.** | **m** | **J** | **X** | **Y** | **D** | **A** | **B** | **R⁸** | **Fp°C** | **Isomer** |
|---|---|---|---|---|---|---|---|---|---|---|
| II-2 | 1 | 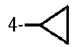 | 2-C₂H₅ | 6-CH₃ | H | -(CH₂)₂-CHOCH₃-(CH₂)₂- | | CH₃ | 123 | β |
| II-3 | 1 | 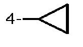 | 2-C₂H₅ | 6-CH₃ | H | -CH₂-CHOC₄H₉-(CH₂)₃- | | CH₃ | *2.89(m,1H, CHO CH₂) 3.58(s,2H, Ar-CH₂-CO) | β |
| II-4 | 1 | 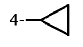 | 2-CH₃ | 6-CH₃ | H | -(CH₂)₂-CHOCH₃-(CH₂)₂- | | CH₃ | *3.12(m,1H, CHO CH₃) 6.80(s,2H,Ar-H) | β |
| II-5 | 1 | 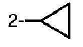 | 6-C₂H₅ | 4-CH₃ | H | CH₃ | CH₃ | CH₃ | 114 | |
| II-6 | 1 | 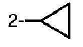 | 6-C₂H₅ | 4-CH₃ | H | -(CH₂)₂-CHOCH₃-(CH₂)₂- | | CH₃ | 96-98 | β |
| II-7 | 1 | 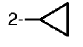 | 4- CH₃ | 6-CH₃ | H | -(CH₂)₂-CHOCH₃-(CH₂)₂- | | CH₃ | *3.12(m,1H, CHO CH₃) 3.80(m,2H, Ar-CH₂-CO) | β |
| II-8 | 1 | 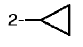 | 4- CH₃ | 6-CH₃ | H | CH₃ | CH₃ | CH₃ | *1.43(s,6H, C(CH₃)₂) 3.68(s,2H, Ar-CH₂-CO) | - |
| II-9 | 1 | 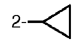 | 4- CH₃ | 6-CH₃ | H | -CH₂-CHOC₂H₅-(CH₂)₃- | | CH₃ | *2.99(m,1H, CHOCH₂-) | β |
| II-10 | 1 | 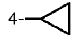 | 2-CH₃ | 6-CH₃ | | -CH₂-CHOC₂H₅-(CH₂)₃- | | CH₃ | 131 | β |
| II-11 | 1 | 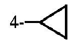 | 2- CH₃ | 6-CH₃ | H | -(CH₂)₂-O-(CH₂)₂- | | CH₃ | 189 | - |
| II-12 | 1 | 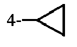 | 2-CH₃ | 6-CH₃ | H | 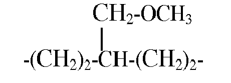 | | CH₃ | 156 | β |
| II-13 | 2 | 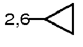 | 4-CH₃ | H | H | -(CH₂)₂-CHOCH₃-(CH₂)₂- | | CH₃ | 120 | β |
| II-14 | 2 | 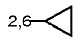 | 4-CH₃ | H | H | CH₃ | CH₃ | CH₃ | 118-119 | - |
| II-15 | 1 | 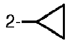 | 4-CH₃ | 6-CH₃ | H | -CH₂-CHOC₄H₉-(CH₂)₃- | | CH₃ | direkt weiter umgesetzt | β |
| II-16 | 1 | 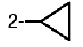 | 6-C₂H₅ | 4-CH₃ | -(CH₂)₃- | | H | CH₃ | *4.52 ppm (m, 1H, CH-N), 0.85 (m, 2H, CH₂ (Cyclopropyl)), 0.62 ppm (m, 2H, CH₂(Cyclopropyl)) | - |
| II-17 | 1 | 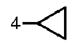 | 2-CH₃ | 6-CH₃ | H | 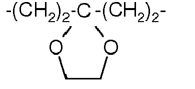 | | CH₃ | 174 | - |
| II-18 | 1 | 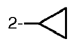 | 4-Cl | 6-CH₃ | H | -(CH₂)₂-CHOCH₃-(CH₂)₂- | | CH₃ | 129-131 | β |
| II-19 | 1 | 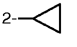 | 6-Cl | 4-CH₃ | H | CH₃ | CH₃ | CH₃ | 122-123 | - |
| II-20 | 1 | 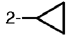 | 6-Cl | 4-CH₃ | H | -(CH₂)₂-CHOCH₃-(CH₂)₂- | | CH₃ | 129-131 | β |
| II-21 | 1 | 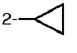 | 6-Cl | 4-CH₃ | H | -CH₂-CHOC₄H₉-(CH₂)₃- | | CH₃ | 100-101 | β |
| II-22 | 1 | 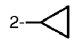 | 6-Cl | 4-CH₃ | H | -CH₂-CHOC₂H₅-(CH₂)₃- | | CH₃ | *3.68 ppm(s, 3H, OCH₃), 3.41 ppm (m, 2H, OCH₂), 0.99 und 0.66 (je m, 2H, CH₂ (Cyclopropyl)) | β |
| II-23 | 2 | 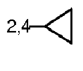 | 6-C₂H₅ | H | H | CH₃ | CH₃ | CH₃ | 124-125 | - |
| II-24 | 2 | 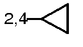 | 6-C₂H₅ | H | H | -(CH₂)₂-CHOCH₃-(CH₂)₂₋ | | CH₃ | - | β |
| II-25 | 2 | 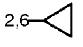 | 4-CH₃ | H | H | 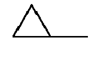 | CH₃ | CH₃ | *3.70 ppm (s, 3H, COO-CH₃), 1.37 ppm (s, 3H, CH₃), 1.89 ppm (m, 2H, Ar-CH (Cyclopropyl)) | - |
| II-26 | 2 | 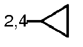 | 6-CH₃ | H | H | CH₃ | CH₃ | CH₃ | *1.42ppm (s, 6H, (CH₃)₂C-NH), 3.80ppm (s, 2H, Ar-CH₂-CO) | - |
| II-27 | 1 | 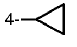 | 4-CH₃ | 6-CH₃ | -(CH₂)₃- | | H | CH₃ | *4.18 ppm (m, 2H, O-CH₂), 3.95 und 3.81 ppm (je m, zusammen 1H, CH-O), 0.94 und 0.67 (je m, 2H, CH₂ (Cyclopropyl)) | - |

| | | | | | | | | | | |
|---|---|---|---|---|---|---|---|---|---|---|
| *¹H-NMR (400 MHz, CDCl₃): Verschiebungen δ in ppm | | | | | | | | | | |

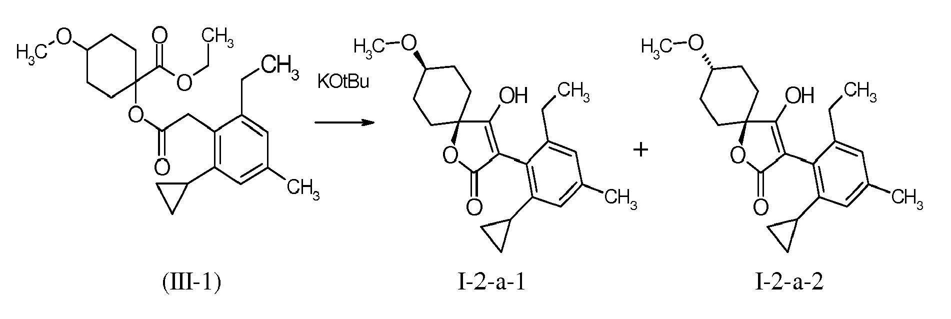

0,59 g (1,466 mmol) der Verbindung gemäß Beispiel (III-1) werden in 10 ml DMF vorgelegt, mit 0,247 g (2,199 mmol) Kalium-tert-butylat versetzt und 12h bei Raumtemperatur gerührt. Das DMF wird abrotiert, der Rückstand zwischen Wasser und Methyl-tert-butylester verteilt, die wässr.Phase mit Salzsäure angesäuert, das Produkt mit Dichlormethan extrahiert und die org.Phase getrocknet und einrotiert.
Zur weiteren Reinigung und Isomerentrennung chromatographiert man das 0,37g Rohprodukt an Kieselgel-RP18 (Acetonitril/Wasser).
Ausbeute:
89 mg I-2-a-1 (17 % der Theorie), logP = 2,81
132 mg I-2-a-2 (25 % der Theorie), logP = 3,07

In Analogie zu den Beispielen (I-2-a-1) und (I-2-a-2) und gemäß den allgemeinen Angaben zur Herstellung erhält man folgende Verbindungen der Formel (I-2-a):

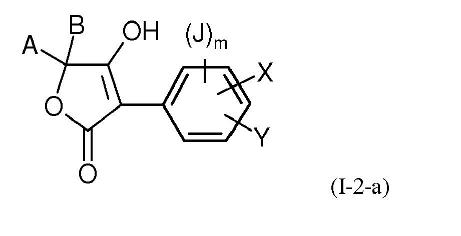

| **Bsp.-Nr.** | **m** | **J** | **X** | **Y** | **A** | **B** | **logP** | **Isomer** |
|---|---|---|---|---|---|---|---|---|
| I-2-a-3 | 1 | 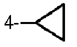 | 2-CH₃ | 6-CH₃ | -(CH₂)₂-CHOCH₃-(CH₂)₂- | | 2.64 | cis |
| I-2-a-4 | 1 | 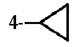 | 2-CH₃ | 6-CH₃ | -(CH₂)₂-CHOCH₃-(CH₂)₂- | | 2.88 | trans |
| I-2-a-5 | 1 | 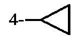 | 2-CH₃ | 6-CH₃ | -(CH₂)₂-O-(CH₂)₂- | | 2.36 | - |

### Beispiel I-2-b-1

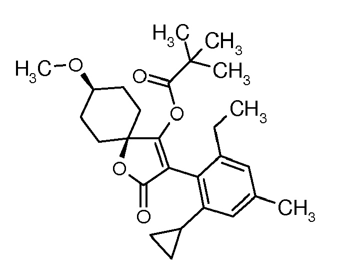

50 mg (0,14 mmol) der Verbindung gemäß Beispiel 1-2-a-1 werden in 5 ml Dichlormethan vorgelegt, mit 17 mg (0,168 mmol) Triethylamin und 20 mg (0,168 mmol) Pivaloylchlorid versetzt und 12h bei Raumtemperatur gerührt. Man engt ein und reingt das Rohprodukt durch Chromatographie an Kieselgel-RP18 (Acetonitril/Wasser).
Ausbeute: 60 mg (97% d.Th.), logP = 5,03

### Beispiel (III-1)

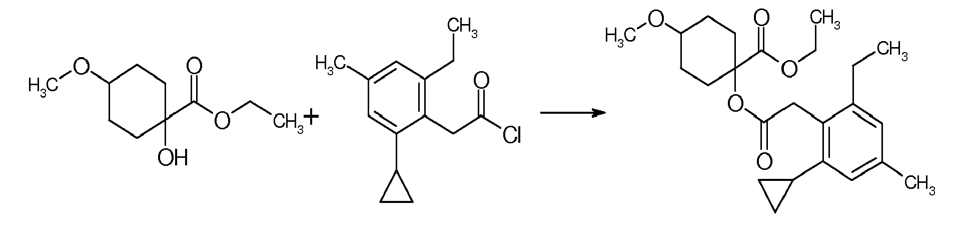

0,854 g (4,224 mmol) 4-Methoxy-1-hydroxy-cyclohexancarbonsäure-ethylester und 1 g (4,224 mmol) 2-Cyclopropyl-6-ethyl-4-methyl-phenylessigsäurechlorid werden 6h bei 120 °C Badtemperatur verrührt, nach dem Abkühlen am Rotationsverdampfer entgast, in Methyl-tert-butylester gelöst, mit 5%iger Natronlauge gewaschen und die org.Phase getrocknet und einrotiert.
Ausbeute: 0,59 g (35% d.Th.), Öl, logP = 5,15

### Beispiel I-6-a-1

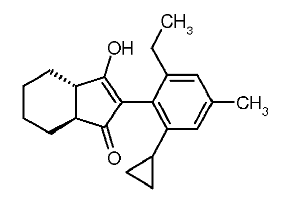

0.58 g (1.7 mmol) der Verbindung gemäß Beispiel VIII-1 und 0.38 g Kalium-tert.-butylat (3.4 mmol) werden in 10 ml wasserfreiem DMF vorgelegt und 3h auf 50 °C erhitzt. Nach Abkühlen wird auf Eiswasser gegeben, mit 2N Salzsäure auf pH 2 angesäuert und mit Essigester extrahiert. Die organische Phase wird noch zweimal mit Wasser gewaschen, getrocknet (Natriumsulfat) und einrotiert. Nach Chromatographie mit Hexan/Essigsäureethylester (v/v=70:30) an Kieselgel erhält man 286 mg (54%) der Verbindung gemäß Beispiel I-6-a-1 in Form farbloser Kristalle.
Fp. 189-190 °C
¹H-NMR (*d*₆-DMSO, 400 MHz): δ = 0.45 und 0.67 (je mc, je 1H), 1.38 und 1.62 (je mc, je 2H), 1.80 (mc, 1H), 2.22 (s, 3H)

### Beispiel VIII-1

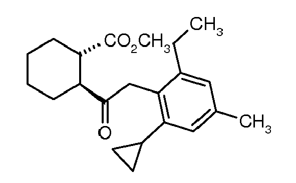

1.50 g (4.6 mmol) der Verbindung gemäß Beispiel XXXVII-1 in 30 ml Aceton werden zusammen mit 1.30 g Kaliumcarbonat und 1.96 g (13.8 mmol) Iodmethan 4 h am Rückfluß gekocht, danach in Essigester aufgenommen, mit Wasser geschüttelt, getrocknet (Magnesiumsulfat) und das Lösemittel abdestilliert. Nach Chromatographie an Kieselgel mit Essigsäureethylester/Hexan (v/v= 70:30) erhält man 1.23 g (78 %) der Verbindung der Formel (VIII-1) als farblosen Feststoff mit Fp. 84-85°C.

### Beispiel XXXVII-1

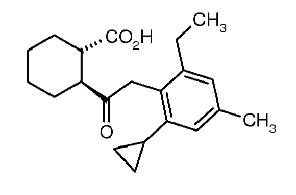

Zu einer Lösung von Lithiumdiethylamid, hergestellt aus 3.5 g Diethylamin in 25 ml THF und 14 ml einer 2.5 molaren Lösung von n-Butyllithium in Hexan werden bei -30 °C langsam 3.38 g (13.9 mmol) 2-Cyclopropyl-6-ethyl-4-methylphenylessigsäuremethylester getropft und noch 30 min. bei Raumtemperatur nachgerührt. Danach werden 2.15 g (13.9 mmol) Cyclohexan-1,2-dicarbonsäureanhydrid, gelöst in 10 ml THF, bei -20 °C zugegeben und 12 h bei Raumtemperatur gerührt. Nach Zugabe von 30 ml ges. Ammoniumchloridlösung, Überschichten mit Essigester, Waschen mit Wasser, Trocknung (Magnesiumsulfat) und Einrotieren erhält man ca. 5.4 g Feststoff, der ohne weitere Aufreinigung mit 2.2 g Kaliumhydroxid in 50 ml Wasser versetzt und 24 h am Rückfluß erhitzt wird. Anschließend wird mit 2N Salzsäure auf pH 2 angesäuert und der dabei ausgefallene Feststoff abgesaugt.

Man erhält 1.68 g (37%) der Verbindung der Formel (XXXVII-1) in Form gelblicher Kristalle mit Fp. 187-188 °C.

### Beispiel I-8-a-1

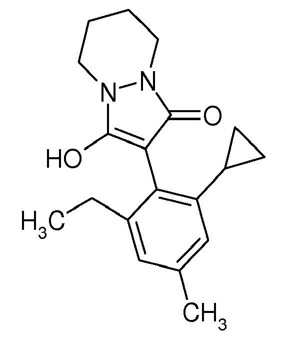

0,992g (9mmol) Kalium-tert.-butylat werden in 35ml wasserfreiem N,N-Dimethylformamid vorgelegt. Dazu tropft man 1,1g der Verbindung gemäß Beispiel XII-1 in N,N-Dimethylformamid und rührt 2h bei 80°C nach. Man gibt Eiswasser zu und säuert mit konzentrierter Salzsäure auf pH1 an, extrahiert mit Methylenchlorid, trocknet und rotiert im Vakuum ein.
Ausbeute: 0,75g (≙ 86% d. Theorie)
¹H-NMR-Daten in CDCl₃, 400 MHz:
δ = 3,75-3,60 (m, 4H); 2,30-2,20 (m, 2H); 1,85-1,75 (m, 4H); 1,55 (m, 1 ^{cy}Pr-H); 0,90 (m, 1 ^{cy}Pr-H); 0,85 (m, 1 ^{cy}Pr-H); 0,75 (m, 1 ^{cy}Pr-H); 0,65 (m, 1 ^{cy}Pr-H) ppm.

### Beispiel I-8-b-1

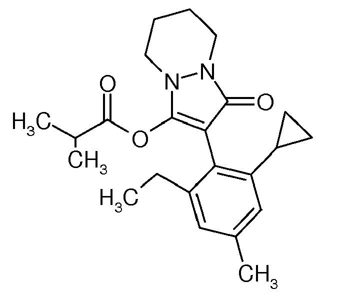

0,15g (0,5mmol) der Verbindung gemäß Beispiel 1-8-a-1 werden mit 0,113g (1mmol) Kaliumcarbonat in Tetrahydrofuran vorgelegt und bei Raumtemperatur Isobuttersäurechlorid (0,04ml) zugegeben. Nach 3h Reaktionszeit wird das Lösungsmittel im Vakuum abgedampft, der Rückstand mit Wasser versetzt, mit Essigsäureethylester extrahiert und erneut im Vakuum eingedampft.
Ausbeute: 0,12g (≙ 70% d. Theorie)
¹H-NMR-Daten in CDCl₃, 400 MHz:
δ = 6,85 (s, 1H); 6,55 (s, 1H); 3,90 (m, 1H), 3,80 (m, 1H); 3,35 (m, 2H); 2,65-2,55 (m, 2H); 2,50 (sept, 1H); 2,25 (s, 3H); 1,95 (m, 2H); 1,85 (m, 2H); 1,80 (m, 1 ^{cy}Pr-H); 1,15 (tr, 3H); 1,05 (m, 6H); 0,75 (m, 2 ^{cy}Pr-H); 0,60 (m, 1 ^{cy}Pr-H); 0,55 (m, 1 ^{cy}Pr-H) ppm.

In Analogie zu Beispiel (I-8-b-1) erhält man **Beispiel (I-8-b-2)** 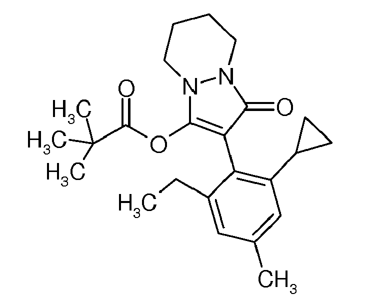 ¹H-NMR-Daten in CDCl₃, 400 MHz:
δ = 6,85 (s, 1H); 6,50 (s, 1H); 3,90 (m, 1H), 3,80 (m, 1H); 3,35 (m, 2H); 2,60-2,40 (m, 2H); 2,25 (s, 3H); 1,95 (m, 2H); 1,85 (m, 2H); 1,80 (m, 1 ^{cy}Pr-H); 1,15 (tr, 3H); 1,05 (s, 9H); 0,80 (m, 2 ^{cy}Pr-H); 0,60 (m, 1 ^{cy}Pr-H); 0,55 (m, 1 ^{cy}Pr-H) ppm.

In Analogie zu Beispiel I-8-b-1 erhält man **Beispiel I-8-c-1** 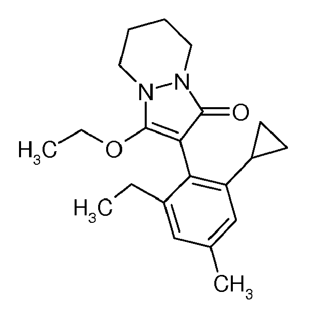 ¹H-NMR-Daten in CDCl₃, 400 MHz:
δ = 6,90 (s, 1H); 6,60 (s, 1H); 4,15 (q, 2H); 3,90-3,80 (m, 2H), 3,40 (tr, 2H); 2,65-2,45 (m, 2H); 2,25 (s, 3H); 1,95 (m, 2H); 1,85 (m, 2H); 1,80 (m, 1 ^{cy}Pr-H); 1,20 (tr, 3H); 1,15 (tr, 3H); 0,75 (m, 2 ^{cy}Pr-H); 0,65 (m, 1 ^{cy}Pr-H); 0,55 (m, 1 ^{cy}Pr-H) ppm.

### Beispiel XII-1

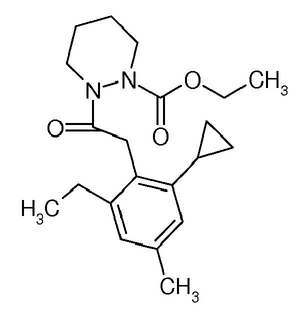

1,7g (8mmol) der Verbindung gemäß Beispiel XXX-3 werden in wasserfreiem Tetrahydrofuran mit 2,28ml (16mmol) Triethylamin versetzt, 2,46g (11mmol) Hexahydropyridazin-ethylcarbazat hinzugefügt, nach 10min. weitere 2,28ml (16mmol) Triethylamin zugetropft und gleich danach 0,73ml (8mmol) Phosphoroxychlorid zugetropft. Die Lösung wird 30min. unter Rückfluss erwärmt, das Lösungsmittel entfernt, der Rückstand in Essigsäuremethylester aufgenommen, zweimal mit Wasser gewaschen, getrocknet und eingedampft. Der Rückstand wird in n-Heptan aufgenommen, über wenig Kieselgel filtriert. Das Filtrat wird eingedampft.
Ausbeute: 2,3g (≙ 74% d. Theorie)
¹H-NMR-Daten in CDCl₃, 400 MHz:
δ = 6,85 (s, 1H); 6,75 (s, 1H); 4,55 (dbr, 1H); 4,30-4,20 (mbr, 3H); 4,00 (d, 1H); 3,80 (d, 1H), 2,95 (mbr, 1H); 2,75 (trbr, 1H); 2,55 (q, 2H); 2,25 (s, 3H); 1,80 (m, 1 ^{cy}Pr-H); 1,75-1,60 (mbr, 4H); 1,15m (tr, 3H); 0,90-0,75 (m, 2 ^{cy}Pr-H); 0,70-0,55 (m, 2 ^{cy}Pr-H) ppm.

### Beispiel XXX-1

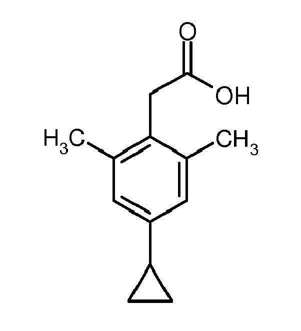

4.8 g der Verbindung gemäß Beispiel (XXXIV-2) werden in 200 ml Tetrahydrofuran vorgelegt, 0,5 g Lithiumhydroxid in 80 ml Wasser gelöst zugegeben und ca. 70 h bei Raumtemperatur gerührt. Das Tetrahydrofuran wird abrotiert, die Wasserphase mit 1 N HCl auf pH 1 gestellt, 10 min bei Raumtemperatur gerührt und der Niederschlag über eine Fritte abgesaugt. Der Rückstand wird in Essigsäureethylester gelöst, über Natriumsulfat getrocknet, einrotiert, in 30 ml nHeptan aufgenommen, 10 min mit Ultraschall behandelt, über Fritte abgesaugt, getrocknet. Man erhält 4.0 g eines farblosen Feststoffes (89 % Ausbeute der Theorie). Fp.: 138°C.

In Analogie zu Beispiel (XXX-1) und gemäß den allgemeinen Angaben zur Herstellung erhält man folgende Verbindungen der Formel (XXX)

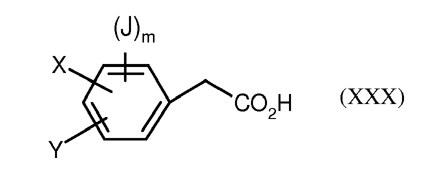

| **Bsp.-Nr.** | **m** | **J** | **X** | **Y** | **Fp °C** |
|---|---|---|---|---|---|
| XXX-2 | 1 | 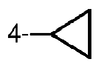 | 2-C₂H₅ | 6-CH₃ | 100 |
| XXX-3 | 1 | 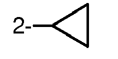 | 6-C₂H₅ | 4-CH₃ | 139 |
| XXX-4 | 1 | 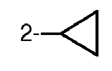 | 4-CH₃ | 6-CH₃ | 144 |
| XXX-5 | 1 | 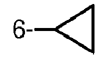 | 2-Cl | 4-CH₃ | 158 |
| XXX-6 | 1 | 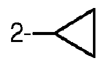 | 6-CH₃ | 4-Cl | 224-226 |
| XXX-7 | 2 | 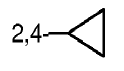 | 6-C₂H₅ | H | *3.91 (s, 2H, CH₂CO₂) 0.93, 0.62 (2m, 4H, CH₂ (Cyclopropyl)) |
| XXX-8 | 2 | 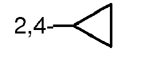 | 6-CH₃ | H | 112 |
| XXX-9 | 2 | 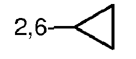 | 4-CH₃ | H | *4.18 (s, 2H, CH₂CO₂) 0.91, 0.64 (2m, 4H, CH₂ (Cyclopropyl)) |

| | | | | | |
|---|---|---|---|---|---|
| *¹H-NMR (400 MHz, CDCl₃): Verschiebungen δ in ppm | | | | | |

### Beispiel XXXIV-1

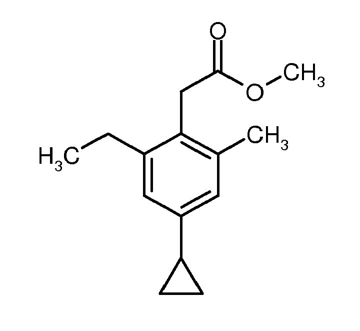

3 g 4-Brom-2-ethyl-6-methyl-phenylessigsäuremethylester werden in 115 ml Toluol und 5.7 ml Wasser vorgelegt und die Apparatur 3 x mit Vakuum/Argon gespült. Dann werden 1.24 g Cyclopropan-boronsäure, 5.27 g Kalium-dihydrogenphosphat, 0.31 g Tricyclohexylphosphin und 0,12 g Palladiumacetat zugegeben und im vorgeheizten Ölbad auf Rückfluss gebracht. 8 h bei Rückfluss rühren. Man gießt auf 500 ml 0,5 N HCl, extrahiert mit 3 x 150 ml Toluol, vereinigt die org. Phasen, wäscht mit 100 ml Natriumchloridlösung, schlechte Phasentrennung, trocknet über Na₂SO₄ und rotiert ein. Der Rückstand wird über eine Biotage-Chromatographiestation mit Essigsäureethylester/n-Heptan (5:95 nach 100:0) aufgetrennt. Es wurden 2.5 g eines öligen Rückstandes erhalten (98 % der Theorie).
*¹H-NMR (400 MHz, CDCl₃): δ: 1.81 (m, 1H, CH-cycPr), 3.66 (s, 2+3H, **CH₂**-COO**CH₃**) ppm.

In Analogie zu Beispiel (XXXIV-1) und gemäß den allgemeinen Angaben zur Herstellung erhält man folgende Verbindungen der Formel (XXXIV)

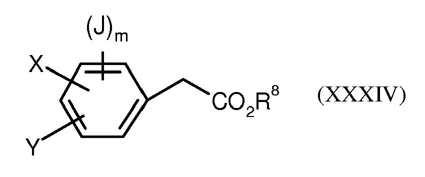

| **Bsp.-Nr.** | **m** | **J** | **X** | **Y** | **R⁸** | **Fp °C** |
|---|---|---|---|---|---|---|
| XXXIV-2 | 1 | 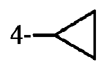 | 2-CH₃ | 6-CH₃ | CH₃ | *1.81 (m,1H,CH-cyc-Pr) 3.64 (s,2H,CH₂CO₂CH₃) 3.67 (s,3H,CH₂-CO₂CH₃) |
| XXXIV-3 | 1 | 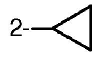 | 6-C₂H₅ | 4-CH₃ | CH₃ | *1.89 (m,1H,CH-cyc-Pr) 3.89 (s,2H,CH₂CO₂CH₂CH₃) 4.13 (s,3H, CO₂CH₂CH₃) |
| XXXIV-4 | 1 | 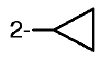 | 4-CH₃ | 6-CH₃ | CH₃ | wurde ohne weitere Charakterisierung zu Beispiel (XXX-4) umgesetzt |
| XXXIV-5 | 1 | 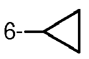 | 2-Cl | 4-CH₃ | CH₃ | *1.86 (m,1H,CH-cyc-Pr) 3.69 (s,3H,CO₂CH₃) |
| XXXIV-6 | 1 | 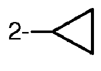 | 4-Cl | 6-CH₃ | CH₃ | *3.88 ppm (s, 2H, CH₂-COO), 3.68 ppm (s, 3H, OCH₃), 0.93 und 0.62 ppm (je m, 2H, CH₂(Cycloproppyl)) |
| XXXIV-7 | 2 | 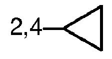 | 6-CH₃ | H | CH₃ | *3.89 ppm (s, 2H, CH₂-COO), 3.68 ppm (s, 3H, OCH₃), 0.90 ppm (m, 4H, CH₂(Cycloproppyl)), 0.66 und 0.60 ppm (je m, 2H, CH₂(Cycloproppyl)) |
| XXXIV-8 | 2 | 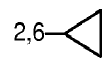 | 4-CH₃ | H | CH₃ | *4.12 ppm (s, 2H, CH₂-COO), 3.68 ppm (s, 3H, OCH₃), 0.89 und 0.62 ppm (je m, 4H, CH₂(Cycloproppyl)) |
| XXXIV-9 | 2 | 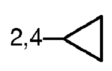 | 6-C₂H₅ | H | CH₃ | *3.90 ppm (s, 2H, CH₂-COO), 3.68 ppm (s, 3H, OCH₃), 0.89 ppm (m, 4H, CH₂(Cycloproppyl)), 0.66 und 0.60 ppm (je m, 2H, CH₂(Cycloproppyl)) |

| | | | | | | |
|---|---|---|---|---|---|---|
| *¹H-NMR (400 MHz, CDCl₃): Verschiebungen δ in ppm | | | | | | |

Die Bestimmung der in der Tabelle angegebenen logP-Werte erfolgte gemäß EEC-Directive 79/831 Annex V.A8 durch HPLC (High Performance Liquid Chromato-graphy) an einer Phasenumkehrsäule (C 18). Temperatur: 43°C.

Eluenten für die Bestimmung im sauren Bereich (pH 2,3): 0,1 % wässrige Phosphorsäure, Acetonitril; linearer Gradient von 10% Acetonitril bis 90 % Acetonitril.

Die Eichung erfolgte mit unverzweigten Alkan-2-onen (mit 3 bis 16 Kohlen-stoff-atomen), deren logP-Werte bekannt sind (Bestimmung der logP-Werte anhand der Retentionszeiten durch lineare Interpolation zwischen zwei aufeinander folgenden Alkanonen).

Die lambda-max-Werte wurden an Hand der UV-Spektren von 200 nm bis 400 nm in den Maxima der chromatographischen Signale ermittelt.

### Beispiel A

### Herbizide Wirkung im Vorauflauf

Samen von mono- bzw. dikotylen Unkraut- bzw. Kulturpflanzen werden in Holzfasertöpfen in sandiger Lehmerde ausgelegt und mit Erde abgedeckt. Die in Form von benetzbaren Pulvern (WP) oder als Emulsionskonzentrate (EC) formulierten Testverbindungen werden dann als wässrige Suspension mit einer Wasseraufwandmenge von umgerechnet 800 1/ha unter Zusatz von 0,2 % Netzmittel auf die Oberfläche der Abdeckerde appliziert.

Nach Behandlung werden die Töpfe im Gewächshaus aufgestellt und unter guten Wachstumsbedingungen für die Testpflanzen gehalten. Die visuelle Bonitur der Schäden an den Versuchspflanzen erfolgt nach einer Versuchszeit von 3 Wochen im Vergleich zu unbehandelten Kontrollen (herbizide Wirkung in Prozent (%): 100 % Wirkung = Pflanzen sind abgestorben, 0 % Wirkung = wie Kontrollpflanzen).

Dabei wurden Lolium multiflorum und Setaria viridis bei einer Aufwandmenge von 320 g/ha z.B. von folgenden Verbindungen mit ≥ 80 % Wirkung erfasst: Beispiel: I-1-a-5, I-1-a-6, I-1-a-7, I-1-a-8, I-1-a-10, I-1-a-11, I-1-a-12, I-1-a-14, I-1-a-15, I-1-a-17, I-1-a-18, I-1-a-19, I-1-b-1, I-1-b-2, I-1-b-6, I-1-b-9, I-1-b-10, I-1-c-1, I-1-c2, I-1-c-7, I-1-c-8, I-1-c-10, I-1-c-12, I-1-c-13, I-1-c-14, I-1-c-15, I-1-c-16, I-1-c-17

### Beispiel B

### Herbizide Wirkung im Nachauflauf

Samen von mono- bzw. dikotylen Unkraut- bzw. Kulturpflanzen werden in Holzfasertöpfen in sandigem Lehmboden ausgelegt, mit Erde abgedeckt und im Gewächshaus unter guten Wachstumsbedingungen angezogen. 2 bis 3 Wochen nach der Aussaat werden die Versuchspflanzen im Einblattstadium behandelt. Die in Form von benetzbaren Pulvern (WP) oder als Emulsionskonzentrate (EC) formulierten Testverbindungen werden dann als wässrige Suspension mit einer Wasseraufwandmenge von umgerechnet 800 1/ha unter Zusatz von 0,2 % Netzmittel auf die grünen Pflanzenteile gesprüht. Nach ca. 3 Wochen Standzeit der Versuchspflanzen im Gewächshaus unter optimalen Wachstumsbedingungen wird die Wirkung der Präparate visuell im Vergleich zur unbehandelten Kontrollen bonitiert (herbizide Wirkung in Prozent (%): 100 % Wirkung = Pflanzen sind abgestorben, 0 % Wirkung = wie Kontrollpflanzen).

Dabei wurden Lolium multiflorum und Setaria viridis bei einer Aufwandmenge von 320 g/ha z. B. von folgenden Verbindungen mit ≥ 90 % Wirkung erfasst: Bsp. I-1-c-1.

Dabei wurden Lolium multiforum und Setaria viridis bei einer Auwandmenge von 80 g/ha z.B. von folgenden Verbindungen mit ≥ 80 % Wirkung erfasst; Beispiel: I-1-a-2, I-1-a-5, I-1-a-6, I-1-a-7, I-1-a-8, I-1-a-10, I-1-a-11, I-1-a-12, I-1-a-14, I-1-a-15, I-1-a-17, I-1-a-18, I-1-a-19, I-1-b-1, I-1-b-6, I-1-b-9, I-1-b-10, I-1-c-3, I-1-c7, I-1-c-8, I-1-c-10, I-1-c-13, I-1-c-14, I-1-c-15,

### Beispiel C

### Herbizide Wirkung im Nachauflauf

Samen von mono- bzw. dikotylen Unkraut- bzw. Kulturpflanzen werden in Holzfasertöpfen oder in Plastiktöpfen in sandiger Lehmerde ausgelegt, mit Erde abgedeckt und im Gewächshaus, während der Vegetationsperiode auch im Freien ausserhalb des Gewächshauses, unter guten Wachstumsbedingungen angezogen. 2- 3 Wochen nach der Aussaat werden die Versuchspflanzen im Ein- bis Drei-Blattstadium behandelt. Die als Spritzpulver (WP) oder Flüssigkeit (EC) formulierten Testverbindungen werden in verschiedenen Dosierungen mit einer Wasseraufwandmenge von umgerechnet 300 1/ha unter Zusatz von Netzmittel (0,2 bis 0,3 %) auf die Pflanzen und die Bodenoberfläche gespritzt. 3 bis 4 Wochen nach Behandlung der Versuchspflanzen wird die Wirkung der Präparate visuell im Vergleich zu unbehandelten Kontrollen bonitiert (herbizide Wirkung in Prozent (%): 100 % Wirkung = Pflanzen sind abgestorben, 0 % Wirkung = wie Kontrollpflanzen).

### Verwendung von Safenern

Soll zusätzlich getestet werden, ob Safener die Pflanzenverträglichkeit von Testsubstanzen bei den Kulturpflanzen verbessern können, werden folgende Möglichkeiten für die Anwendung des Safeners verwendet:
- Samen der Kulturpflanzen werden vor der Aussaat mit der Safenersubstanz gebeizt (Angabe der Safenermenge in Prozent bezogen auf das Samengewicht)
- Kulturpflanzen werden vor der Anwendung der Testsubstanzen mit dem Safener mit einer bestimmten Hektaraufwandmenge gespritzt (üblicherweise 1 Tag vor Anwendung der Prüfsubstanzen)
- der Safener wird zusammen mit der Testsubstanz als Tankmischung appliziert (Angabe der Safenermenge in g/ha oder als Verhältnis zum Herbizid).

Durch Vergleich der Wirkung von Testsubstanzen auf Kulturpflanzen, welche ohne und mit Safener behandelt wurden, kann die Wirkung der Safenersubstanz beurteilt werden.

### Gefäßversuche im Gewächshaus, Safenerbehandlung 1 Tag vor Herbizidapplikation

**Tabelle 1**

| | | 10 Tage nach Applikation | 28 Tage nach Applikation |
|---|---|---|---|
| | Aufwandmenge g a.i./ha | Sommergerste beobachtet (%) | Sommergerste beobachtet (%) |
| **I-1-a-5** | | | |
| | 50 | 80 | 40 |
| | 25 | 70 | 25 |
| | 12,5 | 50 | 15 |
| **I-1-a-5 + Mefenpyr** | | | |
| | 50 + 100 | 60 | 15 |
| | 25 + 100 | 40 | 10 |
| | 12,5 + 100 | 20 | 0 |

**Tabelle 2**

| | | 10 Tage nach Applikation | 28 Tage nach Applikation |
|---|---|---|---|
| | Aufwandmenge g a.i./ha | Sommerweizen beobachtet (%) | Sommerweizen beobachtet (%) |
| **I-1-a-5** | | | |
| | 50 | 70 | 75 |
| | 25 | 70 | 60 |
| | 12,5 | 60 | 40 |
| | 6,25 | 50 | 10 |
| **I-1-a-5 + Mefenpyr** | | | |
| | 50 + 100 | 40 | 25 |
| | 25 + 100 | 20 | 20 |
| | 12,5 + 100 | 10 | 5 |
| | 6,25 + 100 | 3 | 0 |

**Tabelle 3**

| | | 10 Tage nach Applikation |
|---|---|---|
| | Aufwandmenge g a.i./ha | Sommerweizen beobachtet (%) |
| **I-1-a-12** | | |
| | 25 | 50 |
| | 12,5 | 40 |
| | 6,25 | 10 |
| **I-1-a-12 + Mefenpyr** | | |
| | 25 + 100 | 20 |
| | 12,5 + 100 | 15 |
| | 6,25 + 100 | 0 |

**Tabelle 4**

| | | 10 Tage nach Applikation | 28 Tage nach Applikation |
|---|---|---|---|
| | Aufwandmenge g a.i./ha | Sommerweizen beobachtet (%) | Sommerweizen beobachtet (%) |
| **I-1-a-12** | | | |
| | 50 | 60 | 65 |
| | 25 | 60 | 65 |
| | 12,5 | 50 | 25 |
| | 6,25 | 30 | 5 |
| **I-1-a-12 + Mefenpyr** | | | |
| | 50 + 1 00 | 5 | 10 |
| | 25 + 100 | 5 | 5 |
| | 12,5 + 100 | 3 | 0 |
| | 6,25 + 100 | 2 | 0 |

**Tabelle 5**

| | | 28 Tage nach Applikation | |
|---|---|---|---|
| | Aufwandmenge g a.i./ha | Sommergerste beobachtet (%) | Sommerweizen beobachtet (%) |
| **I-1-a-14** | | | |
| | 100 | | 85 |
| | 50 | 70 | 75 |
| | 25 | 60 | 50 |
| | 12,5 | 20 | 20 |
| **I-1-a-14 + Mefenpyr** | | | |
| | 1 00 + 1 00 | | 40 |
| | 50 + 1 00 | 30 | 30 |
| | 25 + 100 | 25 | 20 |
| | 12,5 + 100 | 5 | 5 |

**Tabelle 6**

| | | 10 Tage nach Applikation | 28 Tage nach Applikation |
|---|---|---|---|
| | Aufwandmenge g a.i./ha | Sommergerste beobachtet (%) | Sommergerste beobachtet (%) |
| **I-1-a-18** | | | |
| | 100 | 35 | 20 |
| | 50 | 25 | 10 |
| | 25 | 15 | |
| | 12,5 | 10 | |
| **I-1-a-18 + Mefenpyr** | | | |
| | 1 00 + 50 | 20 | 5 |
| | 50 + 50 | 15 | 5 |
| | 25 + 50 | 10 | |
| | 12,5 + 50 | 5 | |

**Tabelle 7**

| | | 10 Tage nach Applikation |
|---|---|---|
| | Aufwandmenge g a.i./ha | Sommerweizen beobachtet (%) |
| **I-1-a-18** | | |
| | 100 | 40 |
| | 50 | 25 |
| | 25 | 15 |
| | 12,5 | 10 |
| **I-1-a-18 + Mefenpyr** | | |
| | 1 00 + 50 | 10 |
| | 50 + 50 | 5 |
| | 25 + 50 | 5 |
| | 12,5 + 50 | 0 |

**Tabelle 8**

| | | 10 Tage nach Applikation |
|---|---|---|
| | Aufwandmenge g a.i./ha | Sommergerste beobachtet (%) |
| **I-1-a-7** | | |
| | 50 | 60 |
| | 25 | 50 |
| | 12,5 | 10 |
| **I-1-a-7 + Mefenpyr** | 50 + 1 00 | 40 |
| | 25 + 100 | 10 |
| | 12,5 + 100 | 7 |

**Tabelle 9**

| | | 28 Tage nach Applikation |
|---|---|---|
| | Aufwandmenge g a.i./ha | Sommerweizen beobachtet (%) |
| **I-1-a-7** | | |
| | 100 | 80 |
| | 50 | 70 |
| | 25 | 60 |
| | 12,5 | 30 |
| **I-1-a-7 + Mefenpyr** | 100 + 100 | 50 |
| | 50 + 1 00 | 40 |
| | 25 + 100 | 20 |
| | 12,5 + 100 | 10 |

**Tabelle 10**

| | | 10 Tage nach Applikation |
|---|---|---|
| | Aufwandmenge g a.i./ha | Sommerweizen beobachtet (%) |
| **I-1-a-8** | | |
| | 100 | 30 |
| | 50 | 30 |
| | 25 | 25 |
| **I-1-a-8 + Mefenpyr** | 100 + 100 | 5 |
| | 50 + 1 00 | 0 |
| | 25 + 100 | 0 |

**Tabelle 11**

| | | 10 Tage nach Applikation | 28 Tage nach Applikation |
|---|---|---|---|
| | Aufwandmenge g a.i./ha | Sommerweizen beobachtet (%) | Sommerweizen beobachtet (%) |
| **I-1-a-9** | | | |
| | 25 | 65 | 50 |
| | 12,5 | 60 | |
| **I-1-a-9 + Mefenpyr** | 25 + 100 | 50 | 25 |
| | 12,5 + 100 | 15 | |

**Tabelle 12**

| | | 10 Tage nach Applikation | 28 Tage nach Applikation |
|---|---|---|---|
| | Aufwandmenge g a.i./ha | Sommergerste beobachtet (%) | Sommergerste beobachtet (%) |
| **I-1-b-1** | | | |
| | 50 | | 80 |
| | 25 | 65 | 30 |
| | 12,5 | 30 | 10 |
| **I-1-b-1 + Mefenpyr** | 50 + 1 00 | | 30 |
| | 25 + 100 | 30 | 15 |
| | 12,5 + 100 | 5 | 0 |

G

**Tabelle 13**

| | | 10 Tage nach Applikation | 28 Tage nach Applikation |
|---|---|---|---|
| | Aufwandmenge g a.i./ha | Sommerweizen beobachtet (%) | Sommerweizen beobachtet (%) |
| **I-1-b-1** | | | |
| | 100 | | 95 |
| | 50 | | 90 |
| | 25 | 60 | 40 |
| | 12,5 | 50 | |
| **I-1-b-1 + Mefenpyr** | 100 + 100 | | 40 |
| | 50 + 1 00 | | 20 |
| | 25 + 100 | 25 | 0 |
| | 12,5 + 100 | 15 | |

**Tabelle 14**

| | | 10 Tage nach Applikation | 10 Tage nach Applikation |
|---|---|---|---|
| | Aufwandmenge g a.i./ha | Sommergerste beobachtet (%) | Sommerweizen beobachtet (%) |
| **I-1-c-13** | | | |
| | 50 | 30 | 40 |
| | 25 | 20 | 30 |
| | 12,5 | 5 | 20 |
| **I-1-c-13 + Mefenpyr** | 50 + 50 | 10 | 30 |
| | 25 + 50 | 5 | 20 |
| | 12,5 + 50 | 0 | 5 |

**Tabelle 15**

| | | 10 Tage nach Applikation | 28 Tage nach Applikation |
|---|---|---|---|
| | Aufwandmenge g a.i./ha | Sommergerste beobachtet (%) | Sommergerste beobachtet (%) |
| **I-1-c-7** | | | |
| | 100 | 90 | 95 |
| | 50 | 60 | 50 |
| | 25 | 40 | |
| | 12,5 | 10 | |
| **I-1-c-7 + Mefenpyr** | 100 + 100 | 50 | 25 |
| | 50 + 1 00 | 10 | 5 |
| | 25 + 100 | 3 | |
| | 12,5 + 100 | 0 | |

**Tabelle 16**

| | | 10 Tage nach Applikation | 28 Tage nach Applikation |
|---|---|---|---|
| | Aufwandmenge g a.i./ha | Sommerweizen beobachtet (%) | Sommerweizen beobachtet (%) |
| **I-1-c-7** | | | |
| | 50 | | 80 |
| | 25 | 60 | 20 |
| | 12,5 | 20 | 10 |
| **I-1-c-7** + Mefenpyr | 50 + 1 00 | | 20 |
| | 25 + 100 | 30 | 10 |
| | 12,5 + 100 | 10 | 5 |

### Beispiel D

### Phaedon-Test (Spritzbehandlung)

| | |
|---|---|
| Lösungsmittel: | 78 Gewichtsteile Aceton |
| | 1,5 Gewichtsteile Dimethylformamid |
| Emulgator: | 0,5 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Chinakohlblattscheiben *(Brassica pekinensis*) werden mit einer Wirkstoffzubereitung der gewünschten Konzentration gespritzt und nach dem Abtrocknen mit Larven des Meerrettichblattkäfers *(Phaedon cochleariae)* besetzt.

Nach der gewünschten Zeit wird die Wirkung in % bestimmt. Dabei bedeutet 100 %, dass alle Käferlarven abgetötet wurden; 0 % bedeutet, dass keine Käferlarven abgetötet wurden.

Bei diesem Test zeigen z. B. die folgenden Verbindungen der Herstellungsbeispiele eine Wirkung von ≥ 80 % bei einer Aufwandmenge von 500 g/ha: Beispiel: I-1-a-2, 1-1-a-4, 1-1-a-6, 1-1-a-7, I-1-a-10, I-1-a-11, I-1-a-12, I-1-a-14, I-1-a-24, I-1-a-25, I-1-b-5, I-1-b-11, I-1-b-13, I-1-c-7, I-1-c-12, I-1-c-20, I-1-c-22, I-1-c-23, 1-1-d-1,

### Beispiel Nr. E

### Myzus-Test (MYZUPE Spritzbehandlung)

| | |
|---|---|
| Lösungsmittel: | 78 Gewichtsteile Aceton |
| | 1,5 Gewichtsteile Dimethylformamid |
| | |
| Emulgator: | 0,5 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Chinakohlblattscheiben (*Brassica pekinensis*), die von allen Stadien der Grünen Pfirsichblattlaus (*Myzus persicae*) befallen sind, werden mit einer Wirkstoffzubereitung der gewünschten Konzentration gespritzt.

Nach der gewünschten Zeit wird die Wirkung in % bestimmt. Dabei bedeutet 100 %, dass alle Blattläuse abgetötet wurden; 0 % bedeutet, dass keine Blattläuse abgetötet wurden.

Bei diesem Test zeigen z. B. die folgenden Verbindungen der Herstellungsbeispiele eine Wirkung von ≥ 90 % bei einer Aufwandmenge von 500 g/ha: Beispiel: I-1-a-2, I-1-a-4, I-1-a-6, I-1-a-7, I-1-a-9, I-1-a-11, I-1-a-12, I-1-a-14, I-1-a-15, I-1-a-17, I-1-a-20, I-1-a-22, I-1-b-3, I-1-b-4, I-1-b-6, I-1-b-10, I-1-b-13, I-1-c-1, I-1-c-8, I-1-c-9, I-1-c-14, I-1-c-15, I-1-c-19,

### Beispiel Nr. F

### Tetranychus-Test, OP-resistent (TETRUR Spritzbehandlung)

| | |
|---|---|
| Lösungsmittel: | 78 Gewichtsteile Aceton |
| | 1,5 Gewichtsteile Dimethylformamid |
| Emulgator: | 0,5 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Bohnenblattscheiben *(Phaseolus vulgaris),* die von allen Stadien der Gemeinen Spinnmilbe *(Tetranychus urticae)* befallen sind, werden mit einer Wirkstoffzubereitung der gewünschten Konzentration gespritzt.

Nach der gewünschten Zeit wird die Wirkung in % bestimmt. Dabei bedeutet 100 %, dass alle Spinnmilben abgetötet wurden; 0 % bedeutet, dass keine Spinnmilben abgetötet wurden.

Bei diesem Test zeigen z. B. die folgenden Verbindungen der Herstellungsbeispiele eine Wirkung von ≥ 80 % bei einer Aufwandmenge von 100 g/ha: oder 20 g/ha*. Beispiel: I-1-a-4, I-1-a-5, I-1-a-6, I-1-a-7, I-1-a-8, I-1-a-9, I-1-a-10, I-1-a-11, I-1-a-14, I-1-a-17, I-1-a-19, I-1-a-20, I-1-a-25*, I-1-b-4, I-1-b-8, I-1-b-9, I-1-b-13, I-1-b-15, I-1-c-9, I-1-c-12, I-1-c-13, I-1-c-14, I-1-c-15, I-1-c-16, I-1-c-20, I-1-c-25, I-1-d-1, I-8-b-2.

### Beispiel Nr. G

### Myzus persicae -Test, hydroponische Behandlung (MYZUPE sys.)

| | |
|---|---|
| Lösungsmittel: 7 | Gewichtsteile Dimethylformamid |
| Emulgator: | 2 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Die Wirkstoffzubereitung wird mit Wasser gemischt. Die angegebene Konzentration bezieht sich auf die Wirkstoffmenge pro Volumeneinheit Wasser (mg/l = ppm). Man füllt das behandelte Wasser in Gefäße mit einer Erbsenpflanze (*Pisum sativum*), anschließend wird mit der Grünen Pfirsichblattlaus (*Myzus persicae*) infiziert.

Nach der gewünschten Zeit wird die Abtötung in % bestimmt. Dabei bedeutet 100 %, dass alle Blattläuse abgetötet wurden; 0 % bedeutet, dass keine Blattläuse abgetötet wurden.

Bei diesem Test zeigt z. B. die folgende Verbindung der Herstellungsbeispiele eine Wirkung von ≥ 90 % bei einer Konzentration von 20 ppm: Bsp. I-1-a-4.

### Beispiel Nr. H

### Meloidogyne-Test (MELGIN Spritzbehandlung)

| | |
|---|---|
| Lösungsmittel: | 80 Gewichtsteile Aceton |

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser auf die gewünschte Konzentration.

Gefäße werden mit Sand, Wirkstofflösung, *Meloidogyne incognita*-Ei-Larven-Suspension und Salatsamen gefüllt. Die Salatsamen keimen und die Pflänzchen entwickeln sich. An den Wurzeln entwickeln sich die Gallen.

Nach der gewünschten Zeit wird die nematizide Wirkung an Hand der Gallenbildung in % bestimmt. Dabei bedeutet 100 %, dass keine Gallen gefunden wurden; 0 % bedeutet, dass die Zahl der Gallen an den behandelten Pflanzen der der unbehandelten Kontrolle entspricht.

Bei diesem Test zeigen z. B. die folgenden Verbindungen der Herstellungsbeispiele eine Wirkung von ≥ 80 % bei einer Aufwandmenge von 20 ppm: Beispiel: I-1-a-7, I-1-a-12.

### Beispiel Nr. I

### Lucilia cuprina-Test (LUCICU)

| | |
|---|---|
| Lösungsmittel: | Dimethylsulfoxid |

Zur Herstellung einer zweckmäßigen Wirkstoffzubereitung vermischt man 1 Gewichtsteil Wirkstoff mit der angegebenen Menge Lösungsmittel und verdünnt das Konzentrat mit Lösungsmittel auf die gewünschte Konzentration.

Gefäße, die Pferdefleisch enthalten, das mit der Wirkstoffzubereitung der gewünschten Konzentration behandelt wurde, werden mit *Lucilia cuprina* Larven besetzt.

Nach der gewünschten Zeit wird die Abtötung in % bestimmt. Dabei bedeutet 100 %, dass alle Larven abgetötet wurden; 0 % bedeutet, dass keine Larven abgetötet wurden.

Bei diesem Test zeigen z. B. die folgenden Verbindungen der Herstellungsbeispiele eine Wirkung von ≥ 80 % bei einer Aufwandmenge von 100 ppm: Beispiel: I-1-a-4, I-1-b-4, I-1-c-3.

### Beispiel Nr. J

### Boophilus microplus -Test (BOOPMI Injektion)

| | |
|---|---|
| Lösungsmittel: | Dimethylsulfoxid |

Zur Herstellung einer zweckmäßigen Wirkstoffzubereitung vermischt man 1 Gewichtsteil Wirkstoff mit der angegebenen Menge Lösungsmittel und verdünnt das Konzentrat mit Lösungsmittel auf die gewünschte Konzentration.

Die Wirkstofflösung wird in das Abdomen *(Boophilus microplus)* injiziert, die Tiere werden in Schalen überführt und in einem klimatisierten Raum aufbewahrt.

Nach der gewünschten Zeit wird die Wirkung in % bestimmt. Dabei bedeutet 100%, dass keine Zecke fertile Eier gelegt hat.

Bei diesem Test zeigen z. B. die folgenden Verbindungen der Herstellungsbeispiele eine Wirkung von ≥ 80 % bei einer Aufwandmenge von 20 µg/Tier: Beispiel: I-1-a-1, I-1-a-2, I-1-a-4, I-1-a-6, I-1-b-4, I-1-c-3.

### Beispiel K

### Grenzkonzentrations-Test / Bodeninsekten - Behandlung transgener Pflanzen

| | |
|---|---|
| Testinsekt: | Diabrotica balteata - Larven im Boden |
| Lösungsmittel: | 7 Gewichtsteile Aceton |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird auf den Boden gegossen. Dabei spielt die Konzentration des Wirkstoffs in der Zubereitung praktisch keine Rolle, entscheidend ist allein die Wirkstoffgewichtsmenge pro Volumeneinheit Boden, welche in ppm (mg/l) angegeben wird. Man füllt den Boden in 0,251 Töpfe und lässt diese bei 20°C stehen.

Sofort nach dem Ansatz werden je Topf 5 vorgekeimte Maiskörner der Sorte YIELD GUARD (Warenzeichen von Monsanto Comp., USA) gelegt. Nach 2 Tagen werden in den behandelten Boden die entsprechenden Testinsekten gesetzt. Nach weiteren 7 Tagen wird der Wirkungsgrad des Wirkstoffs durch Auszählen der aufgelaufenen Maispflanzen bestimmt (1 Pflanze = 20 % Wirkung).

### Beispiel L

### Heliothis virescens - Test - Behandlung transgener Pflanzen

| | |
|---|---|
| Lösungsmittel: | 7 Gewichtsteile Aceton |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Sojatriebe (Glycine max) der Sorte Roundup Ready (Warenzeichen der Monsanto Comp. USA) werden durch Tauchen in die Wirkstoffzubereitung der gewünschten Konzentration behandelt und mit der Tabakknospenraupe Heliothis virescens besetzt, solange die Blätter noch feucht sind.

Nach der gewünschten Zeit wird die Abtötung der Insekten bestimmt.

### Beispiel M: Wirkungssteigerung durch Ammonium/Phosphoniumsalze in Kombination mit Penetrationsförderern

### Myzus persicae-Test

| | |
|---|---|
| Lösungsmittel: | 7 Gewichtsteile Dimethylformamid |
| Emulgator: | 2 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration. Für die Anwendung mit Penetrationsförderen (Rapsölmethylester 500 EW), Ammonium- oder Phosphoniumsalzen oder Ammoniumsalzen und Penetrationsförderern (Rapsölmethylester 500 EW) werden diese jeweils in einer Konzentration von 1 000 ppm der Spritzbrühe hinzugegeben.

Paprikapflanzen (*Capsicum annuum*), die stark von der Grünen Pfirsichblattlaus (*Myzus persicae*) befallen sind, werden durch Tropfnassspritzung mit der Wirkstoffzubereitung in der gewünschten Konzentration behandelt.

Nach der gewünschten Zeit wird die Abtötung in % bestimmt. Dabei bedeutet 100 %, dass alle Tiere abgetötet wurden; 0 % bedeutet, dass keine Tiere abgetötet wurden.

Bei deisem Test zeigen z.B. die folgenden Verbindungen der Herstellbeispiele gute Wirksamkeit: siehe Tabelle

**Tabelle**

| **Wirkstoff** | **Konzentration (ppm)** | **Abtötung (%) nach 6d** | **+ AS 1000 ppm** | **+ RME 1000 ppm** | **+ AS + RME je 1000 ppm** |
|---|---|---|---|---|---|
| I-1-a-15 | 20 | 95 | 95 | 95 | 98 |
| | 4 | 5 | 15 | 55 | 95 |

| | | | | | |
|---|---|---|---|---|---|
| AS = Ammoniumsulfat | | | | | |

### Beispiel N

### Aphis gossypii -Test

| | |
|---|---|
| Lösungsmittel: | 7 Gewichtsteile Dimethylformamid |
| Emulgator: | 2 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration. Für die Anwendung mit Penetrationsförderen (Rapsölmethylester 500 EW), Ammonium- oder Phosphoniumsalzen oder Ammoniumsalzen und Penetrationsförderern (Rapsölmethylester 500 EW) werden diese jeweils in einer Konzentration von 1 000 ppm a.i. der Spritzbrühe hinzugegeben.

Baumwollpflanzen (*Gossypium hirsutum*), die stark von der Baumwollblattlaus (*Aphis gossypii*) befallen sind, werden durch Tropfnassspritzung mit der Wirkstoffzubereitung in der gewünschten Konzentration behandelt.

Nach der gewünschten Zeit wird die Abtötung in % bestimmt. Dabei bedeutet 100 %, dass alle Blattläuse abgetötet wurden; 0 % bedeutet, dass keine Blattläuse abgetötet wurden.

Bei deisem Test zeigen z.B. die folgenden Verbindungen der Herstellbeispiele gute Wirksamkeit: siehe Tabelle

**Tabelle**

| **Wirkstoff** | **Konzentration (ppm)** | **Abtötung (%) nach 6d** | **+ AS 1000 ppm** | **+ RME 1000 ppm** | **+ AS + RME je 1000 ppm** |
|---|---|---|---|---|---|
| I-1-a-7 | 20 | 55 | 65 | 75 | 99 |

| | | | | | |
|---|---|---|---|---|---|
| AS = Ammoniumsulfat | | | | | |

### Beispiel O

Steigerung der Penetration in die Pflanze durch Ammonium- oder Phosphoniumsalze und synergistische Steigerung der Penetration in die Pflanze durch Ammonium- / Phosphoniumsalze in Kombination mit Penetrationsförderern

In diesem Test wurde die Penetration von Wirkstoffen durch enzymatisch isolierte Kutikeln von Apfelbaumblättern gemessen.

Verwendet wurden Blätter, die in voll entwickeltem Zustand von Apfelbäumen der Sorte Golden Delicious abgeschnitten wurden. Die Isolierung der Kutikeln erfolgte in der Weise, dass
- zunächst auf der Unterseite mit Farbstoff markierte und ausgestanzte Blattscheiben mittels Vakuuminfiltration mit einer auf einen pH Wert zwischen 3 und 4 gepufferten Pectinase-Lösung (0,2 bis 2 %ig) gefüllt wurden,
- dann Natriumazid hinzugefügt wurde und
- die so behandelten Blattscheiben bis zur Auflösung der ursprünglichen Blattstruktur und zur Ablösung der nicht zellulären Kutikula stehen gelassen wurden.

Danach wurden nur die von Spaltöffnungen und Haaren freien Kutikeln der Blattoberseiten weiter verwendet. Sie wurden mehrfach abwechselnd mit Wasser und einer Pufferlösung vom pH Wert 7 gewaschen. Die erhaltenen sauberen Kutikel wurden schließlich auf Teflonplättchen aufgezogen und mit einem schwachen Luftstrahl geglättet und getrocknet.

Im nächsten Schritt wurden die so gewonnenen Kutikelmembranen für Membran-Transport-Untersuchungen in Diffusionszellen (= Transportkammern) aus Edelstahl eingelegt. Dazu wurden die Kutikeln mit einer Pinzette mittig auf die mit Silikonfett bestrichenen Ränder der Diffusionszellen plaziert und mit einem ebenfalls gefetteten Ring verschlossen. Die Anordnung war so gewählt worden, dass die morphologische Außenseite der Kutikeln nach außen, also zur Luft, gerichtet war, während die ursprüngliche Innenseite dem Inneren der Diffusionszelle zugewandt war.

Die Diffusionszellen waren mit einer 30%igen Ethylenglykol/Wasser-Lösung befüllt. Zur Bestimmung der Penetration wurden jeweils 10 µl der Spritzbrühe der nachstehenden Zusammensetzung auf die Außenseite der Kutikula appliziert. Das Ansetzen der Spritzbrühe erfolgt mit lokalem Leitungswasser mittlerer Wasserhärte.

Nach dem Auftragen der Spritzbrühen ließ man das Wasser verdunsten, drehte die Kammern um und stellte sie in thermostatisierte Wannen, in denen die Temperatur und Luftfeuchte über der Kutikula durch einen leichten Luftstrom auf die Kutikula mit dem Spritzbelag einstellbar war (20°C, 60 % rh). In regelmäßigen Abständen wurden von einem Autosampler Aliquots entnommen und der Wirkstoffgehalt mit HPLC bestimmt.

Die Versuchsergebnisse gehen aus der folgenden Tabelle hervor. Bei den angegebenen Zahlen handelt es sich um Durchschnittswerte aus 8 bis 10 Messungen. Deutlich ist zu sehen, dass zusammen mit RME ein überadditiver (synergistischer) Effekt auftritt.

**Tabelle 1**

| Wirkstoff | | | | |
|---|---|---|---|---|
| | Penetration nach 24h / % | | | |
| | a.i. | a.i. + AS (1 g/l) | a.i. + RME (1 g/l) | a.i. + RME (1 g/l) + AS (1 g/l) |
| Beispiel I-1-a-21 200 ppm in Aceton / Wasser 4:6 | 2 | 1.1 | 35.6 | 56.9 |

| | | | | |
|---|---|---|---|---|
| RME=Rapsölmethylester (Einsatz formuliert als 500 EW, Konzentrationsangabe in g Wirkstoff/e) AS = Ammoniumsulfat | | | | |

## Patentansprüche

1. Verbindungen der Formel (I) 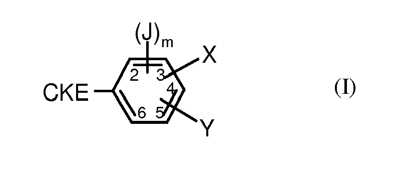 in welcher
J für gegebenenfalls substituiertes Cycloalkyl steht, welches gegebenenfalls durch Heteroatome unterbrochen sein kann,
X für Wasserstoff, Alkyl, Halogen, Halogenalkyl, Alkoxy oder Halogenalkoxy steht,
Y für Wasserstoff, Alkyl, Halogenalkyl, Halogen, Alkoxy oder Halogenalkoxy steht,
m für eine Zahl 1, 2 oder 3 steht,
mit der Maßgabe, dass mindestens einer der Reste J, X oder Y in der 2-Position des Phenylrestes steht und dabei ungleich Wasserstoff ist,
CKE für die Gruppe 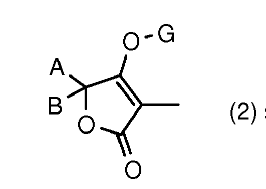 steht,
worin
A für Wasserstoff, jeweils gegebenenfalls durch Halogen substituiertes Alkyl, Alkenyl, Alkoxyalkyl, Alkylthioalkyl, gesättigtes oder ungesättigtes, gegebenenfalls substituiertes Cycloalkyl, in welchem gegebenenfalls mindestens ein Ring-atom durch ein Heteroatom ersetzt ist, oder jeweils gegebenenfalls durch Halogen, Alkyl, Halogenalkyl, Alkoxy, Halogenalkoxy, Cyano oder Nitro substituiertes Aryl, Arylalkyl oder Hetaryl steht,
B für Wasserstoff, Alkyl oder Alkoxyalkyl steht, oder
A und B gemeinsam mit dem Kohlenstoffatom an das sie gebunden sind für einen gesättigten oder ungesättigten, gegebenenfalls mindestens ein Heteroatom enthaltenden unsubstituierten oder substituierten Cyclus stehen,
G für Wasserstoff (a) oder für eine der Gruppen 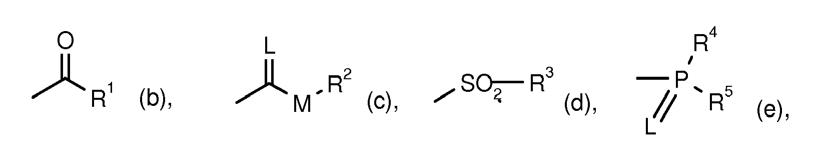 E (f) oder 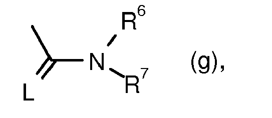 steht,
worin
E für ein Metallionäquivalent oder ein Ammoniumion steht,
L für Sauerstoff oder Schwefel steht,
M für Sauerstoff oder Schwefel steht,
R¹ für jeweils gegebenenfalls durch Halogen substituiertes Alkyl, Alkenyl, Alkoxyalkyl, Alkylthioalkyl, Polyalkoxyalkyl oder gegebenenfalls durch Halogen, Alkyl oder Alkoxy substituiertes Cycloalkyl, das durch mindestens ein Heteroatom unterbrochen sein kann, jeweils gegebenenfalls substituiertes Phenyl, Phenylalkyl, Hetaryl, Phenoxyalkyl oder Hetaryloxyalkyl steht,
R² für jeweils gegebenenfalls durch Halogen substituiertes Alkyl, Alkenyl, Alkoxyalkyl, Polyalkoxyalkyl oder für jeweils gegebenenfalls substituiertes Cycloalkyl, Phenyl oder Benzyl steht,
R³, R⁴ und R⁵ unabhängig voneinander für jeweils gegebenenfalls durch Halogen substituiertes Alkyl, Alkoxy, Alkylamino, Dialkylamino, Alkylthio, Alkenylthio, Cycloalkylthio oder für jeweils gegebenenfalls substituiertes Phenyl, Benzyl, Phenoxy oder Phenylthio stehen,
R⁶ und R⁷ unabhängig voneinander für Wasserstoff, jeweils gegebenenfalls durch Halogen substituiertes Alkyl, Cycloalkyl, Alkenyl, Alkoxy, Alkoxyalkyl, für gegebenenfalls substituiertes Phenyl, für gegebenenfalls substituiertes Benzyl stehen, oder gemeinsam mit dem N-Atom, an das sie gebunden sind, für einen gegebenenfalls durch Sauerstoff oder Schwefel unterbrochenen Cyclus stehen.

2. Verbindungen der Formel (I) gemäß Anspruch 1, in welcher
J für C₃-C₈-Cycloalkyl steht, welches gegebenenfalls durch Sauerstoff unterbrochen sein kann und welches gegebenenfalls durch Halogen, C₁-C₆-Alkyl, C₁-C₆-Alkoxy, C₁-C₆-Haloalkyl oder gegebenenfalls durch C₁-C₄-Alkyl, C₁-C₄-Alkoxy, Halogen, C₁-C₄-Halogenalkyl, C₁-C₄-Halogenalkoxy substituiertes Phenyl oder C₃-C₆-Cycloalkyl substituiert sein kann,
X für Wasserstoff, Halogen, C₁-C₆-Alkyl, C₁-C₄-Halogenalkyl, C₁-C₆-Alkoxy oder C₁-C₄-Halogenalkoxy steht,
Y für Wasserstoff, C₁-C₆-Alkyl, C₁-C₄-Halogenalkyl, Halogen, C₁-C₆-Alkoxy oder C₁-C₄-Halogenalkoxy steht,
m für eine Zahl 1, 2 oder 3 steht
mit der Maßgabe, dass mindestens einer der Reste J, X oder Y in der 2-Position des Phenylrestes steht und dabei ungleich Wasserstoff ist,
CKE für die Gruppe 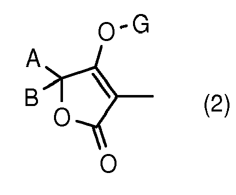 steht,
A für Wasserstoff oder jeweils gegebenenfalls durch Halogen substituiertes C₁-C₁₂-Alkyl, C₃-C₈-Alkenyl, C₁-C₁₀-Alkoxy-C₁-C₈-alkyl, C₁-C₁₀-Alkylthio-C₁-C₆-alkyl, gegebenenfalls durch Halogen, C₁-C₆-Alkyl oder C₁-C₆-Alkoxy substituiertes C₃-C₈-Cycloalkyl, in welchem gegebenenfalls ein oder zwei nicht direkt benachbarte Ringglieder durch Sauerstoff und/oder Schwefel ersetzt sind oder für jeweils gegebenenfalls durch Halogen, C₁-C₆-Alkyl, C₁-C₆- Halogenalkyl, C₁-C₆-Alkoxy, C₁-C₆-Halogenalkoxy, Cyano oder Nitro substituiertes Phenyl, Naphthyl, Hetaryl mit 5 bis 6 Ringatomen, Phenyl-C₁-C₆-alkyl oder Naphthyl-C₁-C₆-alkyl steht,
B für Wasserstoff, C₁-C₁₂-Alkyl oder C₁-C₈-Alkoxy-C₁-C₆-alkyl steht oder
A, B und das Kohlenstoffatom an das sie gebunden sind, für gesättigtes C₃-C₁₀-Cycloalkyl oder ungesättigtes C₅-C₁₀-Cycloalkyl stehen, worin gegebenenfalls ein Ringglied durch Sauerstoff oder Schwefel ersetzt ist und welche gegebenenfalls einfach oder zweifach durch C₁-C₈-Alkyl, C₃-C₁₀-Cycloalkyl, C₁-C₈-Halogen-alkyl, C₁-C₈-Alkoxy, C₁-C₄-Alkoxy-C₁-C₄-alkoxy, C₁-C₈-Alkylthio, Halogen oder Phenyl substituiert sind oder
A, B und das Kohlenstoffatom, an das sie gebunden sind, für C₃-C₆-Cycloalkyl stehen, welches durch eine gegebenenfalls ein oder zwei nicht direkt benachbarte Sauerstoff- und/oder Schwefelatome enthaltende gegebenenfalls durch C₁-C₄-Alkyl substituierte Alkylendiyl-, oder durch eine Alkylendioxyl- oder durch eine Alkylendithioyl-Gruppe substituiert ist, die mit dem Kohlenstoffatom, an das sie gebunden ist, einen weiteren fünf- bis achtgliedrigen Ring bildet oder
A, B und das Kohlenstoffatom, an das sie gebunden sind, für C₃-C₈-Cycloalkyl oder C₅-C₈-Cycloalkenyl stehen, in welchen zwei Substituenten gemeinsam mit den Kohlenstoffatomen, an die sie gebunden sind, für jeweils gegebenenfalls durch C₁-C₆-Alkyl, C₁-C₆-Alkoxy oder Halogen substituiertes C₂-C₆-Alkandiyl, C₂-C₆-Alkendiyl oder C₄-C₆-Alkandiendiyl stehen, worin gegebenenfalls eine Methylengruppe durch Sauerstoff oder Schwefel ersetzt ist,
G für Wasserstoff (a) oder für eine der Gruppen 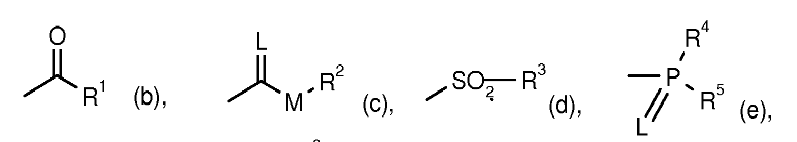 E (f) oder 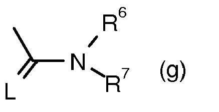 steht, in welchen
E für ein Metallionäquivalent oder ein Ammoniumion steht,
L für Sauerstoff oder Schwefel steht und
M für Sauerstoff oder Schwefel steht,
R¹ für jeweils gegebenenfalls durch Halogen substituiertes C₁-C₂₀-Alkyl, C₂-C₂₀-Alkenyl, C₁-C₈-Alkoxy-C₁-C₈-alkyl, C₁-C₈-Alkylthio-C₁-C₈-alkyl, Poly-C₁-C₈-alkoxy-C₁-C₈-alkyl oder gegebenenfalls durch Halogen, C₁-C₆-Alkyl oder C₁-C₆-Alkoxy substituiertes C₃-C₈-Cycloalkyl, in welchem gegebenenfalls ein oder mehrere nicht direkt benachbarte Ringglieder durch Sauerstoff und/oder Schwefel ersetzt sind,
für gegebenenfalls durch Halogen, Cyano, Nitro, C₁-C₆-Alkyl, C₁-C₆-Alkoxy, C₁-C₆-Halogenalkyl, C₁-C₆-Halogenalkoxy, C₁-C₆-Alkylthio oder C₁-C₆-Alkylsulfonyl substituiertes Phenyl,
für gegebenenfalls durch Halogen, Nitro, Cyano, C₁-C₆-Alkyl, C₁-C₆-Alkoxy, C₁-C₆-Halogenalkyl oder C₁-C₆-Halogenalkoxy substituiertes Phenyl-C₁-C₆-alkyl,
für gegebenenfalls durch Halogen oder C₁-C₆-Alkyl substituiertes 5- oder 6-gliedriges Hetaryl,
für gegebenenfalls durch Halogen oder C₁-C₆-Alkyl substituiertes Phenoxy-C₁-C₆-alkyl oder
für gegebenenfalls durch Halogen, Amino oder C₁-C₆-Alkyl substituiertes 5- oder 6-gliedriges Hetaryloxy-C₁-C₆-alkyl steht,
R² für jeweils gegebenenfalls durch Halogen substituiertes C₁-C₂₀-Alkyl, C₂-C₂₀-Alkenyl, C₁-C₈-Alkoxy-C₂-C₈-alkyl, Poly-C₁-C₈-alkoxy-C₂-C₈-alkyl,
für gegebenenfalls durch Halogen, C₁-C₆-Alkyl oder C₁-C₆-Alkoxy substituiertes C₃-C₈-Cycloalkyl oder
für jeweils gegebenenfalls durch Halogen, Cyano, Nitro, C₁-C₆-Alkyl, C₁-C₆-Alkoxy, C₁-C₆-Halogenalkyl oder C₁-C₆-Halogenalkoxy substituiertes Phenyl oder Benzyl steht,
R³ für gegebenenfalls durch Halogen substituiertes C₁-C₈-Alkyl oder für jeweils gegebenenfalls durch Halogen, C₁-C₆-Alkyl, C₁-C₆-Alkoxy, C₁-C₄-Halogen- alkyl, C₁-C₄-Halogenalkoxy, Cyano oder Nitro substituiertes Phenyl oder Benzyl steht,
R⁴ und R⁵ unabhängig voneinander für jeweils gegebenenfalls durch Halogen substituiertes C₁-C₈-Alkyl, C₁-C₈-Alkoxy, C₁-C₈-Alkylamino, Di-(C₁-C₈-alkyl)amino, C₁-C₈-Alkylthio, C₂-C₈-Alkenylthio, C₃-C₇-Cycloalkylthio oder für jeweils gegebenenfalls durch Halogen, Nitro, Cyano, C₁-C₄-Alkoxy, C₁-C₄-Halogenalkoxy, C₁-C₄-Alkylthio, C₁-C₄-Halogenalkylthio, C₁-C₄-Alkyl oder C₁-C₄-Halogenalkyl substituiertes Phenyl, Phenoxy oder Phenylthio stehen,
R⁶ und R⁷ unabhängig voneinander für Wasserstoff, für jeweils gegebenenfalls durch Halogen substituiertes C₁-C₈-Alkyl, C₃-C₈-Cycloalkyl, C₁-C₈-Alkoxy, C₃-C₈-Alkenyl, C₁-C₈-Alkoxy-C₁-C₈-alkyl, für gegebenenfalls durch Halogen, C₁-C₈-Halogenalkyl, C₁-C₈-Alkyl oder C₁-C₈-Alkoxy substituiertes Phenyl, gegebenenfalls durch Halogen, C₁-C₈-Alkyl, C₁-C₈-Halogenalkyl oder C₁-C₈-Alkoxy substituiertes Benzyl oder zusammen für einen gegebenenfalls durch C₁-C₄-Alkyl substituierten C₃-C₆-Alkylenrest stehen, in welchem gegebenenfalls ein Kohlenstoffatom durch Sauerstoff oder Schwefel ersetzt ist.

3. Verbindungen der Formel (I) gemäß Anspruch 1, in welcher
J für gegebenenfalls durch ein Sauerstoffatom unterbrochenes, gegebenenfalls einfach bis zweifach durch Fluor, Chlor, C₁-C₄-Alkyl, C₃-C₆-Cycloalkyl, C₁-C₄-Alkoxy oder C₁-C₂-Halogenalkyl substituiertes C₃-C₆-Cycloalkyl steht,
X für Wasserstoff, Fluor, Chlor, Brom, Iod, C₁-C₄-Alkyl, Trifluormethyl, C₁-C₄-Alkoxy, Difluormethoxy oder Trifluormethoxy steht,
Y für Wasserstoff, Fluor, Chlor, Brom, Iod, C₁-C₄-Alkyl, Trifluormethyl, C₁-C₄-Alkoxy, Difluormethoxy oder Trifluormethoxy steht,
m für eine Zahl 1 oder 2 steht
mit der Maßgabe, dass mindestens einer der Reste J, X oder Y in der 2-Position des Phenylrestes steht und dabei ungleich Wasserstoff ist,
CKE für die Gruppe 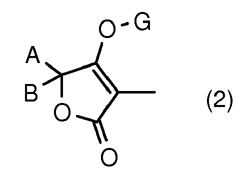 steht,
A für Wasserstoff, jeweils gegebenenfalls einfach bis dreifach durch Fluor oder Chlor substituiertes C₁-C₆-Alkyl, C₁-C₄-Alkoxy-C₁-C₂-alkyl, gegebenenfalls einfach bis zweifach durch C₁-C₂-Alkyl oder C₁-C₂-Alkoxy substituiertes C₃-C₆-Cycloalkyl oder (jedoch nicht im Fall der Verbindungen der Formeln (I-3), (I-4), (I-6) und (I-7)) jeweils gegebenenfalls einfach bis zweifach durch Fluor, Chlor, Brom, C₁-C₄-Alkyl, C₁-C₂-Halogenalkyl, C₁-C₄-Alkoxy, C₁-C₂-Halogenalkoxy, Cyano oder Nitro substituiertes Phenyl oder Benzyl steht,
B für Wasserstoff, C₁-C₄-Alkyl oder C₁-C₂-Alkoxy-C₁-C₂-alkyl steht oder
A, B und das Kohlenstoffatom an das sie gebunden sind, für gesättigtes oder ungesättigtes C₅-C₇-Cycloalkyl stehen, worin gegebenenfalls ein Ringglied durch Sauerstoff oder Schwefel ersetzt ist und welches gegebenenfalls einfach bis zweifach durch C₁-C₆-Alkyl, Trifluormethyl, C₁-C₆-Alkoxy oder C₁-C₃-Alkoxy-C₁-C₃-alkoxy substituiert ist mit der Maßgabe, dass dann Q³ für Wasserstoff oder Methyl steht oder
A, B und das Kohlenstoffatom, an das sie gebunden sind, für C₅-C₆-Cycloalkyl stehen, welches durch eine gegebenenfalls ein oder zwei nicht direkt benachbarte Sauerstoff- oder Schwefelatome enthaltende gegebenenfalls durch Methyl oder Ethyl substituierte Alkylendiyl- oder durch eine Alkylendioxyl- oder durch eine Alkylendithiol-Gruppe substituiert ist, die mit dem Kohlenstoffatom, an das sie gebunden ist, einen weiteren fünf- oder sechsgliedrigen Ring bildet mit der Maßgabe, dass dann Q³ für Wasserstoff oder Methyl steht, oder
A, B und das Kohlenstoffatom, an das sie gebunden sind, für C₃-C₆-Cycloalkyl oder C₅-C₆-Cycloalkenyl stehen, in welchen zwei Substituenten gemeinsam mit den Kohlenstoffatomen, an die sie gebunden sind, für jeweils gegebenenfalls durch C₁-C₂-Alkyl oder C₁-C₂-Alkoxy substituiertes C₂-C₄-Alkandiyl, C₂-C₄-Alkendiyl oder Butadiendiyl stehen, mit der Maßgabe, dass dann Q³ für Wasserstoff oder Methyl steht,
G für Wasserstoff (a) oder für eine der Gruppen 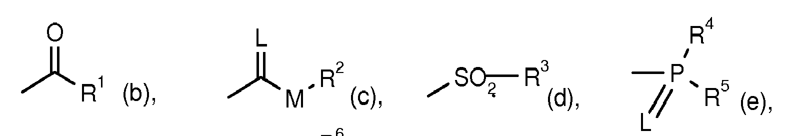 E (f) oder 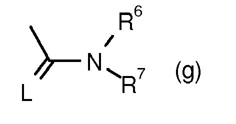 steht
in welchen
E für ein Metallionäquivalent oder ein Ammoniumion steht,
L für Sauerstoff oder Schwefel steht und
M für Sauerstoff oder Schwefel steht,
R¹ für jeweils gegebenenfalls einfach bis dreifach durch Fluor oder Chlor substituiertes C₁-C₈-Alkyl, C₂-C₁₈-Alkenyl, C₁-C₄-Alkoxy-C₁-C₂-alkyl, C₁-C₄-Alkylthio-C₁-C₂-alkyl oder gegebenenfalls einfach bis zweifach durch Fluor, Chlor, C₁-C₂-Alkyl oder C₁-C₂-Alkoxy substituiertes C₃-C₆-Cycloalkyl, in welchem gegebenenfalls ein oder zwei nicht direkt benachbarte Ringglieder durch Sauerstoff ersetzt sind,
für gegebenenfalls einfach bis zweifach durch Fluor, Chlor, Brom, Cyano, Nitro, C₁-C₄-Alkyl, C₁-C₄-Alkoxy, C₁-C₂-Halogenalkyl oder C₁-C₂-Halogenalkoxy substituiertes Phenyl steht,
R² für jeweils gegebenenfalls einfach bis dreifach durch Fluor substituiertes C₁-C₈-Alkyl, C₂-C₈-Alkenyl oder C₁-C₄-Alkoxy-C₂-C₄-alkyl,
für gegebenenfalls einfach durch C₁-C₂-Alkyl oder C₁-C₂-Alkoxy substituiertes C₃-C₆-Cycloalkyl oder
für jeweils gegebenenfalls einfach bis zweifach durch Fluor, Chlor, Brom, Cyano, Nitro, C₁-C₄-Alkyl, C₁-C₃-Alkoxy, Trifluormethyl oder Trifluormethoxy substituiertes Phenyl oder Benzyl steht,
R³ für gegebenenfalls einfach bis dreifach durch Fluor substituiertes C₁-C₆-Alkyl oder für gegebenenfalls einfach durch Fluor, Chlor, Brom, C₁-C₄-Alkyl, C₁-C₄-Alkoxy, Trifluormethyl, Trifluormethoxy, Cyano oder Nitro substituiertes Phenyl steht,
R⁴ für C₁-C₆-Alkyl, C₁-C₆-Alkoxy, C₁-C₆-Alkylamino, Di-(C₁-C₆-alkyl)amino, C₁-C₆-Alkylthio, C₃-C₄-Alkenylthio, C₃-C₆-Cycloalkylthio oder für jeweils gegebenenfalls einfach durch Fluor, Chlor, Brom, Nitro, Cyano, C₁-C₃-Alkoxy, C₁-C₃-Halogenalkoxy, C₁-C₃-Alkylthio, C₁-C₃-Halogenalkylthio, C₁-C₃-Alkyl oder Trifluormethyl substituiertes Phenyl, Phenoxy oder Phenylthio steht,
R⁵ für C₁-C₆-Alkoxy oder C₁-C₆-Alkylthio steht,
R⁶ für Wasserstoff, C₁-C₆-Alkyl, C₃-C₆-Cycloalkyl, C₁-C₆-Alkoxy, C₃-C₆-Alkenyl, C₁-C₆-Alkoxy-C₁-C₄-alkyl, für gegebenenfalls einfach durch Fluor, Chlor, Brom, Trifluormethyl, C₁-C₄-Alkyl oder C₁-C₄-Alkoxy substituiertes Phenyl, für gegebenenfalls einfach durch Fluor, Chlor, Brom, C₁-C₄-Alkyl, Trifluormethyl oder C₁-C₄-Alkoxy substituiertes Benzyl steht,
R⁷ für C₁-C₆-Alkyl, C₃-C₆-Alkenyl oder C₁-C₆-Alkoxy-C₁-C₄-alkyl steht,
R⁶ und R⁷ zusammen für einen gegebenenfalls durch Methyl oder Ethyl substituierten C₄-C₅-Alkylenrest stehen, in welchem gegebenenfalls eine Methylengruppe durch Sauerstoff oder Schwefel ersetzt ist.

4. Verbindungen der Formel (I) gemäß Anspruch 1, in welcher
J für Cyclopropyl, Dicyclopropyl, Cyclobutyl, Cyclopentyl, Cyclohexyl, Oxetanyl, Tetrahydrofurfuryl, Tetrahydropyranyl steht,
X für Wasserstoff, Fluor, Chlor, Brom, Methyl, Ethyl, Propyl, Methoxy oder Ethoxy steht,
Y für Wasserstoff, Chlor, Brom, Methyl, Ethyl, Propyl, Trifluormethyl, Methoxy, Ethoxy oder Trifluormethoxy steht,
m für die Zahl 1 oder 2 steht
mit der Maßgabe, dass mindestens einer der Reste J, X oder Y in der 2-Position des Phenylrestes steht und ungleich Wasserstoff ist,
dabei stehen die Reste J, X und Y in folgenden Phenylsubstitutionsmustern 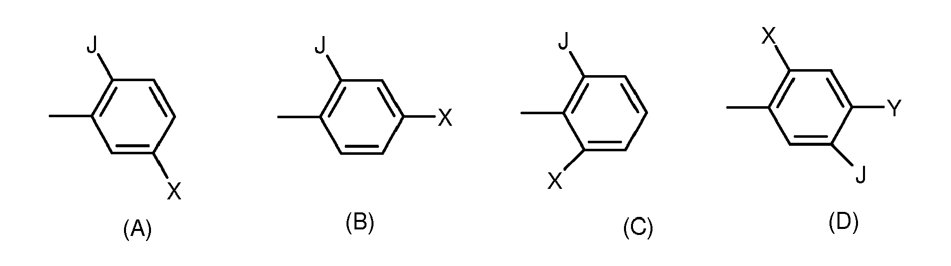 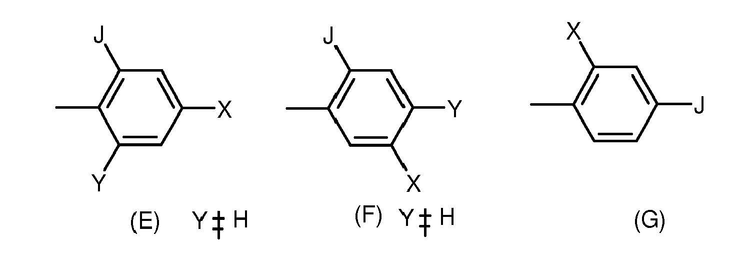 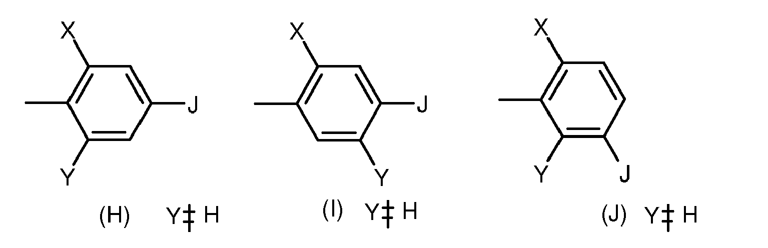 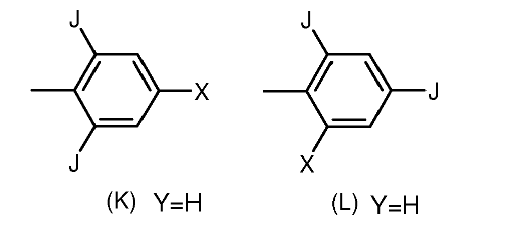 wobei nur im Phenylsubstitutionsmuster (B), (K) und (L) X auch für Wasserstoff stehen darf,
CKE für die Gruppe 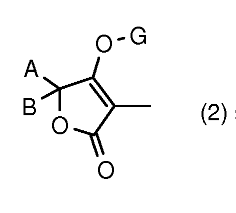 steht,
A für Wasserstoff, jeweils gegebenenfalls einfach bis dreifach durch Fluor substituiertes C₁-C₄-Alkyl oder C₁-C₂-Alkoxy-C₁-C₂-alkyl, für Cyclopropyl, Cyclopentyl oder Cyclohexyl und nur im Fall der Verbindungen der Formel (I-5) für gegebenenfalls einfach bis zweifach durch Fluor, Chlor, Brom, Methyl, Ethyl, n-Propyl, iso-Propyl, Methoxy, Ethoxy, Trifluormethyl, Trifluormethoxy, Cyano oder Nitro substituiertes Phenyl steht,
B für Wasserstoff, Methyl oder Ethyl steht oder
A, B und das Kohlenstoffatom an das sie gebunden sind, für gesättigtes C₅-C₆-Cycloalkyl stehen, in welchem gegebenenfalls ein Ringglied durch Sauerstoff oder Schwefel ersetzt ist und welches gegebenenfalls einfach durch Methyl, Ethyl, Propyl, Isopropyl, Trifluormethyl, Methoxy, Ethoxy, Propoxy, Methoxyethoxy, Butoxy, Methoxymethyl oder Ethoxyethoxy substituiert ist, mit der Maßgabe, dass dann Q³ für Wasserstoff steht oder
A, B und das Kohlenstoffatom, an das sie gebunden sind, für C₆-Cycloalkyl stehen, welches gegebenenfalls durch mit zwei nicht direkt benachbarten Sauerstoffatomen enthaltende Alkylendioxyl-Gruppe substituiert ist, mit der Maßgabe, dass dann Q³ für Wasserstoff steht oder
A, B und das Kohlenstoffatom, an das sie gebunden sind, für C₅-C₆-Cycloalkyl oder C₅-C₆-Cycloalkenyl stehen, worin zwei Substituenten gemeinsam mit den Kohlenstoffatomen, an die sie gebunden sind, für C₂-C₄-Alkandiyl oder C₂-C₄-Alkendiyl oder Butadiendiyl stehen, mit der Maßgabe, dass dann Q³ für Wasserstoff steht,
G für Wasserstoff (a) oder für eine der Gruppen 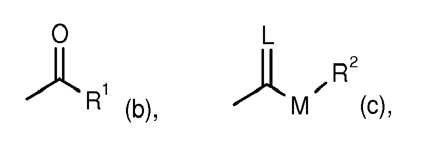 -SO₂-R³ (d) oder E (f) stehen,
in welchen
L für Sauerstoff oder Schwefel steht,
M für Sauerstoff oder Schwefel steht und
E für ein Ammoniumion steht,
R¹ für jeweils gegebenenfalls einfach durch Chlor substituiertes C₁-C₆-Alkyl, C₂-C₁₇-Alkenyl, C₁-C₂-Alkoxy-C₁-alkyl, C₁-C₂-Alkylthio-C₁-alkyl oder jeweils gegebenenfalls einfach durch Fluor, Chlor, Methyl oder Methoxy substituiertes Cyclopropyl oder Cyclohexyl,
für gegebenenfalls einfach durch Fluor, Chlor, Brom, Cyano, Nitro, Methyl, Methoxy, Trifluormethyl oder Trifluormethoxy substituiertes Phenyl steht,
R² für jeweils gegebenenfalls einfach durch Fluor substituiertes C₁-C₈-Alkyl, C₂-C₆-Alkenyl oder C₁-C₄-Alkoxy-C₂-C₃-alkyl, Phenyl oder Benzyl steht,
R³ für C₁-C₈-Alkyl steht.

5. Verbindungen der Formel (I) gemäß Anspruch 1, in welcher
J für Cyclopropyl steht,
X für Chlor, Methyl oder Ethyl steht,
Y für Chlor, Methyl, Ethyl oder Wasserstoff steht,
m für die Zahl 1 oder 2 steht,
mit der Maßgabe, dass mindestens einer der Reste J, X oder Y in der 2-Position des Phenylrestes steht und ungleich Wasserstoff ist,
dabei stehen die Reste J, X und Y in folgenden Phenylsubstitutionsmustern 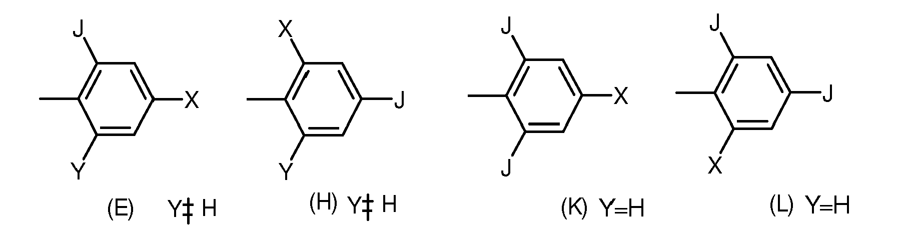
CKE für die Gruppe 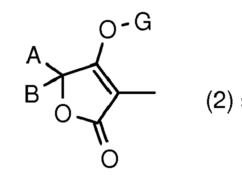 steht,
A für C₁-C₄-Alkyl oder Cyclopropyl steht,
B für Wasserstoff oder Methyl steht oder
A, B und das Kohlenstoffatom, an das sie gebunden sind, für gesättigtes C₅-C₆-Cycloalkyl stehen, in welchem gegebenenfalls ein Ringglied durch Sauerstoff ersetzt ist und welches gegebenenfalls einfach durch Methoxy, Ethoxy, Butoxy oder Methoxymethyl substituiert ist,
A, B und das Kohlenstoffatom, an das sie gebunden sind, für C₆-Cycloalkyl stehen, welches gegebenenfalls durch mit zwei nicht direkt benachbarten Sauerstoffatomen enthaltende C₂-C₃-Alkylendioxyl-Gruppe substituiert ist,
G für Wasserstoff (a) oder für eine der Gruppen 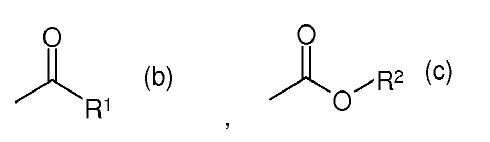 oder SO₂-R³(d) steht,
R¹ für C₁-C₆-Alkyl oder einfach durch Chlor substituiertes Phenyl steht,
R² für C₁-C₈-Alkyl steht,
R³ für C₁-C₈-Alkyl steht. (II)

6. Verfahren zur Herstellung von Verbindungen der Formel (I) gemäß Anspruch 1, **dadurch gekennzeichnet, dass** man zum Gehalt von
(B) Verbindungen der Formel (I-2-a) 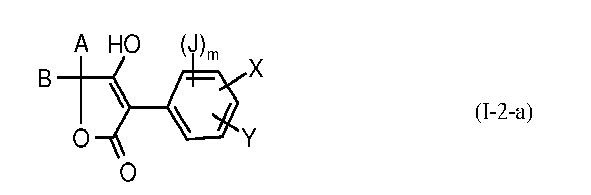 in welcher
A, B, J, m, X und Y die oben angegebenen Bedeutungen haben,
Verbindungen der Formel (III) 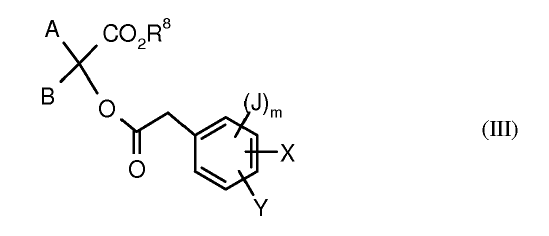 in welcher
A, B, J, m, X , Y und R⁸ die oben angegebenen Bedeutungen haben,
in Gegenwart eines Verdünnungsmittels und in Gegenwart einer Base intramolekular kondensiert,
(K) Verbindungen der oben gezeigten Formel (I-2-b) in welchen A, B, J, m, R¹, X und Y die oben angegebenen Bedeutungen haben, Verbindungen der oben gezeigten Formel (I-2-a), in welchen A, B, J, m, X und Y die oben angegebenen Bedeutungen haben, jeweils
(α) mit Säurehalogeniden der Formel (XV) 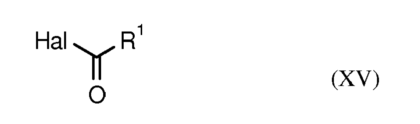 in welcher
R¹ die oben angegebene Bedeutung hat und
Hal für Halogen steht
oder
(β) mit Carbonsäureanhydriden der Formel (XVI)
R¹-CO-O-CO-R¹ (XVI)
in welcher
R¹ die oben angegebene Bedeutung hat,
gegebenenfalls in Gegenwart eines Verdünnungsmittels und gegebenenfalls in Gegenwart eines Säurebindemittels umsetzt;
(L) Verbindungen der oben gezeigten Formel (I-2-c), in welchen A, B, J, m, R², M, X und Y die oben angegebenen Bedeutungen haben und L für Sauerstoff steht, Verbindungen der oben gezeigten Formel (I-2-a), in welchen A, B, J, m, X und Y die oben angegebenen Bedeutungen haben, jeweils
mit Chlorameisensäureestern oder Chlorameisensäurethiolestern der Formel (XVII)
R²-M-CO-Cl (XVII)
in welcher
R² und M die oben angegebenen Bedeutungen haben,
gegebenenfalls in Gegenwart eines Verdünnungsmittels und gegebenenfalls in Gegenwart eines Säurebindemittels umsetzt;
(M) Verbindungen der oben gezeigten Formel (I-2-c), in welchen A, B, J, m, R², M, X und Y die oben angegebenen Bedeutungen haben und L für Schwefel steht, Verbindungen der oben gezeigten Formel (I-2-a), in welchen A, B, J, m, X und Y die oben angegebenen Bedeutungen haben, jeweils
mit Chlormonothioameisensäureestern oder Chlordithioameisensäureestern der Formel (XVIII) 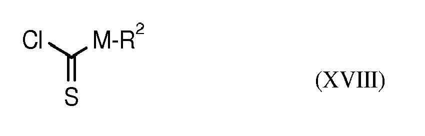 in welcher
M und R² die oben angegebenen Bedeutungen haben,
gegebenenfalls in Gegenwart eines Verdünnungsmittels und gegebenenfalls in Gegenwart eines Säurebindemittels umsetzt
und
(N) Verbindungen der oben gezeigten Formel (I-2-d), in welchen A, B, J, m, R³, X und Y die oben angegebenen Bedeutungen haben, Verbindungen der oben gezeigten Formel (I-2-a), in welchen A, B, J, m, X und Y die oben angegebenen Bedeutungen haben, jeweils
mit Sulfonsäurechloriden der Formel (XIX)
R³-SO₂-Cl (XIX)
in welcher
R³ die oben angegebene Bedeutung hat,
gegebenenfalls in Gegenwart eines Verdünnungsmittels und gegebenenfalls in Gegenwart eines Säurebindemittels umsetzt,
(O) Verbindungen der oben gezeigten Formel (I-2-e), in welchen A, B, J, m, L, R⁴, R⁵, X und Y die oben angegebenen Bedeutungen haben, Verbindungen der oben gezeigten Formel (I-2-a), in welchen A, B, J, m, X und Y die oben angegebenen Bedeutungen haben" jeweils
mit Phosphorverbindungen der Formel (XX) 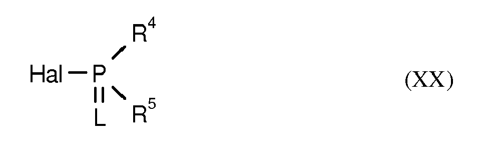 in welcher
L, R⁴ und R⁵ die oben angegebenen Bedeutungen haben und
Hal für Halogen steht,
gegebenenfalls in Gegenwart eines Verdünnungsmittels und gegebenenfalls in Gegenwart eines Säurebindemittels umsetzt,
(P) Verbindungen der oben gezeigten Formel (I-2-f), in welchen A, B, E, J, m, X und Y die oben angegebenen Bedeutungen haben, Verbindungen der oben gezeigten Formel (I-2-a), in welchen A, B, J, m, X und Y die oben angegebenen Bedeutungen haben, jeweils
mit Metallverbindungen oder Aminen der Formeln (XXI) oder (XXII) 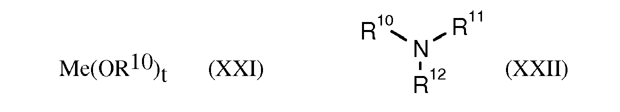 in welchen
Me für ein ein- oder zweiwertiges Metall oder für ein Ammoniumion 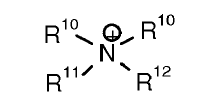
t für die Zahl 1 oder 2 und
R¹⁰, R¹¹, R¹² unabhängig voneinander für Wasserstoff oder Alkyl stehen,
gegebenenfalls in Gegenwart eines Verdünnungsmittels umsetzt,
(Q) Verbindungen der oben gezeigten Formel (I-2-g), in welchen A, B, J, m, L, R⁶, R⁷, X und Y die oben angegebenen Bedeutungen haben, Verbindungen der oben gezeigten Formel (I-2-a), in welchen A, B, J, m, X und Y die oben angegebenen Bedeutungen haben, jeweils
(α) mit Isocyanaten oder Isothiocyanaten der Formel (XXIII)
R⁶-N=C=L (XXIII)
in welcher
R⁶ und L die oben angegebenen Bedeutungen haben,
gegebenenfalls in Gegenwart eines Verdünnungsmittels und gegebenenfalls in Gegenwart eines Katalysators umsetzt oder
(β) mit Carbamidsäurechloriden oder Thiocarbamidsäurechloriden der Formel (XXIV) 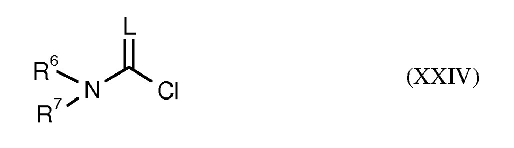 in welcher
L, R⁶ und R⁷ die oben angegebenen Bedeutungen haben,
gegebenenfalls in Gegenwart eines Verdünnungsmittels und gegebenenfalls in Gegenwart eines Säurebindemittels, umsetzt,
(R) Verbindungen der oben gezeigten Formeln (I-2-a), in welchen A, B, G, J, m, X und Y die oben angegebene Bedeutung haben, erhält, Verbindungen der Formeln (I-2'),
in welchen A, B, G, m, X und Y die oben genannte Bedeutung haben und J' bevorzugt für Brom oder Iod steht 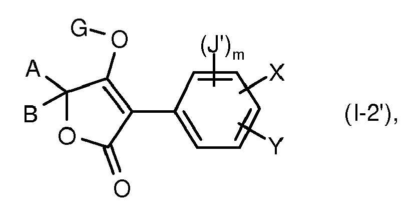 mit kupplungsfähigen Cycloalkylboronsäurederivaten, z.B. Cycloalkylboronsäuren der Formel (XXV) 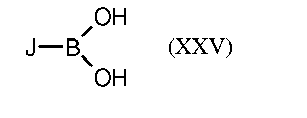 oder deren Ester in Gegenwart eines Lösungsmittels in Gegenwart eines Katalysators und in Gegenwart einer Base kuppelt.

7. Mittel enthaltend einen wirksamen Gehalt an einer Wirkstoffkombination umfassend als Komponenten
(a') mindestens ein substituiertes, cyclisches Ketoenol der Formel (I), in welcher CKE, J, m, X und Y die oben angegebene Bedeutung haben
und
(b') zumindest eine die Kulturpflanzen-Verträglichkeit verbessernde Verbindung aus der folgenden Gruppe von Verbindungen:
4-Dichloracetyl-1-oxa-4-aza-spiro[4.5]-decan (AD-67, MON-4660), 1-Dichloracetyl-hexahydro-3,3,8a-trimethylpyrrolo[1,2-a]-pyrimidin-6(2H)-on (Dicyclonon, BAS-145138), 4-Dichloracetyl-3,4-dihydro-3-methyl-2H-1,4-benzoxazin (Benoxacor), 5-Chlor-chinolin-8-oxy-essigsäure-(1-methyl-hexylester) (Cloquintocet-mexyl - vgl. auch verwandte Verbindungen in EP-A-86750, EP-A-94349, EP-A-191736, EP-A-492366), 3-(2-Chlorbenzyl)-1-(-methyl-1-phenyl-ethyl)-harnstoff (Cumyluron), α-(Cyanomethoximino)-phenylacetonitril (Cyometrinil), 2,4-Dichlor-phenoxyessigsäure (2,4-D), 4-(2,4-Dichlorphenoxy)-buttersäure (2,4-DB), 1-(1-Methyl-1-phenyl-ethyl)-3-(4-methyl-phenyl)-harnstoff (Daimuron, Dymron), 3,6-Dichlor-2-methoxy-benzoesäure (Dicamba), Piperidin-1-thiocarbonsäure-S-1-methyl-1-phenyl-ethylester (Dimepiperate), 2,2-Dichlor-N-(2-oxo-2-(2-propenylamino)-ethyl)-N-(2-propenyl)-acetamid (DKA-24), 2,2-Dichlor-N,N-di-2-propenyl-acetamid (Dichlormid), 4,6-Dichlor-2-phenyl-pyrimidin (Fenclorim), 1-(2,4-Dichlorphenyl)-5-trichlormethyl-1H-1,2,4-triazol-3-carbonsäure-ethylester (Fenchlorazole-ethyl - vgl. auch verwandte Verbindungen in EP-A-174562 und EP-A-346620), 2-Chlor-4-trifluormethyl-thiazol-5-carbonsäure-phenylmethylester (Flurazole), 4-Chlor-N-(1,3-dioxolan-2-yl-methoxy)-a-trifluor-acetophenonoxim (Fluxofenim), 3-Dichloracetyl-5-(2-furanyl)-2,2-dimethyl-oxazolidin (Furilazole, MON-13900), Ethyl-4,5-dihydro-5,5-diphenyl-3-isoxazolcarboxylat (Isoxadifen-ethyl - vgl. auch verwandte Verbindungen in WO-A-95/07897), 1-(Ethoxycarbonyl)-ethyl-3,6-dichlor-2-methoxybenzoat (Lactidichlor), (4-Chlor-o-tolyloxy)-essigsäure (MCPA), 2-(4-Chlor-o-tolyloxy)-propionsäure (Mecoprop), Diethyl-1-(2,4-dichlor-phenyl)-4,5-dihydro-5-methyl-1H-pyrazol-3,5-dicarboxylat (Mefenpyr-diethyl - vgl. auch verwandte Verbindungen in WO-A-91/07874) 2-Dichlormethyl-2-methyl-1,3-dioxolan (MG-191), 2-Propenyl-1-oxa-4-azaspiro[4.5]decane-4-carbodithioate (MG-838), 1,8-Naphthalsäureanhydrid, α-(1,3-Dioxolan-2-yl-methoximino)-phenylacetonitril (Oxabetrinil), 2,2-Dichlor-N-(1,3-dioxolan-2-yl-methyl)-N-(2-propenyl)-acetamid (PPG-1292), 3-Dichloracetyl-2,2-dimethyl-oxazolidin (R-28725), 3-Dichloracetyl-2,2,5-trimethyloxazolidin (R-29148), 4-(4-Chlor-o-tolyl)-buttersäure, 4-(4-Chlor-phenoxy)-buttersäure, Diphenylmethoxyessigsäure, Diphenylmethoxyessigsäure-methylester, Diphenylmethoxyessigsäure-ethylester, 1-(2-Chlor-phenyl)-5-phenyl-1H-pyrazol-3-carbonsäure-methylester, 1-(2,4-Dichlor-phenyl)-5-methyl-1H-pyrazol-3-carbonsäure-ethylester, 1-(2,4-Dichlorphenyl)-5-isopropyl-1H-pyrazol-3-carbonsäure-ethylester, 1-(2,4-Dichlor-phenyl)-5-(1,1-dimethyl-ethyl)-1H-pyrazol-3-carbonsäure-ethylester, 1-(2,4-Dichlor-phenyl)-5-phenyl-1H-pyrazol-3-carbonsäure-ethylester (vgl. auch verwandte Verbindungen in EP-A-269806 und EP-A-333131), 5-(2,4-Dichlor-benzyl)-2-isoxazolin-3-carbonsäure-ethylester, 5-Phenyl-2-isoxazolin-3-carbonsäure-ethylester, 5-(4-Fluor-phenyl)-5-phenyl-2-isoxazolin-3-carbonsäure-ethylester (vgl. auch verwandte Verbindungen in WO-A-91/08202), 5-Chlor-chinolin-8-oxy-essigsäure-(1,3-dimethyl-but-1-yl)-ester, 5-Chlor-chinolin-8-oxy-essigsäure-4-allyloxy-butylester, 5-Chlor-chinolin-8-oxy-essigsäure-1-allyloxy-prop-2-yl-ester, 5-Chlorchinoxalin-8-oxy-essigsäure-methylester, 5-Chlor-chinolin-8-oxy-essigsäure-ethylester, 5-Chlor-chinoxalin-8-oxy-essigsäure-allylester, 5-Chlor-chinolin-8-oxy-essigsäure-2-oxo-prop-1-yl-ester, 5-Chlor-chinolin-8-oxy-malonsäure-diethylester, 5-Chlor-chinoxalin-8-oxy-malonsäure-diallylester, 5-Chlor-chinolin-8-oxy-malonsäure-diethylester (vgl. auch verwandte Verbindungen in EP-A-582198), 4-Carboxy-chroman-4-yl-essigsäure (AC-304415, vgl. EP-A-613618), 4-Chlor-phenoxy-essigsäure, 3,3'-Dimethyl-4-methoxy-benzophenon, 1-Brom-4-chlormethylsulfonyl-benzol, 1-[4-(N-2-Methoxybenzoyl-sulfamoyl)-phenyl]-3-methyl-harnstoff (alias N-(2-Methoxy-benzoyl)-4-[(methylamino-carbonyl)-amino]-benzolsulfonamid), 1-[4-(N-2-Methoxybenzoylsulfamoyl)-phenyl]-3,3-dimethyl-harnstoff, 1-[4-(N-4,5-Dimethylbenzoylsulfamoyl)-phenyl]-3-methyl-harnstoff, 1-[4-(N-Naphthylsulfamoyl)-phenyl]-3,3-dimethyl-harnstoff, N-(2-Methoxy-5-methyl-benzoyl)-4-(cyclopropylaminocarbonyl)-benzolsulfonamid,
und/oder eine der folgenden durch allgemeine Formeln definierten Verbindungen
der allgemeinen Formel (IIa) 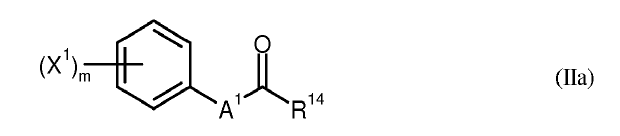 oder der allgemeinen Formel (IIb) 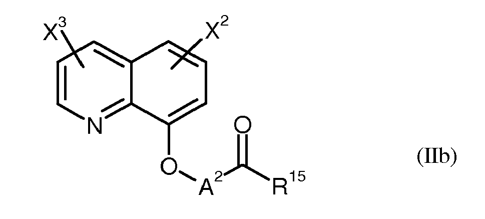 oder der Formel (IIc) 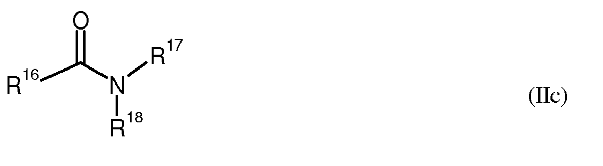
wobei
m für eine Zahl 0, 1, 2, 3, 4 oder 5 steht,
A¹ für eine der nachstehend skizzierten divalenten heterocyclischen Gruppierungen steht, 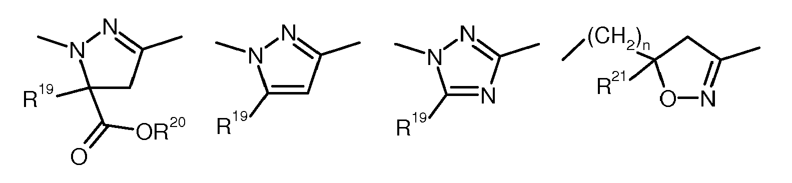
n für eine Zahl 0, 1, 2, 3, 4 oder 5 steht,
A² für gegebenenfalls durch C₁-C₄-Alkyl und/oder C₁-C₄-Alkoxy-carbonyl und/oder C₁-C₄-Alkenyloxy-carbonyl substituiertes Alkandiyl mit 1 oder 2 Kohlenstoffatomen steht,
R¹⁴ für Hydroxy, Mercapto, Amino, C₁-C₆-Alkoxy, C₁-C₆-Alkylthio, C₁-C₆-Alkylamino oder Di-(C₁-C₄-alkyl)-amino steht,
R¹⁵ für Hydroxy, Mercapto, Amino, C₁-C₇-Alkoxy, C₁-C₆-Alkenyloxy, C₁-C₆-Alkenyloxy-C₁-C₆-alkoxy, C₁-C₆-Alkylthio, C₁-C₆-Alkylamino oder Di-(C₁-C₄-alkyl)-amino steht,
R¹⁶ für jeweils gegebenenfalls durch Fluor, Chlor und/oder Brom substituiertes C₁-C₄-Alkyl steht,
R¹⁷ für Wasserstoff, jeweils gegebenenfalls durch Fluor, Chlor und/oder Brom substituiertes C₁-C₆-Alkyl, C₂-C₆-Alkenyl oder C₂-C₆-Alkinyl, C₁-C₄-Alkoxy-C₁-C₄-alkyl, Di-oxolanyl-C₁-C₄-alkyl, Furyl, Furyl-C₁-C₄-alkyl, Thienyl, Thiazolyl, Piperidinyl, oder gegebenenfalls durch Fluor, Chlor und/oder Brom oder C₁-C₄-Alkyl substituiertes Phenyl steht,
R¹⁸ für Wasserstoff, jeweils gegebenenfalls durch Fluor, Chlor und/oder Brom substituiertes C₁-C₆-Alkyl, C₂-C₆-Alkenyl oder C₂-C₆-Alkinyl, C₁-C₄-Alkoxy-C₁-C₄-alkyl, Dioxolanyl-C₁-C₄-alkyl, Furyl, Furyl-C₁-C₄-alkyl, Thienyl, Thiazolyl, Piperidinyl, oder gegebenenfalls durch Fluor, Chlor und/oder Brom oder C₁-C₄-Alkyl substituiertes Phenyl,
R¹⁷ und R¹⁸ auch gemeinsam für jeweils gegebenenfalls durch C₁-C₄-Alkyl, Phenyl, Furyl, einen annellierten Benzolring oder durch zwei Substituenten, die gemeinsam mit dem C-Atom, an das sie gebunden sind, einen 5- oder 6-gliedrigen Carbocyclus bilden, substituiertes C₃-C₆-Alkandiyl oder C₂-C₅-Oxaalkandiyl steht,
R¹⁹ für Wasserstoff, Cyano, Halogen, oder für jeweils gegebenenfalls durch Fluor, Chlor und/oder Brom substituiertes C₁-C₄-Alkyl, C₃-C₆-Cycloalkyl oder Phenyl steht,
R²⁰ für Wasserstoff, gegebenenfalls durch Hydroxy, Cyano, Halogen oder C₁-C₄-Alkoxy substituiertes C₁-C₆-Alkyl, C₃-C₆-Cycloalkyl oder Tri-(C₁-C₄-alkyl)-silyl steht,
R²¹ für Wasserstoff, Cyano, Halogen, oder für jeweils gegebenenfalls durch Fluor, Chlor und/oder Brom substituiertes C₁-C₄-Alkyl, C₃-C₆-Cycloalkyl oder Phenyl steht,
X¹ für Nitro, Cyano, Halogen, C₁-C₄-Alkyl, C₁-C₄-Halogenalkyl, C₁-C₄-Alkoxy oder C₁-C₄-Halogenalkoxy steht,
X² für Wasserstoff, Cyano, Nitro, Halogen, C₁-C₄-Alkyl, C₁-C₄-Halogenalkyl, C₁-C₄-Alkoxy oder C₁-C₄-Halogenalkoxy steht,
X³ für Wasserstoff, Cyano, Nitro, Halogen, C₁-C₄-Alkyl, C₁-C₄-Halogenalkyl, C₁-C₄-Alkoxy oder C₁-C₄-Halogenalkoxy steht,
und/oder die folgenden durch allgemeine Formeln definierten Verbindungen
der allgemeinen Formel (IId) 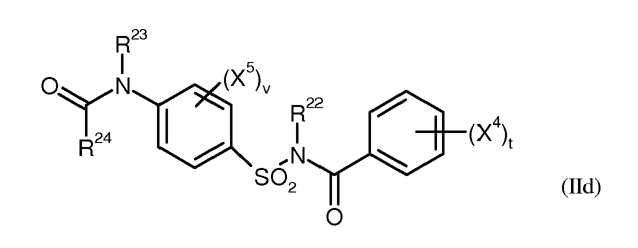 oder der allgemeinen Formel (IIe) 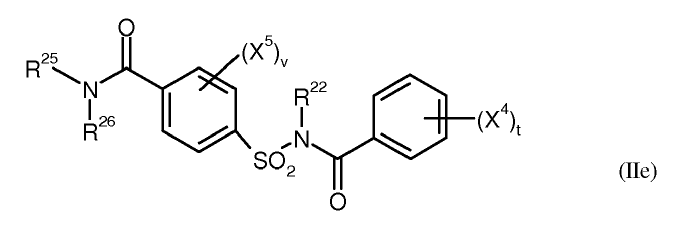 wobei
t für eine Zahl 0, 1, 2, 3, 4 oder 5 steht,
v für eine Zahl 0, 1, 2, 3, 4 oder 5 steht,
R²² für Wasserstoff oder C₁-C₄-Alkyl steht,
R²³ für Wasserstoff oder C₁-C₄-Alkyl steht,
R²⁴ für Wasserstoff, jeweils gegebenenfalls durch Cyano, Halogen oder C₁-C₄-Alkoxy substituiertes C₁-C₆-Alkyl, C₁-C₆-Alkoxy, C₁-C₆-Alkylthio, C₁-C₆-Alkylamino oder Di-(C₁-C₄-alkyl)-amino, oder jeweils gegebenenfalls durch Cyano, Halogen oder C₁-C₄-Alkyl substituiertes C₃-C₆-Cycloalkyl, C₃-C₆-Cycloalkyloxy, C₃-C₆-Cycloalkyl-thio oder C₃-C₆-Cycloalkylamino steht,
R²⁵ für Wasserstoff, gegebenenfalls durch Cyano, Hydroxy, Halogen oder C₁-C₄-Alkoxy substituiertes C₁-C₆-Alkyl, jeweils gegebenenfalls durch Cyano oder Halogen substituiertes C₃-C₆-Alkenyl oder C₃-C₆-Alkinyl, oder gegebenenfalls durch Cyano, Halogen oder C₁-C₄-Alkyl substituiertes C₃-C₆-Cycloalkyl steht,
R²⁶ für Wasserstoff, gegebenenfalls durch Cyano, Hydroxy, Halogen oder C₁-C₄-Alkoxy substituiertes C₁-C₆-Alkyl, jeweils gegebenenfalls durch Cyano oder Halogen substituiertes C₃-C₆-Alkenyl oder C₃-C₆-Alkinyl, gegebenenfalls durch Cyano, Halogen oder C₁-C₄-Alkyl substituiertes C₃-C₆-Cycloalkyl, oder gegebenenfalls durch Nitro, Cyano, Halogen, C₁-C₄-Alkyl, C₁-C₄-Halogenalkyl, C₁-C₄-Alkoxy oder C₁-C₄-Halogenalkoxy substituiertes Phenyl steht, oder zusammen mit R²⁵ für jeweils gegebenenfalls durch C₁-C₄-Alkyl substituiertes C₂-C₆-Alkandiyl oder C₂-C₅-Oxaalkandiyl steht,
X⁴ für Nitro, Cyano, Carboxy, Carbamoyl, Formyl, Sulfamoyl, Hydroxy, Amino, Halogen, C₁-C₄-Alkyl, C₁-C₄-Halogenalkyl, C₁-C₄-Alkoxy oder C₁-C₄-Halogenalkoxy steht, und
X⁵ für Nitro, Cyano, Carboxy, Carbamoyl, Formyl, Sulfamoyl, Hydroxy, Amino, Halogen, C₁-C₄-Alkyl, C₁-C₄-Halogenalkyl, C₁-C₄-Alkoxy oder C₁-C₄-Halogenalkoxy steht.

8. Mittel nach Anspruch 7, bei dem die die Kulturpflanzen-Verträglichkeit verbessernde Verbindung aus der folgenden Gruppe von Verbindungen ausgewählt ist:
Cloquintocet-mexyl, Fenchlorazole-ethyl, Isoxadifen-ethyl, Mefenpyr-diethyl, Furilazole, Fenclorim, Cumyluron, Dymron oder die Verbindungen 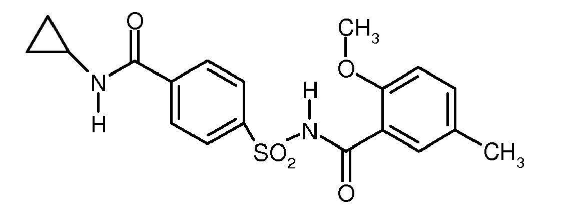 und

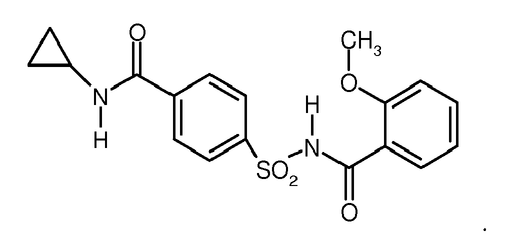

9. Mittel gemäß einem der Ansprüche 7 oder 8, bei denen die Kulturpflanzen-Verträglichkeit verbessernde Verbindung Cloquintocet-mexyl ist.

10. Mittel gemäß einem der Ansprüche 7 oder 8, bei denen die Kulturpflanzen-Verträglichkeit verbessernde Verbindung Mefenpyr-diethyl ist.

11. Mittel zur Bekämpfung von Schädlingen und/oder unerwünschten Pflanzenwuchs, **gekennzeichnet durch** einen Gehalt an mindestens einer Verbindung der Formel (I) gemäß Anspruch 1.

12. Verfahren zur Bekämpfung von tierischen Schädlingen und/oder unerwünschtem Pflanzenbewuchs, **dadurch gekennzeichnet, dass** man Verbindungen der Formel (I) gemäß Anspruch 1 auf Schädlinge, unerwünschtem Pflanzenwuchs und/oder ihren Lebensraum einwirken lässt.

13. Verwendung von Verbindungen der Formel (I) gemäß Anspruch 1 zur Bekämpfung von tierischen Schädlingen und/oder unerwünschtem Pflanzenbewuchs.

14. Verfahren zur Herstellung von Mitteln zur Bekämpfung von Schädlingen und/oder unerwünschtem Pflanzenbewuchs, **dadurch gekennzeichnet, dass** man Verbindungen der Formel (I) gemäß Anspruch 1 mit Streckmitteln und/oder oberflächenaktiven Stoffen vermischt.

15. Verwendung von Verbindungen der Formel (I) gemäß Anspruch 1 zur Herstellung von Mitteln zur Bekämpfung von Schädlingen und/oder unerwünschtem Pflanzenwuchs.

16. Verfahren zum Bekämpfen von unerwünschtem Pflanzenwuchs, **dadurch gekennzeichnet, dass** man ein Mittel gemäß Anspruch 7 auf die Pflanzen oder ihre Umgebung einwirken lässt.

17. Verwendung eines Mittels gemäß Anspruch 7 zum Bekämpfen von unerwünschten Pflanzenwuchs.

18. Verfahren zum Bekämpfen von unerwünschtem Pflanzenwuchs, **dadurch gekennzeichnet, dass** man eine Verbindung der Formel (I) gemäß Anspruch 1 und die die Kulturpflanzenverträglichkeit verbessernde Verbindung gemäß Anspruch 7 in zeitlich naher Abfolge getrennt auf die Pflanzen oder ihre Umgebung einwirken lässt.

19. Verbindungen der Formel (III) 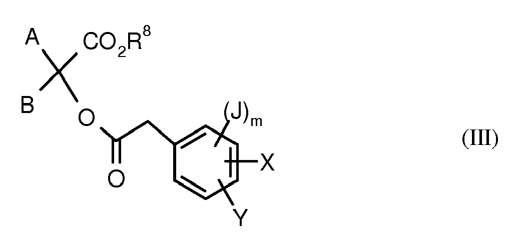 in welcher
A, B, J, m, X und Y und R⁸ die oben angegebenen Bedeutungen haben.

20. Zusammensetzung umfassend
- mindestens eine Verbindung der Formel (I) gemäß Anspruch 1 oder ein Mittel gemäß Anspruch 7 und
- mindestens ein Salz der Formel (III') 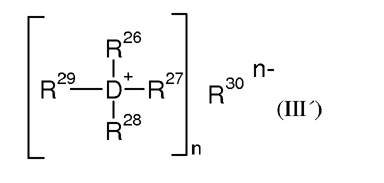
in welcher
D für Stickstoff oder Phosphor steht,
R²⁶, R²⁷, R²⁸ und R²⁹ unabhängig voneinander für Wasserstoff oder jeweils gegebenenfalls substituiertes C₁-C₈-Alkyl oder einfach oder mehrfach, ungesättigtes, gegebenenfalls substituiertes C₁-C₈-Alkylen stehen, wobei die Substituenten aus Halogen, Nitro und Cyano ausgewählt sein können,
n für 1, 2, 3 oder 4 steht,
R³⁰ für ein anorganisches oder organisches Anion steht.

21. Zusammensetzung gemäß Anspruch 20, **dadurch gekennzeichnet, dass** sie mindestens einen Penetrationsförderer enthält.

22. Verfahren zur Steigerung der Wirkung von Schädlingsbekämpfungsmitteln und/oder Herbiziden enthaltend einen Wirkstoff der Formel (I) gemäß Anspruch 1 oder ein Mittel gemäß Anspruch 7, **dadurch gekennzeichnet, dass** das anwendungsfertige Mittel (Spritzbrühe) unter Einsatz eines Salzes der Formel (III') gemäß Anspruch 20 zubereitet wird.

23. Verfahren gemäß Anspruch 22, **dadurch gekennzeichnet, dass** die Spritzbrühe unter Einsatz eines Penetrationsförderers zubereitet wird.
